# EUROPEAN PATENT SPECIFICATION

(11) **EP 3 618 842 B1**
(45) Date of publication and mention of the grant of the patent: **18.10.2023**
(21) Application number: 18730161.9
(22) Date of filing: 01.05.2018
(51) Int. Cl.: A61K 35/17, A61K 31/454, A61P 35/00

(54) **COMBINATION OF A CELL THERAPY AND AN IMMUNOMODULATORY COMPOUND**
KOMBINATION EINER ZELLTHERAPIE UND EINER IMMUNMODULATORISCHEN VERBINDUNG
COMBINAISON D'UNE THÉRAPIE CELLULAIRE ET D'UN COMPOSÉ IMMUNOMODULATEUR

(30) Priority: 01.05.2017 US 201762492947 P; 29.07.2017 US 201762538670 P; 23.08.2017 US 201762549390 P; 01.11.2017 US 201762580433 P; 08.12.2017 US 201762596753 P
(43) Date of publication of application: 11.03.2020
(73) Proprietor: Juno Therapeutics, Inc., Seattle, WA 98109 (US)
(72) Inventor: PORTS, Michael, Seattle, WA 98109 (US); WORKS, Melissa, Seattle, WA 98109 (US); BATUREVYCH, Oleksandr, Seattle, WA 98109 (US); SALMON, Ruth, Seattle, WA 98109 (US); HAUSE, Ronald, James, Jr., Seattle, WA 98109 (US); JOHNSTONE, Timothy, G., Seattle, WA 98109 (US); KUGLER, David, G., Seattle, WA 98109 (US); JONES, Jon, Seattle, WA 98109 (US); SONI, Neha, Seattle, WA 98109 (US)
(74) Representative: Carpmaels & Ransford LLP
(86) International application number: PCT/US2018/030545
(87) International publication number: WO 2018/204427

(56) References cited:
- WO-A1-2016/014530
- WO-A1-2016/164580
- WO-A1-2016/210262
- WO-A1-2017/058754
- WO-A1-2018/093591
- WO-A1-2018/102786
- WO-A1-2018/102787
- WO-A2-2018/071873
- WO-A2-2018/075820
- WO-A2-2018/085731
- WO-A2-2018/102785
- US-A1- 2014 271 635
- US-A1- 2015 283 178
- Joseph A Fraietta ET AL: "Regular Article IMMUNOBIOLOGY Ibrutinib enhances chimeric antigen receptor T-cell engraftment and efficacy in leukemia", Blood, 3 March 2016 (2016-03-03), XP55449528, DOI: 10.1182/blood-2015-11- Retrieved from the Internet: URL:http://www.bloodjournal.org/content/bl oodjournal/127/9/1117.full.pdf?sso-checked =true [retrieved on 2018-02-08]
- DREW C. DENIGER ET AL: "A Pilot Trial of the Combination of Vemurafenib with Adoptive Cell Therapy in Patients with Metastatic Melanoma", CLINICAL CANCER RESEARCH, vol. 23, no. 2, 15 January 2017 (2017-01-15), pages 351-362, XP55495523, US ISSN: 1078-0432, DOI: 10.1158/1078-0432.CCR-16-0906
- YIRAN ZHENG ET AL: "Enhancing Adoptive Cell Therapy of Cancer through Targeted Delivery of Small-Molecule Immunomodulators to Internalizing or Noninternalizing Receptors", ACS NANO, vol. 11, no. 3, 1 March 2017 (2017-03-01), pages 3089-3100, XP55495519, US ISSN: 1936-0851, DOI: 10.1021/acsnano.7b00078
- DANNY N. KHALIL ET AL: "The future of cancer treatment: immunomodulation, CARs and combination immunotherapy", NATURE REVIEWS CLINICAL ONCOLOGY, vol. 13, no. 5, 15 March 2016 (2016-03-15) , pages 273-290, XP55290129, NY, US ISSN: 1759-4774, DOI: 10.1038/nrclinonc.2016.25
- GUSTAV J. ULLENHAG ET AL: "Clinical and Immune Effects of Lenalidomide in Combination with Gemcitabine in Patients with Advanced Pancreatic Cancer", PLOS ONE, vol. 12, no. 1, 18 January 2017 (2017-01-18), page e0169736, XP055495937, DOI: 10.1371/journal.pone.0169736
- PAVEL OTÁHAL ET AL: "Lenalidomide enhances antitumor functions of chimeric antigen receptor modified T cells", ONCOIMMUNOLOGY, vol. 5, no. 4, 2 April 2016 (2016-04-02), page e1115940, XP055495939, US ISSN: 2162-4011, DOI: 10.1080/2162402X.2015.1115940
- Xiuli Wang ET AL: "Lenalidomide Enhances the Function of CS1 Chimeric Antigen Receptor Redirected-T Cells Against Multiple Myeloma | Blood Journal", Blood, 1 December 2016 (2016-12-01), XP055495944, Retrieved from the Internet: URL:http://www.bloodjournal.org/content/12 8/22/812 [retrieved on 2018-07-30]

## Description

### Field

The present disclosure relates in some aspects to methods, compositions and uses involving immunotherapies, in particular adoptive cell therapy, in particular T cell therapy, and an immunomodulatory compound, such as a structural or functional analog or derivative of thalidomide and/or an inhibitor of E3-ubiquitin ligase. The provided methods, compositions and uses include those for combination therapies involving the administration or use of one or more immunomodulatory compounds in conjunction with a T cell therapy, in particular a genetically engineered T cell therapy involving cells engineered with a recombinant receptor, in particular chimeric antigen receptor (CAR)-expressing T cells. In some aspects, features of the methods and cells provide for increased or improved activity, efficacy, persistence, expansion and/or proliferation of T cells for adoptive cell therapy or endogenous T cells recruited by immunotherapeutic agents.

### Background

Various strategies are available for immunotherapy, for example administering engineered T cells for adoptive therapy. For example, strategies are available for engineering T cells expressing genetically engineered antigen receptors, such as CARs, and administering compositions containing such cells to subjects. Improved strategies are needed to improve efficacy of the cells, for example, improving the persistence, activity and/or proliferation of the cells upon administration to subjects. Provided are methods, compositions, kits, and systems that meet such needs.

Otáhal et al. (2016) Oncoimmunology, 5(4):e1115940, describes how lenalidomide enhances antitumor functions of chimeric antigen receptor modified T cells.

Wang et al. (2016) Blood, 128(22):812, describes how lenalidomide enhances the function of CS1 chimeric antigen receptor redirected-t cells against multiple myeloma.

### Summary

The present invention is set out in the appended set of claims.

Any references herein to methods of treatment of the human or animal body by therapy refer to compounds, compositions, or medicaments for use in said methods.

Provided herein are combination therapies involving administration of an immunotherapy involving T cell function or activity, in particular a T cell therapy, and an immunomodulatory compound, such as a structural or functional analog or derivative of thalidomide and/or an inhibitor of E3-ubiquitin ligase. In some aspects, the provided methods enhance or modulate proliferation and/or activity of T cell activity associated with administration of an immunotherapy or immunotherapeutic agent, in particular a composition including cells for adoptive cell therapy, in particular a T cell therapy, in particular CAR-expressing T cells. In the present invention, the combination therapy generally involves administration of an immunomodulatory compound, such as a structural or functional analog of thalidomide and/or an inhibitor of E3-ubiquitin ligase (e.g. lenalidomide (3-(4-amino-1-oxo-1,3-dihydro-2H-isoindol-2-yl)piperidine-2,6-dione)), and administration of the T cell therapy, in particular a composition including cells for adoptive cell therapy, in particular a T cell therapy, in particular CAR-expressing T cells.

Provided herein are methods of treatment that involve: (a) administering a T cell therapy to a subject having a disease or condition; and (b) administering to the subject an immunomodulatory compound. In particular, the present invention provides a T cell therapy and an immunomodulatory compound for use in a method of treating cancer, wherein the method comprises:
(a) administering a T cell therapy to a subject having a disease or condition that is cancer, wherein the T cell therapy is or comprises genetically engineered cells expressing a recombinant receptor that specifically binds to an antigen, wherein the recombinant receptor is a chimeric antigen receptor (CAR), and wherein the CAR comprises an extracellular domain comprising an antigen-binding domain that specifically binds to a tumor antigen, a spacer, and an intracellular signaling region; and
(b) administering to the subject an immunomodulatory compound, wherein said immunomodulatory compound is selected from the group consisting of: thalidomide analogs; thalidomide derivatives; compounds that interact with and/or bind to cereblon (CRBN) and/or one or more members of the CRBN E3 ubiquitin-ligase complex; inhibitors of Ikaros (IKZF1); inhibitors of Aiolos (IKZF3); and compounds that enhance or promote ubiquitination and/or degradation of Ikaros (IKZF1) and/or Aiolos (IKZF3),

wherein initiation of administration of the immunomodulatory compound is carried out 7 to 21 days after initiation of administration of the T cell therapy, and
wherein the immunomodulatory compound is administered orally.

Provided herein are methods of treatment that involve administering an immunomodulatory compound to a subject having a disease or condition, wherein, at the time of initiation of administration of the immunomodulatory compound, the subject has been previously administered a T cell therapy for treatment of the disease or condition and optionally a blood or biopsy sample of the subject contains detectable levels of T cells of an engineered T cell therapy. In particular, the present invention further provides an immunomodulatory compound for use in a method of treating cancer, the method comprising administering an immunomodulatory compound to a subject having a disease or condition that is cancer, wherein, at the time of initiation of administration of the immunomodulatory compound, the subject has been previously administered a T cell therapy for treatment of the disease or condition, wherein the T cell therapy is or comprises genetically engineered cells expressing a recombinant receptor that specifically binds to an antigen, wherein the recombinant receptor is a chimeric antigen receptor (CAR), and wherein the CAR comprises an extracellular domain comprising an antigen-binding domain that specifically binds to a tumor antigen, a spacer, and an intracellular signaling region,
wherein said immunomodulatory compound is selected from the group consisting of: thalidomide analogs; thalidomide derivatives; compounds that interact with and/or bind to cereblon (CRBN) and/or one or more members of the CRBN E3 ubiquitin-ligase complex; inhibitors of Ikaros (IKZF1); inhibitors of Aiolos (IKZF3); and compounds that enhance or promote ubiquitination and/or degradation of Ikaros (IKZF1) and/or Aiolos (IKZF3); and
wherein initiation of administration of the immunomodulatory compound is carried out 7 to 21 days after initiation of administration of the T cell therapy, and
wherein the immunomodulatory compound is administered orally.

In some embodiments, the method thereby prevents, reduces or ameliorates one or more symptoms or outcomes of the disease or condition.

In some embodiments of any of the methods provided herein, (a) the amount of the immunomodulatory compound administered is insufficient, as a single agent and/or in the absence of administration of the T cell therapy, to ameliorate, reduce or prevent the disease or condition or a symptom or outcome thereof; and/or (b) the amount of the immunomodulatory compound administered is insufficient, as a single agent and/or in the absence of administration of the T cell therapy, to ameliorate, reduce or prevent the disease or condition in the subject or a symptom or outcome thereof; and/or (c) the method thereby reduces or ameliorates a symptom or outcome or burden of the disease or condition to a degree that is greater than the combination of (i) the degree of reduction or amelioration effected by the administration of the immunomodulatory agent alone, optionally on average in a population of subjects having the disease or condition, and (ii) the degree of reduction or amelioration by the administration of the T cell therapy alone, optionally on average in a population of subjects having the disease or condition; and/or (d) the amount of the immunomodulatory compound administered in the method, or administered in one or more doses, is a maintenance-level dose of the compound, or corresponds to a dose of the compound administered to subjects having exhibited a response, optionally a complete response, following administration of the compound for treatment.

In some embodiments of any of the methods provided herein, the disease or condition is refractory or resistant to the immunomodulatory compound and/or has become refractory or resistant thereto following treatment with the immunomodulatory compound; and/or the subject or disease or condition has been determined to have a mutation or factor conferring resistance of the disease or condition to treatment with the immunomodulatory compound.

In some embodiments of any of the methods provided herein, the immunomodulatory compound is selected from: thalidomide analogs, thalidomide derivatives, compounds that interact with and/or bind to cereblon (CRBN) and/or one or more members of the CRBN E3 ubiquitin-ligase complex, inhibitors of Ikaros (IKZF1), inhibitors of Aiolos (IKZF3), compounds that enhance or promote ubiquitination and/or degradation of Ikaros (IKZF1) and/or Aiolos (IKZF3).

In some embodiments of any of the methods provided herein, the administration of the immunomodulatory compound includes: (i) at least one cycle of greater than 30 days beginning upon initiation of the administration of the immunomodulatory compound, wherein the cycle includes administration of the compound, optionally daily or at least daily, for up to 21 consecutive days and/or wherein the last administration of the compound in the cycle is at or less than 21 days after the first administration of the compound in the cycle; and/or (ii) at least two cycles, each of the at least two cycles including administration of the compound for a plurality of consecutive days followed by a rest period during which the immunomodulatory compound is not administered, wherein the rest period is greater than 14 consecutive days; and/or (iii) administration, optionally daily or at least daily, for no more than 14 consecutive days.

In the present invention, the immunomodulatory compound is selected from the group consisting of: thalidomide analogs; thalidomide derivatives; compounds that interact with and/or bind to cereblon (CRBN) and/or one or more members of the CRBN E3 ubiquitin-ligase complex; inhibitors of Ikaros (IKZF1); inhibitors of Aiolos (IKZF3); and compounds that enhance or promote ubiquitination and/or degradation of Ikaros (IKZF1) and/or Aiolos (IKZF3).

In the present invention, the T cell therapy includes cells engineered to express a recombinant receptor. In some embodiments, the engineering includes one or more steps of the ex vivo manufacturing process, optionally selected from among: (1) isolating cells from a biological sample by leukapheresis or apheresis; (2) selecting or enriching cells by immunoaffinity-based methods; (3) introducing a recombinant nucleic acid, optionally a viral vector, into cells; (4) incubating cells, optionally engineered cells, in the presence of one or more stimulating conditions; (5) formulating cells in the presence of a cryoprotectant; and/or (6) formulating cells for administration to a subject, optionally in the presence of a pharmaceutically acceptable excipient.

In some embodiments of any of the methods provided herein, the method includes carrying out the manufacturing process and/or further including engineering T cells to express a recombinant receptor, thereby generating the T cell therapy. In some embodiments of any of the methods provided herein, the method includes contacting cells with an immunomodulatory compound during one or more of the steps of the ex vivo manufacturing process.

In some embodiments of any of the methods provided herein, the T cell therapy includes engineered T cells produced by a manufacturing process including incubation of cells, ex vivo, in the presence of the immunomodulatory compound.

In some embodiments of any of the methods provided herein, the method involves incubating cells in the presence of one or more stimulating conditions, which is carried out in the presence of an immunomodulatory compound.

In some embodiments of any of the methods provided herein, initiation of administration of the immunomodulatory compound in at least one cycle is after initiation of administration of the T cell therapy.

In some embodiments of any of the methods provided herein, the T cell therapy is one in which the peak number of a population of cells of the therapy, which optionally are CD3+ or CD8+ cells of the T cell therapy and are CAR+ T cells, in the blood is ((a) on average in a plurality of subjects treated with the T cell therapy in the absence of administration of the immunomodulatory compound, or (b) in the subject following administration of the T cell therapy) less than 10 cells per µL, less than 5 cells per µL or less than per 1 cells per µL.

In the present invention, the T cell therapy includes cells expressing a recombinant receptor, in particular a CAR. In some embodiments of any of the methods provided herein, the recombinant receptor includes an antigen-binding domain specific for a B cell maturation antigen (BCMA).

In some embodiments of any of the methods provided herein, initiation of administration of the immunomodulatory compound in at least one cycle is carried out after initiation of administration of the T cell therapy. In the invention, initiation of administration of the immunomodulatory compound is carried out 7 to 21 days after initiation of administration of, or after the last dose of, the T cell therapy.

In some embodiments of any of the methods provided herein, initiation of administration of the immunomodulatory compound is carried out at a time that is greater than or greater than about 14 days, 15 days, 16 days, 17 days, 18 days, 19, days, or 20 days after initiation of the administration of the T cell therapy.

In some embodiments of any of the methods provided herein, prior to initiation of administration of the immunomodulatory compound, selecting a subject in which: (i) peak or maximum level of the cells of the T cell therapy are detectable in the blood of the subject; (ii) the number of cells of the T cell therapy detectable in the blood, after having been detectable in the blood, is not detectable or is reduced, optionally reduced compared to a preceding time point after administration of the T cell therapy; (iii) the number of cells of the T cell therapy detectable in the blood is decreased by or more than 1.5-fold, 2.0-fold, 3.0-fold, 4.0-fold, 5.0-fold, 10-fold or more the peak or maximum number cells of the T cell therapy detectable in the blood of the subject after initiation of administration of the T cell therapy; (iv) at a time after a peak or maximum level of the cells of the T cell therapy are detectable in the blood of the subject, the number of cells of or derived from the T cells detectable in the blood from the subject is less than less than 10%, less than 5%, less than 1% or less than 0.1% of total peripheral blood mononuclear cells (PBMCs) in the blood of the subject; (v) the subject exhibits disease progression and/or has relapsed following remission after treatment with the T cell therapy; and/or (iv) the subject exhibits increased tumor burden as compared to tumor burden at a time prior to or after administration of the T cells and prior to initiation of administration of the immunomodulatory compound.

In some embodiments of any of the methods provided herein, the immunomodulatory compound is administered for greater than or greater than about 7 consecutive days, greater than or greater than about 14 consecutive days, greater than or greater than about 21 consecutive days, greater than or greater than about 21 consecutive days, or greater than or greater than about 28 consecutive days. In some embodiments of any of the methods provided herein, the immunomodulatory compound is administered in a cycle including administration daily for a plurality of consecutive days followed by a rest period during which the immunomodulatory compound is not administered. In some embodiments, the rest period during with the immunomodulatory compound is not administered is greater than 7 consecutive days, greater than 14 consecutive days, greater than 21 days, or greater than 28 days.

In some embodiments of any of the methods provided herein, the cycle of administration of the immunomodulatory compound is repeated at least one time. In some embodiments, the immunomodulatory compound is administered for at least 2 cycles, at least 3 cycles, at least 4 cycles, at least 5 cycles, at least 6 cycles, at least 7 cycles, at least 8 cycles, at least 9 cycles, at least 10 cycles, at least 11 cycles, or at least 12 cycles.

In some embodiments of any of the methods provided herein, the administration of the immunomodulatory compound is continued, from at least after initiation of administration of the T cells, until: the number of cells of or derived from the administered T cell therapy detectable in the blood from the subject is increased compared to in the subject at a preceding time point just prior to administration of the immunomodulatory compound or compared to a preceding time point after administration of the T-cell therapy; the number of cells of or derived from the T cell therapy detectable in the blood is within 2.0-fold (greater or less) the peak or maximum number observed in the blood of the subject after initiation of administration of the T cells; the number of cells of the T cell therapy detectable in the blood from the subject is greater than or greater than about 10%, 15%, 20%, 30%, 40%, 50%, or 60% total peripheral blood mononuclear cells (PBMCs) in the blood of the subject; and/or the subject exhibits a reduction in tumor burden as compared to tumor burden at a time immediately prior to the administration of the T cell therapy or at a time immediately prior to the administration of the immunomodulatory compound; and/or the subject exhibits complete or clinical remission.

In some embodiments of any of the methods provided herein, the immunomodulatory compound binds to cereblon (CRBN) and/or the CRBN E3 ubiquitin-ligase complex; and/or is an inhibitor of Ikaros (IKZF1) or Aiolos (IKZF3) transcription factor; and/or enhances ubiquitination or degradation of Ikaros (IKZF1) or Aiolos (IKZF3).

In some embodiments of any of the methods provided herein, the immunomodulatory compound is thalidomide or is a derivative or analogue of thalidomide. In some embodiments, the immunomodulatory compound is lenalidomide (3-(4-amino-1-oxo-1,3-dihydro-2H-isoindol-2-yl)piperidine-2,6-dione) or pomalidomide (4-amino-2-(2,6-dioxopiperidin-3-yl)isoindole-1,3-dione), avadomide (3-(5-amino-2-methyl-4-oxo-4H-quinazolin-3-yl)-piperidine-2,6-dione), a stereoisomer of lenalidomide (3-(4-amino-1-oxo-1,3-dihydro-2H-isoindol-2-yl)piperidine-2,6-dione), pomalidomide (4-amino-2-(2,6-dioxopiperidin-3-yl)isoindole-1,3-dione), avadomide (3-(5-amino-2-methyl-4-oxo-4H-quinazolin-3-yl)-piperidine-2,6-dione) or a pharmaceutically acceptable salt, solvate, hydrate, co-crystal, clathrate, or polymorph thereof. In some embodiments, the immunomodulatory compound is lenalidomide (3-(4-amino-1-oxo-1,3-dihydro-2H-isoindol-2-yl)piperidine-2,6-dione), a stereoisomer of lenalidomide (3-(4-amino-1-oxo-1,3-dihydro-2H-isoindol-2-yl)piperidine-2,6-dione) or a pharmaceutically acceptable salt, solvate, hydrate, co-crystal, clathrate, or polymorph thereof.

In some of any of the embodiments provided herein, the immunomodulatory compound is lenalidomide (3-(4-amino-1-oxo-1,3-dihydro-2H-isoindol-2-yl)piperidine-2,6-dione), pomalidomide (4-amino-2-(2,6-dioxopiperidin-3-yl)isoindole-1,3-dione), or avadomide (3-(5-amino-2-methyl-4-oxo-4H-quinazolin-3-yl)-piperidine-2,6-dione), a stereoisomer of lenalidomide (3-(4-amino-1-oxo-1,3-dihydro-2H-isoindol-2-yl)piperidine-2,6-dione), pomalidomide (4-amino-2-(2,6-dioxopiperidin-3-yl)isoindole-1,3-dione), or avadomide (3-(5-amino-2-methyl-4-oxo-4H-quinazolin-3-yl)-piperidine-2,6-dione), or a pharmaceutically acceptable salt, solvate, hydrate, co-crystal, clathrate, or polymorph thereof.

In some embodiments, the immunomodulatory compound is 3-(4-amino-1-oxo-1,3-dihydro-2H-isoindol-2-yl)piperidine-2,6-dione, or a stereoisomer thereof, or a pharmaceutically acceptable salt, solvate, hydrate, co-crystal, clathrate, or polymorph thereof. In some embodiments, the immunomodulatory compound is 3-(4-amino-1-oxo-1,3-dihydro-2H-isoindol-2-yl)piperidine-2,6-dione. In some embodiments, the immunomodulatory compound is 3-(5-amino-2-methyl-4-oxo-4H-quinazolin-3-yl)-piperidine-2,6-dione, or a stereoisomer thereof, or a pharmaceutically acceptable salt, solvate, hydrate, co-crystal, clathrate, or polymorph thereof. In some embodiments, the immunomodulatory compound is 3-(5-amino-2-methyl-4-oxo-4H-quinazolin-3-yl)-piperidine-2,6-dione.

In the present invention, the immunomodulatory compound is administered orally. In some embodiments, the immunomodulatory compound is administered in a capsule or a tablet.

In some embodiments of any of the methods provided herein, the immunomodulatory compound is administered in an amount from or from about 0.1 mg to about 100 mg, from or from about 0.1 mg to 50 mg, from or from about 0.1 mg to 25 mg, from or from about 0.1 mg to 10 mg, from or from about 0.1 mg to 5 mg, from or from about 0.1 mg to 1 mg, from or from about 1 mg to 100 mg, from or from about 1 mg to 50 mg, from or from about 1 mg to 25 mg, from or from about 1 mg to 10 mg, from or from about 1 mg to 5 mg, from or from about 5 mg to 100 mg, from or from about 5 mg to 50 mg, from or from about 5 mg to 25 mg, from or from about 5 mg to 10 mg, from or from about 10 mg to 100 mg, from or from about 10 mg to 50 mg, from or from 10 mg to 25 mg, from or from about 25 mg to 100 mg, from or from about 25 mg to 50 mg or from or from about 50 mg to 100 mg, each inclusive.

In some embodiments of any of the methods provided herein, the immunomodulatory compound is administered once daily, twice daily, three times daily, four times daily, five times daily, or six times daily. In some embodiments, the immunomodulatory compound is administered at a total daily dosage amount of at least or at least about 0.1 mg per day, 0.5 mg per day, 1.0 mg per day, 2.5 mg per day, 5 mg per day, 10 mg per day, 25 mg per day, 50 mg per day or 100 mg per day.

In some embodiments of any of the methods provided herein, the immunomodulatory compound is administered in an amount greater than or greater than about 1 mg, 2.5 mg, 5 mg, 7.5 mg, 10 mg, 15 mg and less than 25 mg; or the immunomodulatory compound is administered in an amount greater than or greater than about 1 mg per day, 2.5 mg per day, 5 mg per day, 7.5 mg per day, 10 mg per day, 15 mg per day and less than 25 mg per day.

In some embodiments of any of the methods provided herein, the administration of the therapeutically effective amount of immunomodulatory compound stimulates an increased expansion of T cells associated with the T cell therapy compared to the expansion of following administration of the T cell therapy in absence of the immunomodulatory compound.

In some embodiments of any of the methods provided herein, the administration of the therapeutically effective amount of immunomodulatory compound stimulates an increase in T cell-mediated cytolytic activity of T cells associated with the T cell therapy compared to the cytolytic activity following the administration of the T cells in absence of the immunomodulatory compound.

In some embodiments of any of the methods provided herein, the administration of the therapeutically effective amount of immunomodulatory compound stimulates an increase in the cytokine production of T cells associated with the T cell therapy compared to cytokine production following the administration of the T cells in absence of the immunomodulatory compound. In some embodiments, the increase is greater than or greater than about 1.5-fold, 2.0-fold, 3.0-fold, 4.0-fold, 5.0-fold, 10.0-fold or more.

In the present invention, the T cell therapy is or includes genetically engineered cells expressing a recombinant receptor that specifically binds to an antigen. In the present invention, the recombinant antigen receptor is a chimeric antigen receptor (CAR).

In the present invention, the T cell therapy includes a recombinant antigen receptor, in particular a CAR, which includes an extracellular domain containing an antigen-binding domain that specifically binds to an antigen. In the present invention, the antigen is associated with, specific to, and/or expressed on a cell or tissue of a disease, disorder or condition. In the present invention, the disease, disorder or condition is a tumor or a cancer. In the present invention, the antigen is a tumor antigen.

In some embodiments of any of the methods provided herein, the antigen is selected from among ROR1, B cell maturation antigen (BCMA), carbonic anhydrase 9 (CAIX), tEGFR, Her2/neu (receptor tyrosine kinase erbB2), L1-CAM, CD19, CD20, CD22, mesothelin, CEA, and hepatitis B surface antigen, anti-folate receptor, CD23, CD24, CD30, CD33, CD38, CD44, EGFR, epithelial glycoprotein 2 (EPG-2), epithelial glycoprotein 40 (EPG-40), EPHa2, erb-B2, erb-B3, erb-B4, erbB dimers, EGFR vIII, folate binding protein (FBP), FCRL5, FCRH5, fetal acetylcholine receptor, GD2, GD3, HMW-MAA, IL-22R-alpha, IL-13R-alpha2, kinase insert domain receptor (kdr), kappa light chain, Lewis Y, L1-cell adhesion molecule, (L1-CAM), Melanoma-associated antigen (MAGE)-A1, MAGE-A3, MAGE-A6, Preferentially expressed antigen of melanoma (PRAME), survivin, TAG72, B7-H6, IL-13 receptor alpha 2 (IL-13Ra2), CA9, GD3, HMW-MAA, CD171, G250/CAIX, HLA-AI MAGE Al, HLA-A2 NY-ESO-1, PSCA, folate receptor-a, CD44v6, CD44v7/8, avb6 integrin, 8H9, NCAM, VEGF receptors, 5T4, Foetal AchR, NKG2D ligands, CD44v6, dual antigen, a cancer-testes antigen, mesothelin, murine CMV, mucin 1 (MUC1), MUC16, PSCA, NKG2D, NY-ESO-1, MART-1, gp100, G Protein Coupled Receptor 5D (GPCR5D), oncofetal antigen, ROR1, TAG72, VEGF-R2, carcinoembryonic antigen (CEA), Her2/neu, estrogen receptor, progesterone receptor, ephrinB2, CD123, c-Met, GD-2, O-acetylated GD2 (OGD2), CE7, Wilms Tumor 1 (WT-1), a cyclin, cyclin A2, CCL-1, CD138, optionally a human antigen of any of the foregoing; a pathogen-specific antigen; and an antigen associated with a universal tag. In some embodiments, the antigen is or includes CD19, optionally human CD19. In some embodiments, the antigen is or includes a multiple myeloma-associated antigen, optionally a BCMA, optionally human BCMA.

In some embodiments of any of the methods provided herein, the antigen-binding domain is or includes an antibody or an antibody fragment thereof, which optionally is a single chain fragment. In some embodiments, the fragment includes antibody variable regions joined by a flexible linker. In some embodiments, the fragment includes an scFv.

In the present invention, the T cell therapy includes a recombinant receptor, in particular a CAR, that further includes a spacer, optionally derived from an immunoglobulin, optionally containing a hinge region. In the present invention, the recombinant antigen receptor, in particular the CAR, includes an intracellular signaling region. In some embodiments, the intracellular signaling region includes an intracellular signaling domain. In some embodiments, the intracellular signaling domain is or includes a primary signaling domain, a signaling domain that is capable of inducing a primary activation signal in a T cell, a signaling domain of a T cell receptor (TCR) component, and/or a signaling domain containing an immunoreceptor tyrosine-based activation motif (ITAM). In some embodiments of any of the methods provided herein, the intracellular signaling domain is or includes an intracellular signaling domain of a CD3 chain, optionally a CD3-zeta (CD3ζ) chain, or a signaling portion thereof.

In some embodiments of any of the methods provided herein, the recombinant receptor, in particular the CAR, further includes a transmembrane domain disposed between the extracellular domain and the intracellular signaling region, wherein the transmembrane domain is optionally transmembrane domain of CD8 or CD28. In some embodiments, the intracellular signaling region further includes a costimulatory signaling region. In some embodiments, costimulatory signaling region includes an intracellular signaling domain of a T cell costimulatory molecule or a signaling portion thereof. In some embodiments, the costimulatory signaling region includes an intracellular signaling domain of a CD28, a 4-1BB or an ICOS or a signaling portion thereof. In some embodiments, the costimulatory signaling region containing an intracellular signaling domain of 4-1BB. In some embodiments of any of the methods provided herein, the costimulatory signaling region is between the transmembrane domain and the intracellular signaling region.

In some embodiments of any of the methods provided herein, the T cell therapy includes: T cells selected from central memory T cells, effector memory T cells, naive T cells, stem central memory T cells, effector T cells and regulatory T cells; and/or a plurality of cells, the plurality containing at least 50 % of a population of cells selected from CD4+ T cells, CD8+ T cells, central memory T cells, effector memory T cells, naive T cells, stem central memory T cells, effector T cells and regulatory T cells.

In some embodiments of any of the methods provided herein, the T cell therapy includes T cells that are CD4+ or CD8+. In some embodiments, the T cell therapy includes primary cells derived from a subject. In some embodiments, the T cell therapy includes cells that are autologous to the subject. In some embodiments, the T cell therapy includes T cells that are allogeneic to the subject. In some embodiments of any of the methods provided herein, the subject is a human.

In some embodiments of any of the methods provided herein, the T cell therapy includes the administration of from or from about 1 × 105 to 1 × 108 total recombinant receptor-expressing cells, total T cells, or total peripheral blood mononuclear cells (PBMCs), from or from about 5 × 10⁵ to 1 × 10⁷ total recombinant receptor-expressing cells, total T cells, or total peripheral blood mononuclear cells (PBMCs) or from or from about 1 × 10⁶ to 1 × 10⁷ total recombinant receptor-expressing cells, total T cells, or total peripheral blood mononuclear cells (PBMCs), each inclusive.

In some embodiments of any of the methods provided herein, the T cell therapy includes the administration of no more than 1 × 10⁸ total recombinant receptor-expressing cells, total T cells, or total peripheral blood mononuclear cells (PBMCs), no more than 1 × 10⁷ total recombinant receptor-expressing cells, total T cells, or total peripheral blood mononuclear cells (PBMCs), no more than 0.5 × 10⁷ total recombinant receptor-expressing cells, total T cells, or total peripheral blood mononuclear cells (PBMCs), no more than 1 × 10⁶ total recombinant receptor-expressing cells, total T cells, or total peripheral blood mononuclear cells (PBMCs), no more than 0.5 × 10⁶ total recombinant receptor-expressing cells, total T cells, or total peripheral blood mononuclear cells (PBMCs).

In some embodiments of any of the methods provided herein, the amount of cells administered in the T cell therapy is less than the amount in another method in which the T cell therapy is administered without administration of the immunomodulatory compound, optionally which other method results in a similar or lower degree of amelioration or reduction or prevention of the disease or condition or symptom or burden thereof, as compared to that resulting from the method. In some embodiments, the amount of cells administered is 1.5-fold, 2-fold, 3-fold, 4-fold, 5-fold, or 10-fold less than that administered in the other method.

In some embodiments of any of the methods provided herein, the T cell therapy is administered as a single pharmaceutical composition containing the cells. In some embodiments of any of the methods provided herein, the T cell therapy includes a dose of cell that is a split dose, wherein the cells of the dose are administered in a plurality of compositions, collectively containing the cells of the dose, over a period of no more than three days.

In some embodiments of any of the methods provided herein, the method further includes administering a lymphodepleting chemotherapy prior to administration of the T cell therapy.

In the present invention, the disease or condition is cancer. In some embodiments, the cancer is a B cell malignancy and/or a myeloma, lymphoma or leukemia. In some embodiments, the cancer is mantle cell lymphoma (MCL), multiple myeloma (MM), acute lymphoblastic leukemia (ALL), adult ALL, chronic lymphoblastic leukemia (CLL), non-Hodgkin lymphoma (NHL), or Diffuse Large B-Cell Lymphoma (DLBCL). In some embodiments, cancer is a non-hematological cancer or is a solid tumor.

In some embodiments of any of the methods provided herein, the T cell therapy exhibits increased or prolonged expansion and/or persistence in the subject as compared to a method in which the T cell therapy is administered to the subject in the absence of the immunomodulatory compound.

In some embodiments of any of the methods provided herein, the method reduces tumor burden to a greater degree and/or for a greater period of time as compared to the reduction that would be observed with a comparable method in which the T cell therapy is administered to the subject in the absence of the immunomodulatory compound and/or in which the immunomodulatory compound is administered in the absence of the T cell therapy, optionally at the same dose or dosing schedule.

### Brief Description of the Drawings

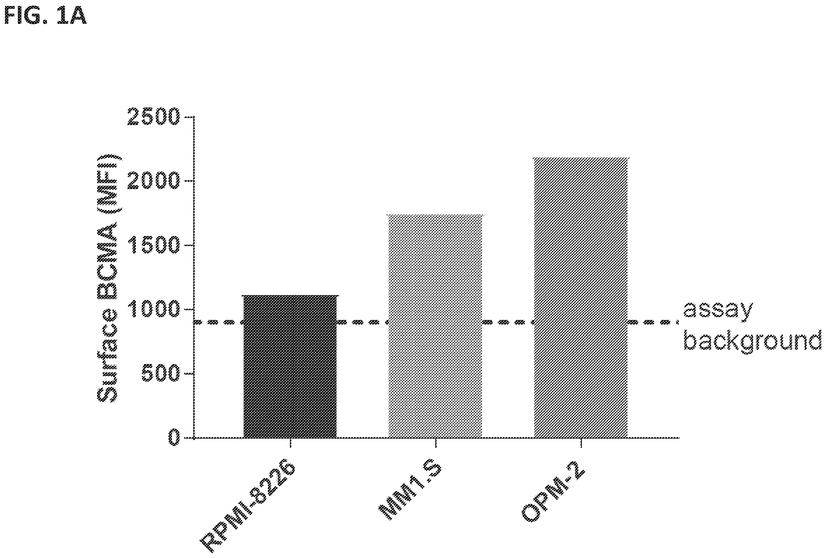

**FIG.1A** shows the surface BCMA expression of multiple myeloma cells lines (RPMI-8226, MM1.S, and OPM-2). The dotted line indicates background and BCMA-negative cell lines were stained with anti-BCMA antibody. MFI, median fluorescence intensity.

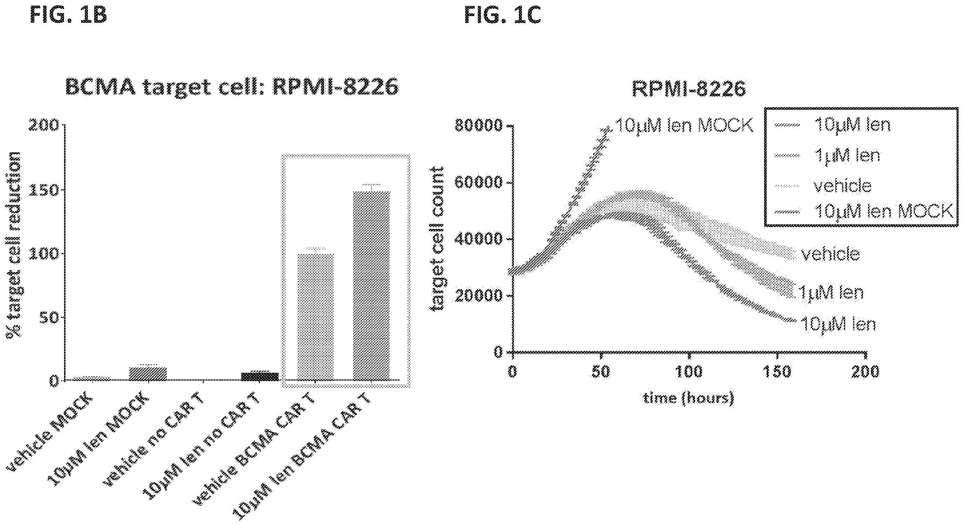

**FIG.1B** shows the percent of reduction of BCMA-expressing target cells (RPMI-8226) by anti-BCMA CAR+ T cells in the presence and absence of lenalidomide (10 µM) after 6 days of co-culture. **FIG. 1C** shows the effect of lenalidomide on the cytolytic activity of anti-BCMA CAR+ T cells against RPMI-8226 target cells.

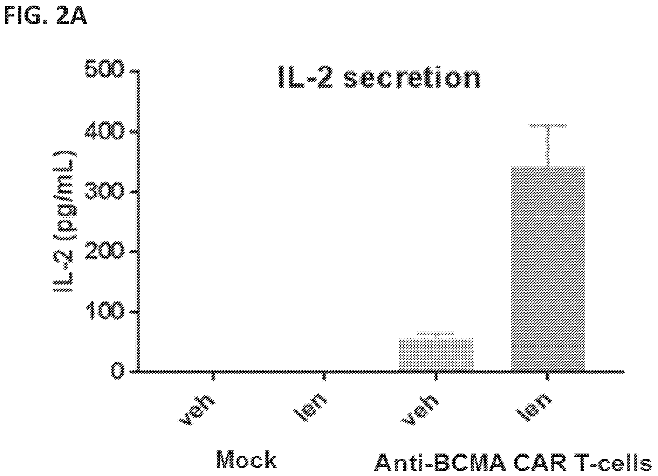

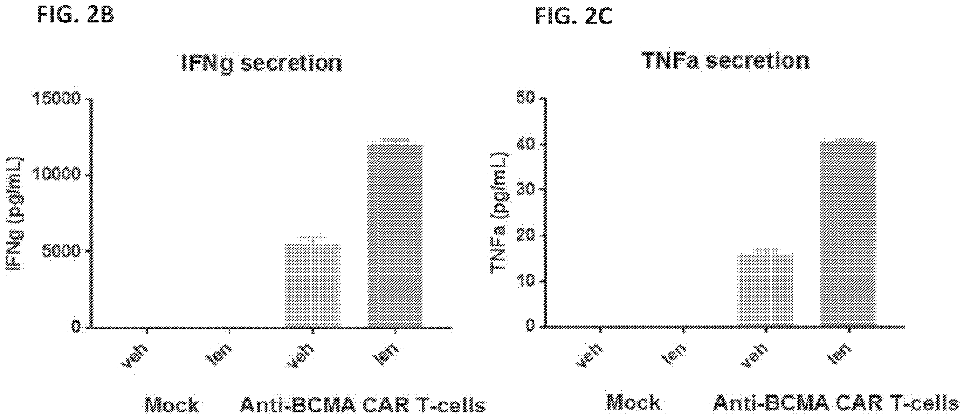

**FIGs. 2A-2C** show the amount of IL-2 (FIG. 2A), IFNγ (FIG. 2B), and TNF-α (FIG. 2C) observed in supernatants after incubation of RPMI-8226 target cells with anti-BCMA CAR T cells in the presence and absence of lenalidomide.

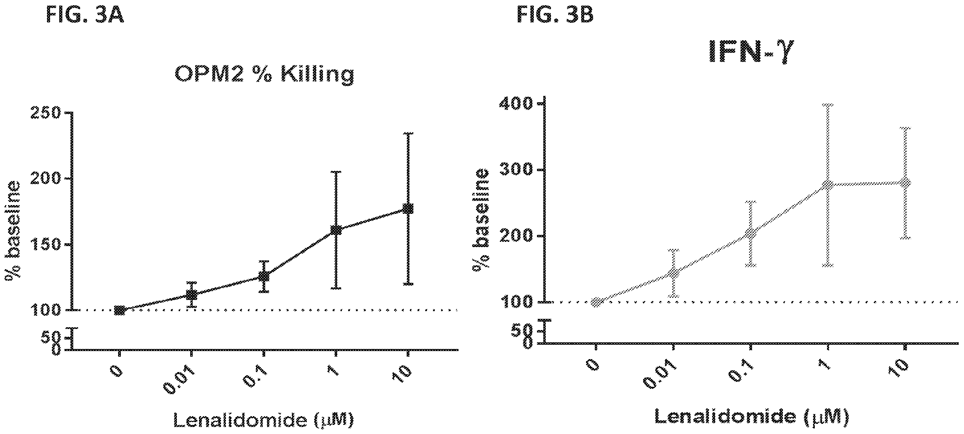

**FIG. 3A** shows the effect of increasing concentrations of lenalidomide on the cytolytic activity of anti-BCMA CAR+ T cells against OPM2 target cells.

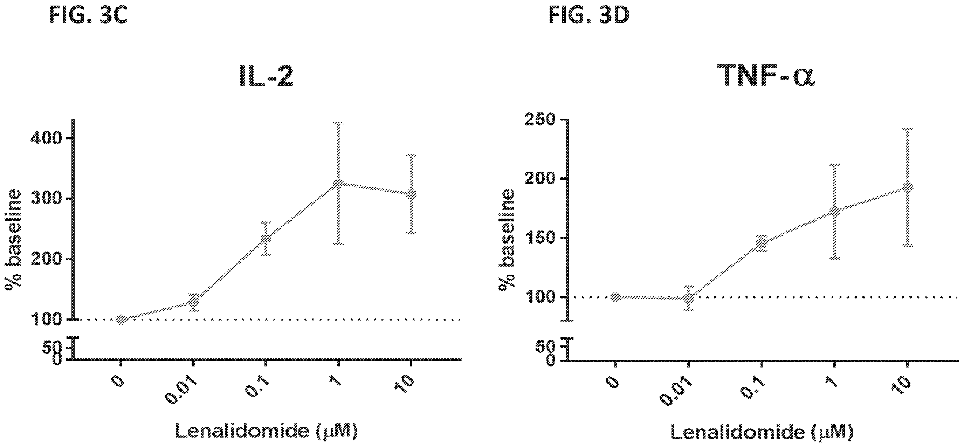

FIGs. 3B-D show the amount of IFNγ (FIG. 3B), IL-2 (FIG. 3C), and TNF-α (FIG. 3D) observed in supernatants after incubation of OPM2 target cells with anti-BCMA CAR T cells in the presence of increasing concentrations of lenalidomide, or in the absence of lenalidomide.

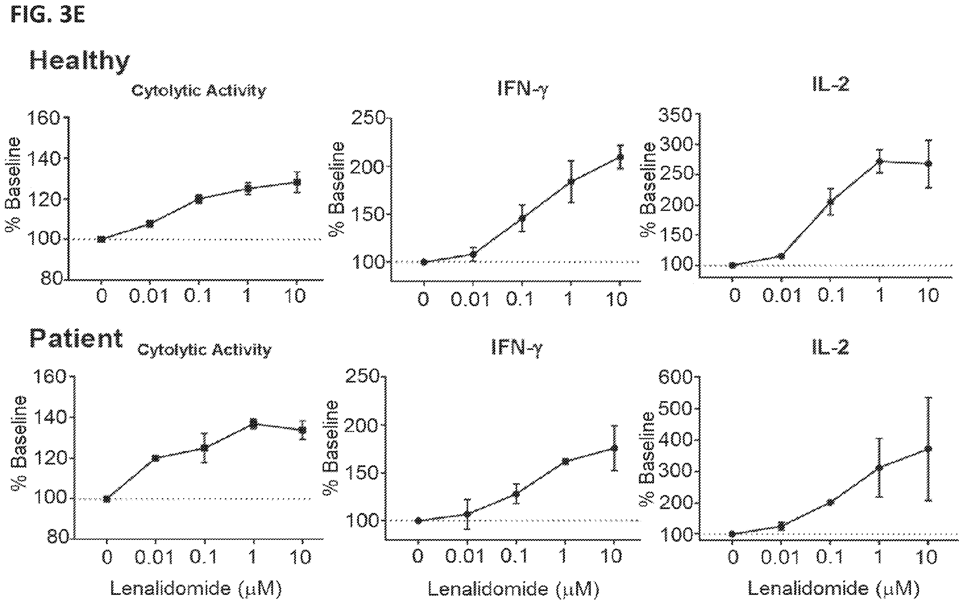

**FIG. 3E** shows the antigen-specific anti-BCMA CAR-T cytolytic activity and cytokine production of anti-BCMA CAR+ T cells derived from representative healthy donors and multiple myeloma patients against OPM-2 target cells in the presence of varying concentrations of lenalidomide (0.01 µM, 0.1 µM, 1.0 µM or 10 µM lenalidomide) or in the absence of lenalidomide.

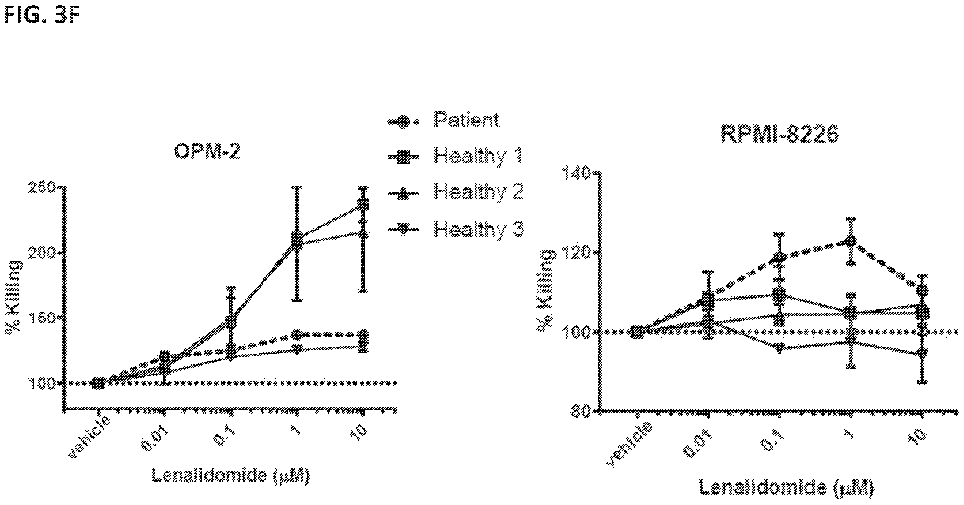

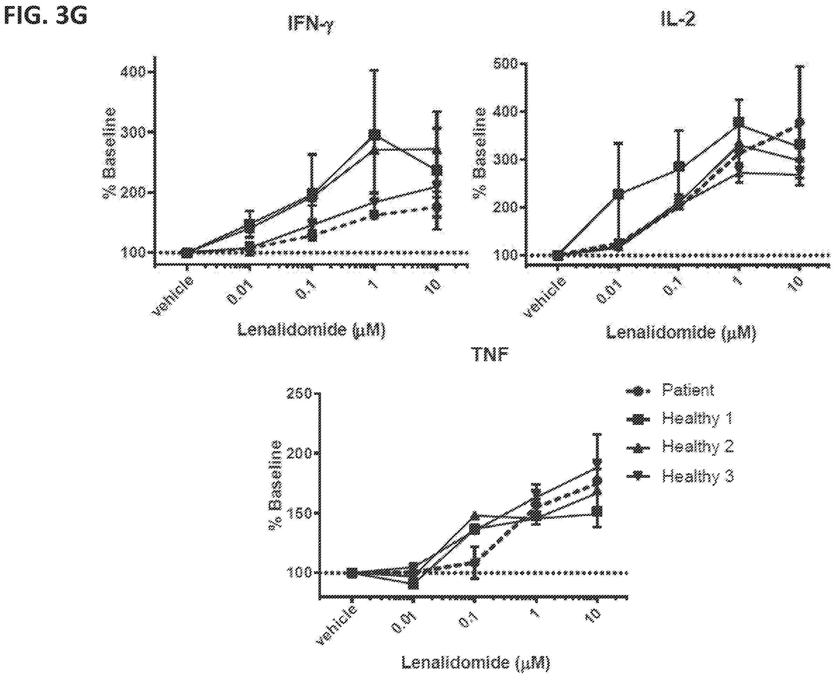

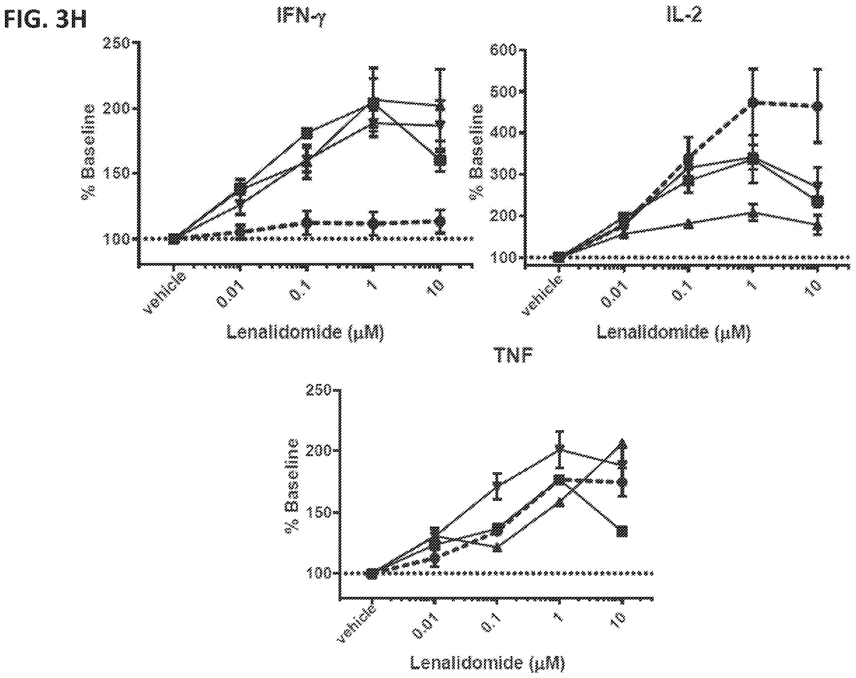

**FIG. 3F** shows the antigen-specific anti-BCMA CAR-T cytolytic activity of anti-BCMA CAR+ T cells derived from three healthy donors and one multiple myeloma patient against OPM-2 and RPMI-8226 target cells in the presence of varying concentrations of lenalidomide (0.01 µM, 0.1 µM, 1.0 µM or 10 µM lenalidomide) or in the absence of lenalidomide. **FIG. 3G** shows cytokine production of anti-BCMA CAR+ T cells derived from three healthy donors and one multiple myeloma patient against OPM-2 target cells in the presence of varying concentrations of lenalidomide (0.01 µM, 0.1 µM, 1.0 µM or 10 µM lenalidomide) or in the absence of lenalidomide.. **FIG. 3H** shows cytokine production of anti-BCMA CAR+ T cells derived from three healthy donors and one multiple myeloma patient against RPMI-8226 target cells in the presence of varying concentrations of lenalidomide (0.01 µM, 0.1 µM, 1.0 µM or 10 µM lenalidomide) or in the absence of lenalidomide.

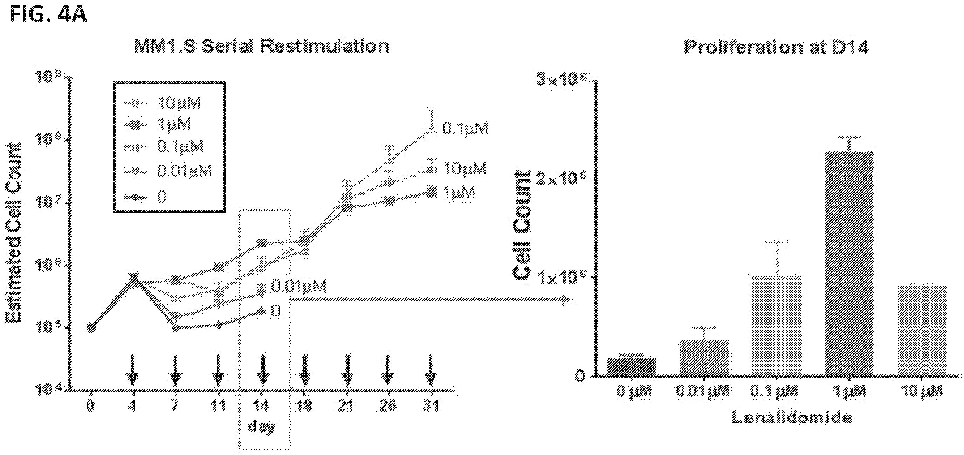

**FIG. 4A** depicts the expansion of anti-BCMA CAR T cells after restimulation in the presence of varying concentrations of lenalidomide.

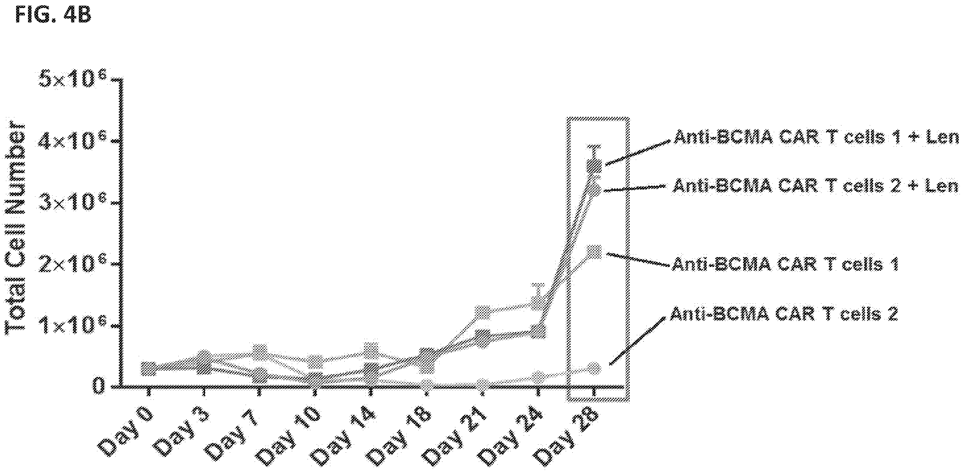

**FIG. 4B** depicts the expansion of anti-BCMA CAR T cells after restimulation both in the presence and absence of lenalidomide.

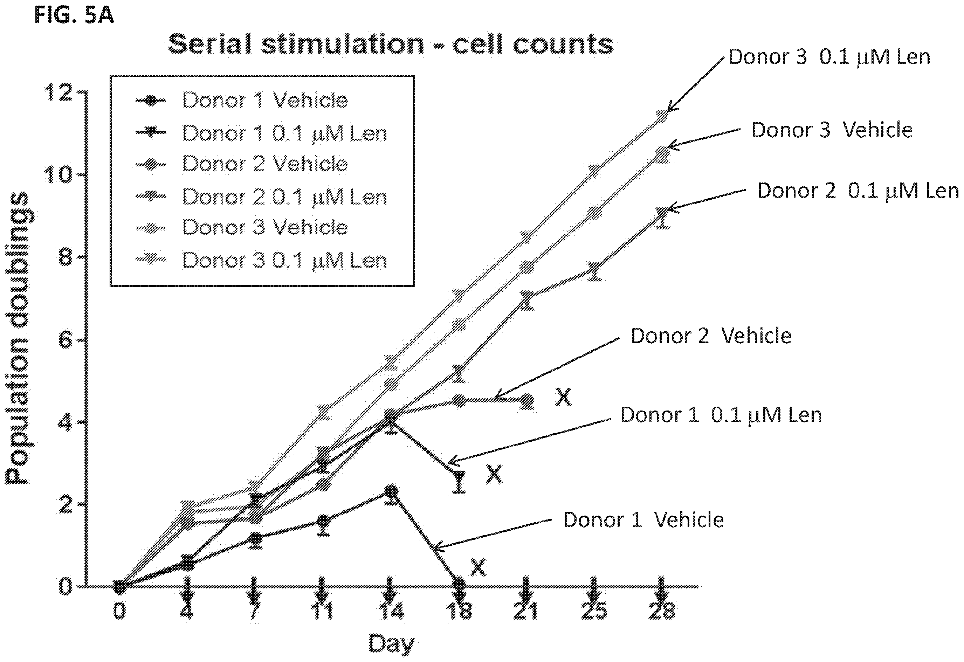

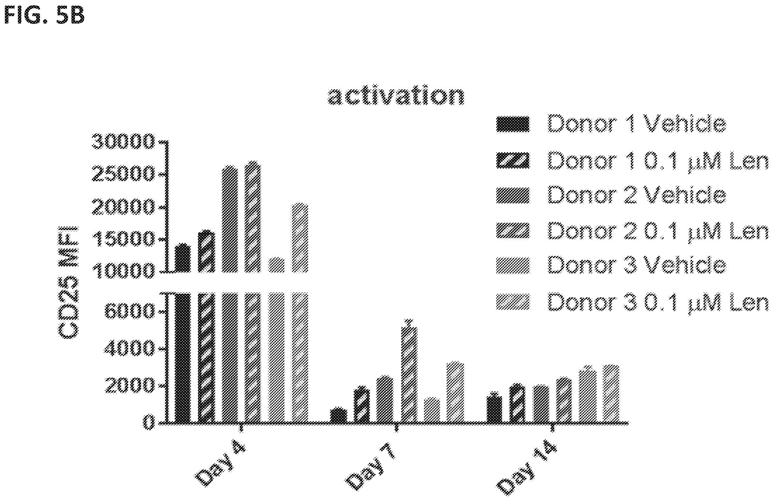

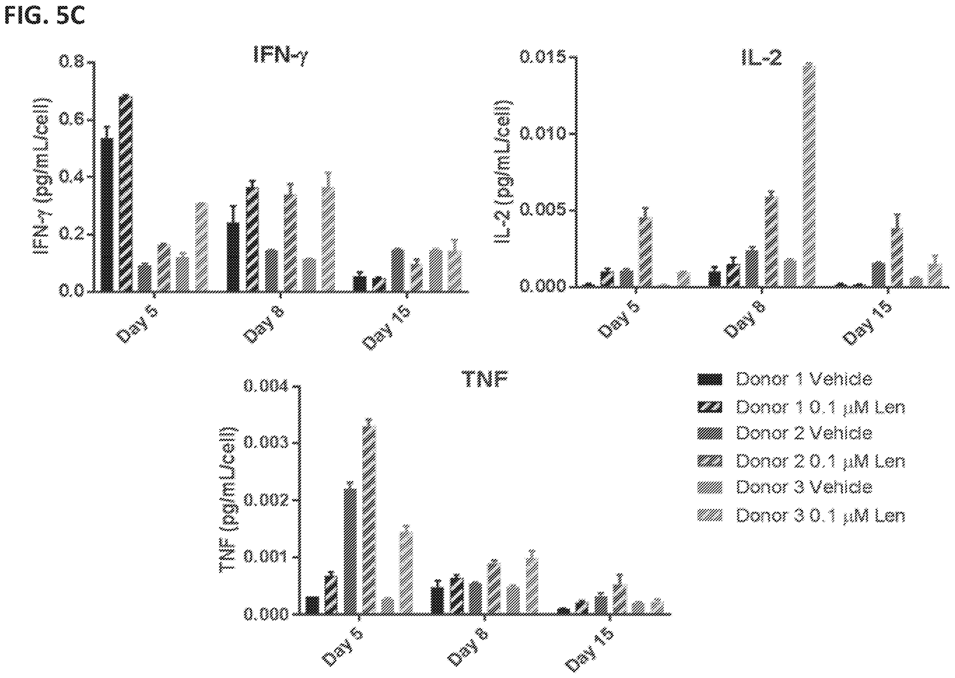

**FIG. 5A** shows the cell counts (projected population doublings) of the anti-BCMA CAR+ T cells from three donors for each time point in the restimulation assay, in the presence of a vehicle or 0.1 µM lenalidomide. The "x" indicates insufficient cells for re-plating in the assay. **FIG. 5B** shows CD25 median fluorescent intensity (MFI) (gated on live CD3⁺ CAR⁺). **FIG. 5C** shows cytokine production normalized for cell number plated (top and left bottom panels) and CD25 median fluorescent intensity (MFI) (gated on live CD3⁺ CAR⁺) (right bottom panel).

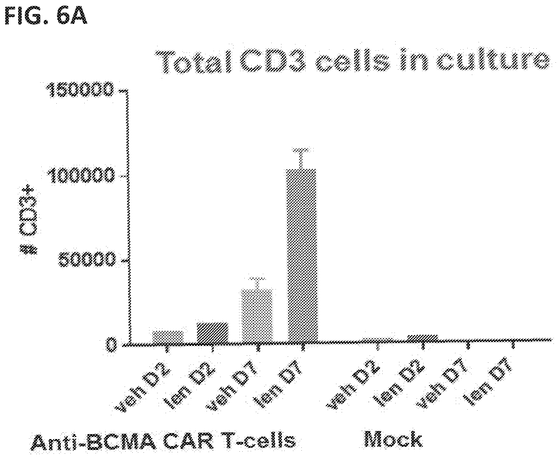

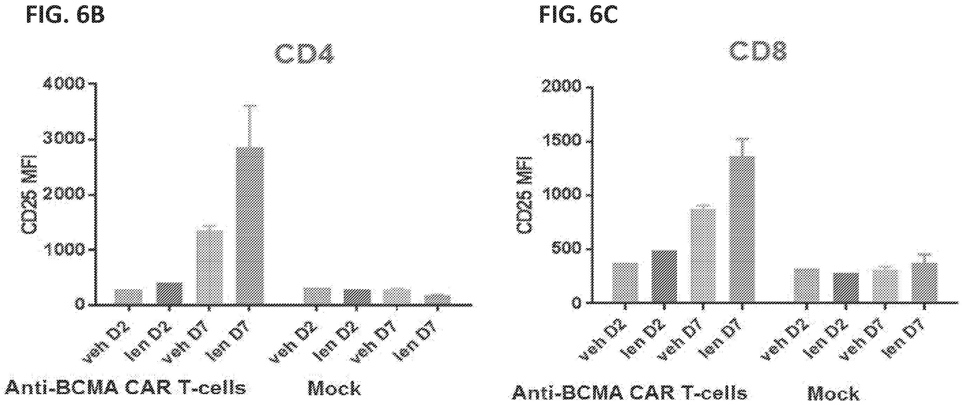

**FIG. 6A** shows the total number of CD3+ cells in culture on days 2 and 7 after incubating anti-BCMA CAR T-Cells or T cells that did not express a CAR (mock) in the presence or absence of lenalidomide. **FIGs. 6B** and **6C** show the CD25+ expression in CD4+ (FIG. 6B) and CD8+ (FIG. 6C) T cells in culture on days 2 and 7 after incubating anti-BCMA CAR T-Cells or T cells that did not express a CAR (mock) in the presence or absence of lenalidomide.

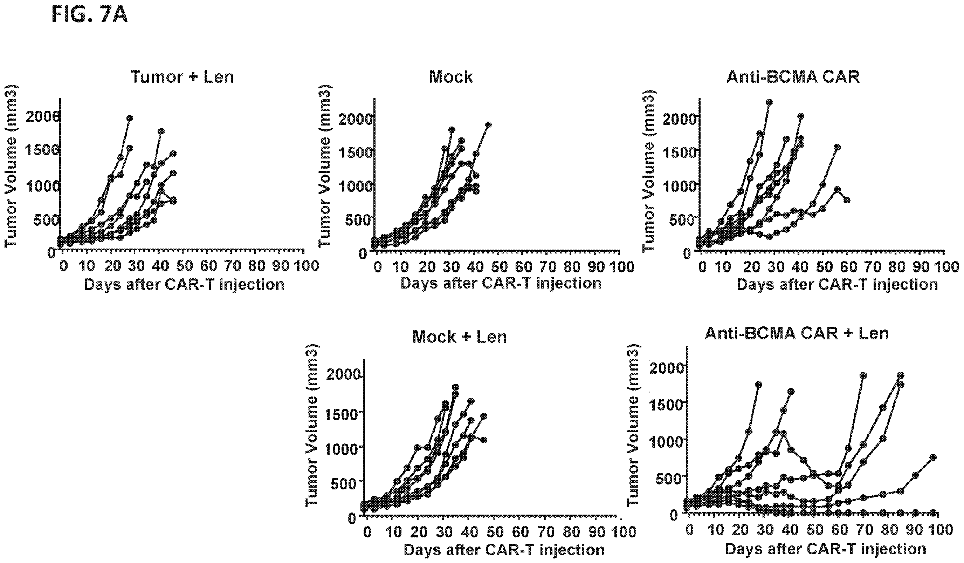

**FIG. 7A** shows the tumor volume of mice over time after administration of a low dose of anti-BCMA CAR+ T cells in the presence and absence of lenalidomide.

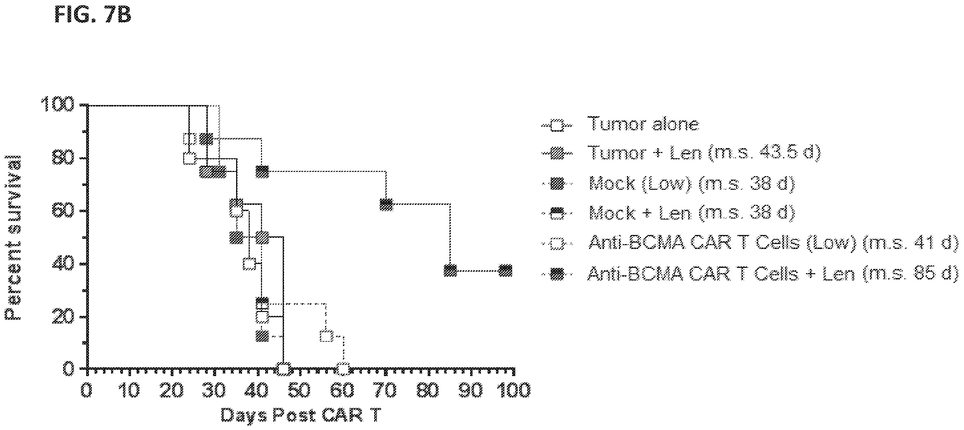

**FIG. 7B** shows the percent survival of mice that were administered a low dose of anti-BCMA CAR+ T cells in the presence and absence of lenalidomide. For the control groups, T cells that did not express a CAR (mock) were administered in the presence and absence of lenalidomide, and lenalidomide without T cells was also administered.

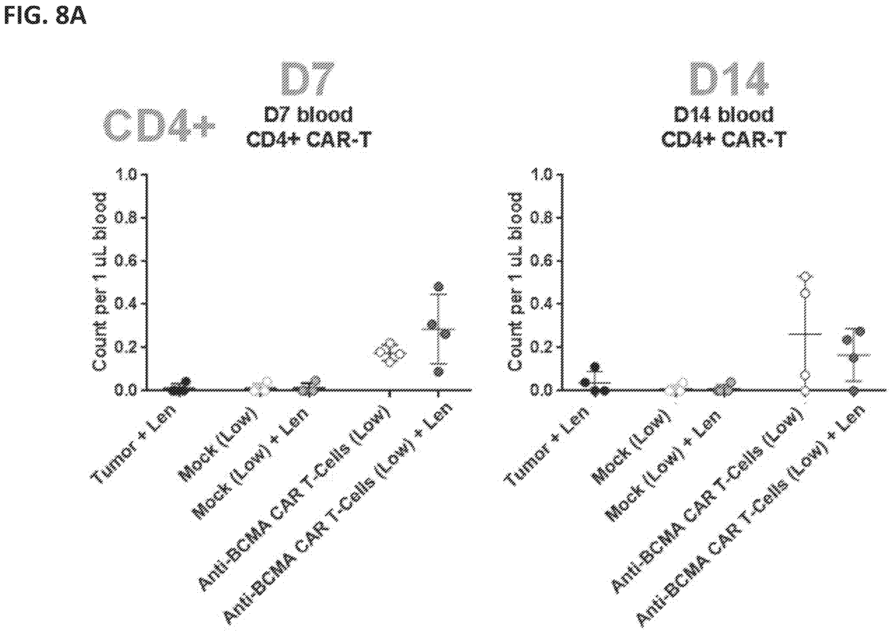

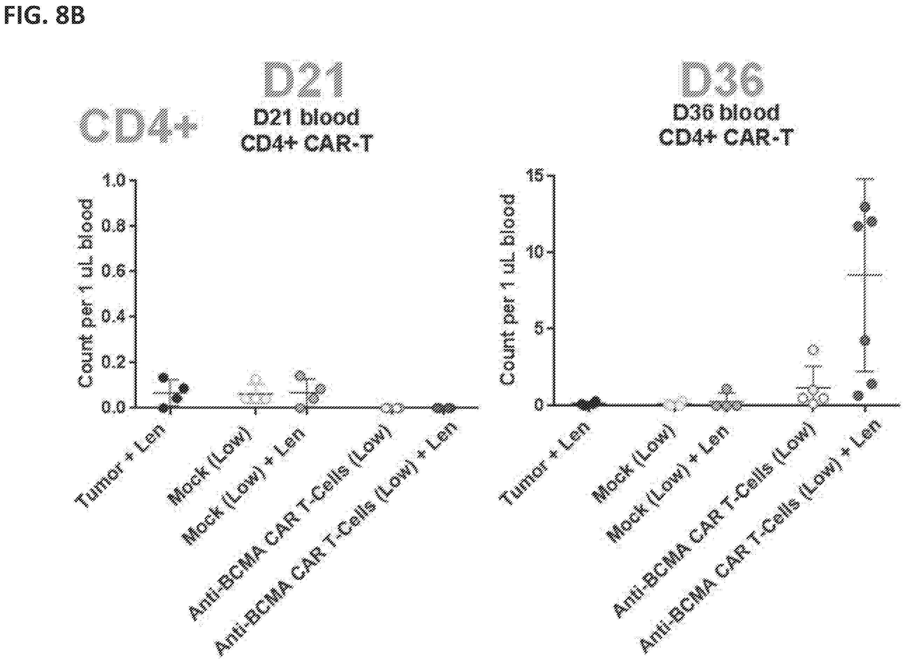

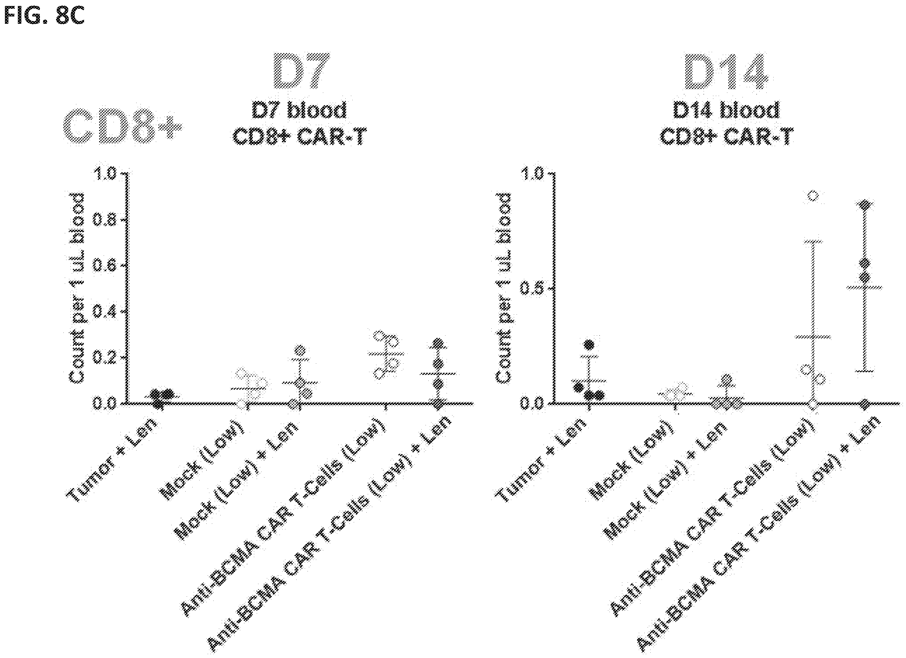

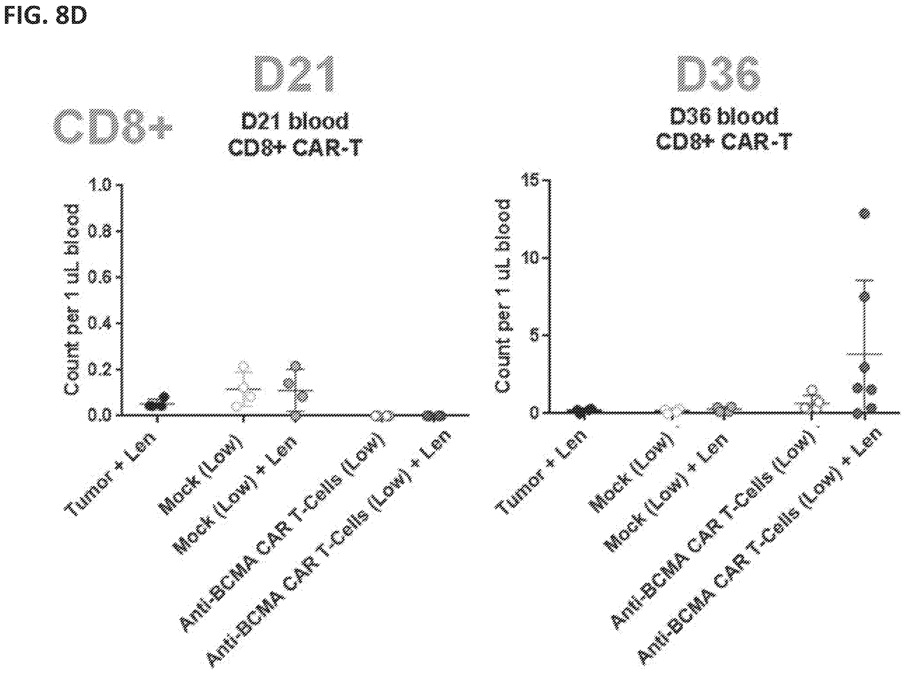

**FIG. 8A** shows the levels of CD4+ CAR+ T cells in the blood from mice treated with anti-BCMA CAR+ T cells and lenalidomide compared to the other treatment groups at days 7, and 14. **FIG. 8B** shows the levels of CD4+ CAR+ T cells in the blood from mice treated with anti-BCMA CAR+ T cells and lenalidomide compared to the other treatment groups at days 21 and 36. **FIG. 8C** shows the levels of CD8+ CAR+ T cells in the blood from mice treated with anti-BCMA CAR+ T cells and lenalidomide compared to the other treatment groups at days 7 and 14. **FIG. 8D** shows the levels of CD8+ CAR+ T cells in the blood from mice treated with anti-BCMA CAR+ T cells and lenalidomide compared to the other treatment groups at days 21 and 36.

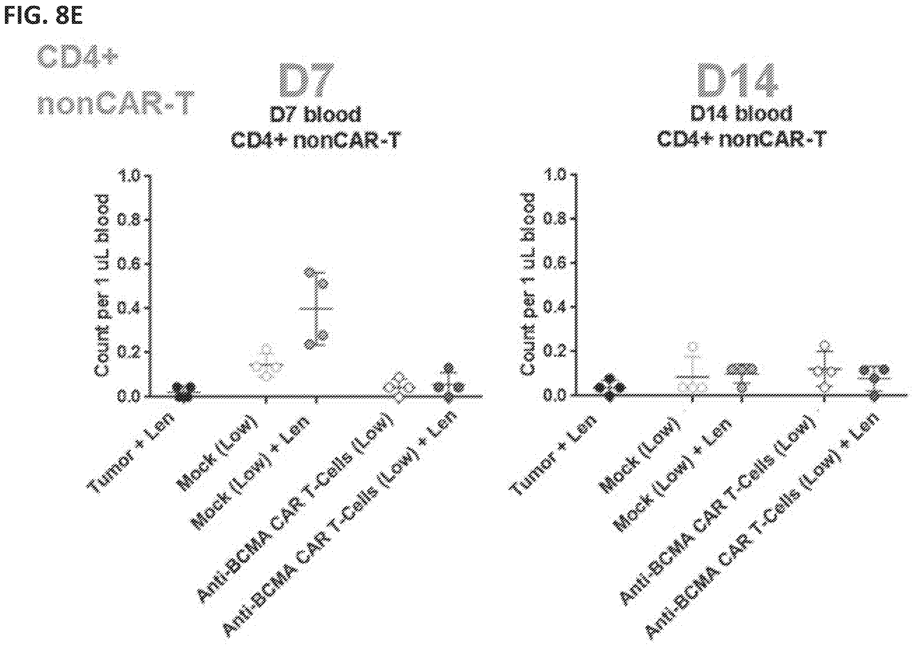

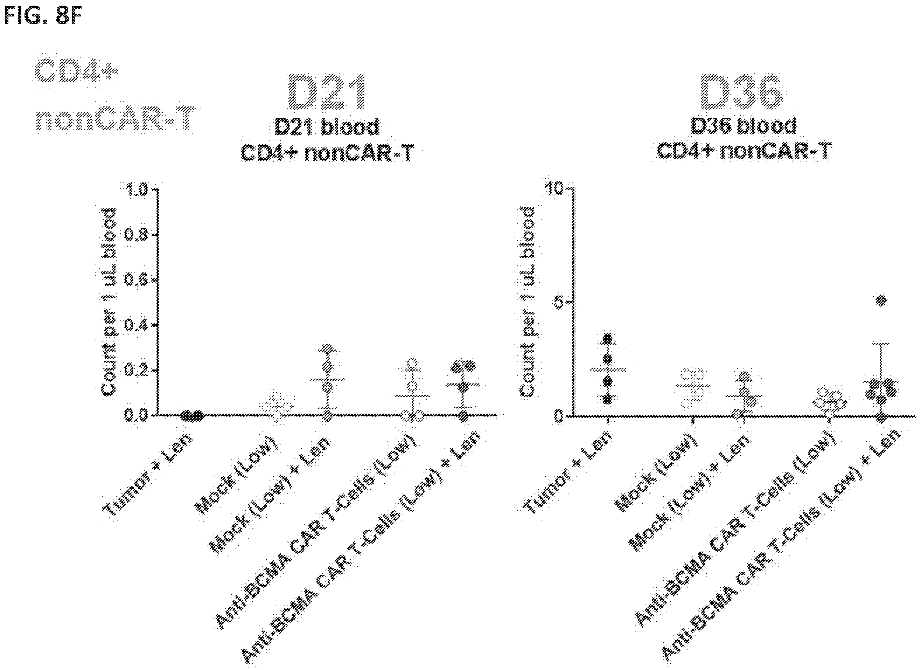

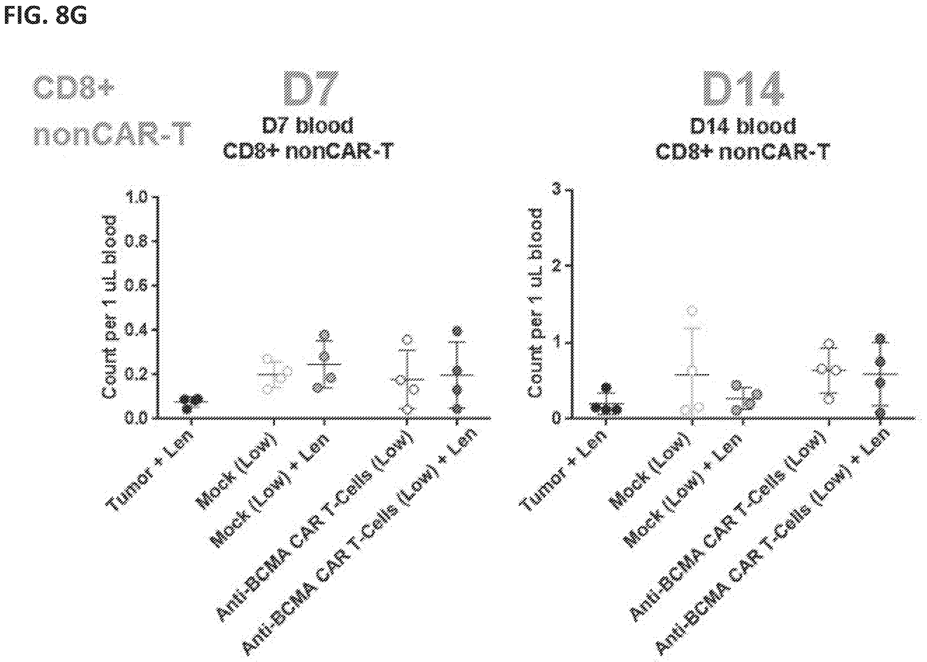

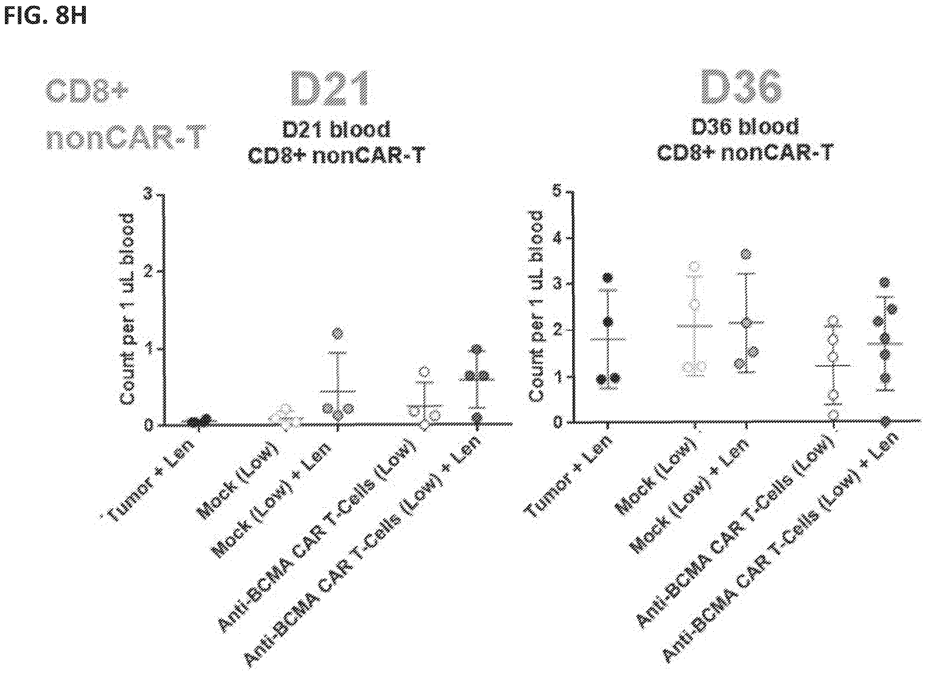

**FIG. 8E** shows the levels of CD4+ CAR+ T cells in the blood from mice treated with non-CAR+ T cells and lenalidomide compared to the other treatment groups at days 7, and 14. **FIG. 8F** shows the levels of CD4+ CAR+ T cells in the blood from mice treated with non-CAR+ T cells and lenalidomide compared to the other treatment groups at days 21 and 36. **FIG. 8G** shows the levels of CD8+ CAR+ T cells in the blood from mice treated with non-CAR+ T cells and lenalidomide compared to the other treatment groups at days 7 and 14. **FIG. 8H** shows the levels of CD8+ CAR+ T cells in the blood from mice treated with non-CAR+ T cells and lenalidomide compared to the other treatment groups at days 21 and 36.

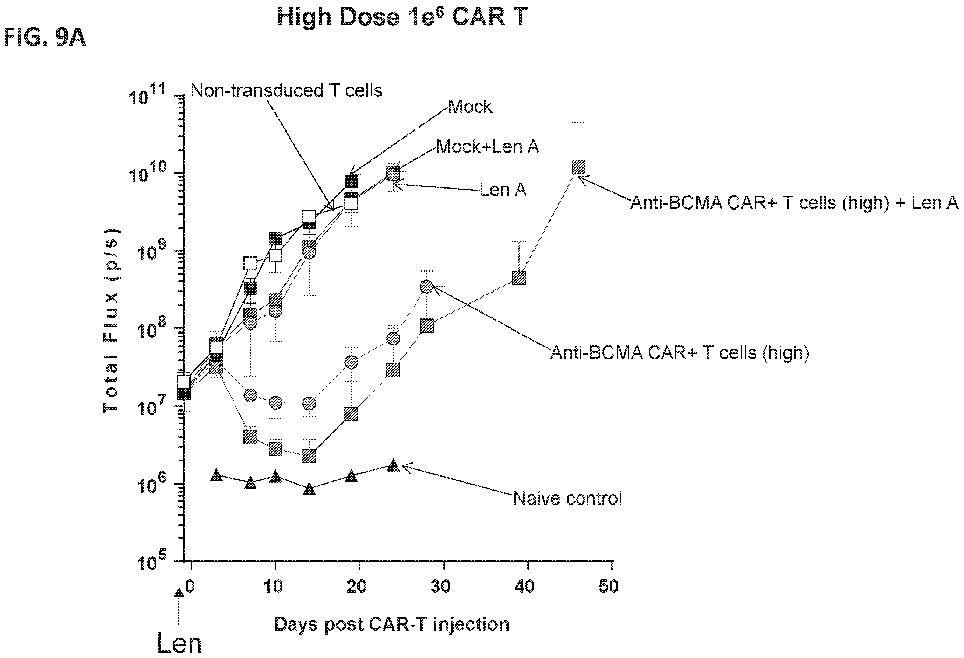

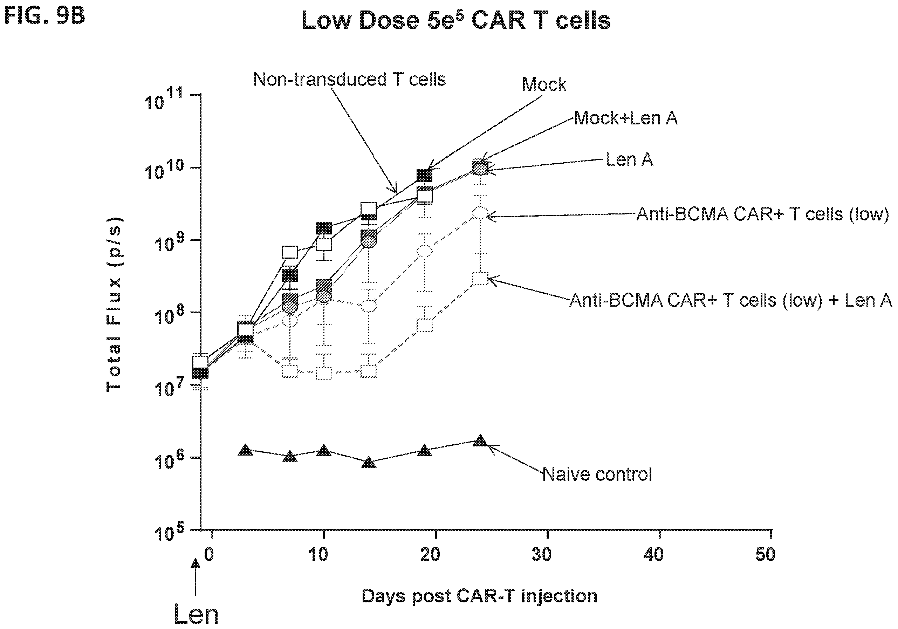

**FIGs. 9A** **and** **9B** depict tumor burden results of mice treated under Regimen A (LenA), in which mice were administered lenalidomide one day prior to receiving CAR+ T cells.

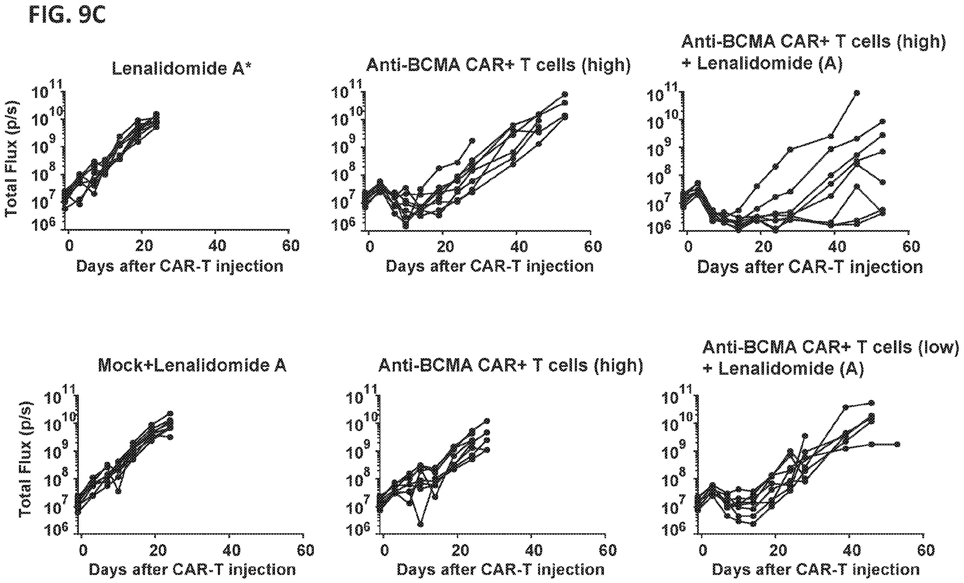

**FIG. 9C** shows the tumor burden of individual mice through up to day 53.

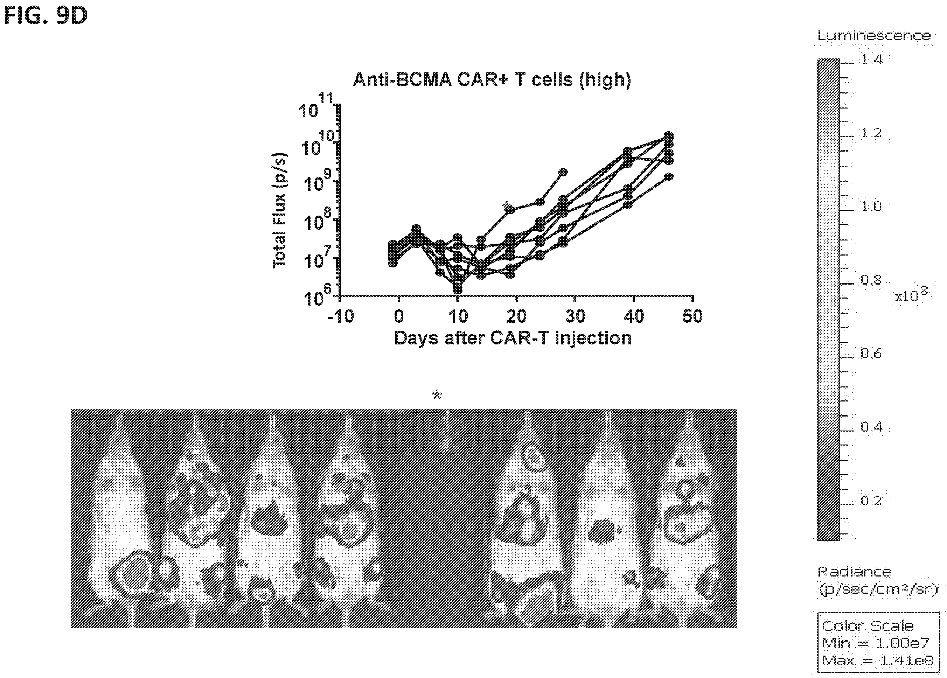

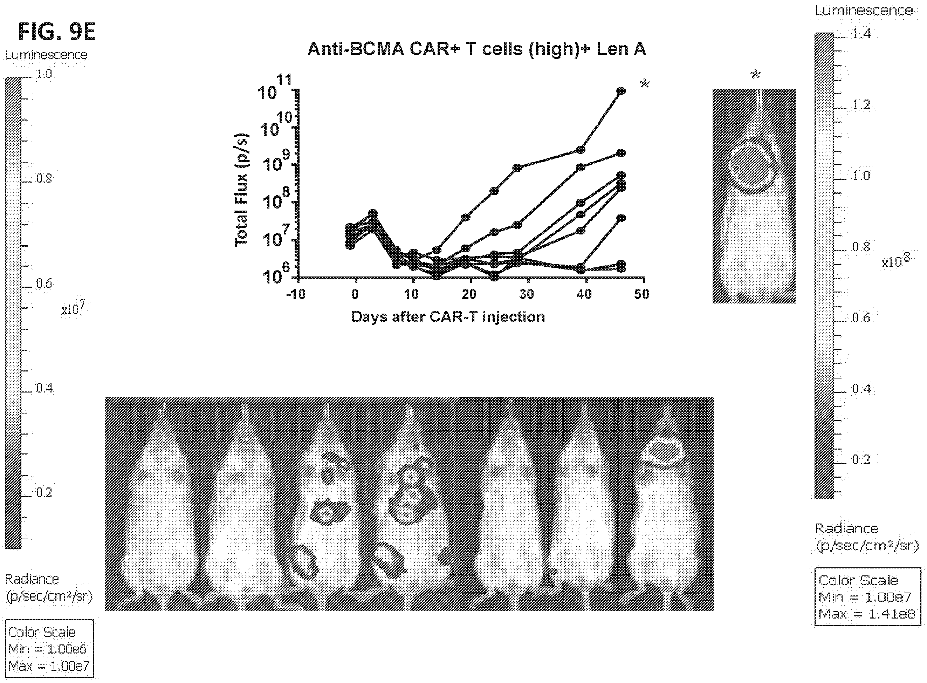

**FIG. 9D** shows tumor imaging results at day 46 post CAR+ cell administration for individual mice having received the higher CAR+ dose (1 × 10⁶), with lenalidomide at day -1 (Len A). **FIG. 9E** shows tumor imaging results at day 46 post CAR+ cell administration for individual mice having received the higher CAR+ dose (1 × 10⁶) without lenalidomide at day -1 (Len A).

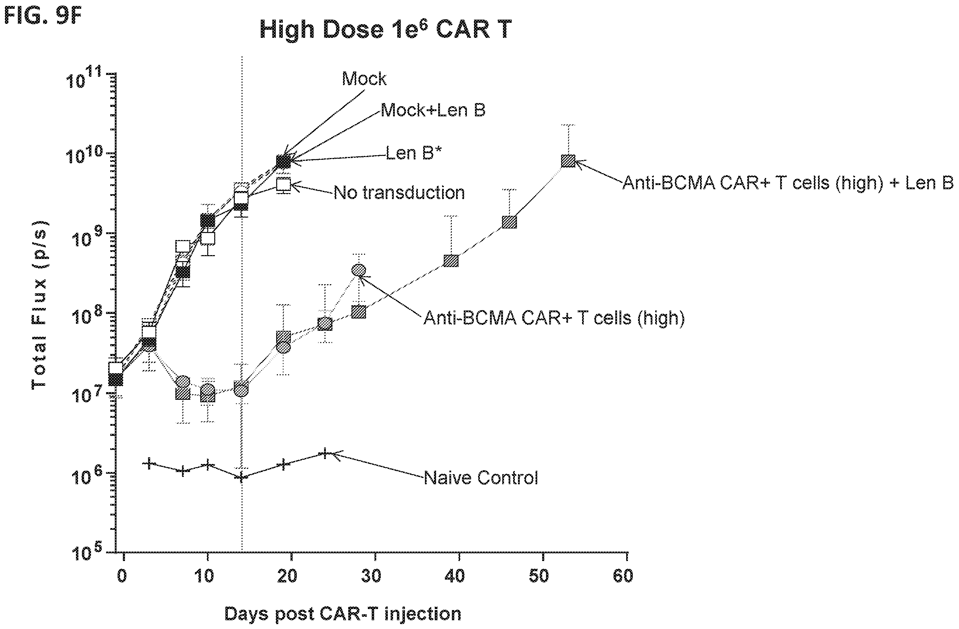

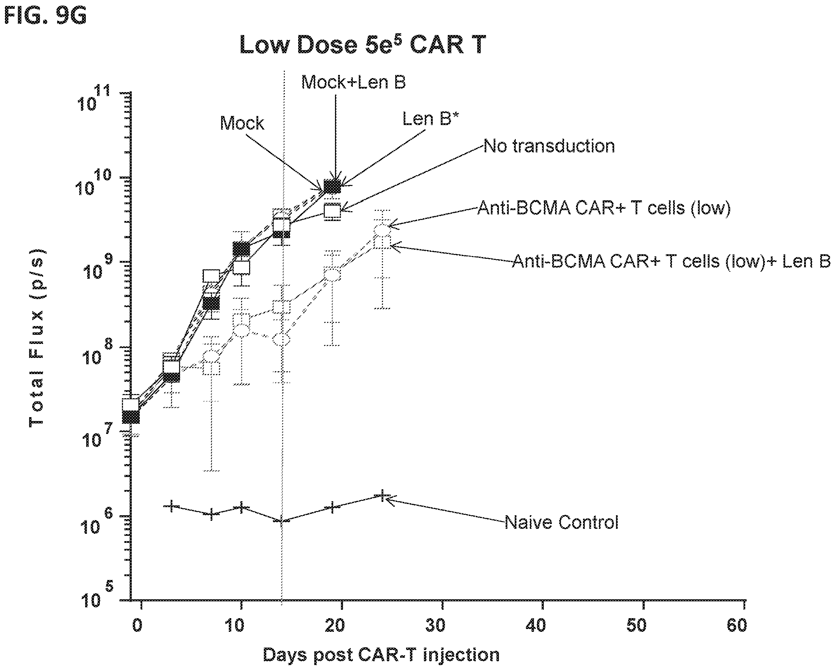

**FIGs. 9F** **and** **9G** depict tumor burden results of mice treated under Regimen B (LenB), in which administration of lenalidomide was initiated at day 14 post CAR+T administration.

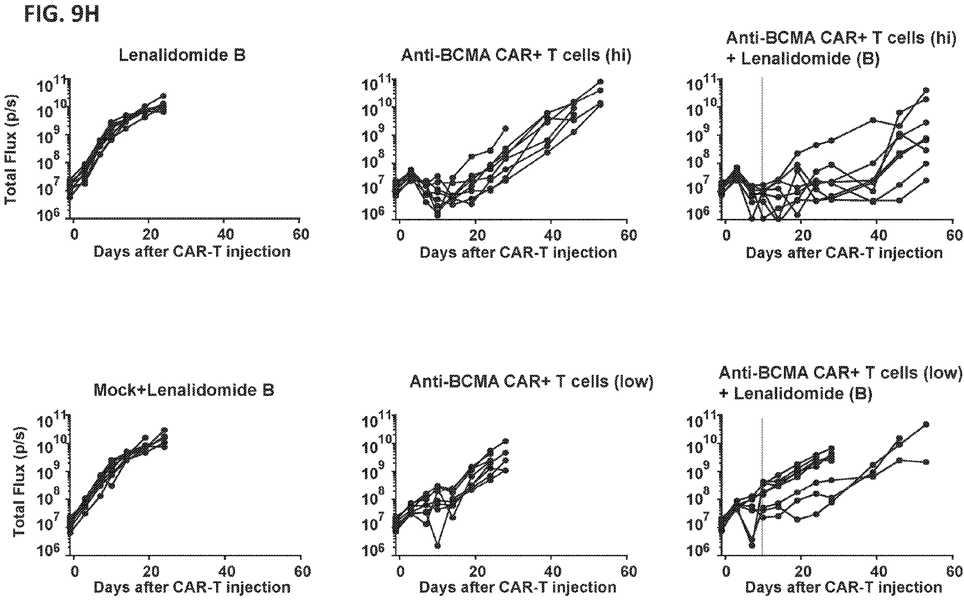

**FIG. 9H** shows the tumor burden of individual mice through up to day 53.

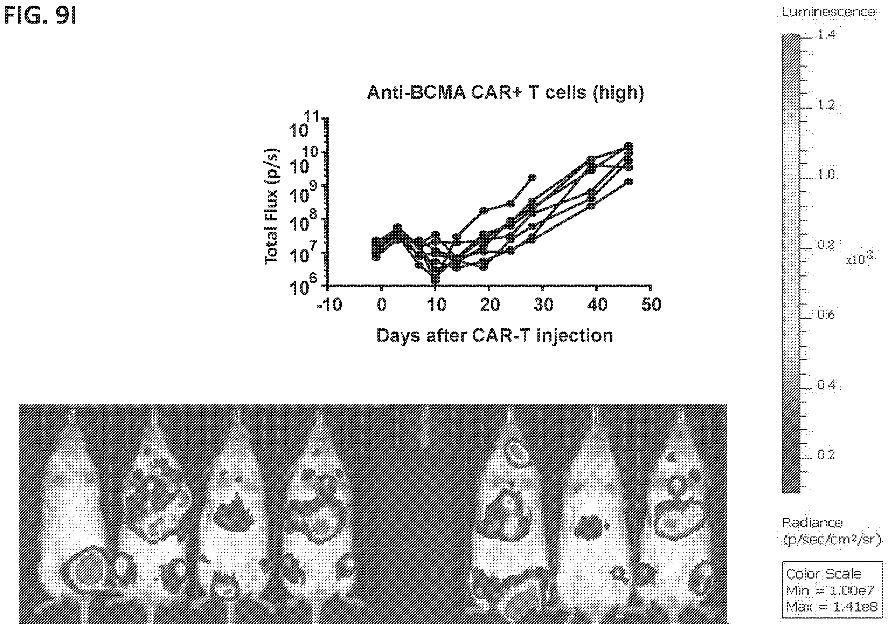

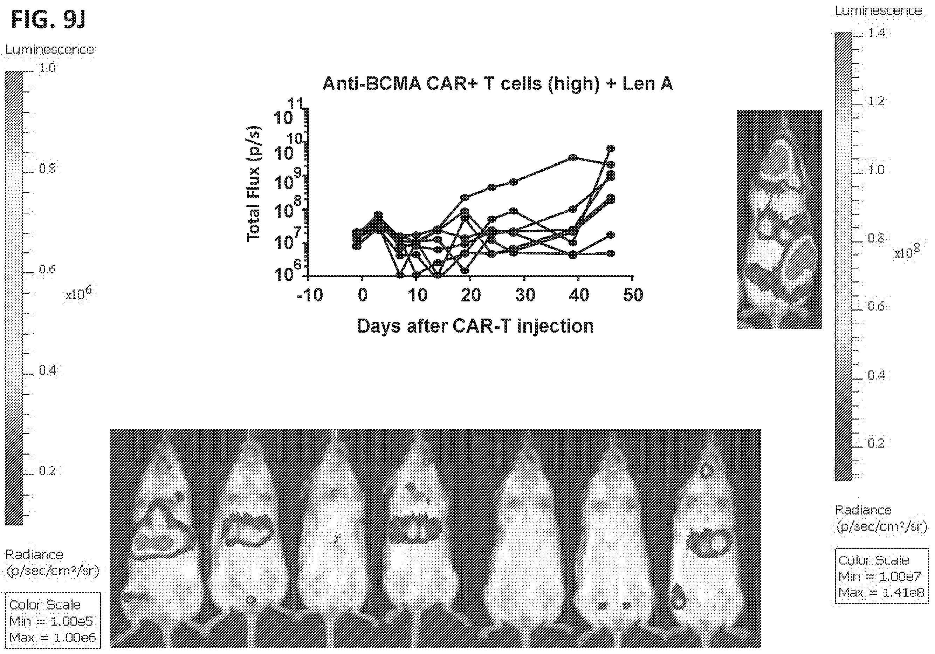

**FIG. 9I** shows tumor imaging results (day 46 post-CAR+ cell administration) for individual mice having received the higher CAR+ dose (1 × 10⁶), with lenalidomide at day -1 (Len A). **FIG. 9J** shows tumor imaging results (day 46 post-CAR+ cell administration) for individual mice having received the higher CAR+ dose (1 × 10⁶), without lenalidomide at day - 1 (Len A).

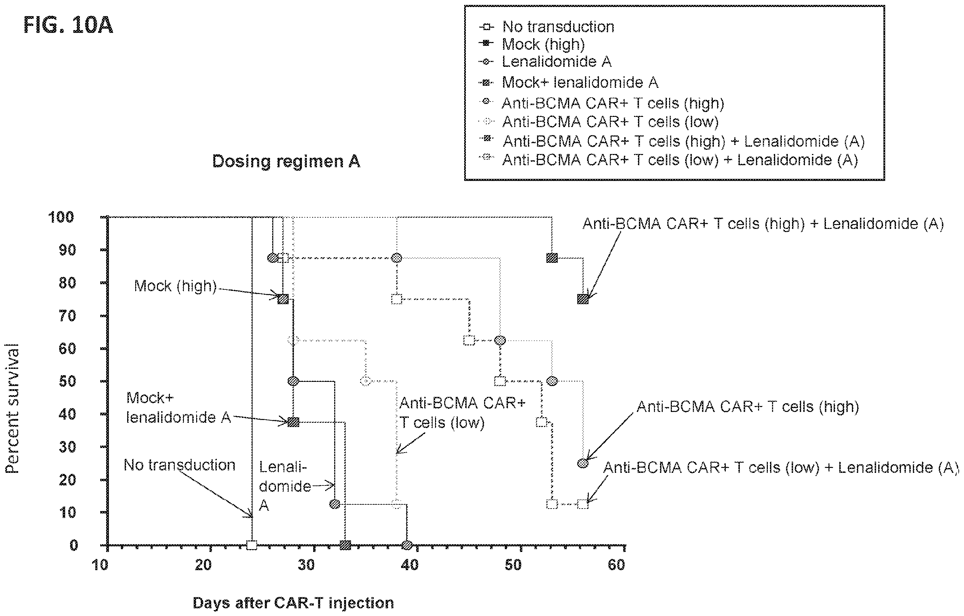

**FIGs. 10A-10D** show the survival of mice in the presence or absence of lenalidomide. Lenalidomide was administered via Regimen A (Len A; administration of lenalidomide initiated at day -1) or Regimen B (Len B; administration of lenalidomide initiated at day 14) in combination with low (5 × 10⁵ or 5e⁵) or high (1 × 10⁶ or 1e⁶) doses of CAR+ T cells. For the control groups, T cells that did not express a CAR (mock) were administered in the presence and absence of lenalidomide via both Regimen A and Regimen B, and lenalidomide without T cells was also administered via both Regimen A and Regimen B.

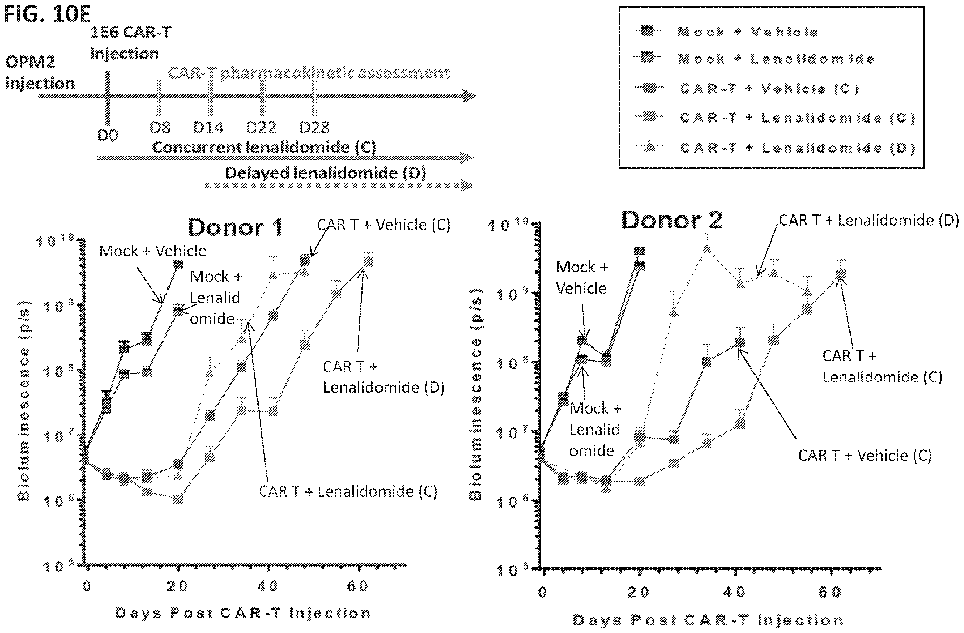

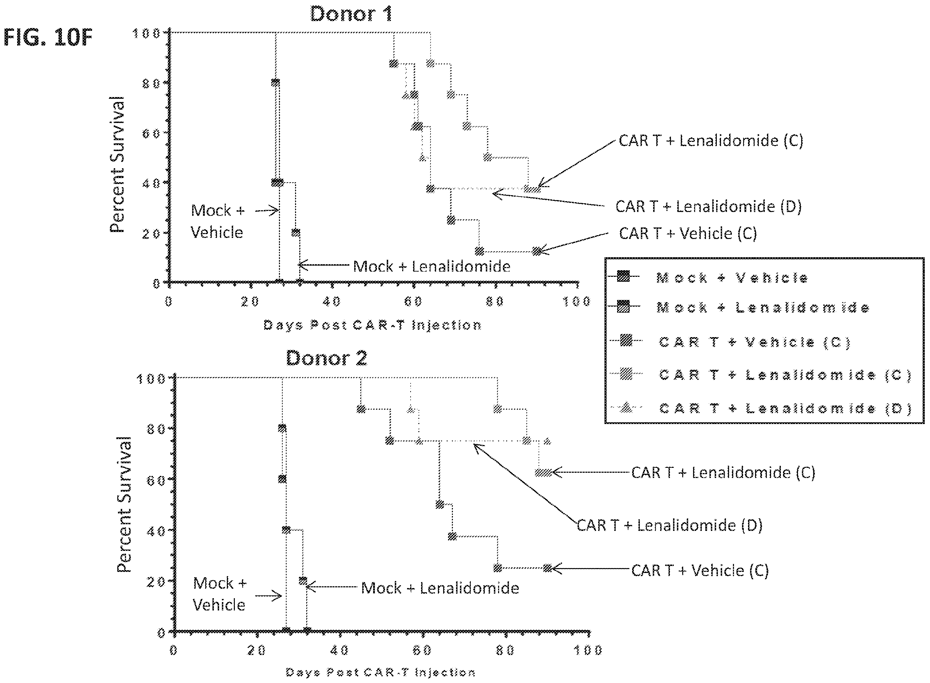

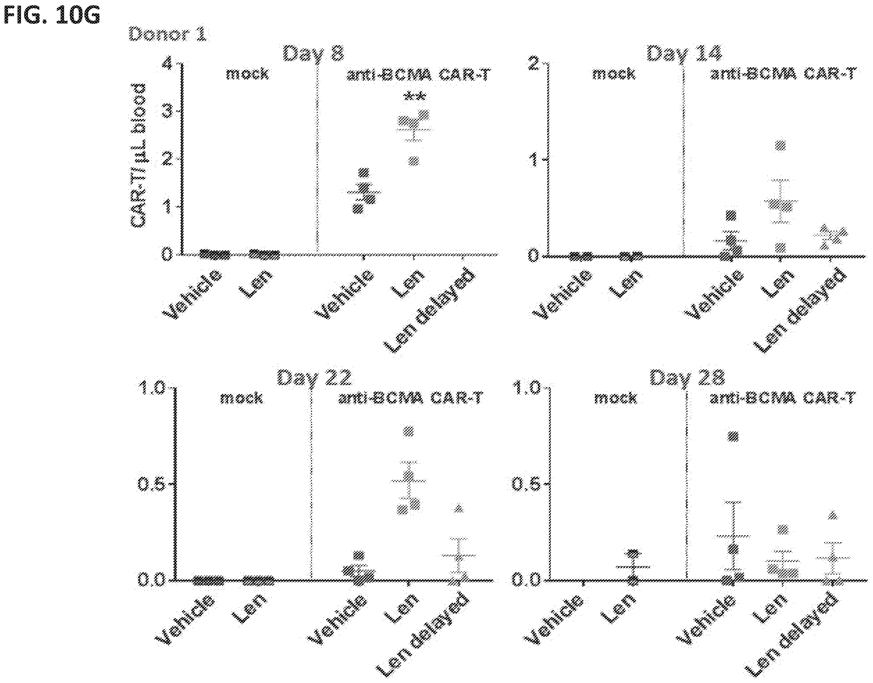

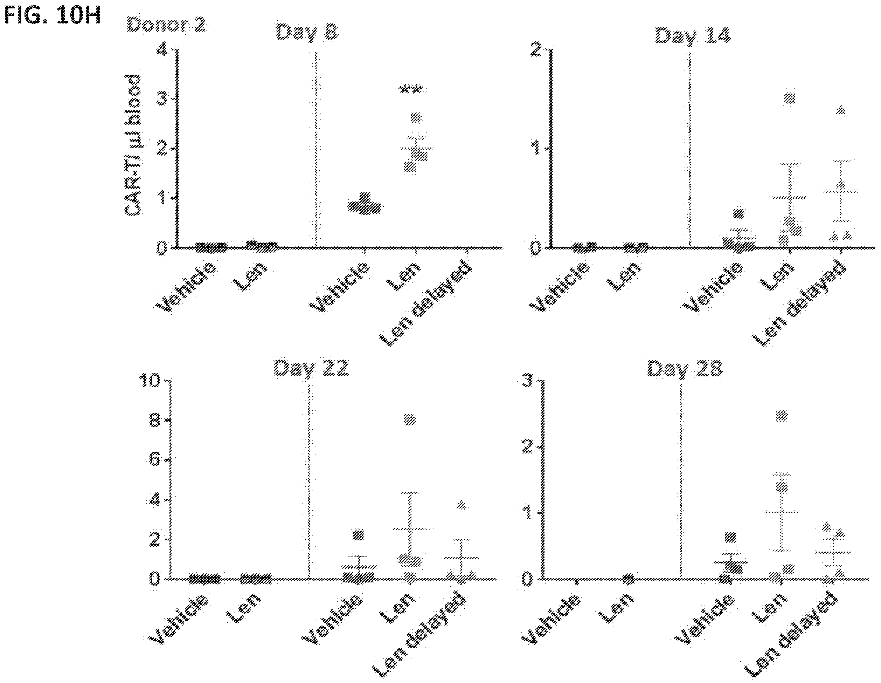

**FIG. 10E** shows the results of tumor burden assessment for mice having received the higher CAR+ dose (1 × 10⁶) and given a daily intraperitoneal administration of 10 mg/kg lenalidomide or vehicle control initiated at either day -1 (one day prior to CAR-T administration) (concurrent lenalidomide (lenalidomide (C) or vehicle (vehicle (C)) or at day 14 post-CAR-T (or mock) cell administration (delayed lenalidomide (D)). Results are shown through day 60, as analyzed by the bioluminescence measured by flow cytometry. **FIG. 10F** shows the percent survival of mice in the presence or absence of lenalidomide. **FIGs. 10G** and **10H** show the flow cytometric analysis of mock control cells and CAR-T cells in the blood of the mice at days 8, 14, 22, and 28 following injection of the CAR-T cells from two donors.

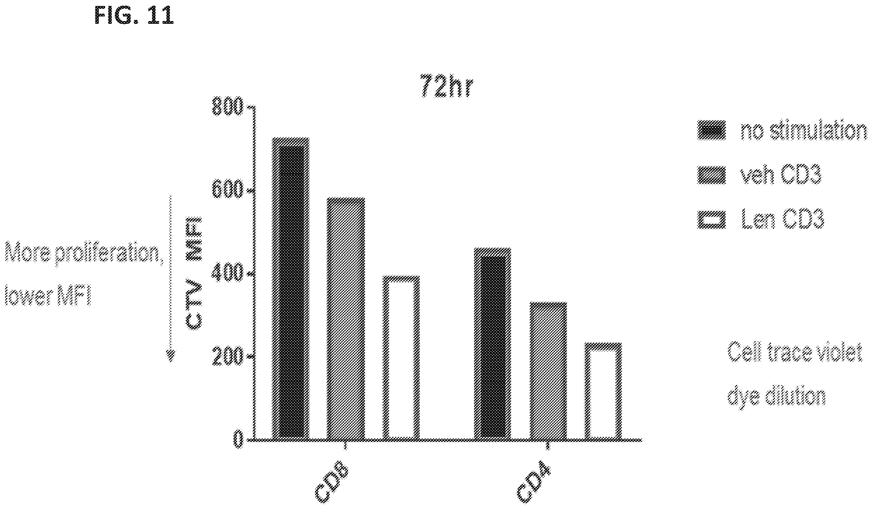

**FIG. 11** shows the number of CD4+ and CD8+ T cells in cultures of anti-CD19 CAR T cells stimulated with a suboptimal concentration of anti-CD3 in the presence and absence of lenalidomide.

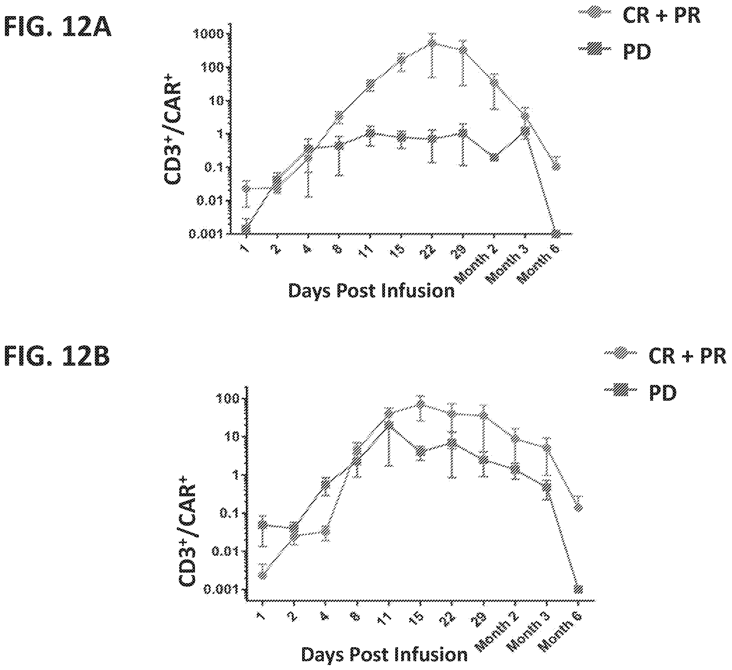

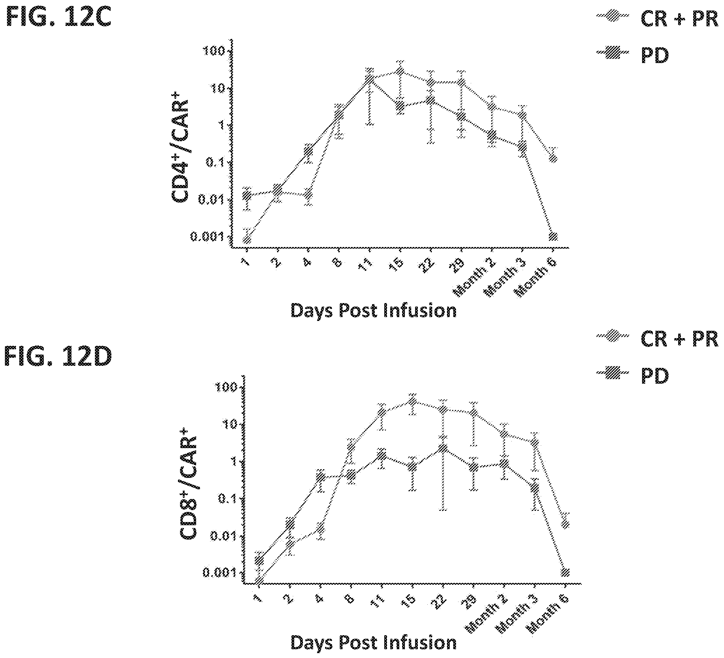

**FIG. 12A** shows the number of CD3⁺/CAR⁺ T cells in peripheral blood measured at certain time points post-infusion for subjects grouped by best overall response.
**FIG. 12B** shows CD3⁺/CAR⁺ T cells in peripheral blood measured at certain time points post-infusion for subjects who achieved a response, grouped by continued response at 3 months.
FIGs. 12C-2D show CD4⁺/CAR⁺ T and CD8⁺/CAR⁺ T cell levels in peripheral blood measured at certain time points post-infusion for subjects who achieved a response, grouped by continued response at 3 months.

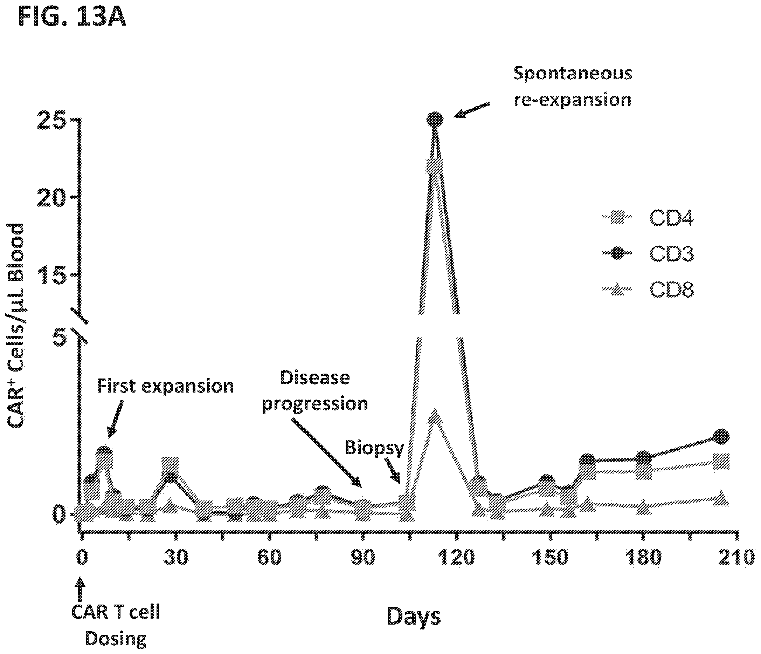

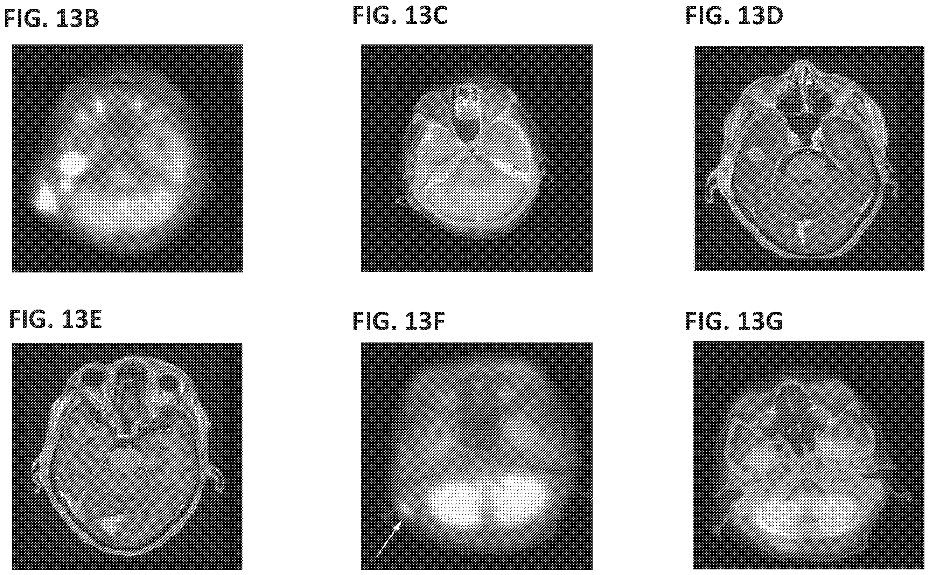

**FIG. 13A** shows the number of CD3+/CAR+, CD4+/CAR+, CD8+/CAR+ T cells in peripheral blood of a subject with chemorefractory transformed DLBCL measured at certain time points. **FIG. 13B** depicts a pretreatment axial PET-CT image showing an intracranial abnormality in the right middle cranial foss and extensive abnormality in subcutaneous tissues in the right posterior auricular region. **FIG. 13C** is a post-treatment PET-CT image depicting resolution of the abnormality in FIG. 13B after treatment with anti-CD19 CAR+ T cells. **FIG. 13D** is a pretreatment brain MRI (high-resolution Ti-weighted image with the use of contrast material; axial view) showing a homogeneously enhancing mass in the right middle cranial fossa. **FIG. 13E** is a post-treatment MRI image showing near-complete resolution of the enhancing mass. **FIG. 13F** is an axial PET-CT image at relapse showing right posteriour auricular tumor recurrence associated with intense uptake of ¹⁸F-flurodeoxyglycose (arrow). **FIG. 13G** is a PET-CT imaging showing resolution of the posterior auricular tumor after incisional biopsy and re-expansion of CAR+ T cells.

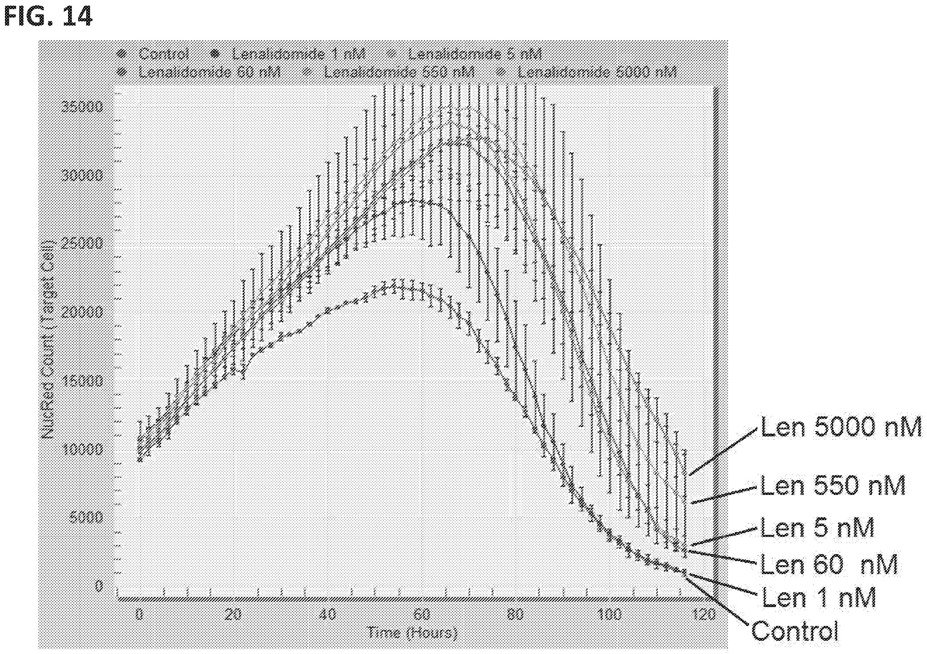

**FIG. 14** shows the level of viable target cells over a period of approximately 120 hours when anti-CD19 CAR+ T cells are incubated with K562-CD19 effector cells at an effector to target cell (E:T) ratio of 5:1 in the presence or absence of 1 nM, 5 nM, 60 nM, 550 nM or 5000 nM lenalidomide or in the absence of lenalidomide (control).

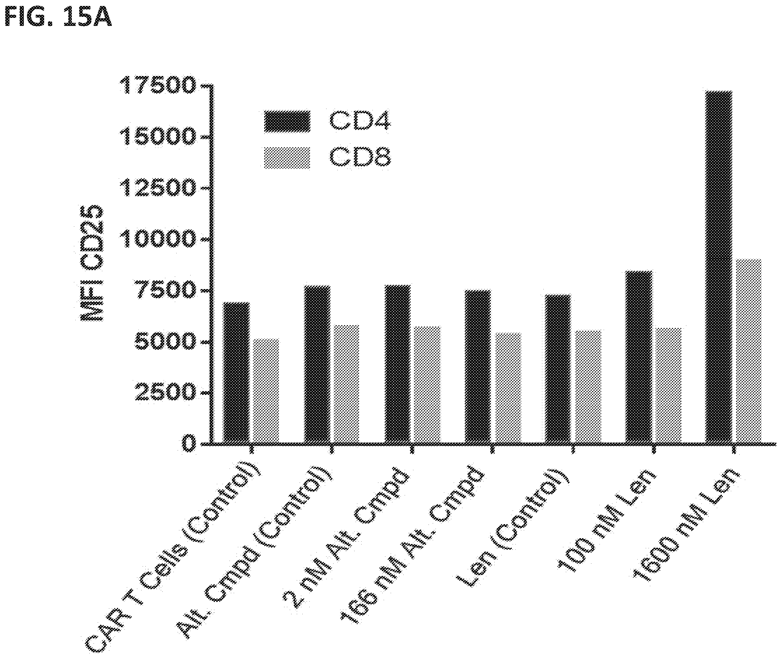

**FIG. 15A** shows the levels of CD25+ expression in both CD4+ and CD8+T cells when anti-CD19 CAR+ T cells are incubated with K562-CD19 effector cells in the presence of lenalidomide or an alternative compound targeting a kinase.

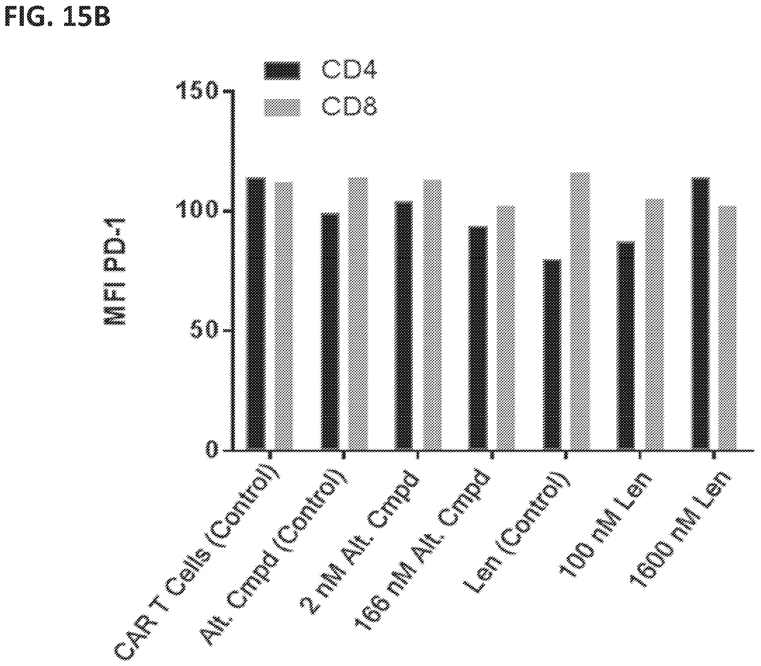

**FIG. 15B** shows the levels of CD25+ expression in both CD4+ and CD8+T cells when anti-CD19 CAR+ T cells are incubated with PD-1 effector cells in the presence of lenalidomide or an alternative compound targeting a kinase.

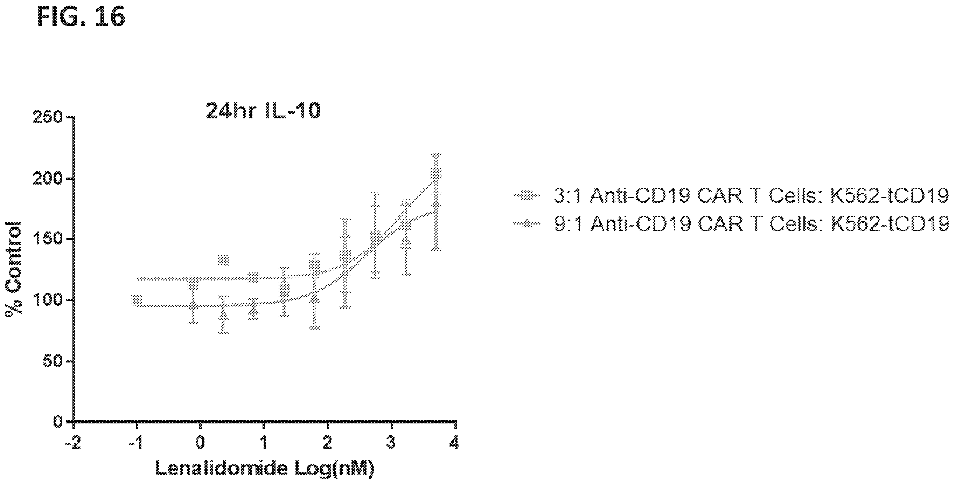

**FIG. 16** shows the amount of IL-10 in culture supernatants after incubating anti-CD19 CAR+ T cells with K562-CD19 effector cells at an effector to target cell (E:T) ratio of 3:1 or 9:1, in the presence or absence of various concentrations of lenalidomide.

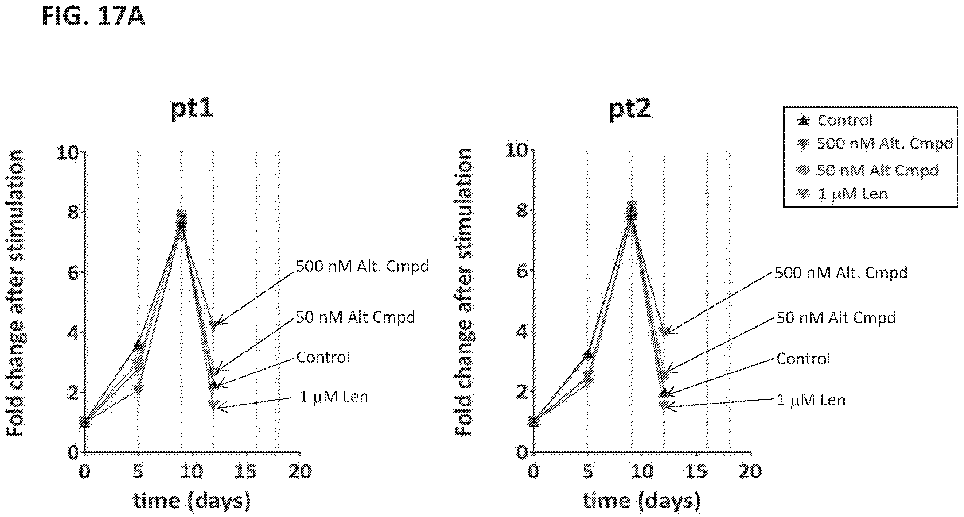

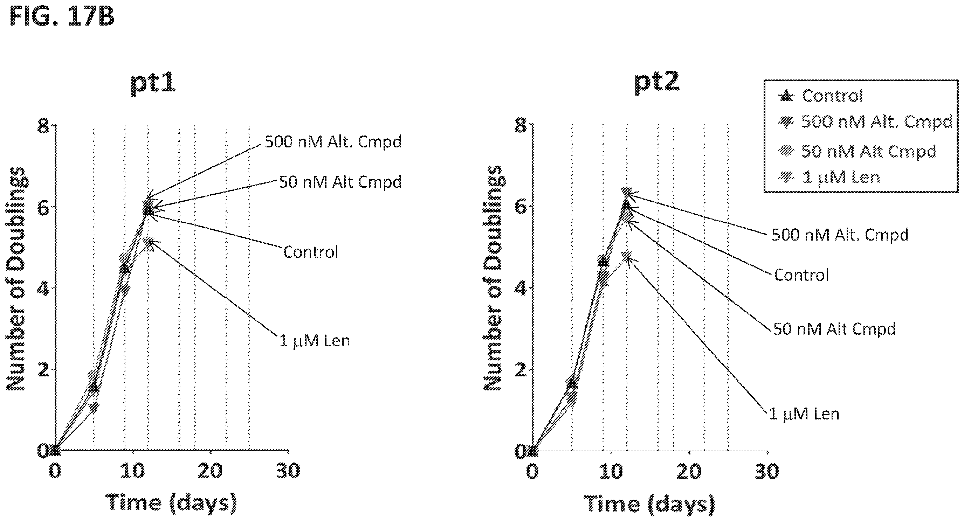

**FIG. 17A** shows the fold-change of cell number after stimulation of anti-CD19 CAR+ T cells from two donors (pt 1 and pt 2) with K562-CD19 effector cells in the presence or absence of 1 µM lenalidomide or 50 nM or 500 nM of an alternative compound targeting a kinase. **FIG. 17B** shows the number of cell doublings compared to the initial number after the 2^{nd} and 4^{th} stimulations.

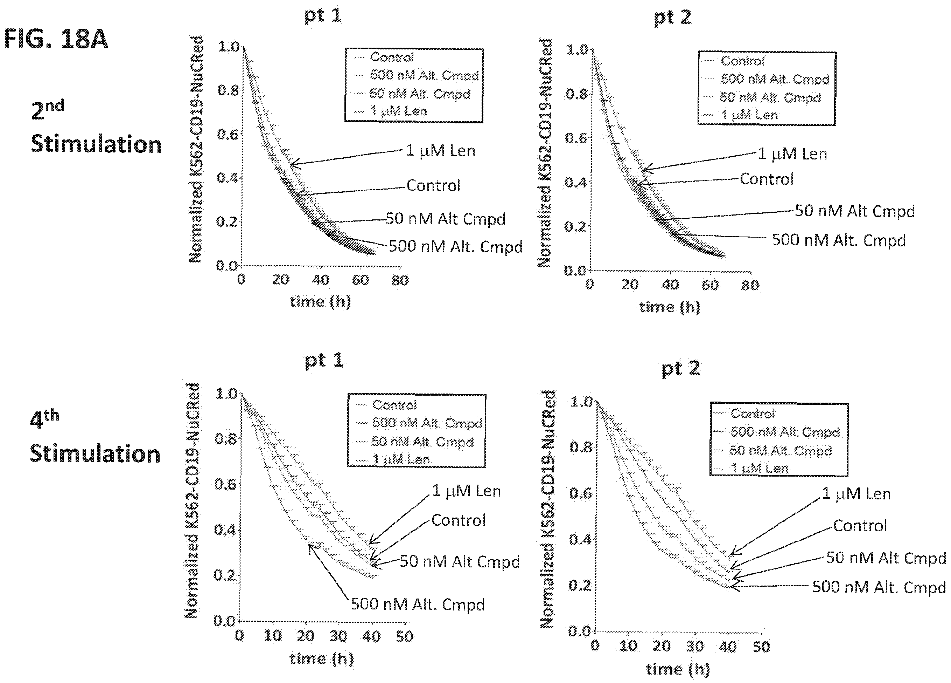

**FIG. 18A** shows the cytolytic activity of the anti-CD19 CAR+ T cells from two donor cells (pt 1 and pt 2) restimulated with K562-CD19 cells (labeled with NucLight Red (NLR)) and in the presence of 1 µM lenalidomide or 50 nM or 500 nM of the alternative compound targeting a kinase.

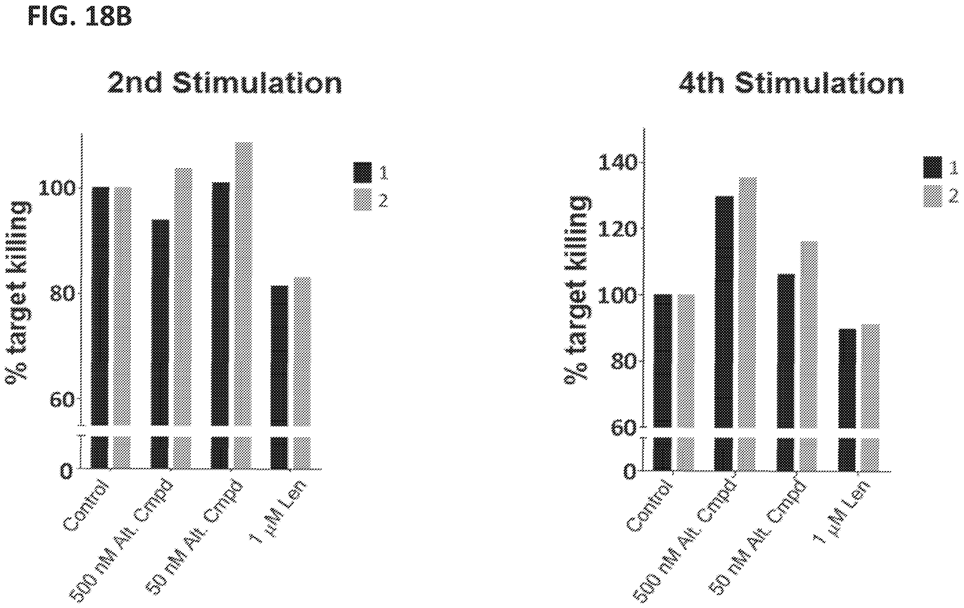

**FIG. 18B** shows the percent target cell killing of the anti-CD19 CAR+ T cells from two donor cells (1 or 2) restimulated with K562-CD19 cells compared to the vehicle-only control (set at 100%).

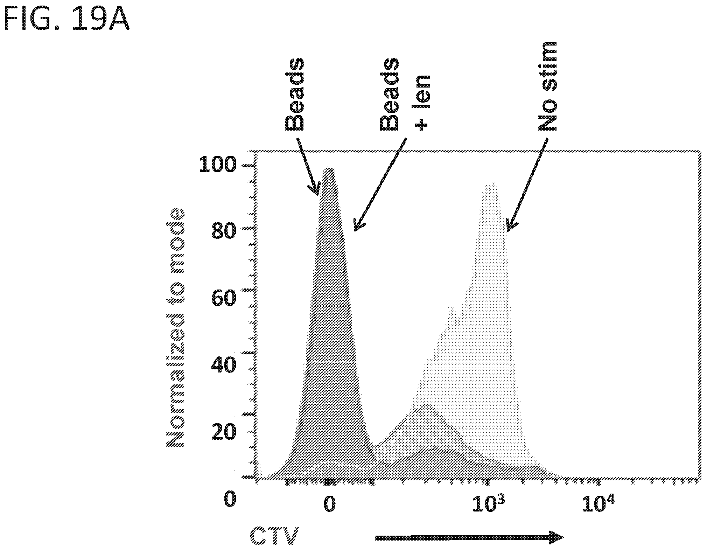

**FIG. 19A** shows a histogram plot of CTV staining of total cells in an anti-BCMA CAR+ T cell composition after incubation with beads (200 µg/mL BCMA-conjugated bead composition) at a ratio of 1:1 T cells to beads and in the presence or absence of 5µM lenalidomide.

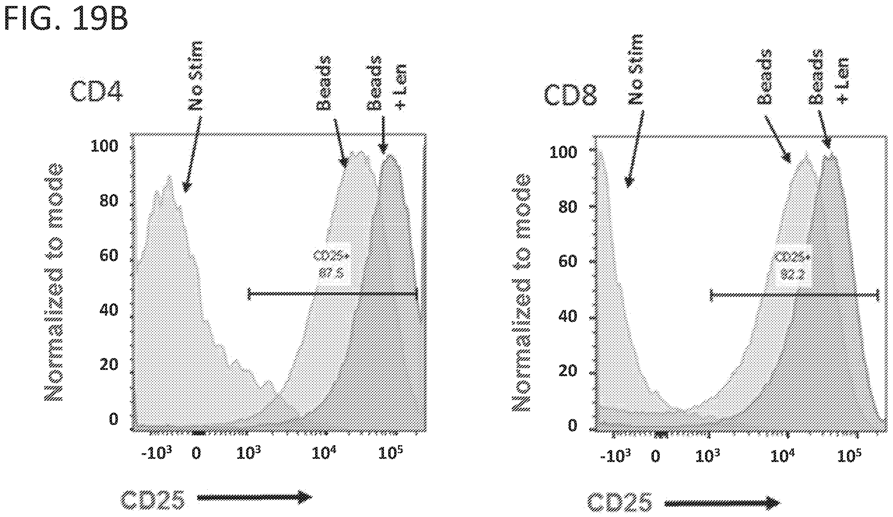

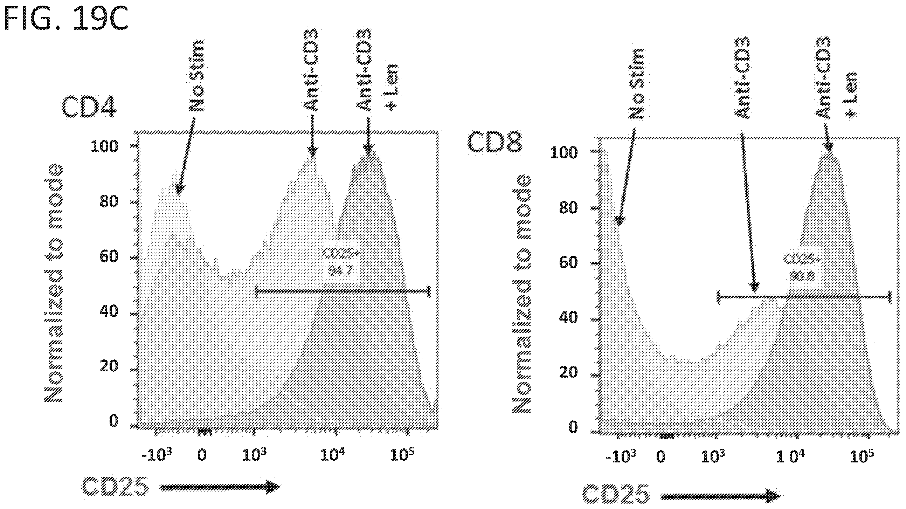

**FIG. 19B** **and** **FIG. 19C** show flow cytometry histograms for CD25 in CD4+ T cells (left panel) or CD8+ T cells (right panel) present in an anti-BCMA CAR+ T cell composition after incubation with beads (200 µg/mL BCMA-conjugated bead composition) at a ratio of 1:1 T cells to beads or immobilized anti-CD3, respectively, in the presence or absence of lenalidomide.

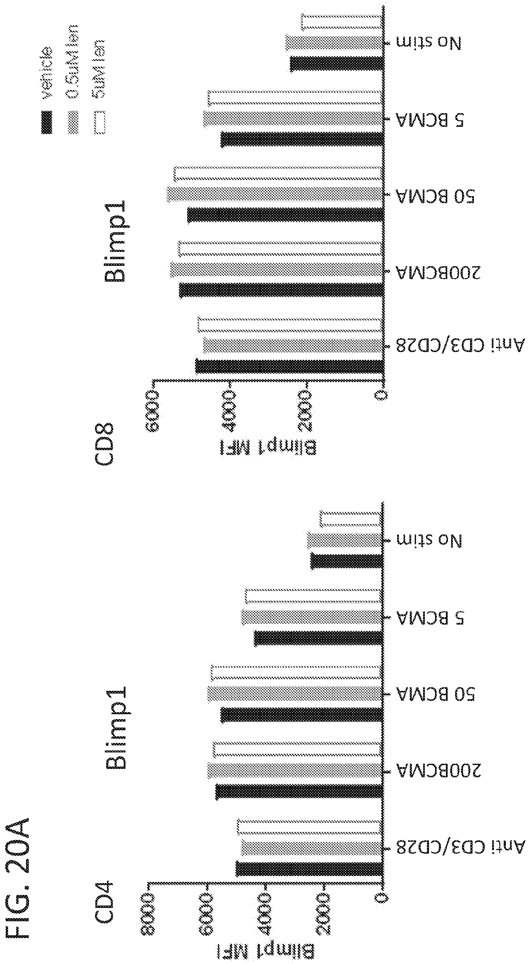

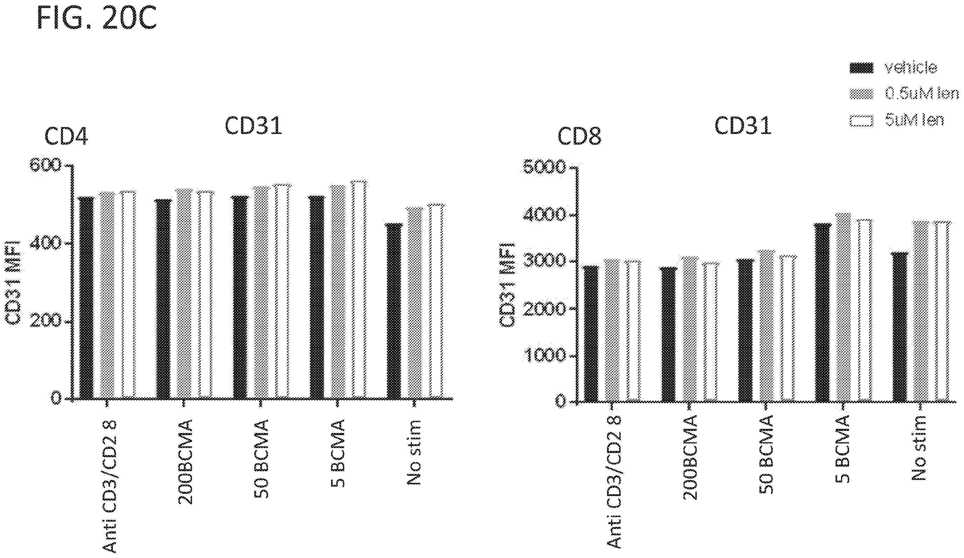

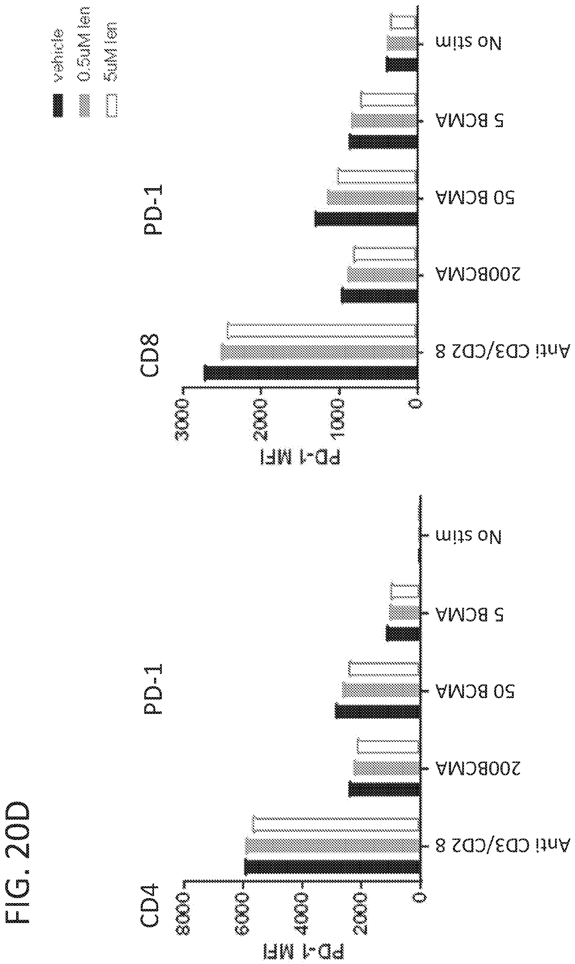

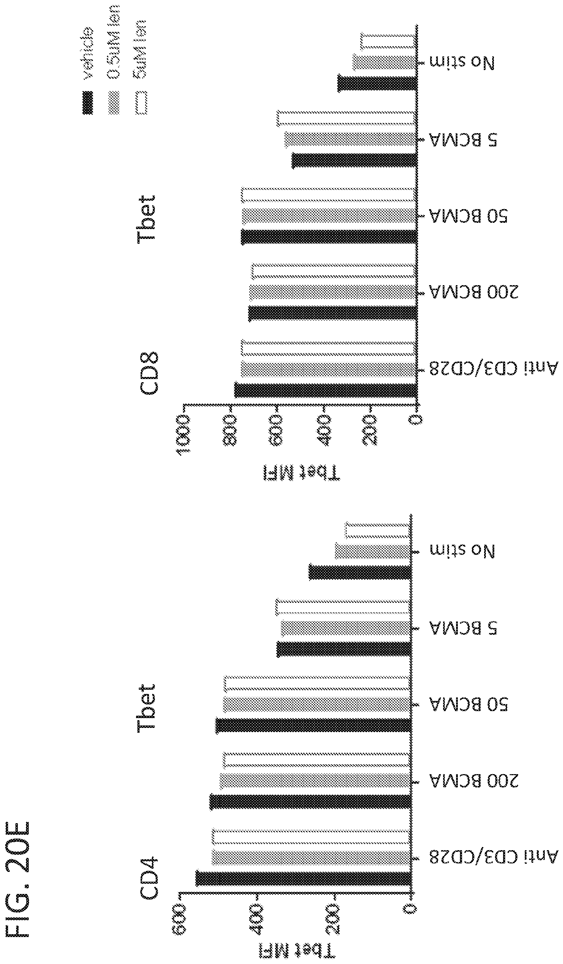

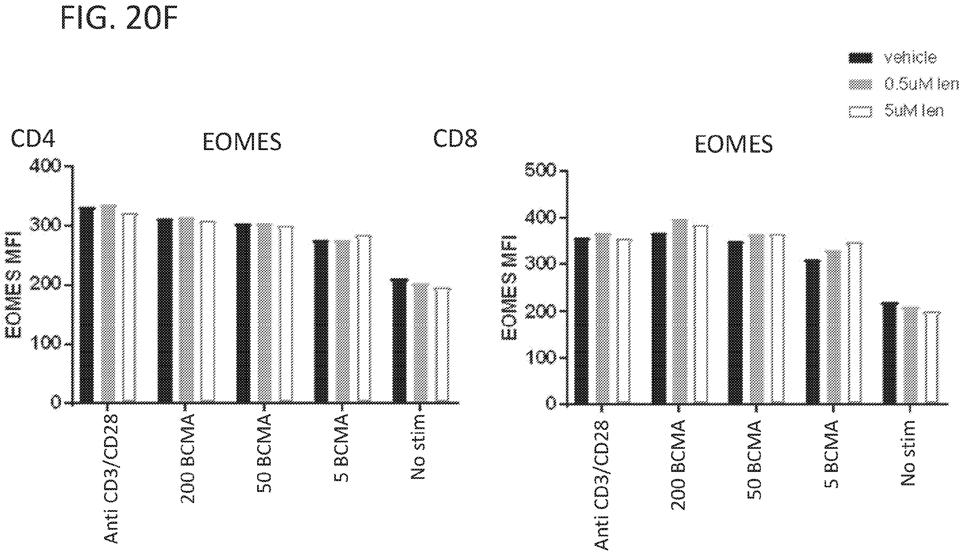

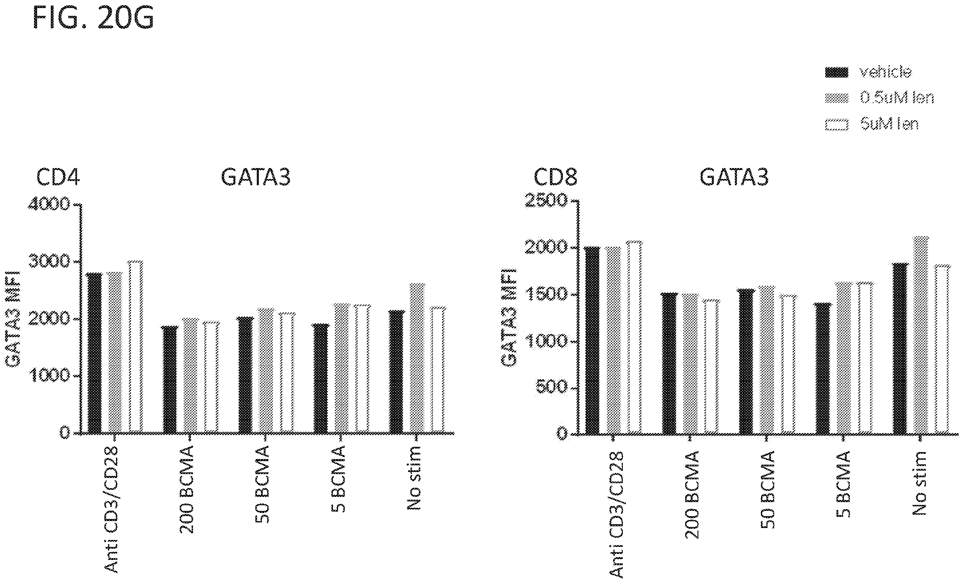

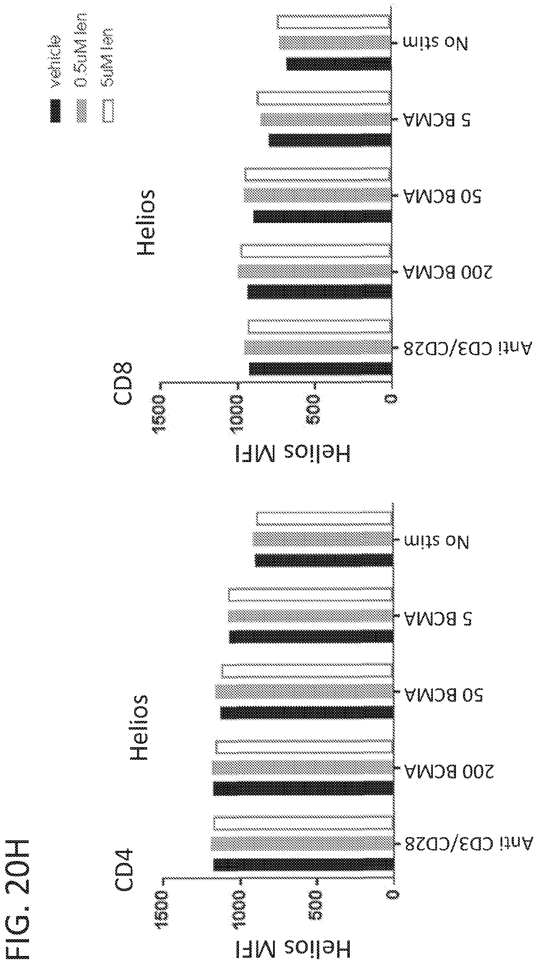

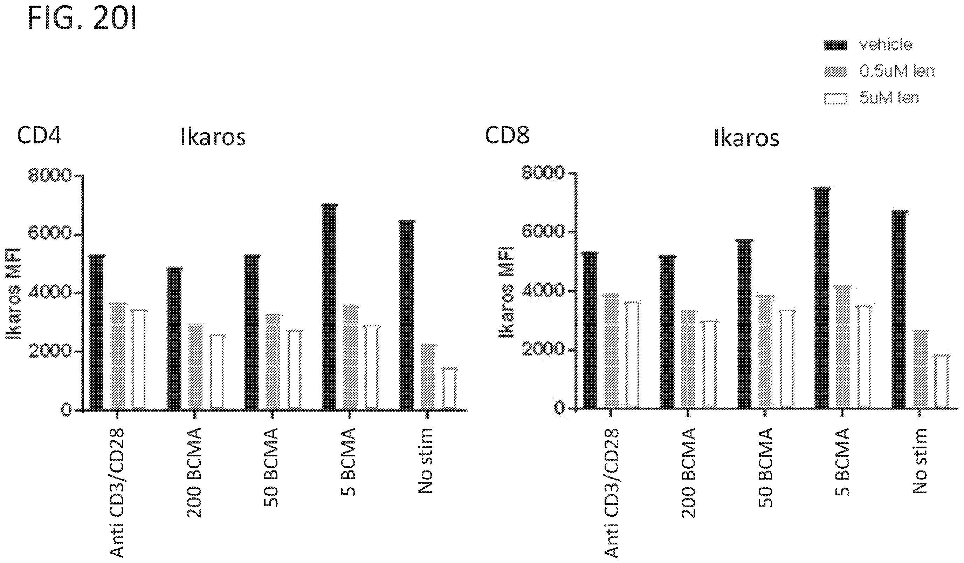

**FIGS. 20A-20I** show graphs displaying the levels of transcription factors and activation markers in or on CD4+ T cells (left panels) or CD8+ T cells (right panels) present in an anti-BCMA CAR+ T cell composition after incubation without stimulation or with different amounts of BCMA-conjugated bead or anti-CD3 and anti-CD28 conjugated beads and in the presence of 0 µM, 0.5 µM, or 50 µM lenalidomide. Levels of Blimp 1 (**FIG. 20A**), CD25 (**FIG**. **20B**), CD31 (**FIG. 20C**), PD-1 (**FIG. 20D**), Tbet (**FIG. 20E**), EOMES (**FIG. 20F**), GATA3 (**FIG. 20G**), Helios (**FIG. 20H**), and Ikaros (**FIG. 20I**) are shown. 200 BCMA, 50 BCMA, and 5 BCMA indicate BCMA-conjugated beads generated by incubating BCMA with the beads in an amount of 200, 50, and 5 µg of BCMA per approximately 4×10⁸ beads, respectively.

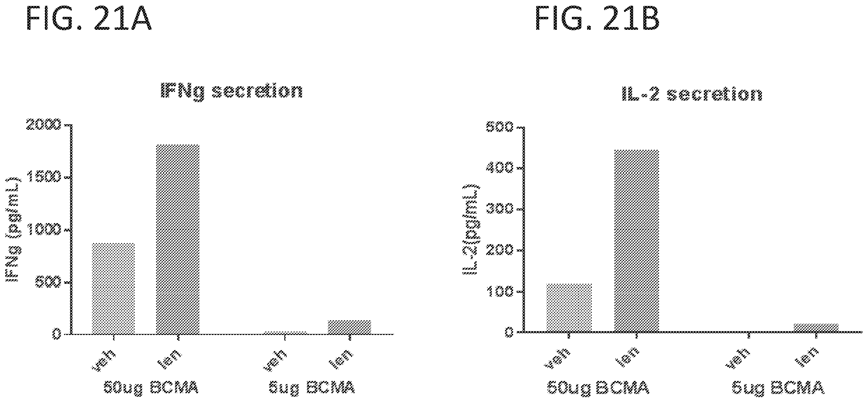

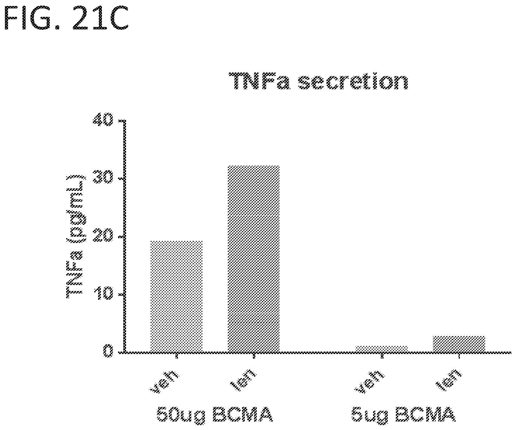

FIG. 21A-C shows graphs displaying the levels of extracellular IFN-gamma (**FIG. 21A**)**,** IL-2 (**FIG. 21B**), and TNF alpha (**FIG. 21C**) from cultures following incubation of an anti-BCMA CAR+ T cell composition with two different amounts of BCMA-conjugated beads in the presence or absence of 5µM lenalidomide. 50µg BCMA and 5µg BCMA indicate BCMA-conjugated beads generated by incubating BCMA with the beads in an amount of 50 and 5 µg of BCMA per approximately 4×10⁸ beads, respectively.

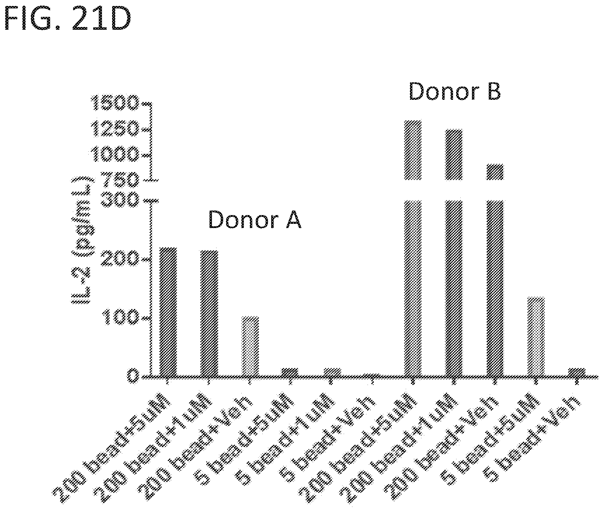

**FIG. 21D** shows a graph displaying the levels of extracellular IL-2 from cultures following incubation of an anti-BCMA CAR+ T cell composition from two different donors with different amounts of BCMA-conjugated beads in the presence of 0 µM, 1 µM, or 5 µM lenalidomide. 200 BCMA and 5 BCMA indicate BCMA-conjugated beads generated by incubating BCMA with the beads in an amount of 200 µg and 5 µg of BCMA per approximately 4×10⁸ beads, respectively.

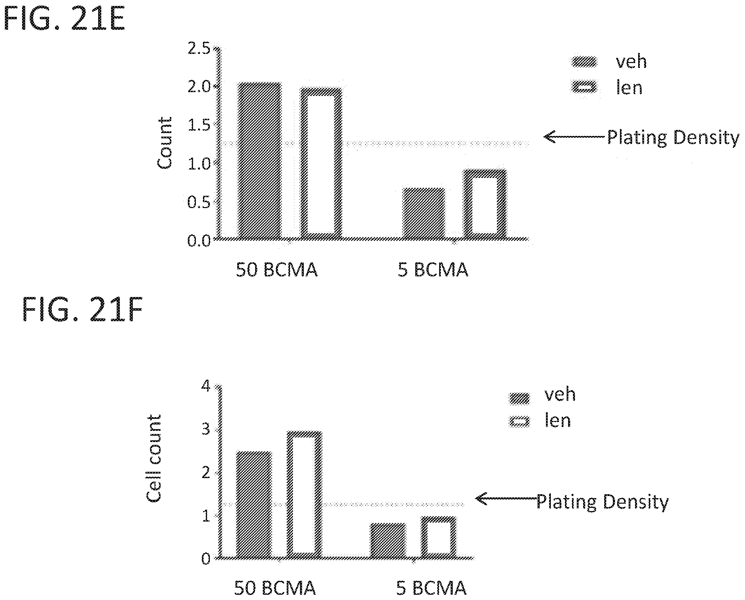

**FIG. 21E and FIG. 21F** shows total cell count following culture of an anti-BCMA CAR+ T cell composition after incubation for 4 days (**FIG. 21E**) or 7 days (**FIG. 21F**) with different amounts of BCMA-conjugated beads in the presence of 5 µM lenalidomide. 50 BCMA and 5 BCMA indicate BCMA-conjugated beads generated by incubating BCMA antigen with the beads in an amount of 50 µg and 5 µg of BCMA per approximately 4×10⁸ beads, respectively.

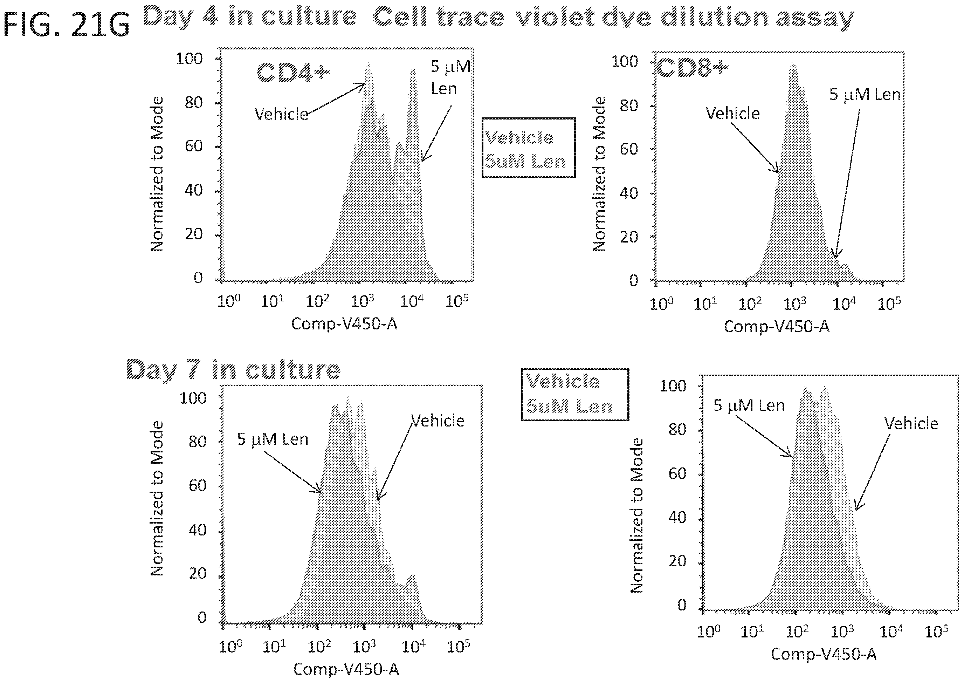

**FIG. 21G** shows histogram plots of CTV staining of CD4+ T cells or CD8+ T cells in an anti-BCMA CAR+ T cell composition after incubation for 4 or 7 days with BCMA-conjugated beads in the presence of 5µM lenalidomide or absence of lenalidomide (vehicle).

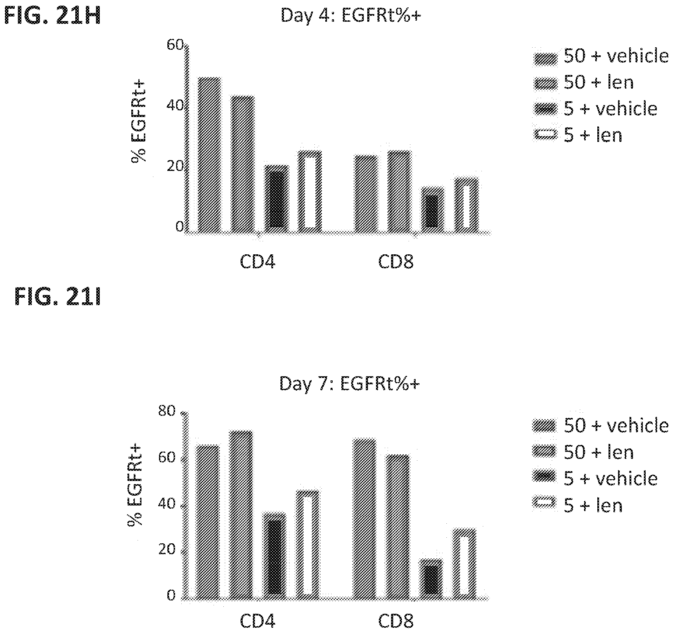

**FIG. 21H** and 21I show graphs displaying the percentage of cells positive for the surrogate marker EGFRt as determined with an anti-EGFR antibody following incubation of an anti-BCMA CAR+ T cell composition for 4 days (FIG. 21H) or 7 days (FIG. 21I) with different amounts of BCMA-conjugated beads in the presence of 5µM lenalidomide or absence of lenalidomide (vehicle). "50" and "5" indicate beads generated by incubating BCMA with the beads in an amount of 50 µg and 5 µg of BCMA per approximately 4×10⁸ beads, respectively.

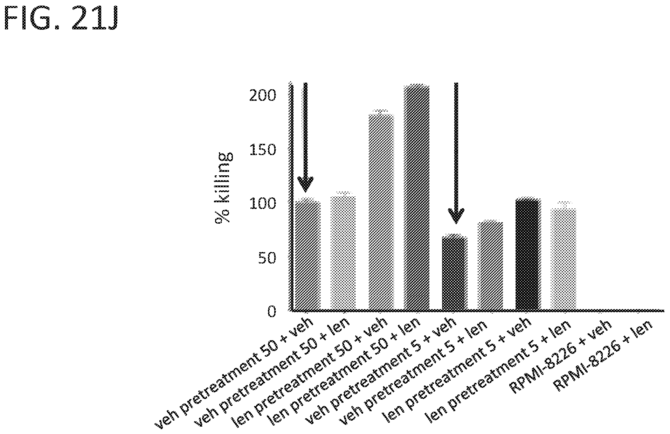

**FIG. 21J** shows the percent cell killing of RPMI-8226 target cells by anti-BCMA CAR+ T cells effector cells that had been incubated with different amounts of BCMA-conjugated beads in the presence of 5µM lenalidomide or absence of lenalidomide (vehicle). Cytolytic activity of compositions containing a ratio of effector cells to target cells of 3: 1 or 1: 1 and in the further presence or absence of lenalidomide are shown. "50" and "5" indicate BCMA conjugated beads generated by incubating BCMA with the beads in an amount of 50 and 5 µg of BCMA per approximately 4×10⁸ beads, respectively.

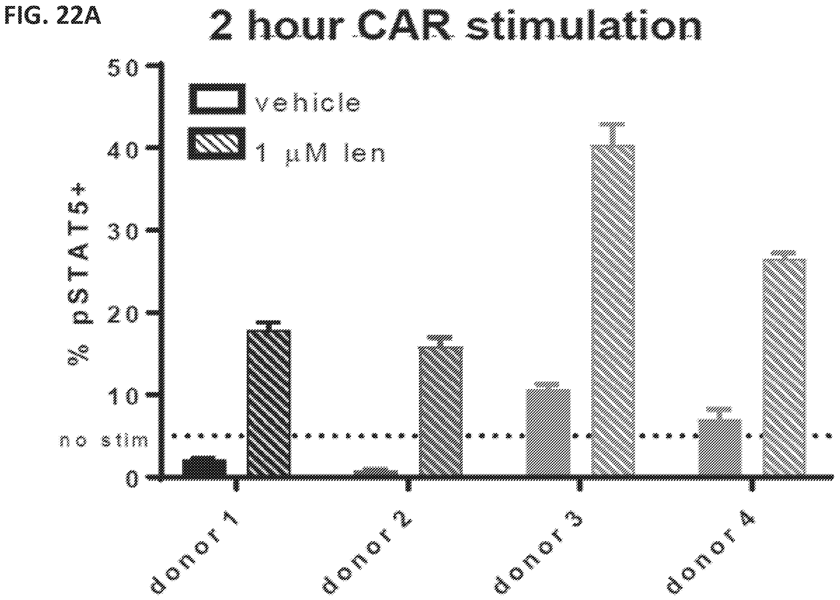

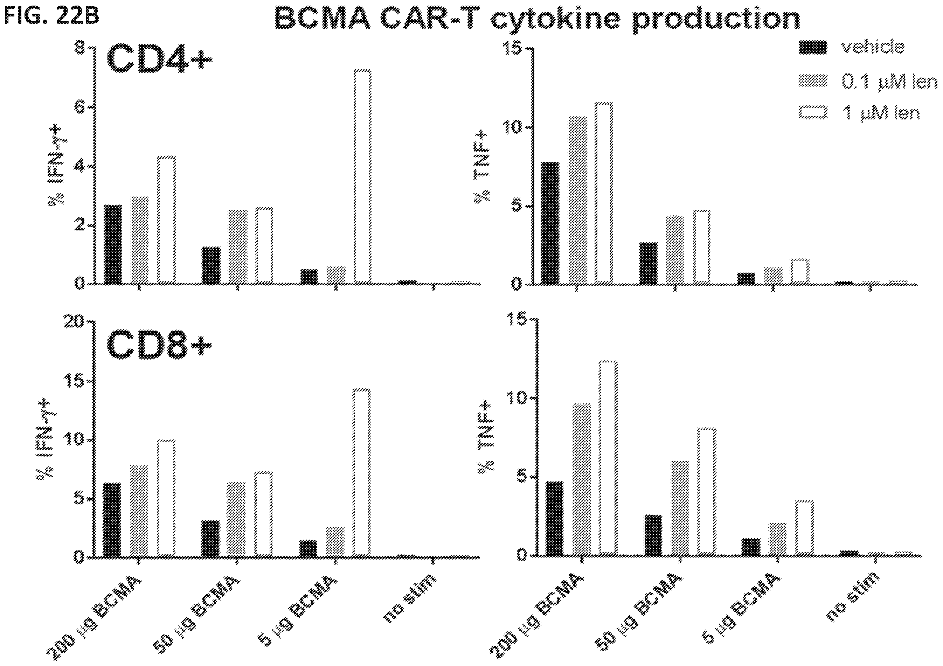

**FIG. 22A** shows the flow cytometric analysis of phosphorylated STATS after 2 hours of CAR stimulation (stim) with 50 µg BCMA beads. No stimulation control shown with dotted line. **FIG. 22B** shows the flow cytometric analysis of intracellular cytokine levels on a representative normal CAR T donor after 24 hours of BCMA bead stimulation (gated on transduced, live CD3+).

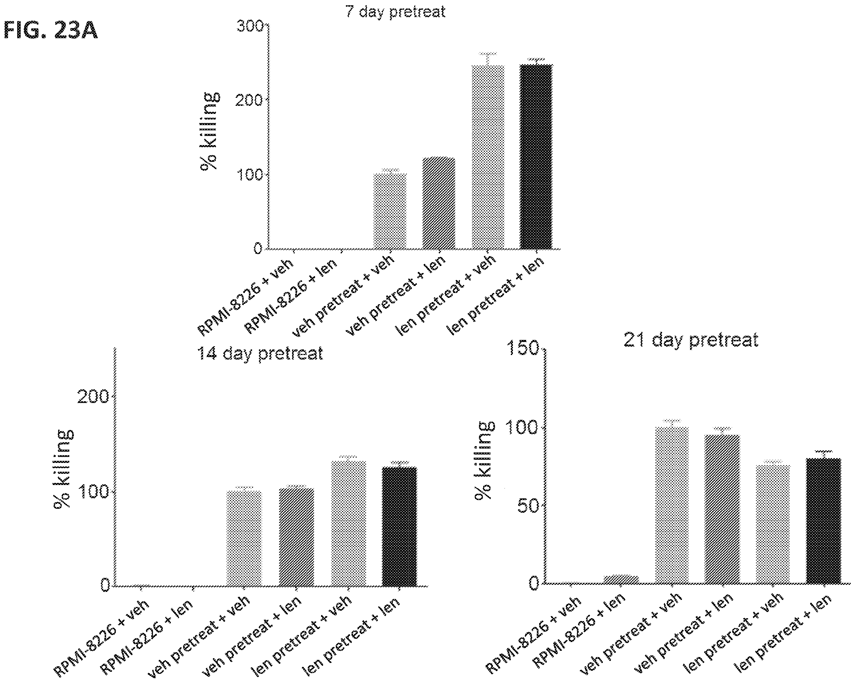

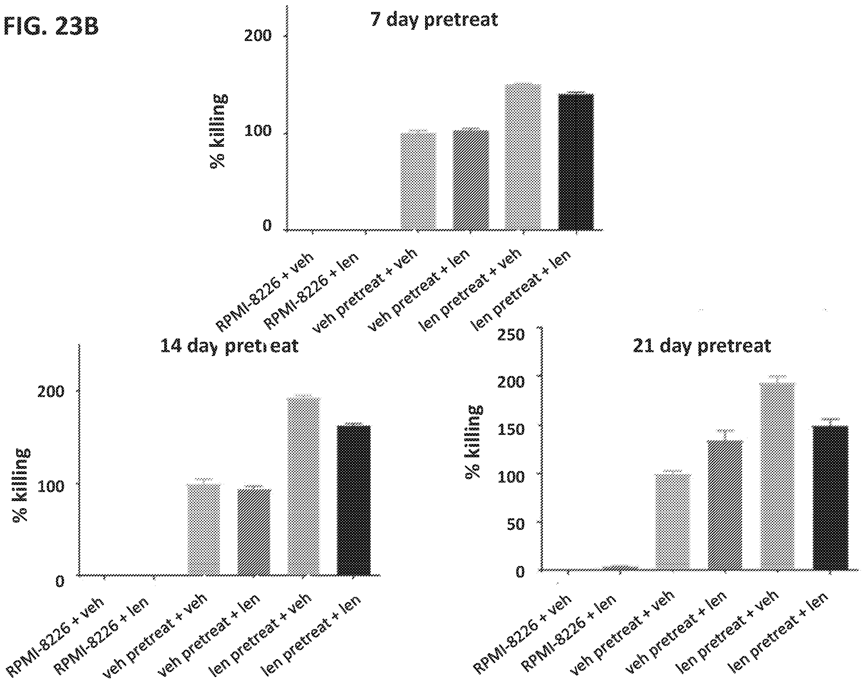

**FIG. 23A-23B** depicts results of a serial restimulation assay of anti-BCMA CAR T cell compositions that had been incubated for seven days with BCMA-conjugated beads (50 µg/mL). Results from three different donor compositions are shown. FIG. 23A and FIG. 23B show the cytolytic activity of the anti-BCMA CAR+ T cells at each of the time points for two different donors.

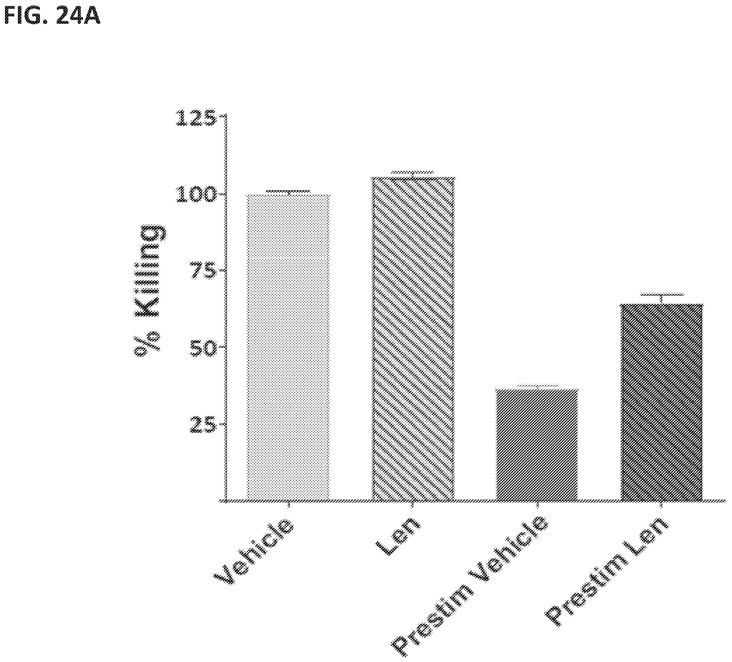

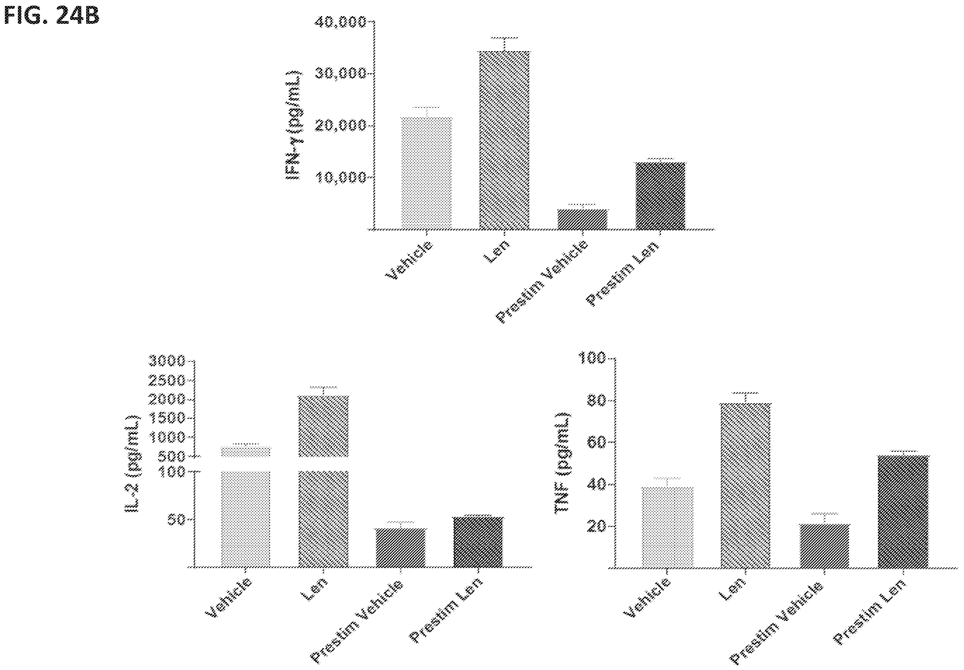

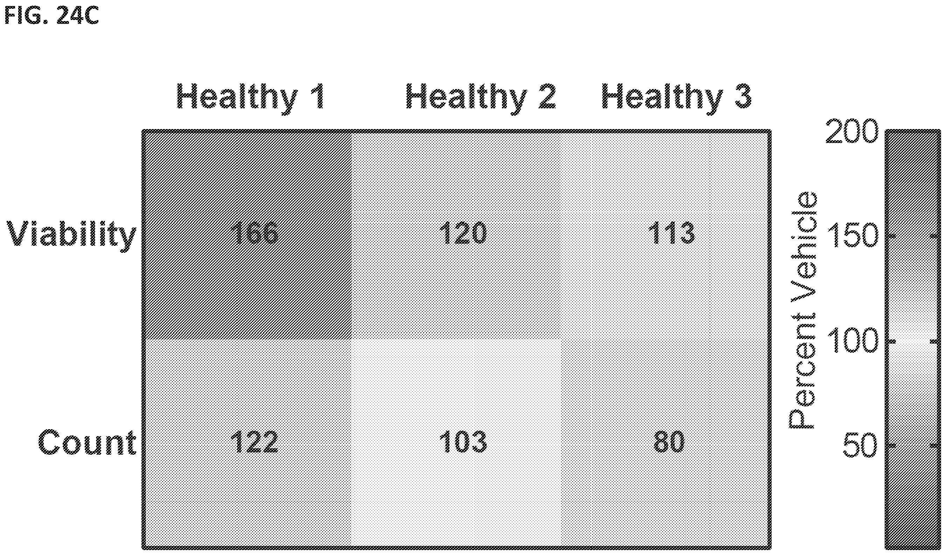

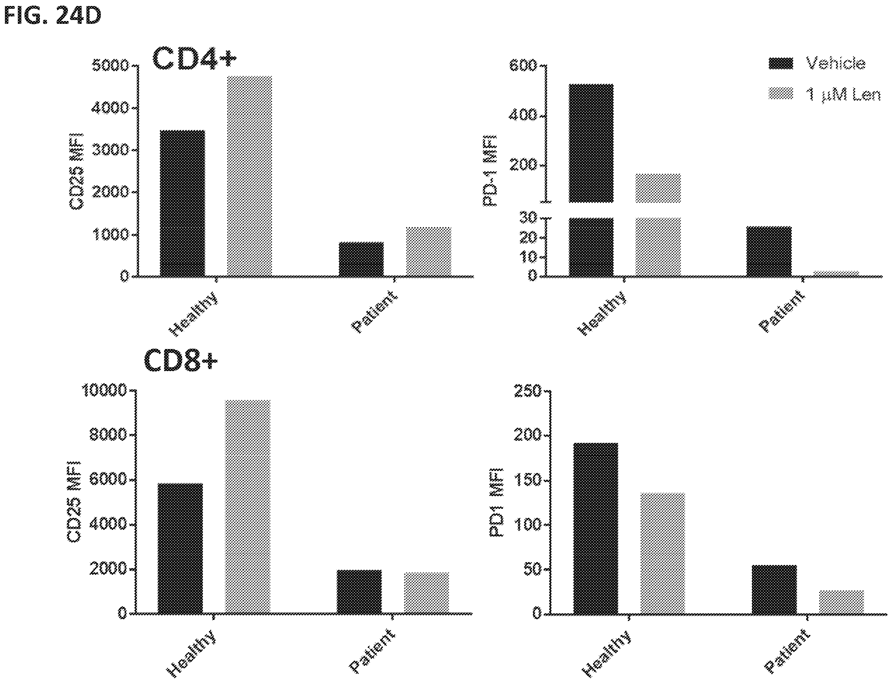

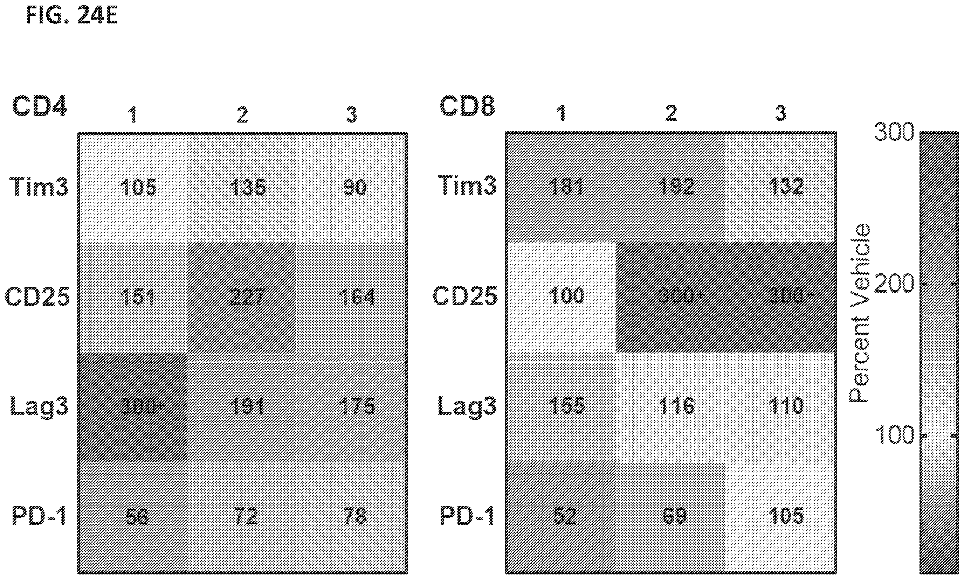

**FIG. 24A** shows results for CAR antigen-specific cytolytic activity and **FIG. 24B** shows results for cytokine production for anti-BCMA CAR-T cells that had been prestimulated with BCMA beads (compared to freshly-thawed (non-prestimulated) anti-BCMA CAR-T cells) in the co-cultures, comparing cells cultured in the presence versus absence of lenalidomide. **FIG. 24C** shows the overall viability and cell count assessed for three anti-BCMA CAR T donors. **FIG. 24D** shows results of flow cytometric analysis of surface CD25 and PD-1 expression (mean fluorescent intensity (MFI), for CD4+ or CD8+ anti-BCMA CAR T-cells after stimulation (pretreatment) with BCMA beads for 7 days, in the presence or absence of 1 µM lenalidomide. **FIG. 24E** shows the flow cytometric analysis across CAR T donors for median fluorescence intensity (MFI; CD25 and Tim3) or percentage positive PD-1 and Lag3 on the surface of T-cell markers in CD4+ CAR+ and CD8+ CAR+ subsets (gated on live CD3+ cells). Values shown are percentage baseline (Veh) MFI, viability, or count.

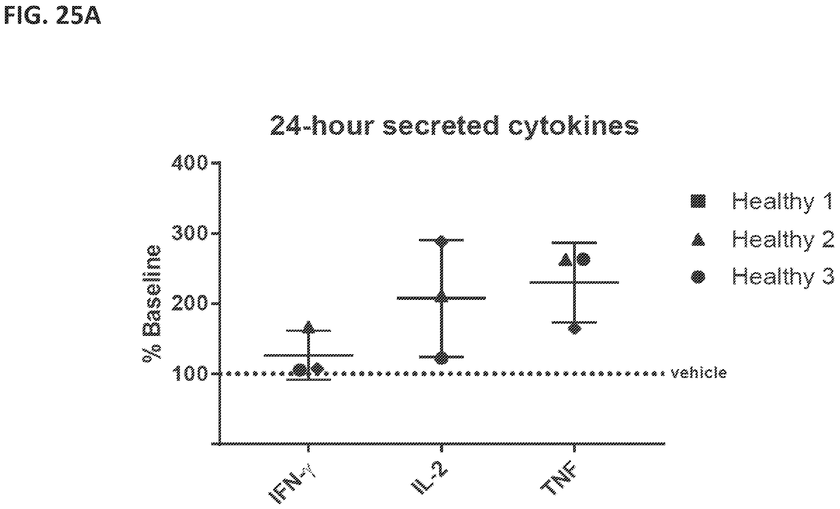

**FIG. 25A** shows the analysis of effector cytokine production following CAR-specific stimulation on 50 µg BCMA beads for 24 hours in the presence of 1 µM lenalidomide compared with baseline (vehicle) response for each of three donors.

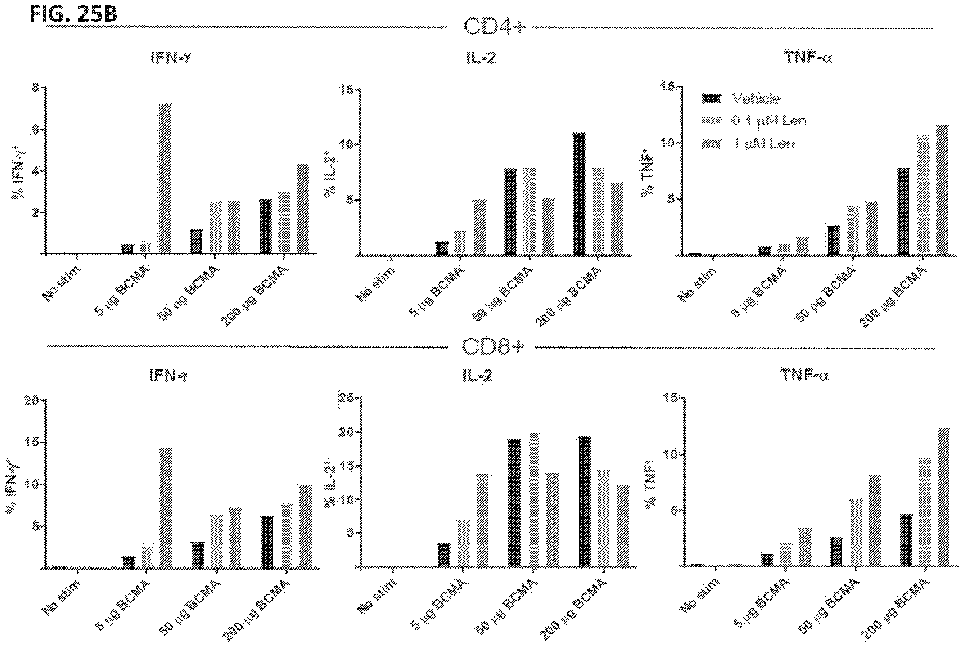

**FIG. 25B** shows the effects of anti-BCMA CAR T cells activated on different concentrations of BCMA beads (i.e., 5 µg, 50 µg, and 200 µg) in the presence or absence of lenalidomide (0.1 µM or 1 µM) on CAR T effector cytokine production.

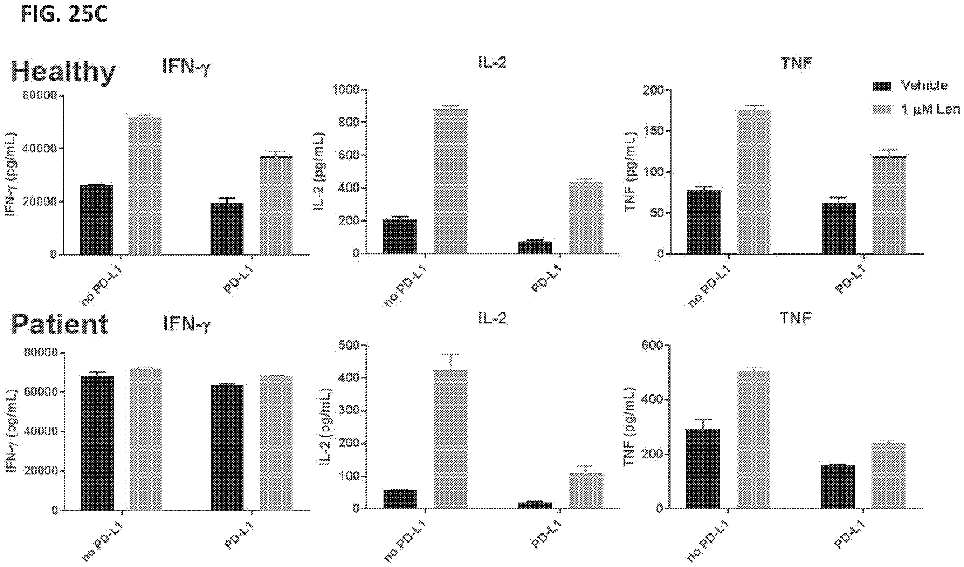

**FIG. 25C** shows the cytokine production of anti-BCMA CAR T cells derived from representative healthy donors and multiple myeloma patients stimulated on BCMA beads with or without addition of PD-L1 on the beads, in the presence of 1 µM lenalidomide) or in the absence of lenalidomide.

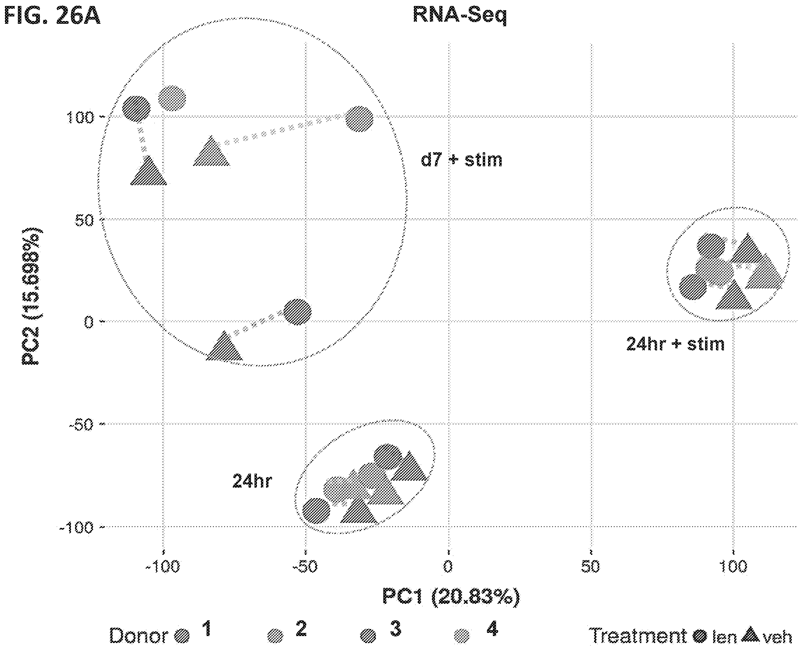

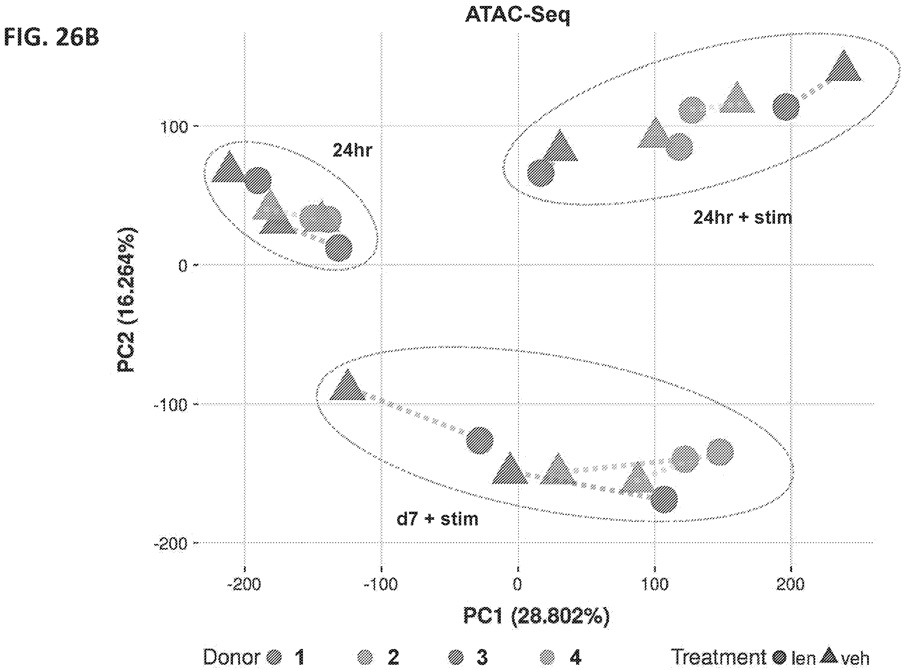

**FIGS. 26A** and **26B** show results of principal component analysis (PCA) for gene expression (based on RNA-seq results; **FIG. 26A**) and chromatin accessibility (based on ATAC-seq results; **FIG. 26B**), in anti-BCMA CAR-expressing T cells generated from 4 different donors (Donors 1-4), stimulated with BCMA-conjugated beads, for 24 hours (24 hr + stim) or 7 days (d7 + stim), or cultured without stimulation for 24 hours (24 hr), in the presence or absence of lenalidomide.

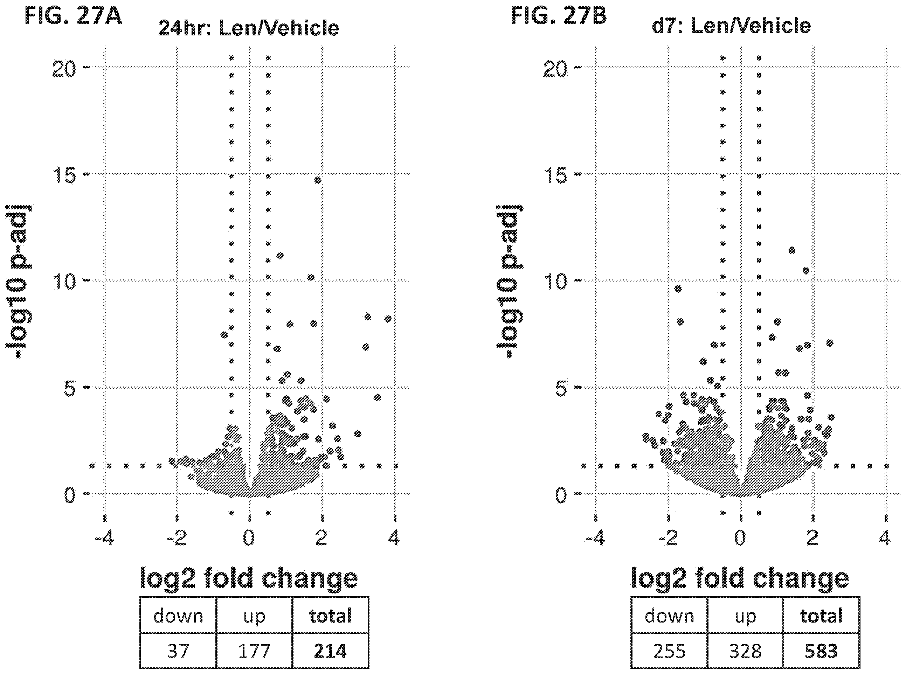

**FIGS. 27A** and **27B** show volcano plots depicting statistical significance of expression (logic of adjusted p-value) with the log₂ fold-change in gene expression, including genes or peaks that show increased (right side) or decreased (left side) expression, in CAR+ T cells stimulated with BCMA-conjugated beads, for 24 hours (24 hr + stim, **FIG. 27A**) or 7 days (d7 + stim, **FIG. 27B**), in the presence or absence of lenalidomide. The tables indicate the number of genes or peaks that showed statistically significant increase (up) or decrease (down) in expression.

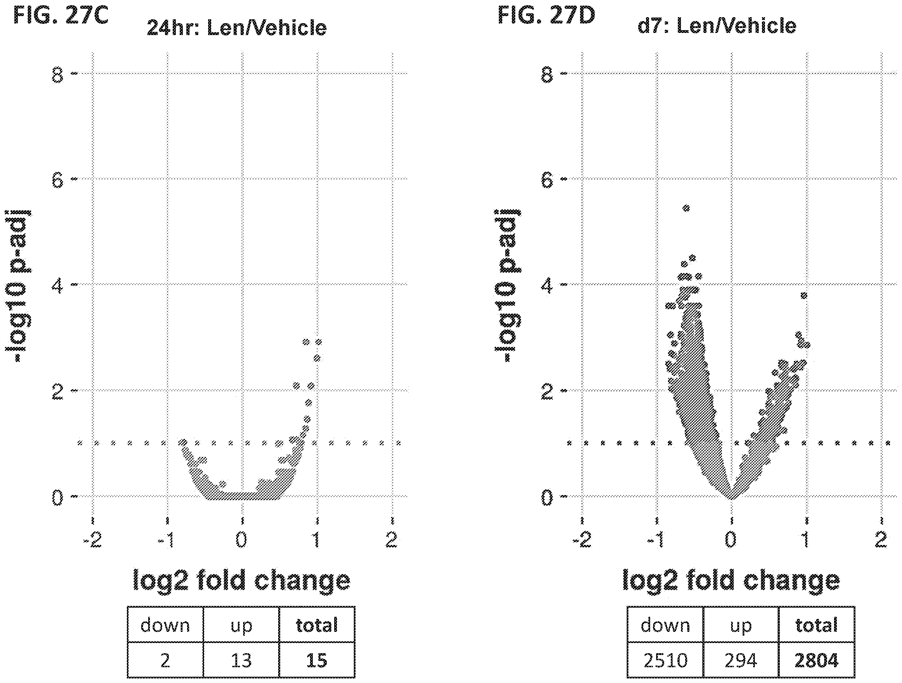

**FIGS. 27C** and **27D** show volcano plots depicting statistical significance of expression (logic of adjusted p-value) with the log₂ fold-change in g chromatin accessibility, including genes or peaks that show increased (right side) or decreased (left side) accessibility, in CAR+ T cells stimulated with BCMA-conjugated beads, for 24 hours (24 hr + stim, **FIG. 27C**) or 7 days (d7 + stim, **FIG. 27D**). The tables indicate the number of genes or peaks that showed statistically significant increase (up) or decrease (down) in accessibility.

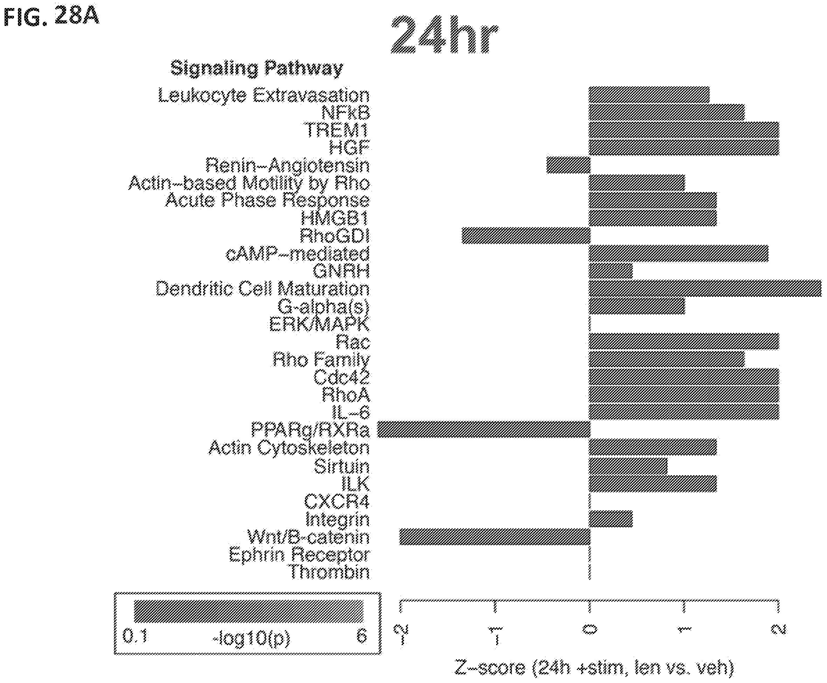

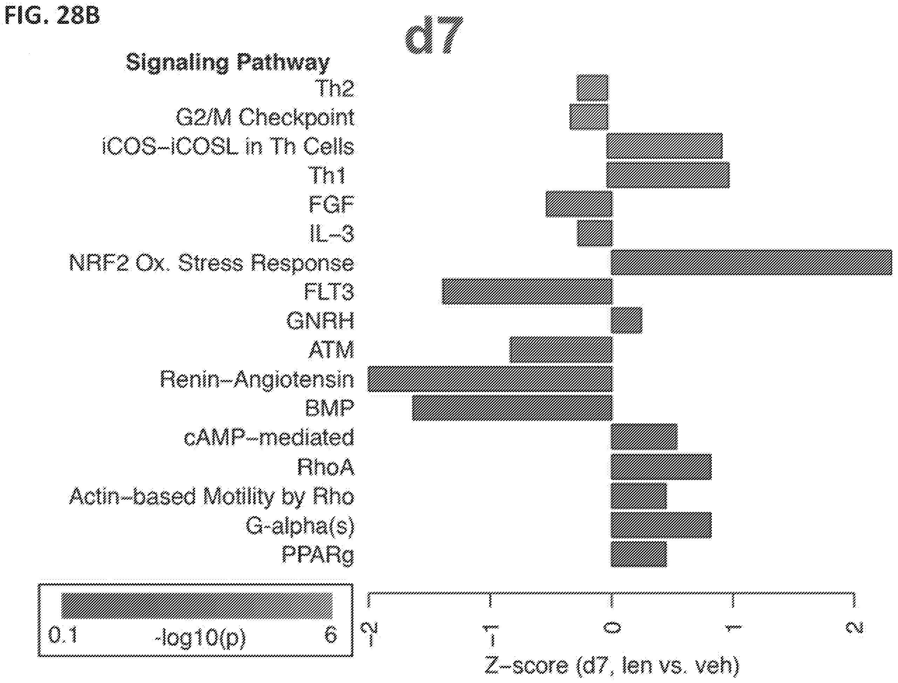

**FIGS. 28A** and **28B** depict directionality and significance of expression for the genes in biological signaling pathways that were enriched in the sets of genes whose expression was statistically significantly increased or decreased, in CAR+ T cells stimulated with BCMA-conjugated beads, for 24 hours (24 hr + stim, **FIG. 28A**) or 7 days (d7 + stim, **FIG. 28B**).

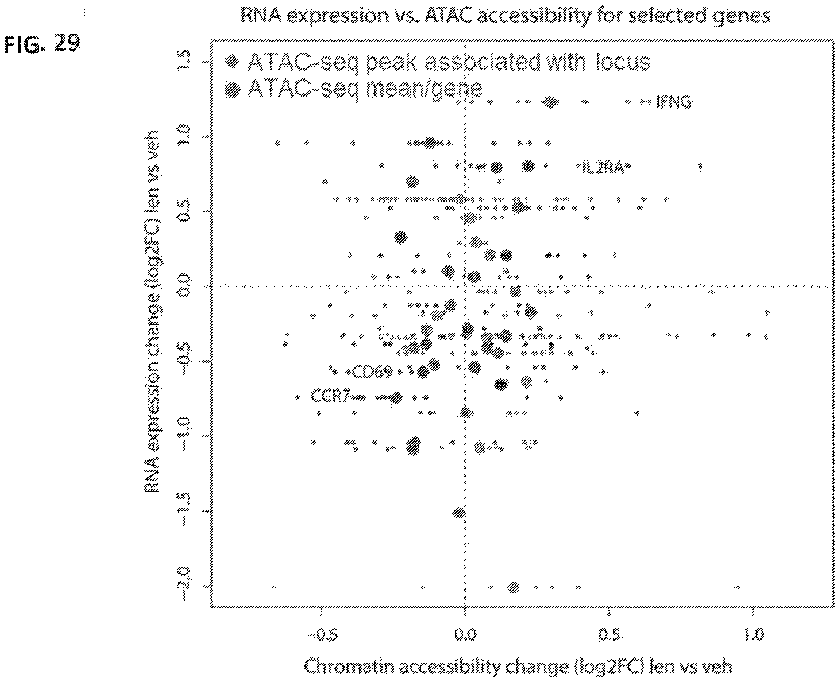

**FIG. 29** shows a plot comparing individual chromatin accessibility peaks (diamond) and the mean chromatin accessibility changes for each gene (circle), with the gene expression changes, for selected genes involved in T cell activation and signaling.

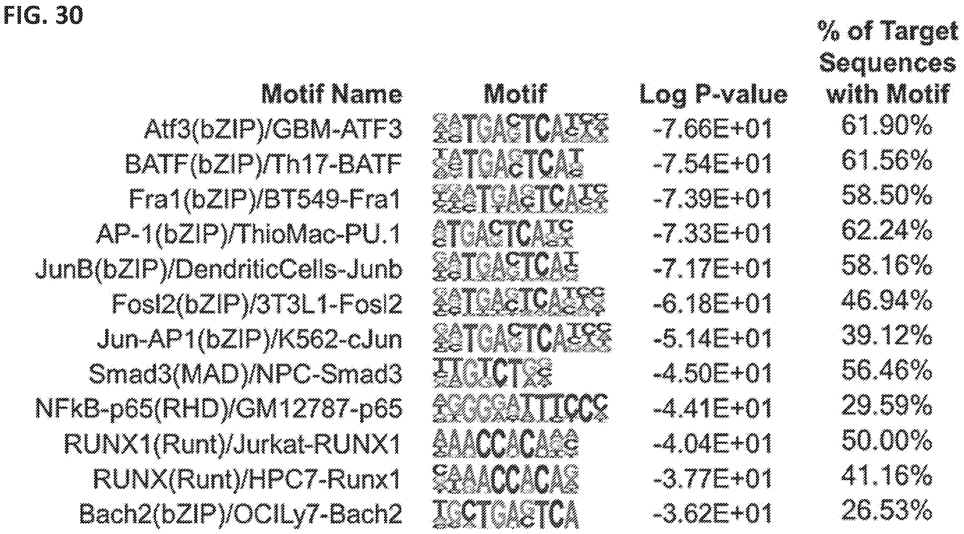

**FIG. 30** shows motif enrichment analysis, enrichment log p-value, prevalence and transcription factors predicted to bind the motifs for peaks with increased accessibility in the presence of lenalidomide in day 7 cultures.

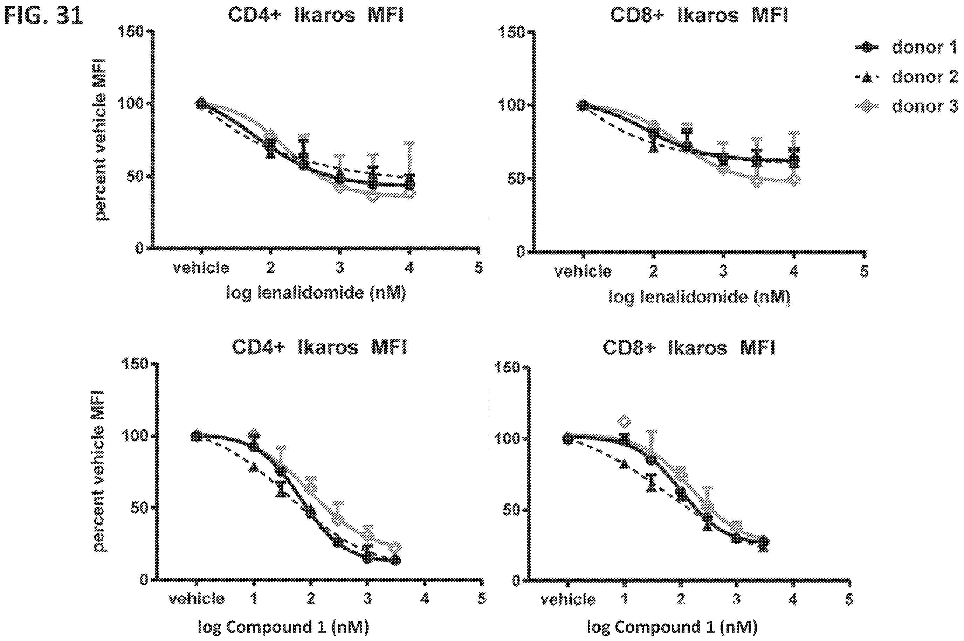

**FIG. 31** shows flow cytometry analysis of intracellular Ikaros expression on both CD4+ anti-CD19 CAR-expressing T cells and CD8+ anti-CD19 CAR-expressing T cells. CAR-expressing T cells were stimulated with CAR-T anti-idiotypic antibody (5 µg/mL) treated across a concentration range of lenalidomide or Compound 1. Median fluorescence intensity (MFI) values for Ikaros were normalized and calculated as a percentage relative to as a percentage relative to vehicle control.

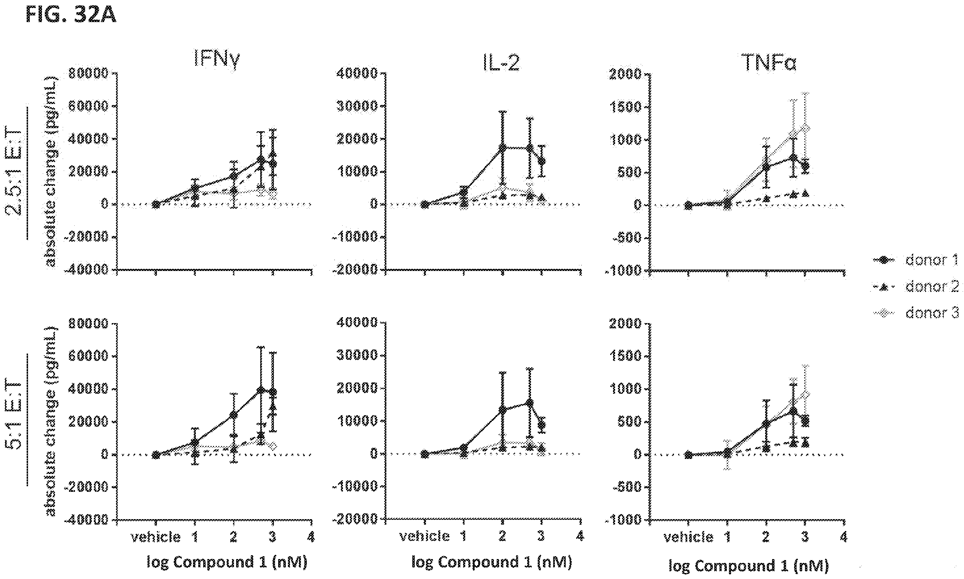

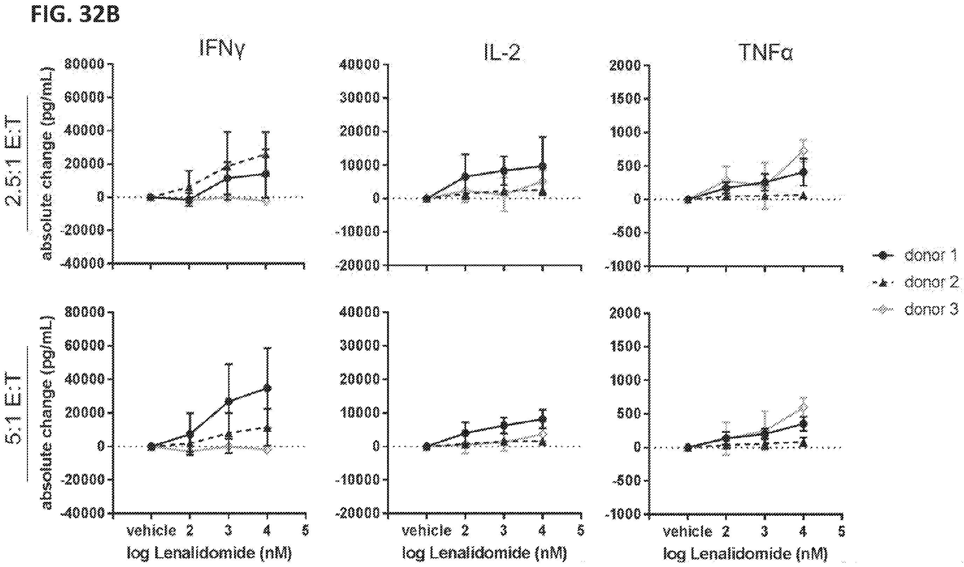

**FIGS. 32A** and **32B** show analysis of cytokine production of anti-CD19 CAR-expressing T cells in the presence of Compound 1 (**FIG. 32A**) or lenalidomide (**FIG. 32B**) following incubation with target cells. Multiplex cytokine assay of supernatants taken at 24 hours from triplicate wells of anti-CD19 CAR-expressing T cells co-cultured with K562.CD19 target cells in the presence of several concentrations of Compound 1 or lenalidomide. IFN-γ, IL-2, and TNF-α concentrations were determined for CAR-expressing T cell from three different donors over two E:T ratios. Data represents the mean +/- S.D. across 3 experiments.

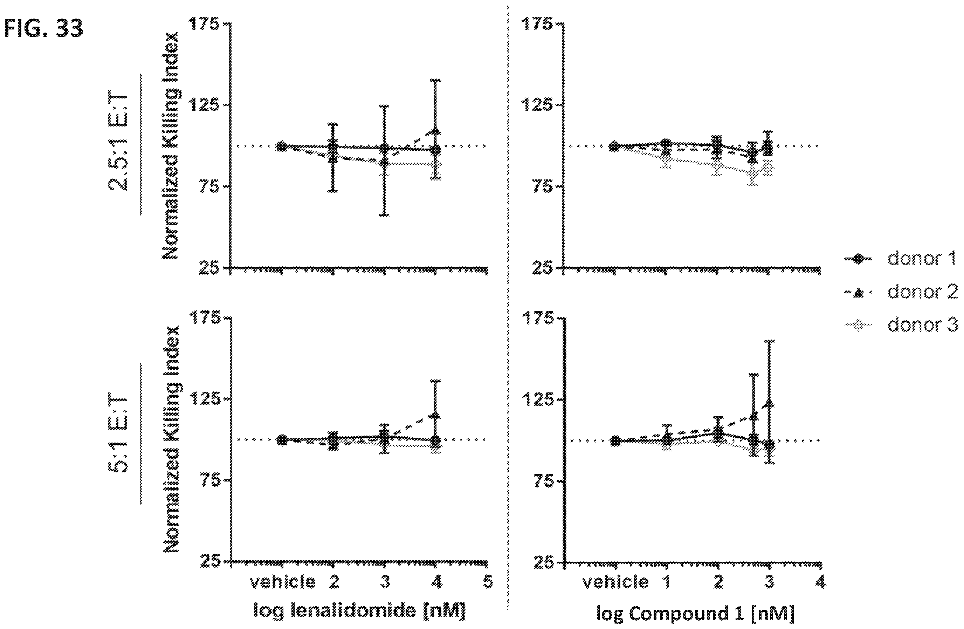

**FIG. 33** shows analysis of cytolytic function of anti-CD19 CAR-expressing T cells in the presence of Compound 1 or lenalidomide following incubation with target cells. Anti-CD19 CAR-expressing T cells from three different donors were co-cultured with K562.CD19 target cells in triplicate at two E:T ratios in the presence of Compound 1 or lenalidomide over 5 days. Results were calculated as a normalized killing index. Data represents the mean +/- S.D. across 3 experiments.

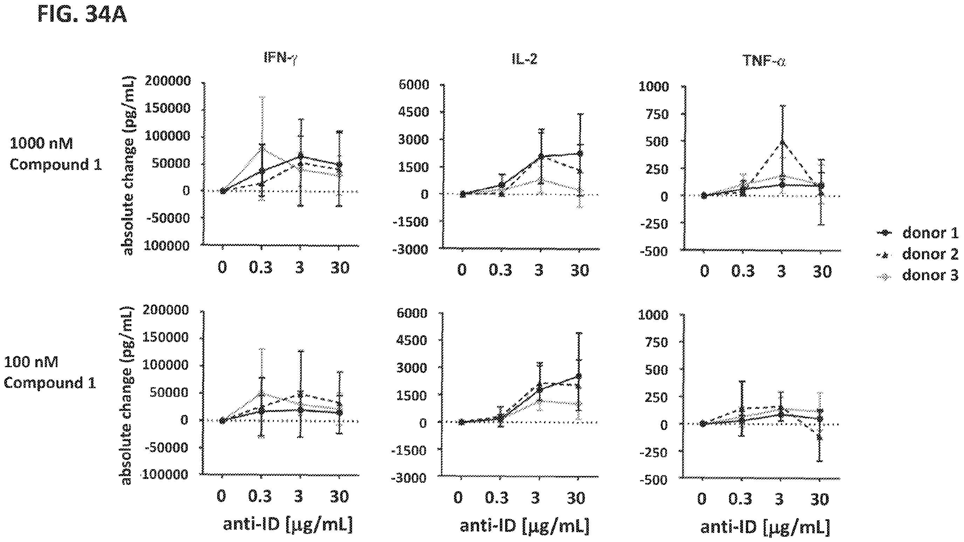

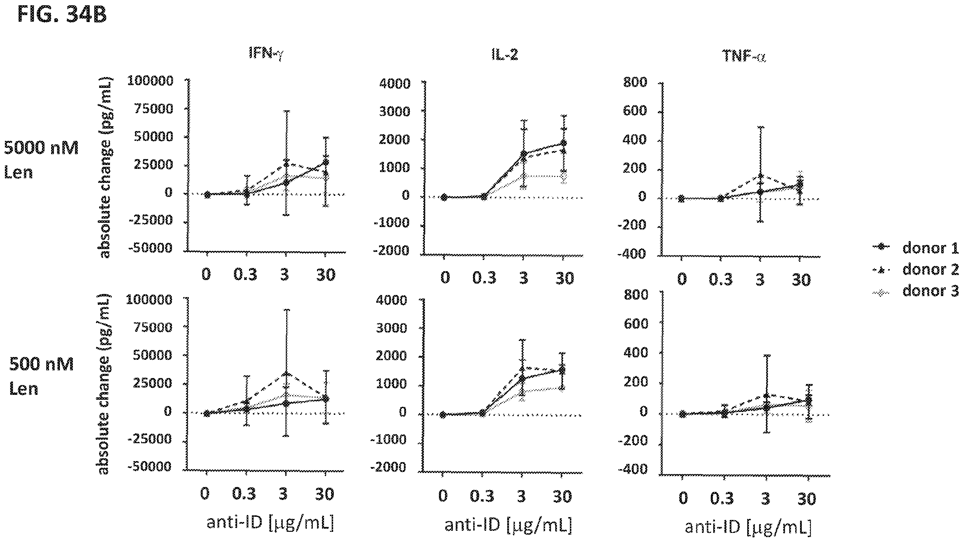

**FIGS. 34A** and **34B** show analysis of cytokine production of anti-CD19 CAR-expressing T cells in the presence of Compound 1 (**FIG. 34A**) or lenalidomide (**FIG. 34B**) following anti-idiotypic antibody stimulation. Multiplex cytokine assay of supernatants taken at 24 hours from triplicate wells of anti-CD19 CAR-expressing T cells co-cultured with agonist anti-idiotypic antibody in the presence of 100 or 1000 nM Compound 1 (**FIG. 34A**), or 500 or 5000 nM lenalidomide (**FIG. 34B**). IFN-γ, IL-2, and TNF-α concentrations were determined for CAR-expressing T cells from three different donors. Data represents the mean +/- S.D. across 3 experiments.

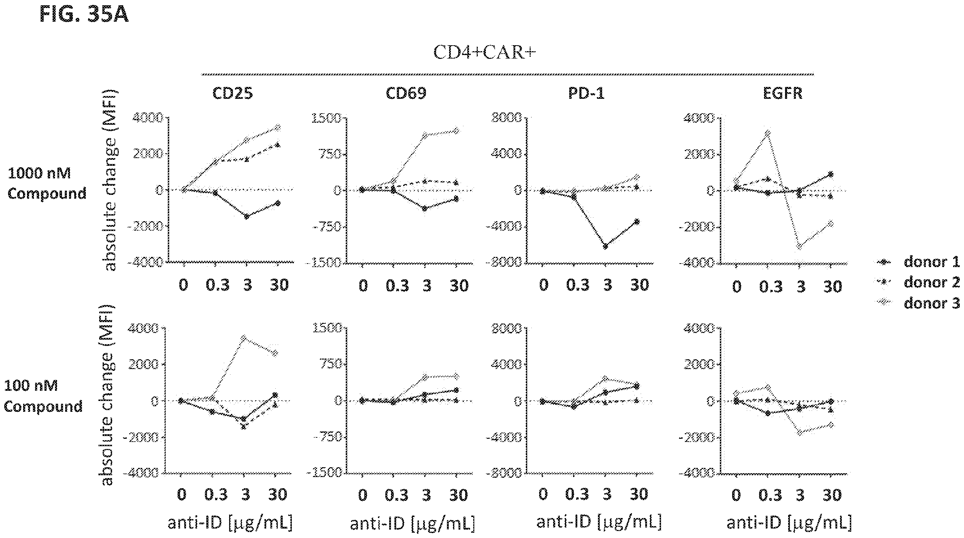

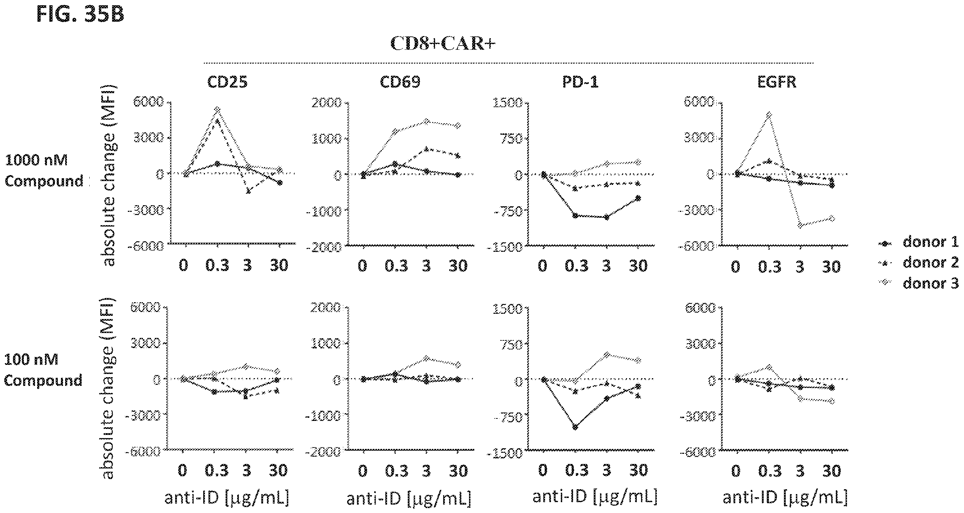

**FIGS. 35A** and **35B** show analysis of surface marker expressions on CD4+ anti-CD19 CAR-expressing T cells (**FIG. 35A**) and CD8+ anti-CD19 CAR-expressing T cells (**FIG. 35B**) in the presence of Compound 1 following anti-idiotypic antibody stimulation. Anti-CD19 CAR-expressing T cells from three different donors were stimulated with anti-idiotypic antibody at 0, 0.3, 3, or 30 µg/mL in the presence of 100 or 1000 nM of Compound 1. Cells were analyzed by flow cytometry at day 4. The absolute change in median fluorescence intensity relative to vehicle control for each concentration of anti-idiotypic antibody was calculated. Data are representative of 3 experiments.

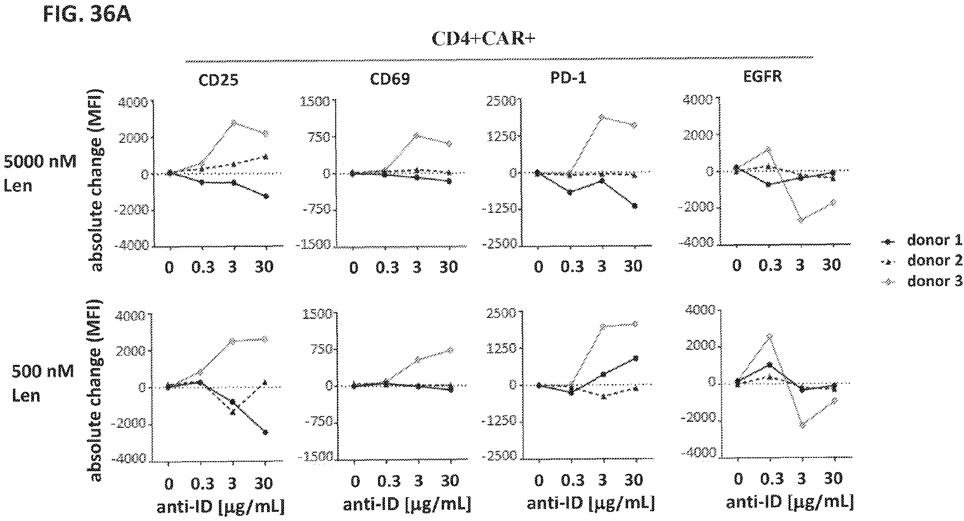

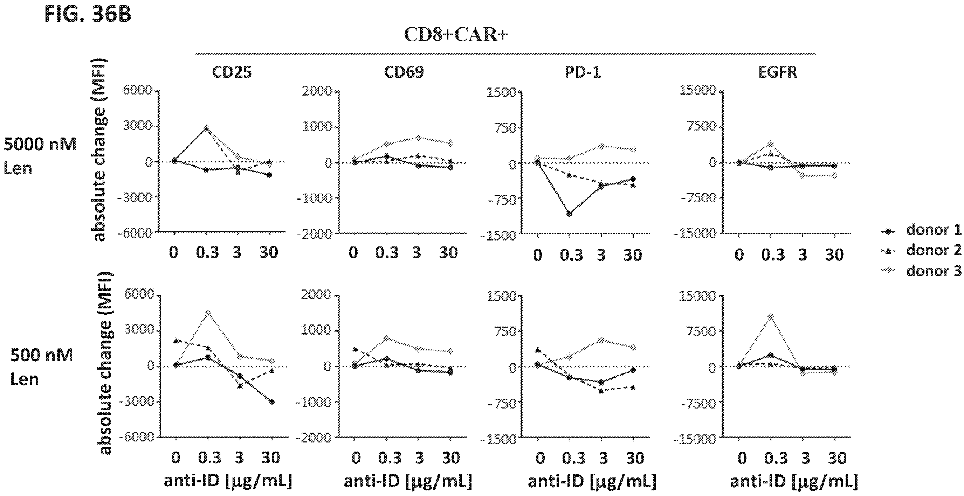

**FIGS. 36A** and **36B** show analysis of surface marker expressions on CD4+ anti-CD19 CAR-expressing T cells (**FIG. 36A**) and CD8+ anti-CD19 CAR-expressing T cells (**FIG. 36B**) in the presence of lenalidomide following anti-idiotypic antibody stimulation. Anti-CD19 CAR-expressing T cells from three different donors were stimulated with anti-idiotypic antibody at 0, 0.3, 3, or 30 µg/mL in the presence of 500 or 5000 nM of lenalidomide. Cells were analyzed by flow cytometry at day 4. The absolute change in median fluorescence intensity relative to vehicle control for each concentration of anti-idiotypic antibody was calculated. Data are representative of 3 experiments.

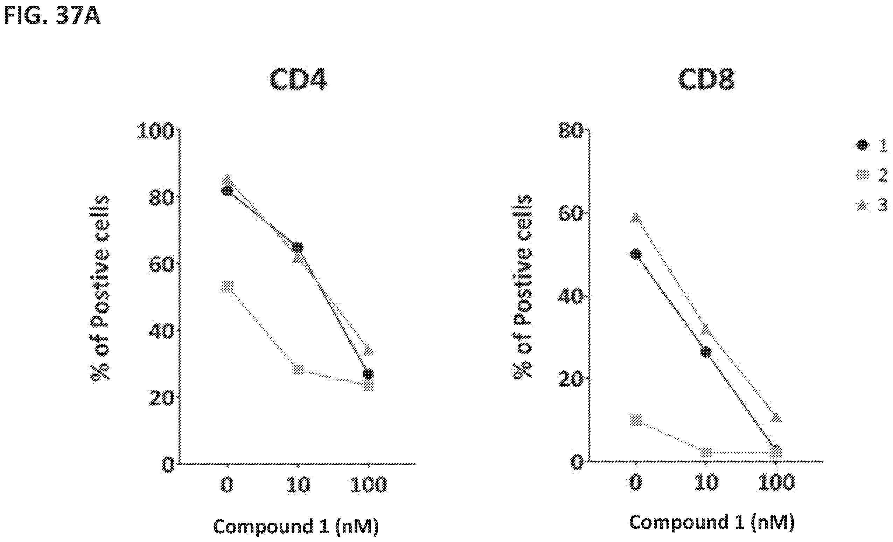

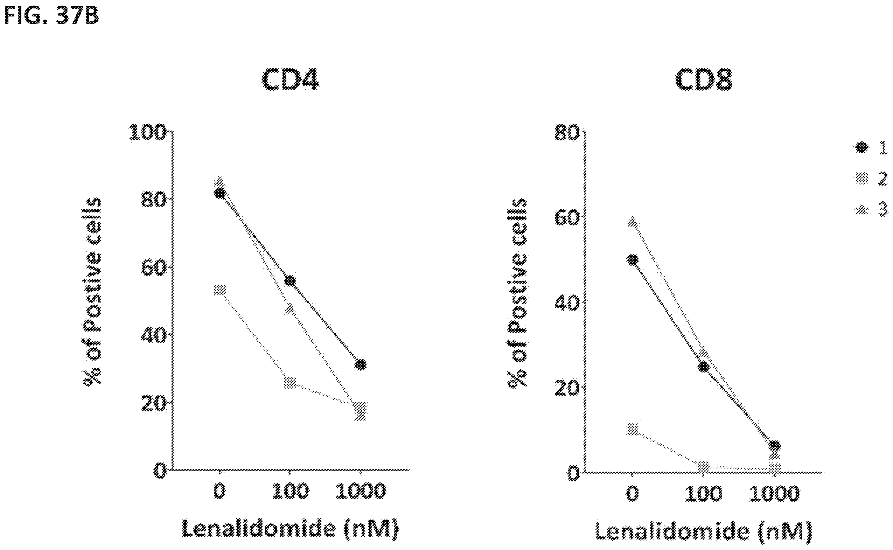

**FIGS. 37A** and **37B** show analysis of CD28 surface expression on CD4+ and CD8+ anti-CD19 CAR-expressing T cells in the presence of Compound 1 (**FIG. 37A**) or lenalidomide (**FIG. 37B**) after serial stimulation. Anti-CD19 CAR-expressing T cells from three different donors were stimulated with K562.CD19 at an E:T ratio of 2.5:1 every 3-4 days in the presence of Compound 1 (**FIG. 37A**) or lenalidomide (**FIG. 37B**). The percentage of cells positive for CD28 was measured by flow cytometry at day 28.

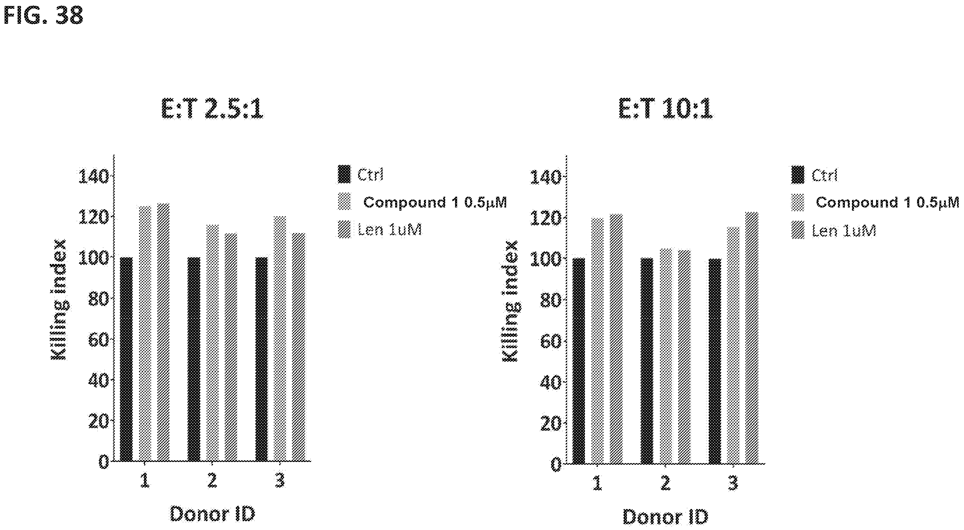

**FIG. 38** shows analysis of cytolytic function of anti-CD19 CAR-expressing T cells in the presence of Compound 1 or lenalidomide following serial stimulation. Anti-CD19 CAR-expressing T cells from three different donors after 24 days of serial stimulation were co-cultured with irradiated K562.CD19 target cells in triplicate at two E:T ratios in the presence of Compound 1 or lenalidomide. Results were calculated as a normalized killing index.

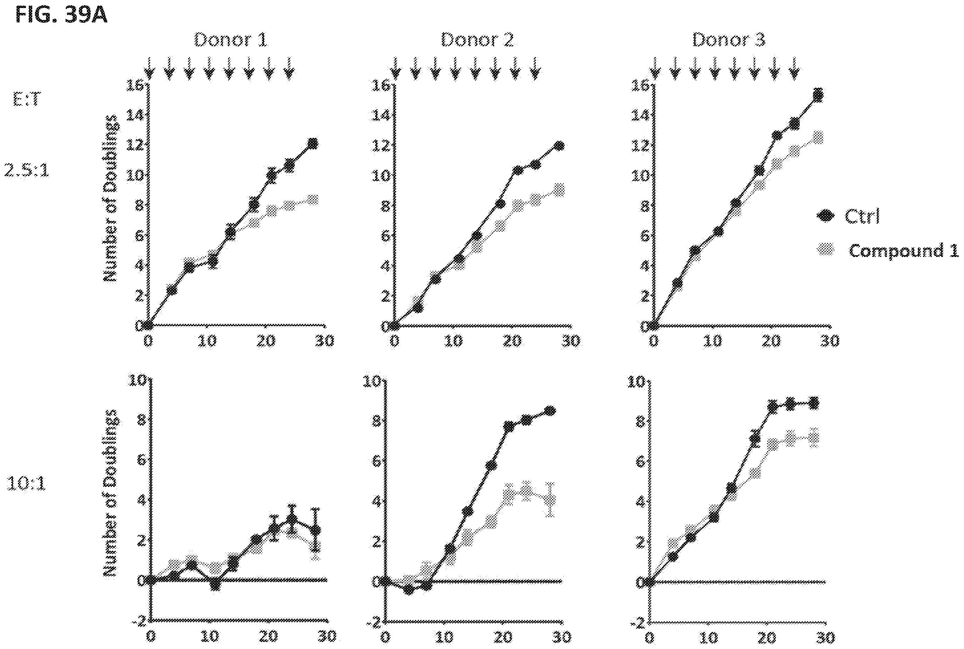

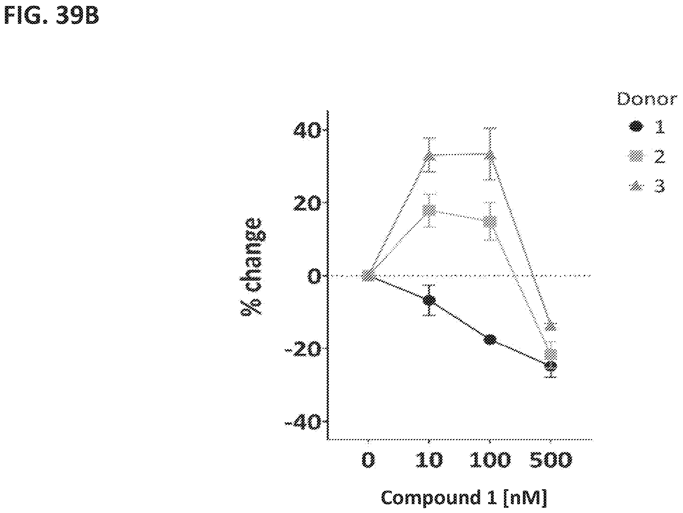

**FIGS. 39A** and **39B** show analysis of population doublings of anti-CD19 CAR-expressing T cells during a 28-day serial stimulation period in the presence of absence of Compound 1. Anti-CD19 CAR-expressing T cells from three different donors were stimulated with with K562.CD19 target cells at an E:T ratio of 2.5:1 or 10:1 every 3-4 days in the presence of 500 nM Compound 1 for 28 days (represented by x-axis). Cells were counted after each stimulation and cell doublings were calculated. (**FIG. 39A**) Percentage change in cell doublings at day 24 of serial stimulation in the presence of 10 nM, 100 nM or 500 nM Compound 1 was shown in **FIG. 39B****.** Data represents mean+/-S.E.M of triplicated treatments from 3 donors. Each arrow represents a re-stimulation time point.

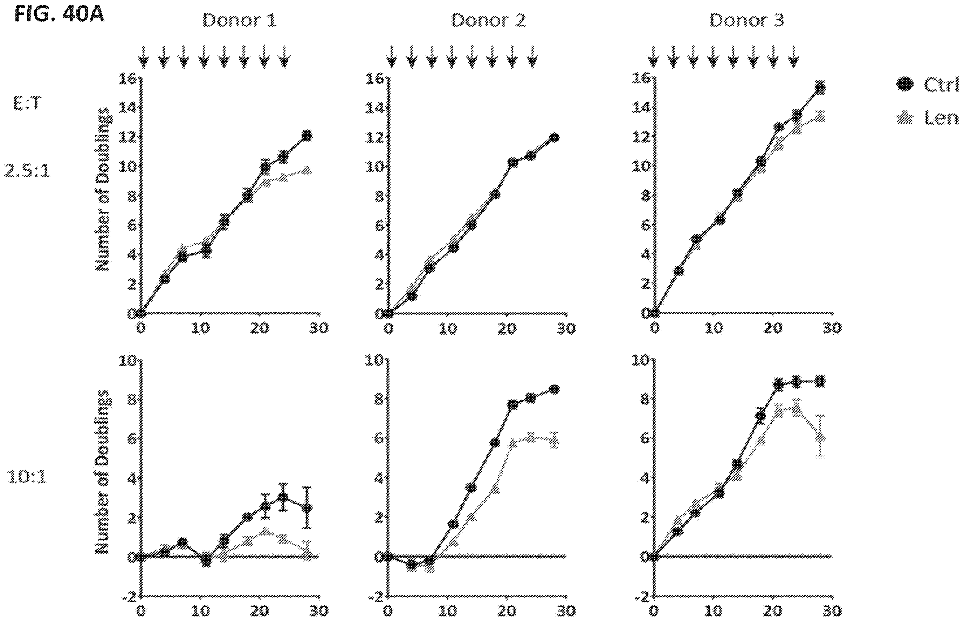

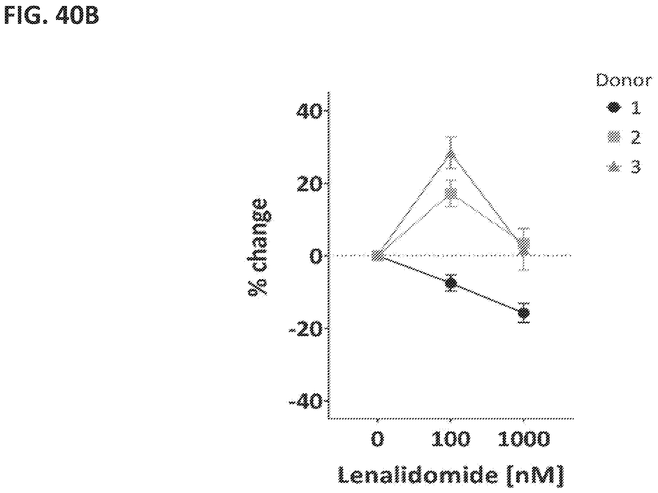

**FIGS. 40A** and **40B** show analysis of population doublings of anti-CD19 CAR-expressing T cells during a 28-day serial stimulation period in the presence of absence of lenalidomide. Anti-CD19 CAR-expressing T cells from three different donors were stimulated with with K562.CD19 target cells at an E:T ratio of 2.5:1 or 10:1 every 3-4 days in the presence of 1000 nM lenalidomide for 28 days (represented by x-axis). Cells were counted after each stimulation and cell doublings were calculated. (**FIG. 40A**) Percentage change in cell doublings at day 24 of serial stimulation in the presence of 100 nM or 1000 nM lenalidomide was shown in **FIG. 40B****.** Data represents mean+/-S.E.M of triplicated treatments from three donors. Each arrow represents a re-stimulation time point.

### Detailed Description

Embodiments of the invention are described in some of the instances and aspects disclosed below.

Disclosed herein are combination therapies involving administration of an immunotherapy involving T cell function or activity, such as a T cell therapy, and an immunomodulatory compound, such as a structural or functional analog or derivative of thalidomide and/or an inhibitor of E3-ubiquitin ligase. In some aspects, the disclosed methods enhance or modulate proliferation and/or activity of T cell activity associated with administration of an immunotherapy or immunotherapeutic agent, such as a composition including cells for adoptive cell therapy, e.g., such as a T cell therapy (e.g. CAR-expressing T cells). In some instances, the combination therapy involves administration of an immunomodulatory compound, such as a structural or functional analog of thalidomide and/or an inhibitor of E3-ubiquitin ligase, and administration of the T cell therapy, such as a composition including cells for adoptive cell therapy, e.g., such as a T cell therapy (e.g. CAR-expressing T cells).

T cell-based therapies, such as adoptive T cell therapies (including those involving the administration of cells expressing chimeric receptors specific for a disease or disorder of interest, such as chimeric antigen receptors (CARs) and/or other recombinant antigen receptors, as well as other adoptive immune cell and adoptive T cell therapies) can be effective in the treatment of cancer and other diseases and disorders. The engineered expression of recombinant receptors, such as chimeric antigen receptors (CARs), on the surface of T cells enables the redirection of T-cell specificity. In clinical studies, CAR-T cells, for example anti-CD19 CAR-T cells, have produced durable, complete responses in both leukemia and lymphoma patients (Porter et al. (2015) Sci Transl Med., 7:303ra139; Kochenderfer (2015) J. Clin. Oncol., 33: 540-9; Lee et al. (2015) Lancet, 385:517-28; Maude et al. (2014) N Engl J Med, 371: 1507-17).

In certain contexts, available approaches to adoptive cell therapy may not always be entirely satisfactory. In some contexts, optimal efficacy can depend on the ability of the administered cells to recognize and bind to a target, *e.g.,* target antigen, to traffic, localize to and successfully enter appropriate sites within the subject, tumors, and environments thereof. In some contexts, optimal efficacy can depend on the ability of the administered cells to become activated, expand, to exert various effector functions, including cytotoxic killing and secretion of various factors such as cytokines, to persist, including long-term, to differentiate, transition or engage in reprogramming into certain phenotypic states (such as long-lived memory, less-differentiated, and effector states), to avoid or reduce immunosuppressive conditions in the local microenvironment of a disease, to provide effective and robust recall responses following clearance and re-exposure to target ligand or antigen, and avoid or reduce exhaustion, anergy, peripheral tolerance, terminal differentiation, and/or differentiation into a suppressive state.

In some instances, the exposure and persistence of engineered cells is reduced or declines after administration to the subject. Yet, observations indicate that, in some cases, increased exposure of the subject to administered cells expressing the recombinant receptors (e.g., increased number of cells or duration over time) may improve efficacy and therapeutic outcomes in adoptive cell therapy. Preliminary analysis conducted following the administration of different CD19-targeting CAR-expressing T cells to subjects with various CD19-expressing cancers in multiple clinical trials revealed a correlation between greater and/or longer degree of exposure to the CAR-expressing cells and treatment outcomes. Such outcomes included patient survival and remission, even in individuals with severe or significant tumor burden.

In some aspects, the disclosed methods and uses provide for or achieve improved or more durable responses or efficacy as compared to certain alternative methods, such as in particular groups of subjects treated. In some instances, the methods are advantageous by virtue of administering T cell therapy, such as a composition including cells for adoptive cell therapy, e.g., such as a T cell therapy (*e.g.* CAR-expressing T cells), and an immunomodulatory compound, such as a structural or functional analog or derivative of thalidomide and/or an inhibitor of E3 ubiquitin ligase, e.g. lenalidomide.

The disclosed methods are based on observations that the immunomodulatory compound, such as a structural or functional analog or derivative of thalidomide and/or an inhibitor of E3 ubiquitin ligase, e.g. lenalidomide, improves T cell function, including functions related to the expansion, proliferation and persistence of T cells. Lenalidomide is an immunomodulatory drug currently approved for the treatment of multiple myeloma (MM) and mantle cell lymphoma (MCL), and clinically tested in the therapy of diffuse large B-cell lymphoma of activated B cell immunophenotype. In some cases, lenalidomide increases antitumor immune responses at least partially by modulating the activity of E3 ubiquitin ligase Cereblon (CRBN), which leads to increased ubiquitinylation of Ikaros and Aiolos transcription factors, which in turn results in changed expression of various receptors on the surface of tumor cells (*see* e.g., Otáhal et al. (2016) Oncoimmunology., April; 5(4): e1115940).

The provided findings indicate that combination therapy of the immunomodulatory compound, such as a structural or functional analog or derivative of thalidomide and/or an inhibitor of E3 ubiquitin ligase, e.g. lenalidomide, in methods involving T cells, such as involving administration of adoptive T cell therapy, achieves improved function of the T cell therapy. In some instances, combination of the cell therapy (e.g., administration of engineered T cells) with the immunomodulatory compound, e.g., lenalidomide, improves or enhances one or more functions and/or effects of the T cell therapy, such as persistence, expansion, cytotoxicity, and/or therapeutic outcomes, e.g., ability to kill or reduce the burden of tumor or other disease or target cell.

In particular aspects, it is found herein that an immunomodulatory compound, such as a structural or functional analog or derivative of thalidomide and/or an inhibitor of E3 ubiquitin ligase, e.g. lenalidomide, promotes continued function and/or survival of cells of a T cell therapy (e.g. CAR-T cells) after activation, including after encounter with antigen. In some aspects, lenalidomide increases the ability of such T cells to persist or function long-term, such as by preventing exhaustion or cell death. In some instances, such improvements can result in a combination therapy exhibiting improved overall responses, e.g. reduction in tumor burden, and/or increased survival compared to in subjects treated with a monotherapy involving administration of the T cell therapy (e.g. CAR-T cell) or immunomodulatory compound (e.g. lenalidomide) alone. In some aspects, the disclosed methods increase overall response and/or survival by or more than 1.5-fold, 2.0-fold, 3.0-fold, 4.0-fold, 5.0-fold, 10-fold or more compared to an alternative treatment, such as compared to a monotherapy involving administration of the T cell therapy (e.g. CAR-T cell) or immunomodulatory compound (e.g. lenalidomide) alone.

In some instances, the combination with the immunomodulatory compound, while improving one or more outcomes or functional attributes, does not affect one or more side effects or unwanted changes in the T cells, such as does not reduce the ability of the cells to become activated, secrete one or more desired cytokines, expand and/or persist, e.g., as measured in an in vitro assay as compared to such cells cultured under conditions otherwise the same but in the absence of the immunomodulatory compound. Thus in some instances, disclosed are methods and combinations that result in improvements in T cell function or phenotype, e.g., in intrinsic T cell functionality and/or intrinsic T cell phenotype, generally without compromising one or more other desired properties of functionality, e.g., of CAR-T cell functionality.

In some instances, the disclosed methods can potentiate T cell therapy, e.g. CAR-T cell therapy, which, in some aspects, can improve outcomes for treatment. In some instances, the methods are particularly advantageous in subjects in which the cells of the T cell therapy exhibit weak expansion, have become exhausted, exhibit a reduced or decreased persistence in the subject and/or in subjects that have a cancer that is resistant or refractory to other therapies, is an aggressive or high-risk cancer, and/or that is or is likely to exhibit a relatively lower response rate to a CAR-T cell therapy administered without the immunomodulatory compound compared to another type of cancer or compared to administration with a different CAR-T cell therapy.

In some aspects, the disclosed methods can enhance, increase or potentiate T cell therapy, such as to overcome lack of persistence and/or exhaustion of T cells, e.g. in subjects in which, at or about day 12-15 after initiation of administration of the T cell therapy, less than 10 µL, such as less than 5 µL or less than 1 µL of such cells, or a CD8+ or CD3+ subset thereof, are detectable in the blood. In some instances, a subject having received administration of a T cell therapy, e.g. CAR-T cell, is monitored for the presence, absence or level of T cells of the therapy in the subject, such as in a biological sample of the subject, e.g. in the blood of the subject. In some instances, an immunomodulatory compound, such as a structural or functional analog or derivative of thalidomide and/or an inhibitor of E3 ubiquitin ligase, e.g. lenalidomide, is administered to a subject having received the T cell therapy (e.g. CAR-T cells) but in which such cells have weakly expanded and/or are at or below a threshold level in a sample of the subject, e.g. blood sample, at a time when strong or robust expansion of the CAR-T cells in the subject is typically observed in a plurality of subjects administered a T cell therapy (e.g. CAR-T), in some cases, this same T cell therapy (e.g. same CAR-T cells). In some aspects, an immunomodulatory compound, such as a structural or functional analog or derivative of thalidomide and/or an inhibitor of E3 ubiquitin ligase, e.g., lenalidomide, is administered if, at or about day 12-15 after initiation of administration of the T cell therapy, less than 10 µL, such as less than 5 µL or less than 1 µL of such cells, or a CD8+ or CD3+ subset thereof, are detectable in the blood.

In certain aspects, the disclosed methods can enhance, increase or potentiate T cell therapy in subjects in which a peak response to the T cell therapy has been observed but in which the response, e.g. presence of T cells and/or reduction in tumor burden, has become reduced or is no longer detectable. In some aspects, an immunomodulatory compound, such as a structural or functional analog or derivative of thalidomide and/or an inhibitor of E3 ubiquitin ligase, e.g. lenalidomide, is administered to a subject within a week, such as within 1, 2 or 3 days after: (i) peak or maximum level of the cells of the T cell therapy are detectable in the blood of the subject; (ii) the number of cells of the T cell therapy detectable in the blood, after having been detectable in the blood, is not detectable or is reduced, optionally reduced compared to a preceding time point after administration of the T cell therapy; (iii) the number of cells of the T cell therapy detectable in the blood is decreased by or more than 1.5-fold, 2.0-fold, 3.0-fold, 4.0-fold, 5.0-fold, 10-fold or more the peak or maximum number cells of the T cell therapy detectable in the blood of the subject after initiation of administration of the T cell therapy; (iv) at a time after a peak or maximum level of the cells of the T cell therapy are detectable in the blood of the subject, the number of cells of or derived from the T cells detectable in the blood from the subject is less than less than 10%, less than 5%, less than 1% or less than 0.1% of total peripheral blood mononuclear cells (PBMCs) in the blood of the subject; (v) the subject exhibits disease progression and/or has relapsed following remission after treatment with the T cell therapy; and/or (iv) the subject exhibits increased tumor burden as compared to tumor burden at a time prior to or after administration of the T cells and prior to initiation of administration of the immunomodulatory compound.

In some instances, the methods can be used for treating a disease or condition, e.g. a B cell malignancy or hematological malignancy, and in particular such diseases, conditions or malignancies in which responses, e.g. complete response, to treatment with the T cell therapy alone, such as a composition including cells for adoptive cell therapy, e.g., such as a T cell therapy (*e.g.* CAR-expressing T cells), is relatively low compared to treatment with other T cell therapies or treatment of other diseases or malignancies (e.g. a CR in a less than or less than about 60%, less than about 50% or less than about 45% of the subjects so treated) and/or in which the subject is not responsive to treatment with the immunomodulatory compound, such as a structural or functional analog or derivative of thalidomide and/or an inhibitor of E3 ubiquitin ligase, e.g. lenalidomide, alone.

In some instances, the combination therapy disclosed herein is for use in a subject having a cancer in which after initiation of administration of the T cell therapy, such as a composition including cells for adoptive cell therapy, e.g., CAR-expressing T cells, the subject has relapsed following remission after treatment with the T cell therapy. In some instances, subjects that have relapsed following such remission are administered an immunomodulatory compound, such as a structural or functional analog or derivative of thalidomide and/or an inhibitor of E3 ubiquitin ligase, e.g. lenalidomide. In some instances, the combination therapy disclosed herein is for use in a subject having a disease or condition, e.g. cancer, in which the amount of the immunomodulatory compound administered is insufficient, as a single agent and/or in the absence of administration of the T cell therapy, to ameliorate, reduce or prevent the disease or condition or a symptom or outcome thereof, such as is insufficient to ameliorate, reduce or prevent the disease or condition in the subject or a symptom or outcome thereof. In some instances, the method thereby reduces or ameliorates a symptom or outcome or burden of the disease or condition to a degree that is greater than the combination of (i) the degree of reduction or amelioration effected by the administration of the immunomodulatory agent alone, optionally on average in a population of subjects having the disease or condition, and (ii) the degree of reduction or amelioration by the administration of the T cell therapy alone, optionally on average in a population of subjects having the disease or condition. In some instance, the method reduces or ameliorates such symptoms, outcomes or burdens of the disease, e.g. compared to on average in a population of subjects having the disease or condition, by greater than or greater than about 1.5-fold, 2.0-fold, 3.0-fold, 4.0-fold, 5.0-fold, 6.0-fold, 7.0-fold, 8.0-fold, 9.0-fold, 10.0 fold, 20.0-fold, 30.0-fold, 40.0-fold, 50.0-fold or more.

In some instances, the provided combination therapy is used in connection with treating certain diseases or conditions, e.g., cancer, in which optimal stimulation of a recombinant antigen receptor, e.g. CAR-T cell, is difficult to achieve and/or is not consistently observed. In some instances, less than optimal stimulation may be a result of low or inaccessible levels of disease antigen in vivo, e.g. at or on the tumor. In some instances, certain cancers, such as NHL, e.g. high-risk or aggressive NHL, such as DLBCL, and/or chronic lymphocytic leukemia (CLL) can be associated with defects in or reduction in intrinsic T cell functionality, which, in some cases, is influenced by the disease itself. For example, the pathogenesis of many cancers, such as CLL and NHL, e.g. DLBCL, can be associated with immunodeficiency, leading to promotion of tumor growth and immune evasion, such as due to immunosuppression of T cells, e.g. driven by one or more factors in the tumor microenvironment. In some cases, alleviating intrinsic T cell defects obtained from cancers of such patients for use in connection with adoptive cell therapy could provide for more potent responses to adoptive T cell therapy, e.g. CAR-T cell therapy. In some cases, less than optimal stimulation can be due to differences in expression level of the CAR on engineered T cells administered to the subject. In any of such instances, administration of the immunomodulatory compound, such as a structural or functional analog or derivative of thalidomide and/or an inhibitor of E3 ubiquitin ligase, e.g., lenalidomide, can enhance the stimulation or activity of such T cells in vivo in the subject.

In some instances of the disclosed methods, one or more properties of administered genetically engineered cells can be improved or increased or greater compared to administered cells of a reference composition, such as increased or longer expansion and/or persistence of such administered cells in the subject or an increased or greater recall response upon restimulation with antigen. In some instances, the increase can be at least a 1.2-fold, at least 1.5-fold, at least 2-fold, at last 3-fold, at least 4-fold, at least 5-fold, at least 6-fold, at least 7-fold, at least 8-fold, at least 9-fold, or at least 10-fold increase in such property or feature compared to the same property or feature upon administration of a reference cell composition. In some instances, the increase in one or more of such properties or features can be observed or is present within 7 days, 14 days, 21 days, within one months, two months, three months, four months, five months, six months, or 12 months after administration of the genetically engineered cells and the initiation of administration of the immunomodulatory compound, such as a structural or functional analog or derivative of thalidomide and/or an inhibitor of E3 ubiquitin ligase, e.g., lenalidomide.

In some instances, a reference cell composition can be a composition of T cells from the blood of a subject not having or not suspected of having the cancer or is a population of T cells obtained, isolated, generated, produced, incubated and/or administered under the same or substantially the conditions, except not having been incubated or administered in the presence of the immunomodulatory compound. In some instances, the reference cell composition contains genetically engineered cells that are substantially the same, including expression of the same recombinant receptor, e.g., CAR. In some aspects, such T cells are treated identically or substantially identically, such as manufactured similarly, formulated similarly, administered in the same or about the same dosage amount and other similar factors.

In some instances, the disclosed methods result in genetically engineered cell with increased persistence and/or better potency in a subject to which it is administered. In some instances, the persistence of genetically engineered cells, such as CAR-expressing T cells, in the subject is greater as compared to that which would be achieved by alternative methods, such as those involving administration of a reference cell composition, e.g. administration of the T cell therapy but in the absence of administration of the immunomodulatory compound. In some instances, the persistence is increased at least or about at least 1.5-fold, 2-fold, 3-fold, 4-fold, 5-fold, 6-fold, 7-fold, 8-fold, 9-fold, 10-fold, 20-fold, 30-fold, 50-fold, 60-fold, 70-fold, 80-fold, 90-fold, 100-fold or more.

In some instances, the degree or extent of persistence of administered cells can be detected or quantified after administration to a subject. For example, in some aspects, quantitative PCR (qPCR) is used to assess the quantity of cells expressing the recombinant receptor (e.g., CAR-expressing cells) in the blood or serum or organ or tissue (e.g., disease site) of the subject. In some aspects, persistence is quantified as copies of DNA or plasmid encoding the receptor, e.g., CAR, per microgram of DNA, or as the number of receptor-expressing, e.g., CAR-expressing, cells per microliter of the sample, e.g., of blood or serum, or per total number of peripheral blood mononuclear cells (PBMCs) or white blood cells or T cells per microliter of the sample. In some instances, flow cytometric assays detecting cells expressing the receptor generally using antibodies specific for the receptors also can be performed. Cell-based assays may also be used to detect the number or percentage of functional cells, such as cells capable of binding to and/or neutralizing and/or inducing responses, e.g., cytotoxic responses, against cells of the disease or condition or expressing the antigen recognized by the receptor. In any of such instances, the extent or level of expression of another marker associated with the recombinant receptor (e.g. CAR-expressing cells) can be used to distinguish the administered cells from endogenous cells in a subject.

Also disclosed are methods for engineering, preparing, and producing the cells, compositions containing the cells and/or immunomodulatory compound, and kits and devices containing and for using, producing and administering the cells and/or immunomodulatory compound, such as in accord with the disclosed combination therapy methods.

The section heading used herein are for organizational purposes only and are not to be construed as limiting the subject matter described.

### I. COMBINATION THERAPY

Disclosed herein are methods for combination therapy for treating a disease or disorder, e.g. a cancer or proliferative disease, that includes administering to a subject a combination therapy of 1) an immunomodulatory compound, such as a structural or functional analog or derivative of thalidomide and/or an inhibitor of E3 ubiquitin ligase, e.g. lenalidomide, and 2) a T cell therapy, e.g. CAR-expressing cell, e.g. T cells. In some instances, the T cell therapy is an adoptive immune cell therapy comprising T cells that specifically recognize and/or target an antigen associated with a disease or disorder, e.g. a cancer or proliferative disease. Also disclosed are combinations and articles of manufacture, such as kits, that contain a composition comprising the T cell therapy and/or a composition comprising the immunomodulatory compound, and uses of such compositions and combinations to treat or prevent diseases, conditions, and disorders, including cancers.

In some instances, such methods can include administration of the immunomodulatory compound, such as a structural or functional analog or derivative of thalidomide and/or an inhibitor of E3 ubiquitin ligase, e.g. lenalidomide, prior to, simultaneously with, during, during the course of (including once and/or periodically during the course of), and/or subsequently to, the administration (e.g., initiation of administration) of the T cell therapy (e.g. CAR-expressing T cells). In some instances, the administrations can involve sequential or intermittent administrations of the immunomodulatory compound and T cell therapy.

In some instances, the cell therapy is adoptive cell therapy. In some instances, the cell therapy is or comprises a tumor infiltrating lymphocytic (TIL) therapy, a transgenic TCR therapy or a recombinant-receptor expressing cell therapy (optionally T cell therapy), which optionally is a chimeric antigen receptor (CAR)-expressing cell therapy. In some instances, the therapy is a B cell targeted therapy. In some instances, the therapy targets B cell maturation antigen (BCMA). In some instances, the therapy targets CD19. In some instances, the cells and dosage regimens for administering the cells can include any as described in the following subsection A under "Administration of T Cell therapy."

In some instances, the immunomodulatory compound potentiates T-cell functionality. In some instances, the immunomodulatory compound drives anti-myeloma activity. In some instances, the immunomodulatory compound alters the suppressive microenvironment. In some instances, the immunomodulatory compound is a structural or functional analog or derivative of thalidomide. In some instances, the immunomodulatory compound is an inhibitor of E3 ubiquitin ligase. In some instances, the immunomodulatory compound is lenalidomide or a compound with the same or similar properties of lenalidomide, including analogs or derivatives, a stereoisomer of lenalidomide or a pharmaceutically acceptable salt, solvate, hydrate, co-crystal, clathrate, or polymorph thereof. In some instances, the dosage regimens for administering the immunomodulatory compound can include any as described in the following subsection B under "Administration of the Immunomodulatory Compound."

In some instances, the T cell therapy (e.g. CAR-expressing T cells) and immunomodulatory compound are provided as pharmaceutical compositions for administration to the subject. In some instances, the pharmaceutical compositions contain therapeutically effective amounts of one or both of the agents for combination therapy, *e.g.,* T cells for adoptive cell therapy and an immunomodulatory compound as described. In some instances, the agents are formulated for administration in separate pharmaceutical compositions. In some instances, any of the pharmaceutical compositions disclosed herein can be formulated in dosage forms appropriate for each route of administration.

In some instances, the combination therapy, which includes administering the T cell therapy, including engineered cells, such as CAR-T cell therapy, and the immunomodulatory compound is administered to a subject or patient having a disease or condition to be treated (*e.g.* cancer) or at risk for having the disease or condition (*e.g.* cancer). In some aspects, the methods treat, *e.g.,* ameliorate one or more symptom of, the disease or condition, such as by lessening tumor burden in a cancer expressing an antigen recognized by the immunotherapy or immunotherapeutic agent, e.g. recognized by an engineered T cell.

In some instances, the disease or condition that is treated can be any in which expression of an antigen is associated with and/or involved in the etiology of a disease condition or disorder, *e.g.* causes, exacerbates or otherwise is involved in such disease, condition, or disorder. Exemplary diseases and conditions can include diseases or conditions associated with malignancy or transformation of cells (*e.g.* cancer), autoimmune or inflammatory disease, or an infectious disease, *e.g.* caused by bacterial, viral or other pathogens. Exemplary antigens, which include antigens associated with various diseases and conditions that can be treated, include any of antigens described herein. In particular instances, the recombinant receptor expressed on engineered cells of a combination therapy, including a chimeric antigen receptor or transgenic TCR, specifically binds to an antigen associated with the disease or condition.

In some instances, the disease or condition is a tumor, such as a solid tumor, lymphoma, leukemia, blood tumor, metastatic tumor, or other cancer or tumor type.

In some instances, the cancer or proliferative disease is a B cell malignancy or hematological malignancy. In some instances the cancer or proliferative disease is lymphoblastic leukemia (ALL), non-Hodgkin's lymphoma (NHL), or chronic lymphocytic leukemia (CLL). In some instances, the cancer is CLL. In some instances, the methods can be used to treat a myeloma, a lymphoma or a leukemia. In some instances, the methods can be used to treat a non-Hodgkin lymphoma (NHL), an acute lymphoblastic leukemia (ALL), a chronic lymphocytic leukemia (CLL), a diffuse large B-cell lymphoma (DLBCL), acute myeloid leukemia (AML), or a myeloma, *e.g.,* a multiple myeloma (MM). In some instances, the methods can be used to treat a MM or a DBCBL.

In some instances, the antigen associated with the disease or disorder is selected from the group consisting of ROR1, B cell maturation antigen (BCMA), tEGFR, Her2, Ll-CAM, CD19, CD20, CD22, mesothelin, CEA, and hepatitis B surface antigen, anti-folate receptor, CD23, CD24, CD30, CD33, CD38, CD44, EGFR, EGP-2, EGP-4, EPHa2, ErbB2, 3, or 4, erbB dimers, EGFR vIII, FBP, FCRL5, FCRH5, fetal acethycholine e receptor, GD2, GD3, HMW-MAA, IL-22R-alpha, IL-13R-alpha2, kdr, kappa light chain, Lewis Y, L1-cell adhesion molecule, (L1-CAM), Melanoma-associated antigen (MAGE)-A1, MAGE-A3, MAGE-A6, Preferentially expressed antigen of melanoma (PRAME), survivin, EGP2, EGP40, TAG72, B7-H6, IL-13 receptor a2 (IL-13Ra2), CA9, GD3, HMW-MAA, CD171, G250/CAIX, HLA-AI MAGE Al, HLA-A2 NY-ESO-1, PSCA, folate receptor-a, CD44v6, CD44v7/8, avb6 integrin, 8H9, NCAM, VEGF receptors, 5T4, Foetal AchR, NKG2D ligands, CD44v6, dual antigen, and an antigen associated with a universal tag, a cancer-testes antigen, mesothelin, MUC1, MUC16, PSCA, NKG2D Ligands, NY-ESO-1, MART-1, gp100, G Protein Coupled Receptor 5D (GPCR5D), oncofetal antigen, ROR1, TAG72, VEGF-R2, carcinoembryonic antigen (CEA), prostate specific antigen, PSMA, Her2/neu, estrogen receptor, progesterone receptor, ephrinB2, CD123, c-Met, GD-2, O-acetylated GD2 (OGD2), CE7, Wilms Tumor 1 (WT-1), a cyclin, cyclin A2, CCL-1, CD138, and a pathogen-specific antigen. In some instances, the antigen is associated with or is a universal tag.

In some instances the cancer or proliferative disease expresses BCMA. In some instances, the disclosed methods employ a recombinant receptor-expressing T cell (*e.g.* CAR-T cell) that targets BCMA.

In some instances, the methods can be used to treat a non-hematologic cancer, such as a solid tumor. In some instances, the methods can be used to treat a bladder, lung, brain, melanoma (*e.g.* small-cell lung, melanoma), breast, cervical, ovarian, colorectal, pancreatic, endometrial, esophageal, kidney, liver, prostate, skin, thyroid, or uterine cancers. In some instances, the cancer or proliferative disease is cancer is a pancreatic cancer, bladder cancer, colorectal cancer, breast cancer, prostate cancer, renal cancer, hepatocellular cancer, lung cancer, ovarian cancer, cervical cancer, pancreatic cancer, rectal cancer, thyroid cancer, uterine cancer, gastric cancer, esophageal cancer, head and neck cancer, melanoma, neuroendocrine cancers, CNS cancers, brain tumors, bone cancer, or soft tissue sarcoma.

In some instances, the disease or condition is an infectious disease or condition, such as, but not limited to, viral, retroviral, bacterial, and protozoal infections, immunodeficiency, Cytomegalovirus (CMV), Epstein-Barr virus (EBV), adenovirus, BK polyomavirus. In some instances, the disease or condition is an autoimmune or inflammatory disease or condition, such as arthritis, *e.g.,* rheumatoid arthritis (RA), Type I diabetes, systemic lupus erythematosus (SLE), inflammatory bowel disease, psoriasis, scleroderma, autoimmune thyroid disease, Graves disease, Crohn's disease, multiple sclerosis, asthma, and/or a disease or condition associated with transplant.

For the prevention or treatment of disease, the appropriate dosage of immunomodulatory compound (e.g., lenalidomide) and/or immunotherapy, such as a T cell therapy (*e.g.* CAR-expressing T cells), may depend on the type of disease to be treated, the particular immunomodulatory compound, cells and/or recombinant receptors expressed on the cells, the severity and course of the disease, route of administration, whether the immunomodulatory compound and/or the T cell therapy are administered for preventive or therapeutic purposes, previous therapy, frequency of administration, the subject's clinical history and response to the cells, and the discretion of the attending physician. The compositions and cells are in some instances suitably administered to the subject at one time or over a series of treatments. Exemplary dosage regimens and schedules for the provided combination therapy are described.

In some instances, the T cell therapy and the immunomodulatory compound are administered as part of a further combination treatment, which can be administered simultaneously with or sequentially to, in any order, another therapeutic intervention. In some contexts, the T cell therapy, e.g. engineered T cells, such as CAR-expressing T cells, are co-administered with another therapy sufficiently close in time such that the T cell therapy enhances the effect of one or more additional therapeutic agents, or vice versa. In some instances, the cells are administered prior to the one or more additional therapeutic agents. In some instances, the T cell therapy, e.g. engineered T cells, such as CAR-expressing T cells, are administered after the one or more additional therapeutic agents. In some instances, the combination therapy methods further include a lymphodepleting therapy, such as administration of a chemotherapeutic agent. In some instances, the combination therapy further comprises administering another therapeutic agent, such as an anti-cancer agent, a checkpoint inhibitor, or another immune modulating agent. Uses include uses of the combination therapies in such methods and treatments, and uses of such compositions in the preparation of a medicament in order to carry out such combination therapy methods. In some instances, the methods and uses thereby treat the disease or condition or disorder, such as a cancer or proliferative disease, in the subj ect.

Prior to, during or following administration of the immunotherapy (*e.g.* T cell therapy, such as CAR-T cell therapy) and/or an immunomodulatory compound, the biological activity of the T cell therapy, *e.g.* the biological activity of the engineered cell populations, in some instances is measured, *e.g.,* by any of a number of known methods. Parameters to assess include the ability of the engineered cells to destroy target cells, persistence and other measures of T cell activity, such as measured using any suitable method known in the art, such as assays described further below in Section III. In some instances, the biological activity of the cells, *e.g.,* T cells administered for the T cell based therapy, is measured by assaying cytotoxic cell killing, expression and/or secretion of one or more cytokines, proliferation or expansion, such as upon restimulation with antigen. In some aspects the biological activity is measured by assessing the disease burden and/or clinical outcome, such as reduction in tumor burden or load. In some instances, administration of one or both agents of the combination therapy and/or any repeated administration of the therapy, can be determined based on the results of the assays before, during, during the course of or after administration of one or both agents of the combination therapy.

In some instances, the combined effect of the immunomodulatory compound in combination with the cell therapy can be synergistic compared to treatments involving only the immunomodulatory compound or monotherapy with the cell therapy. For example, in some instances, the methods disclosed herein result in an increase or an improvement in a desired therapeutic effect, such as an increased or an improvement in the reduction or inhibition of one or more symptoms associated with cancer.

In some instances, the immunomodulatory compound increases the expansion or proliferation of the engineered T cells, such as CAR T-Cells. In some instances, the increase in expansion or proliferation is observed in vivo upon administration to a subject. In some instances, the increase in the number of engineered T cells, e.g. CAR-T cells, is increased by greater than or greater than about 1.2-fold, 1.5-fold, 2.0-fold, 3.0-fold, 4.0-fold, 5.0-fold, 6.0-fold, 7.0-fold, 8.0-fold, 9.0-fold, 10.0 fold or more.

### A. ADMINISTRATION OF T CELL THERAPY

In some instances of the methods, compositions, combinations, kits and uses disclosed herein, the combination therapy includes administering to a subject an immune cell therapy, such as a T cell therapy (*e.g.* CAR-expressing T cells). Administration of such therapies can be initiated prior to, subsequent to, simultaneously with administration of one or more immunomodulatory compound as described.

In some instances, the cell-based therapy is or comprises administration of cells, such as immune cells, for example T cell or NK cells, that target a molecule expressed on the surface of a lesion, such as a tumor or a cancer. In some instances, the immune cells express a T cell receptor (TCR) or other antigen-binding receptor. In some instances, the immune cells express a recombinant receptor, such as a transgenic TCR or a chimeric antigen receptor (CAR). In some instances, the cells are autologous to the subject. In some instances, the cells are allogeneic to the subject.

In some aspects, the T cell therapy is or comprises a tumor infiltrating lymphocytic (TIL) therapy, a transgenic TCR therapy or a T cell therapy comprising genetically engineered cells, such as a recombinant-receptor expressing cell therapy. In some instances, the recombinant receptor specifically binds to a ligand, such as one associated with a disease or condition, *e.g.* associated with or expressed on a cell of a tumor or cancer. In some instances, the T cell therapy includes administering T cells engineered to express a chimeric antigen receptor (CAR).

In some instances, the provided cells express and/or are engineered to express receptors, such as recombinant receptors, including those containing ligand-binding domains or binding fragments thereof, and T cell receptors (TCRs) and components thereof, and/or functional non-TCR antigen receptors, such as chimeric antigen receptors (CARs). In some instances, the recombinant receptor contains an extracellular ligand-binding domain that specifically binds to an antigen. In some instances, the recombinant receptor is a CAR that contains an extracellular antigen-recognition domain that specifically binds to an antigen. In some instances, the ligand, such as an antigen, is a protein expressed on the surface of cells. In some instances, the CAR is a TCR-like CAR and the antigen is a processed peptide antigen, such as a peptide antigen of an intracellular protein, which, like a TCR, is recognized on the cell surface in the context of a major histocompatibility complex (MHC) molecule.

Among the engineered cells, including engineered cells containing recombinant receptors, are described in Section II below. Exemplary recombinant receptors, including CARs and recombinant TCRs, as well as methods for engineering and introducing the receptors into cells, include those described, for example, in international patent application publication numbers WO200014257, WO2013126726, WO2012/129514, WO2014031687, WO2013/166321, WO2013/071154, WO2013/123061, WO2016/0046724, WO2016/014789, WO2016/090320, WO2016/094304, WO2017/025038, WO2017/173256, U.S. patent application publication numbers US2002131960, US2013287748, US20130149337, U.S. Patent Nos. 6,451,995, 7,446,190, 8,252,592, 8,339,645, 8,398,282, 7,446,179, 6,410,319, 7,070,995, 7,265,209, 7,354,762, 7,446,191, 8,324,353, 8,479,118, and 9,765,342, and European patent application number EP2537416, and/or those described by Sadelain et al., Cancer Discov., 3(4): 388-398 (2013); Davila et al., PLoS ONE 8(4): e61338 (2013); Turtle et al., Curr. Opin. Immunol., 24(5): 633-39 (2012); Wu et al., Cancer, 18(2): 160-75 (2012). In some aspects, the genetically engineered antigen receptors include a CAR as described in U.S. Patent No.: 7,446,190, and those described in International Patent Application Publication No.: WO/2014055668 A1.

In some instances, the antigen is or includes αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), truncated epidermal growth factor protein (tEGFR), type III epidermal growth factor receptor mutation (EGFR vIII), epithelial glycoprotein 2 (EPG-2), epithelial glycoprotein 40 (EPG-40), ephrinB2, ephrine receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPCR5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(II,-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some instances include antigens associated with a B cell malignancy, such as any of a number of known B cell marker. In some instances, the antigen is or includes CD20, CD19, CD22, ROR1, CD45, CD21, CD5, CD33, Igkappa, Iglambda, CD79a, CD79b or CD30.

In some instances, the antigen is or includes a pathogen-specific or pathogen-expressed antigen. In some instances, the antigen is a viral antigen (such as a viral antigen from HIV, HCV, HBV, etc.), bacterial antigens, and/or parasitic antigens.

In some instances, the combination therapy includes administration to a subject cells, e.g. T cells, expressing a recombinant receptor that specifically recognize and/or target an antigen associated with the cancer and/or present on a universal tag . In some instances, the antigen recognized or targeted by the T cells is ROR1, B cell maturation antigen (BCMA), carbonic anhydrase 9 (CAIX), tEGFR, Her2/neu (receptor tyrosine kinase erbB2), L1-CAM, CD19, CD20, CD22, mesothelin, CEA, and hepatitis B surface antigen, anti-folate receptor, CD23, CD24, CD30, CD33, CD38, CD44, EGFR, epithelial glycoprotein 2 (EPG-2), epithelial glycoprotein 40 (EPG-40), EPHa2, erb-B2, erb-B3, erb-B4, erbB dimers, EGFR vIII, folate binding protein (FBP), FCRL5, FCRH5, fetal acetylcholine receptor, GD2, GD3, HMW-MAA, IL-22R-alpha, IL-13R-alpha2, kinase insert domain receptor (kdr), kappa light chain, Lewis Y, L1-cell adhesion molecule, (L1-CAM), Melanoma-associated antigen (MAGE)-A1, MAGE-A3, MAGE-A6, Preferentially expressed antigen of melanoma (PRAME), survivin, TAG72, B7-H6, IL-13 receptor alpha 2 (IL-13Ra2), CA9, GD3, HMW-MAA, CD171, G250/CAIX, HLA-AI MAGE Al, HLA-A2, PSCA, folate receptor-a, CD44v6, CD44v7/8, avb6 integrin, 8H9, NCAM, VEGF receptors, 5T4, Foetal AchR, NKG2D ligands, CD44v6, dual antigen, a cancer-testes antigen, mesothelin, murine CMV, mucin 1 (MUC1), MUC16, PSCA, NKG2D, NY-ESO-1, MART-1, gp100, G Protein Coupled Receptor 5D (GPCR5D), oncofetal antigen, ROR1, TAG72, VEGF-R2, carcinoembryonic antigen (CEA), Her2/neu, estrogen receptor, progesterone receptor, ephrinB2, CD123, c-Met, GD-2, O-acetylated GD2 (OGD2), CE7, Wilms Tumor 1 (WT-1), a cyclin, cyclin A2, CCL-1, CD138, optionally a human antigen of any of the foregoing; a pathogen-specific antigen.

Methods for administration of engineered cells for adoptive cell therapy are known and may be used in connection with the disclosed methods and compositions. For example, adoptive T cell therapy methods are described, *e.g.,* in US Patent Application Publication No. 2003/0170238 to Gruenberg et al; US Patent No. 4,690,915 to Rosenberg; Rosenberg (2011) Nat Rev Clin Oncol. 8(10):577-85). See, *e.g.,* Themeli et al., (2013) Nat Biotechnol. 31(10): 928-933; Tsukahara et al., (2013) Biochem Biophys Res Commun 438(1): 84-9; Davila et al., (2013) PLoS ONE 8(4): e61338.

In some instances, the cell therapy, e.g., adoptive T cell therapy, is carried out by autologous transfer, in which the cells are isolated and/or otherwise prepared from the subject who is to receive the cell therapy, or from a sample derived from such a subject. Thus, in some aspects, the cells are derived from a subject, e.g., patient, in need of a treatment and the cells, following isolation and processing are administered to the same subject.

In some instances, the cell therapy, *e.g.,* adoptive T cell therapy, is carried out by allogeneic transfer, in which the cells are isolated and/or otherwise prepared from a subject other than a subject who is to receive or who ultimately receives the cell therapy, *e.g.,* a first subject. In such instances, the cells then are administered to a different subject, e.g., a second subject, of the same species. In some instances, the first and second subjects are genetically identical. In some instances, the first and second subjects are genetically similar. In some instances, the second subject expresses the same HLA class or supertype as the first subject.

In certain instances, the cells, or individual populations of sub-types of cells, are administered to the subject at a range of about one million to about 100 billion cells and/or that amount of cells per kilogram of body weight, such as, e.g., 1 million to about 50 billion cells (e.g., about 5 million cells, about 25 million cells, about 500 million cells, about 1 billion cells, about 5 billion cells, about 20 billion cells, about 30 billion cells, about 40 billion cells, or a range defined by any two of the foregoing values), such as about 10 million to about 100 billion cells (e.g., about 20 million cells, about 30 million cells, about 40 million cells, about 60 million cells, about 70 million cells, about 80 million cells, about 90 million cells, about 10 billion cells, about 25 billion cells, about 50 billion cells, about 75 billion cells, about 90 billion cells, or a range defined by any two of the foregoing values), and in some cases about 100 million cells to about 50 billion cells (e.g., about 120 million cells, about 250 million cells, about 350 million cells, about 450 million cells, about 650 million cells, about 800 million cells, about 900 million cells, about 3 billion cells, about 30 billion cells, about 45 billion cells) or any value in between these ranges and/or per kilogram of body weight. Dosages may vary depending on attributes particular to the disease or disorder and/or patient and/or other treatments.

In some instances, for example, where the subject is a human, the dose includes fewer than about 1 × 10⁸ total recombinant receptor (e.g., CAR)-expressing cells, T cells, or peripheral blood mononuclear cells (PBMCs), e.g., in the range of about 1 × 10⁶ to 1 × 10⁸ such cells, such as 2 × 10⁶, 5 × 10⁶, 1 × 10⁷, 5 × 10⁷, or 1 × 10⁸ or total such cells, or the range between any two of the foregoing values.

The cells can be administered by any suitable means. The cells are administered in a dosing regimen to achieve a therapeutic effect, such as a reduction in tumor burden. Dosing and administration may depend in part on the schedule of administration of the immunomodulatory compound, which can be administered prior to, subsequent to and/or simultaneously with initiation of administration of the T cell therapy. Various dosing schedules of the T cell therapy include but are not limited to single or multiple administrations over various time-points, bolus administration, and pulse infusion.

### 1. Compositions and formulations

In some instances, the dose of cells of the T cell therapy, such a T cell therapy comprising cells engineered with a recombinant antigen receptor, *e.g.* CAR or TCR, is provided as a composition or formulation, such as a pharmaceutical composition or formulation. Such compositions can be used in accord with the disclosed methods, such as in the prevention or treatment of diseases, conditions, and disorders.

In some instances, the T cell therapy, such as engineered T cells (*e.g.* CAR T cells), are formulated with a pharmaceutically acceptable carrier. In some aspects, the choice of carrier is determined in part by the particular cell or agent and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001% to about 2% by weight of the total composition. Carriers are described, *e.g.,* by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.* Zn-protein complexes); and/or non-ionic surfactants such as polyethylene glycol (PEG).

Buffering agents in some aspects are included in the compositions. Suitable buffering agents include, for example, citric acid, sodium citrate, phosphoric acid, potassium phosphate, and various other acids and salts. In some aspects, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

The formulations can include aqueous solutions. The formulation or composition may also contain more than one active ingredient useful for the particular indication, disease, or condition being prevented or treated with the cells or agents, where the respective activities do not adversely affect one another. Such active ingredients are suitably present in combination in amounts that are effective for the purpose intended. Thus, in some instances, the pharmaceutical composition further includes other pharmaceutically active agents or drugs, such as chemotherapeutic agents, *e.g.,* asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc.

The pharmaceutical composition in some instances contains cells in amounts effective to treat or prevent the disease or condition, such as a therapeutically effective or prophylactically effective amount. Therapeutic or prophylactic efficacy in some instances is monitored by periodic assessment of treated subjects. For repeated administrations over several days or longer, depending on the condition, the treatment is repeated until a desired suppression of disease symptoms occurs. However, other dosage regimens may be useful and can be determined. The desired dosage can be delivered by a single bolus administration of the composition, by multiple bolus administrations of the composition, or by continuous infusion administration of the composition.

The cells may be administered using standard administration techniques, formulations, and/or devices. Disclosed are formulations and devices, such as syringes and vials, for storage and administration of the compositions. With respect to cells, administration can be autologous or heterologous. For example, immunoresponsive cells or progenitors can be obtained from one subject, and administered to the same subject or a different, compatible subject. Peripheral blood derived immunoresponsive cells or their progeny (e.g., in vivo, ex vivo or in vitro derived) can be administered via localized injection, including catheter administration, systemic injection, localized injection, intravenous injection, or parenteral administration. When administering a therapeutic composition (e.g., a pharmaceutical composition containing a genetically modified immunoresponsive cell), it will generally be formulated in a unit dosage injectable form (solution, suspension, emulsion).

Formulations include those for oral, intravenous, intraperitoneal, subcutaneous, pulmonary, transdermal, intramuscular, intranasal, buccal, sublingual, or suppository administration. In some instances, the agent or cell populations are administered parenterally. The term "parenteral," as used herein, includes intravenous, intramuscular, subcutaneous, rectal, vaginal, and intraperitoneal administration. In some instances, the agent or cell populations are administered to a subject using peripheral systemic delivery by intravenous, intraperitoneal, or subcutaneous injection.

Compositions in some instances are provided as sterile liquid preparations, e.g., isotonic aqueous solutions, suspensions, emulsions, dispersions, or viscous compositions, which may in some aspects be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol) and suitable mixtures thereof.

Sterile injectable solutions can be prepared by incorporating the cells in a solvent, such as in admixture with a suitable carrier, diluent, or excipient such as sterile water, physiological saline, glucose, dextrose, or the like. The compositions can also be lyophilized. The compositions can contain auxiliary substances such as wetting, dispersing, or emulsifying agents (e.g., methylcellulose), pH buffering agents, gelling or viscosity enhancing additives, preservatives, flavoring agents, colors, and the like, depending upon the route of administration and the preparation desired. Standard texts may in some aspects be consulted to prepare suitable preparations.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. Prolonged absorption of the injectable pharmaceutical form can be brought about by the use of agents delaying absorption, for example, aluminum monostearate and gelatin.

The formulations to be used for in vivo administration are generally sterile. Sterility may be readily accomplished, e.g., by filtration through sterile filtration membranes.

For the prevention or treatment of disease, the appropriate dosage may depend on the type of disease to be treated, the type of agent or agents, the type of cells or recombinant receptors, the severity and course of the disease, whether the agent or cells are administered for preventive or therapeutic purposes, previous therapy, the subject's clinical history and response to the agent or the cells, and the discretion of the attending physician. The compositions are in some instances suitably administered to the subject at one time or over a series of treatments.

In some cases, the cell therapy is administered as a single pharmaceutical composition comprising the cells. In some instances, a given dose is administered by a single bolus administration of the cells or agent. In some instances, it is administered by multiple bolus administrations of the cells or agent, for example, over a period of no more than 3 days, or by continuous infusion administration of the cells or agent.

### 2. Dosage Schedule and administration

In some instances, a dose of cells is administered to subjects in accord with the disclosed combination therapy methods. In some instances, the size or timing of the doses is determined as a function of the particular disease or condition in the subject. One may empirically determine the size or timing of the doses for a particular disease in view of the provided description.

In certain instances, the cells, or individual populations of sub-types of cells, are administered to the subject at a range of about 0.1 million to about 100 billion cells and/or that amount of cells per kilogram of body weight of the subject, such as, *e.g.,* 0.1 million to about 50 billion cells (*e.g.,* about 5 million cells, about 25 million cells, about 500 million cells, about 1 billion cells, about 5 billion cells, about 20 billion cells, about 30 billion cells, about 40 billion cells, or a range defined by any two of the foregoing values), 1 million to about 50 billion cells (*e.g.,* about 5 million cells, about 25 million cells, about 500 million cells, about 1 billion cells, about 5 billion cells, about 20 billion cells, about 30 billion cells, about 40 billion cells, or a range defined by any two of the foregoing values), such as about 10 million to about 100 billion cells (*e.g.,* about 20 million cells, about 30 million cells, about 40 million cells, about 60 million cells, about 70 million cells, about 80 million cells, about 90 million cells, about 10 billion cells, about 25 billion cells, about 50 billion cells, about 75 billion cells, about 90 billion cells, or a range defined by any two of the foregoing values), and in some cases about 100 million cells to about 50 billion cells (*e.g.,* about 120 million cells, about 250 million cells, about 350 million cells, about 450 million cells, about 650 million cells, about 800 million cells, about 900 million cells, about 3 billion cells, about 30 billion cells, about 45 billion cells) or any value in between these ranges and/or per kilogram of body weight of the subject. Dosages may vary depending on attributes particular to the disease or disorder and/or patient and/or other treatments. In some instances, such values refer to numbers of recombinant receptor-expressing cells; in other instances, they refer to number of T cells or PBMCs or total cells administered.

In some instances, the cell therapy comprises administration of a dose comprising a number of cell from or from about 1 × 10⁵ to 1 × 10⁸ total recombinant receptor-expressing cells, total T cells, or total peripheral blood mononuclear cells (PBMCs), from or from about 5 × 10⁵ to 1 × 10⁷ total recombinant receptor-expressing cells, total T cells, or total peripheral blood mononuclear cells (PBMCs) or from or from about 1 × 10⁶ to 1 × 10⁷ total recombinant receptor-expressing cells, total T cells, or total peripheral blood mononuclear cells (PBMCs), each inclusive. In some instances, the cell therapy comprises administration of a dose of cells comprising a number of cells at least or about at least 1 × 10⁵ total recombinant receptor-expressing cells, total T cells, or total peripheral blood mononuclear cells (PBMCs), such at least or at least 1 × 10⁶, at least or about at least 1 × 10⁷, at least or about at least 1 × 10⁸ of such cells.

In some instances, for example, where the subject is a human, the dose includes fewer than about 5 × 10⁸ total recombinant receptor (e.g., CAR)-expressing cells, T cells, or peripheral blood mononuclear cells (PBMCs), e.g., in the range of about 1 × 10⁶ to 5 × 10⁸ such cells, such as 2 × 10⁶, 5 × 10⁶, 1 × 10⁷, 5 × 10⁷, 1× 10⁸, or 5 × 10⁸ total such cells, or the range between any two of the foregoing values.

In some instances, the number is with reference to the total number of CD3+ or CD8+, in some cases also recombinant receptor-expressing (e.g. CAR+) cells. In some instances, the cell therapy comprises administration of a dose comprising a number of cell from or from about 1 × 10⁵ to 1 × 10⁸ CD3+ or CD8+ total T cells or CD3+ or CD8+ recombinant receptor-expressing cells, from or from about 5 × 10⁵ to 1 × 10⁷ CD3+ or CD8+ total T cells or CD3+ or CD8+ recombinant receptor-expressing cells, or from or from about 1 × 10⁶ to 1 × 10⁷ CD3+ or CD8+ total T cells or CD3+ or CD8+recombinant receptor-expressing cells, each inclusive. In some instances, the cell therapy comprises administration of a dose comprising a number of cell from or from about 1 × 10⁵ to 1 × 10⁸ total CD3+/CAR+ or CD8+/CAR+ cells, from or from about 5 × 10⁵ to 1 × 10⁷ total CD3+/CAR+ or CD8+/CAR+ cells, or from or from about 1 × 10⁶ to 1 × 10⁷ total CD3+/CAR+ or CD8+/CAR+ cells, each inclusive.

In some instances, the dose of genetically engineered cells comprises from or from about 1 × 10⁵ to 5 × 10⁸ total CAR-expressing T cells, 1 × 10⁵ to 2.5 × 10⁸ total CAR-expressing T cells, 1 × 10⁵ to 1 × 10⁸ total CAR-expressing T cells, 1 × 10⁵ to 5 × 10⁷ total CAR-expressing T cells, 1 × 10⁵ to 2.5 × 10⁷ total CAR-expressing T cells, 1 × 10⁵ to 1 × 10⁷ total CAR-expressing T cells, 1 × 10⁵ to 5 × 10⁶ total CAR-expressing T cells, 1 × 10⁵ to 2.5 × 10⁶ total CAR-expressing T cells, 1 × 10⁵ to 1 × 10⁶ total CAR-expressing T cells, 1 × 10⁶ to 5 × 10⁸ total CAR-expressing T cells, 1 × 10⁶ to 2.5 × 10⁸ total CAR-expressing T cells, 1 × 10⁶ to 1 × 10⁸ total CAR-expressing T cells, 1 × 10⁶ to 5 × 10⁷ total CAR-expressing T cells, 1 × 10⁶ to 2.5 × 10⁷ total CAR-expressing T cells, 1 × 10⁶ to 1 × 10⁷ total CAR-expressing T cells, 1 × 10⁶ to 5 × 10⁶ total CAR-expressing T cells, 1 × 10⁶ to 2.5 × 10⁶ total CAR-expressing T cells, 2.5 × 10⁶ to 5 × 10⁸ total CAR-expressing T cells, 2.5 × 10⁶ to 2.5 × 10⁸ total CAR-expressing T cells, 2.5 × 10⁶ to 1 × 10⁸ total CAR-expressing T cells, 2.5 × 10⁶ to 5 × 10⁷ total CAR-expressing T cells, 2.5 × 10⁶ to 2.5 × 10⁷ total CAR-expressing T cells, 2.5 × 10⁶ to 1 × 10⁷ total CAR-expressing T cells, 2.5 × 10⁶ to 5 × 10⁶ total CAR-expressing T cells, 5 × 10⁶ to 5 × 10⁸ total CAR-expressing T cells, 5 × 10⁶ to 2.5 × 10⁸ total CAR-expressing T cells, 5 × 10⁶ to 1 × 10⁸ total CAR-expressing T cells, 5 × 10⁶ to 5 × 10⁷ total CAR-expressing T cells, 5 × 10⁶ to 2.5 × 10⁷ total CAR-expressing T cells, 5 × 10⁶ to 1 × 10⁷ total CAR-expressing T cells, 1 × 10⁷ to 5 × 10⁸ total CAR-expressing T cells, 1 × 10⁷ to 2.5 × 10⁸ total CAR-expressing T cells, 1 × 10⁷ to 1 × 10⁸ total CAR-expressing T cells, 1 × 10⁷ to 5 × 10⁷ total CAR-expressing T cells, 1 × 10⁷ to 2.5 × 10⁷ total CAR-expressing T cells, 2.5 × 10⁷ to 5 × 10⁸ total CAR-expressing T cells, 2.5 × 10⁷ to 2.5 × 10⁸ total CAR-expressing T cells, 2.5 × 10⁷ to 1 × 10⁸ total CAR-expressing T cells, 2.5 × 10⁷ to 5 × 10⁷ total CAR-expressing T cells, 5 × 10⁷ to 5 × 10⁸ total CAR-expressing T cells, 5 × 10⁷ to 2.5 × 10⁸ total CAR-expressing T cells, 5 × 10⁷ to 1 × 10⁸ total CAR-expressing T cells, 1 × 10⁸ to 5 × 10⁸ total CAR-expressing T cells, 1 × 10⁸ to 2.5 × 10⁸ total CAR-expressing T cells, or 2.5 × 10⁸ to 5 × 10⁸ total CAR-expressing T cells.

In some instances, the dose of genetically engineered cells comprises at least or at least about 1 × 10⁵ CAR-expressing cells, at least or at least about 2.5 × 10⁵ CAR-expressing cells, at least or at least about 5 × 10⁵ CAR-expressing cells, at least or at least about 1 × 10⁶ CAR-expressing cells, at least or at least about 2.5 × 10⁶ CAR-expressing cells, at least or at least about 5 × 10⁶ CAR-expressing cells, at least or at least about 1 × 10⁷ CAR-expressing cells, at least or at least about 2.5 × 10⁷ CAR-expressing cells, at least or at least about 5 × 10⁷ CAR-expressing cells, at least or at least about 1 × 10⁸ CAR-expressing cells, at least or at least about 2.5 × 10⁸ CAR-expressing cells, or at least or at least about 5 × 10⁸ CAR-expressing cells.

In some instances, the cell therapy comprises administration of a dose comprising a number of cell from or from about 1 × 10⁵ to 5 × 10⁸ total recombinant receptor-expressing cells, total T cells, or total peripheral blood mononuclear cells (PBMCs), from or from about 5 × 10⁵ to 1 × 10⁷ total recombinant receptor-expressing cells, total T cells, or total peripheral blood mononuclear cells (PBMCs) or from or from about 1 × 10⁶ to 1 × 10⁷ total recombinant receptor-expressing cells, total T cells, or total peripheral blood mononuclear cells (PBMCs), each inclusive. In some instances, the cell therapy comprises administration of a dose of cells comprising a number of cells at least or at least about 1 × 10⁵ total recombinant receptor-expressing cells, total T cells, or total peripheral blood mononuclear cells (PBMCs), such at least or at least 1 × 10⁶, at least or at least about 1 × 10⁷, at least or at least about 1 × 10⁸ of such cells. In some instances, the number is with reference to the total number of CD3+ or CD8+, in some cases also recombinant receptor-expressing (e.g. CAR+) cells. In some instances, the cell therapy comprises administration of a dose comprising a number of cell from or from about 1 × 10⁵ to 5 × 10⁸ CD3+ or CD8+ total T cells or CD3+ or CD8+ recombinant receptor-expressing cells, from or from about 5 × 10⁵ to 1 × 10⁷ CD3+ or CD8+ total T cells or CD3+ or CD8+ recombinant receptor-expressing cells, or from or from about 1 × 10⁶ to 1 × 10⁷ CD3+ or CD8+ total T cells or CD3+ or CD8+recombinant receptor-expressing cells, each inclusive. In some instances, the cell therapy comprises administration of a dose comprising a number of cell from or from about 1 × 10⁵ to 5 × 10⁸ total CD3+/CAR+ or CD8+/CAR+ cells, from or from about 5 × 10⁵ to 1 × 10⁷ total CD3+/CAR+ or CD8+/CAR+ cells, or from or from about 1 × 10⁶ to 1 × 10⁷ total CD3+/CAR+ or CD8+/CAR+ cells, each inclusive.

In some instances, the T cells of the dose include CD4+ T cells, CD8+ T cells or CD4+ and CD8+ T cells.

In some instances, for example, where the subject is human, the CD8+ T cells of the dose, including in a dose including CD4+ and CD8+ T cells, includes between about 1 × 10⁶ and 5 × 10⁸ total recombinant receptor (e.g., CAR)-expressing CD8+cells, e.g., in the range of about 5 × 10⁶ to 1 × 10⁸ such cells, such cells 1 × 10⁷, 2.5 × 10⁷, 5 × 10⁷, 7.5 × 10⁷, 1 × 10⁸, or 5 × 10⁸ total such cells, or the range between any two of the foregoing values. In some instances, the patient is administered multiple doses, and each of the doses or the total dose can be within any of the foregoing values. In some instances, the dose of cells comprises the administration of from or from about 1 × 10⁷ to 0.75 × 10⁸ total recombinant receptor-expressing CD8+ T cells, 1 × 10⁷ to 2.5 × 10⁷ total recombinant receptor-expressing CD8+ T cells, from or from about 1 × 10⁷ to 0.75 × 10⁸ total recombinant receptor-expressing CD8+ T cells, each inclusive. In some instances, the dose of cells comprises the administration of or about 1 × 10⁷, 2.5 × 10⁷, 5 × 10⁷ 7.5 × 10⁷, 1 × 10⁸, or 5 × 10⁸ total recombinant receptor-expressing CD8+ T cells.

In some instances, the dose of cells, e.g., recombinant receptor-expressing T cells, is administered to the subject as a single dose or is administered only one time within a period of two weeks, one month, three months, six months, 1 year or more.

In some instances, the cell therapy comprises administration of a dose comprising a number of cells that is at least or at least about or is or is about 0.1 × 10⁶ cells/kg body weight of the subject, 0.2 × 10⁶ cells/kg, 0.3 × 10⁶ cells/kg, 0.4 × 10⁶ cells/kg, 0.5 × 10⁶ cells/kg, 1 × 10⁶ cell/kg, 2.0 × 10⁶ cells/kg, 3 × 10⁶ cells/kg or 5 × 10⁶ cells/kg.

In some instances, the cell therapy comprises administration of a dose comprising a number of cells is between or between about 0.1 × 10⁶ cells/kg body weight of the subject and 1.0 × 10⁷ cells/kg, between or between about 0.5 × 10⁶ cells/kg and 5 × 10⁶ cells/kg, between or between about 0.5 × 10⁶ cells/kg and 3 × 10⁶ cells/kg, between or between about 0.5 × 10⁶ cells/kg and 2 × 10⁶ cells/kg, between or between about 0.5 × 10⁶ cells/kg and 1 × 10⁶ cell/kg, between or between about 1.0 × 10⁶ cells/kg body weight of the subject and 5 × 10⁶ cells/kg, between or between about 1.0 × 10⁶ cells/kg and 3 × 10⁶ cells/kg, between or between about 1.0 × 10⁶ cells/kg and 2 × 10⁶ cells/kg, between or between about 2.0 × 10⁶ cells/kg body weight of the subject and 5 × 10⁶ cells/kg, between or between about 2.0 × 10⁶ cells/kg and 3 × 10⁶ cells/kg, or between or between about 3.0 × 10⁶ cells/kg body weight of the subject and 5 × 10⁶ cells/kg, each inclusive.

In some instances, the dose of cells comprises between at or about 2 × 10⁵ of the cells/kg and at or about 2 × 10⁶ of the cells/kg, such as between at or about 4 × 10⁵ of the cells/kg and at or about 1 × 10⁶ of the cells/kg or between at or about 6 × 10⁵ of the cells/kg and at or about 8 × 10⁵ of the cells/kg. In some instances, the dose of cells comprises no more than 2 × 10⁵ of the cells (*e.g.* antigen-expressing, such as CAR-expressing cells) per kilogram body weight of the subject (cells/kg), such as no more than at or about 3 × 10⁵ cells/kg, no more than at or about 4 × 10⁵ cells/kg, no more than at or about 5 × 10⁵ cells/kg, no more than at or about 6 × 10⁵ cells/kg, no more than at or about 7 × 10⁵ cells/kg, no more than at or about 8 × 10⁵ cells/kg, nor more than at or about 9 × 10⁵ cells/kg, no more than at or about 1 × 10⁶ cells/kg, or no more than at or about 2 × 10⁶ cells/kg. In some instances, the dose of cells comprises at least or at least about or at or about 2 × 10⁵ of the cells (*e.g.* antigen-expressing, such as CAR-expressing cells) per kilogram body weight of the subject (cells/kg), such as at least or at least about or at or about 3 × 10⁵ cells/kg, at least or at least about or at or about 4 × 10⁵ cells/kg, at least or at least about or at or about 5 × 10⁵ cells/kg, at least or at least about or at or about 6 × 10⁵ cells/kg, at least or at least about or at or about 7 × 10⁵ cells/kg, at least or at least about or at or about 8 × 10⁵ cells/kg, at least or at least about or at or about 9 × 10⁵ cells/kg, at least or at least about or at or about 1 × 10⁶ cells/kg, or at least or at least about or at or about 2 × 10⁶ cells/kg.

In the context of adoptive cell therapy, administration of a given "dose" of cells encompasses administration of the given amount or number of cells as a single composition and/or single uninterrupted administration, *e.g.,* as a single injection or continuous infusion, and also encompasses administration of the given amount or number of cells as a split dose, provided in multiple individual compositions or infusions, over a specified period of time, which is no more than 3 days. Thus, in some contexts, the dose is a single or continuous administration of the specified number of cells, given or initiated at a single point in time. In some contexts, however, the dose is administered in multiple injections or infusions over a period of no more than three days, such as once a day for three days or for two days or by multiple infusions over a single day period.

Thus, in some aspects, the cells of the dose are administered in a single pharmaceutical composition. In some instances, the cells of the dose are administered in a plurality of compositions, collectively containing the cells of the dose.

The term "split dose" refers to a dose that is split so that it is administered over more than one day. This type of dosing is encompassed by the present methods and is considered to be a single dose. In some instances, the cells of a split dose are administered in a plurality of compositions, collectively comprising the cells of the dose, over a period of no more than three days.

Thus, the dose of cells may be administered as a split dose. For example, in some instances, the dose may be administered to the subject over 2 days or over 3 days. Exemplary methods for split dosing include administering 25% of the dose on the first day and administering the remaining 75% of the dose on the second day. In other instances, 33% of the dose may be administered on the first day and the remaining 67% administered on the second day. In some aspects, 10% of the dose is administered on the first day, 30% of the dose is administered on the second day, and 60% of the dose is administered on the third day. In some instances, the split dose is not spread over more than 3 days.

In some instances, the dose of cells is generally large enough to be effective in reducing disease burden.

In some instances, the cells are administered at a desired dosage, which in some aspects includes a desired dose or number of cells or cell type(s) and/or a desired ratio of cell types. Thus, the dosage of cells in some instances is based on a total number of cells (or number per kg body weight) and a desired ratio of the individual populations or sub-types, such as the CD4+ to CD8+ ratio. In some instances, the dosage of cells is based on a desired total number (or number per kg of body weight) of cells in the individual populations or of individual cell types. In some instances, the dosage is based on a combination of such features, such as a desired number of total cells, desired ratio, and desired total number of cells in the individual populations.

In some instances, the populations or sub-types of cells, such as CD8⁺ and CD4⁺ T cells, are administered at or within a tolerated difference of a desired dose of total cells, such as a desired dose of T cells. In some aspects, the desired dose is a desired number of cells or a desired number of cells per unit of body weight of the subject to whom the cells are administered, e.g., cells/kg. In some aspects, the desired dose is at or above a minimum number of cells or minimum number of cells per unit of body weight. In some aspects, among the total cells, administered at the desired dose, the individual populations or sub-types are present at or near a desired output ratio (such as CD4⁺ to CD8⁺ ratio), *e.g.,* within a certain tolerated difference or error of such a ratio.

In some instances, the cells are administered at or within a tolerated difference of a desired dose of one or more of the individual populations or sub-types of cells, such as a desired dose of CD4+ cells and/or a desired dose of CD8+ cells. In some aspects, the desired dose is a desired number of cells of the sub-type or population, or a desired number of such cells per unit of body weight of the subject to whom the cells are administered, e.g., cells/kg. In some aspects, the desired dose is at or above a minimum number of cells of the population or sub-type, or minimum number of cells of the population or sub-type per unit of body weight.

Thus, in some instances, the dosage is based on a desired fixed dose of total cells and a desired ratio, and/or based on a desired fixed dose of one or more, *e.g.,* each, of the individual sub-types or sub-populations. Thus, in some instances, the dosage is based on a desired fixed or minimum dose of T cells and a desired ratio of CD4⁺ to CD8⁺ cells, and/or is based on a desired fixed or minimum dose of CD4⁺ and/or CD8⁺ cells.

In some instances, the cells are administered at or within a tolerated range of a desired output ratio of multiple cell populations or sub-types, such as CD4+ and CD8+ cells or sub-types. In some aspects, the desired ratio can be a specific ratio or can be a range of ratios. for example, in some instances, the desired ratio (*e.g.,* ratio of CD4⁺to CD8⁺ cells) is between at or about 5:1 and at or about 5:1 (or greater than about 1:5 and less than about 5:1), or between at or about 1:3 and at or about 3:1 (or greater than about 1:3 and less than about 3:1), such as between at or about 2:1 and at or about 1:5 (or greater than about 1:5 and less than about 2:1, such as at or about 5:1, 4.5:1, 4:1, 3.5:1, 3:1, 2.5:1, 2:1, 1.9:1, 1.8:1, 1.7:1, 1.6:1, 1.5:1, 1.4:1, 1.3:1, 1.2:1, 1.1:1, 1:1, 1:1.1, 1:1.2, 1:1.3, 1:1.4, 1:1.5, 1:1.6, 1:1.7, 1:1.8, 1:1.9: 1:2, 1:2.5, 1:3, 1:3.5, 1:4, 1:4.5, or 1:5. In some aspects, the tolerated difference is within about 1%, about 2%, about 3%, about 4% about 5%, about 10%, about 15%, about 20%, about 25%, about 30%, about 35%, about 40%, about 45%, about 50% of the desired ratio, including any value in between these ranges.

In particular instances, the numbers and/or concentrations of cells refer to the number of recombinant receptor (*e.g.,* CAR)-expressing cells. In other instances, the numbers and/or concentrations of cells refer to the number or concentration of all cells, T cells, or peripheral blood mononuclear cells (PBMCs) administered.

In some aspects, the size of the dose is determined based on one or more criteria such as response of the subject to prior treatment, *e.g.* chemotherapy, disease burden in the subject, such as tumor load, bulk, size, or degree, extent, or type of metastasis, stage, and/or likelihood or incidence of the subject developing toxic outcomes, *e.g.,* CRS, macrophage activation syndrome, tumor lysis syndrome, neurotoxicity, and/or a host immune response against the cells and/or recombinant receptors being administered.

In some instances, administration of the immunomodulatory compound in combination with the cells is able to significantly increase the expansion or proliferation of the cells, and thus a lower dose of cells can be administered to the subject. In some cases, the disclosed methods allow a lower dose of such cells to be administered, to achieve the same or better efficacy of treatment as the dose in a method in which the cell therapy is administered without administering the immunomodulatory compound, such as at least 1.5-fold, 2-fold, 3-fold, 4-fold, 5-fold or 10-fold less than the dose in a method in which the cell therapy is administered without administering the immunomodulatory compound, *e.g.*,lenalidomide*.*

In some instances, for example, the dose contains between or between about 5.0 × 10⁶ and 2.25 × 10⁷, 5.0 × 10⁶ and 2.0 × 10⁷, 5.0 × 10⁶ and 1.5 × 10⁷, 5.0 × 10⁶ and 1.0 × 10⁷, 5.0 × 10⁶ and 7.5 × 10⁶, 7.5 × 10⁶ and 2.25 × 10⁷, 7.5 × 10⁶ and 2.0 × 10⁷, 7.5 × 10⁶ and 1.5 × 10⁷, 7.5 × 10⁶ and 1.0 × 10⁷, 1.0 × 10⁷ and 2.25 × 10⁷, 1.0 × 10⁷ and 2.0 × 10⁷, 1.0 × 10⁷ and 1.5 × 10⁷, 1.5 × 10⁷ and 2.25 × 10⁷, 1.5 × 10⁷ and 2.0 × 10⁷, 2.0 × 10⁷ and 2.25 × 10⁷. In some instances, the dose of cells contains a number of cells, that is between at least or at least about 5 × 10⁶, 6 × 10⁶, 7 × 10⁶, 8 × 10⁶, 9 × 10⁶, 10 × 10⁶ and about 15 × 10⁶ recombinant-receptor expressing cells, such as recombinant-receptor expressing cells that are CD8+. In some instances, such dose, such as such target number of cells refers to the total recombinant-receptor expressing cells in the administered composition.

In some instances, for example, the lower dose contains less than about 5 × 10⁶ cells, recombinant receptor (e.g. CAR)-expressing cells, T cells, and/or PBMCs per kilogram body weight of the subject, such as less than about 4.5 × 10⁶, 4 × 10⁶, 3.5 × 10⁶, 3 ×10⁶, 2.5 × 10⁶, 2 × 10⁶, 1.5 × 10⁶, 1 × 10⁶, 5 × 10⁵, 2.5 × 10⁵, or 1 × 10⁵ such cells per kilogram body weight of the subject. In some instances, the lower dose contains less than about 1 × 10⁵, 2 × 10⁵, 5 × 10⁵, or 1 × 10⁶ of such cells per kilogram body weight of the subject, or a value within the range between any two of the foregoing values. In some instances, such values refer to numbers of recombinant receptor-expressing cells; in other instances, they refer to number of T cells or PBMCs or total cells administered.

In some instances, the subject receives multiple doses, e.g., two or more doses or multiple consecutive doses, of the cells. In some instances, two doses are administered to a subject. In some instances, the subject receives the consecutive dose, e.g., second dose, is administered approximately 4, 5, 6, 7, 8, 9, 10, 11, 12, 13, 14, 15, 16, 17, 18, 19, 20 or 21 days after the first dose. In some instances, multiple consecutive doses are administered following the first dose, such that an additional dose or doses are administered following administration of the consecutive dose. In some aspects, the number of cells administered to the subject in the additional dose is the same as or similar to the first dose and/or consecutive dose. In some instances, the additional dose or doses are larger than prior doses. In some instances, one or more subsequent dose of cells can be administered to the subject. In some instances, the subsequent dose of cells is administered greater than or greater than about 7 days, 14 days, 21 days, 28 days or 35 days after initiation of administration of the first dose of cells. The subsequent dose of cells can be more than, approximately the same as, or less than the first dose. In some instances, administration of the T cell therapy, such as administration of the first and/or second dose of cells, can be repeated.

In some instances, initiation of administration of the cell therapy, *e.g.* the dose of cells or a first dose of a split dose of cells, is administered before (prior to), concurrently with or after (subsequently or subsequent to) the administration of the immunomodulatory compound, *e.g.,* lenalidomide.

In some instances, the dose of cells, or the subsequent dose of cells, is administered concurrently with initiating administration of the immunomodulatory compound in accord with the combination therapy methods. In some instances, the dose of cells, or the subsequent dose of cells, is administered on the same day as initiating administration of the immunomodulatory compound in accord with the combination therapy methods. In some instances, the dose of cells, or the subsequent dose of cells, is administered within 1 day, within 2 days, within 3 days, within 4 days, within 5 days, within 6 days, or within 7 days of initiating administration of the immunomodulatory compound in accord with the combination therapy methods.

In some instances, the dose of cells, or the subsequent dose of cells, is administered prior to starting or initiating administration of the immunomodulatory compound in accord with the provided combination therapy. In some instances, the dose of cells is administered at least or at least about 1 hour, at least or at least about 2 hours, at least or at least about 3 hours, at least or at least about 6 hours, at least or at least about 12 hours, at least or at least about 1 day, at least or at least about 2 days, at least or at least about 3 days, at least or about at least 4 days, at least or at least about 5 days, at least or about at least 6 days, at least or at least about 7 days, at least or about at least 12 days, at least or at least about 14 days, at least or about at least 15 days, at least or at least about 21 days, at least or at least about 28 days, at least or about at least 30 days, at least or at least about 35 days, at least or at least about 42 days, at least or about at least 60 days or at least or about at least 90 days prior to administering the immunomodulatory compound in accord with the provided combination therapy.

In some instances, the administration of the immunomodulatory compound (e.g., lenalidomide) immunomodulatory compound in accord with the provided combination therapy is at a time in which the prior administration of the immunotherapy (*e.g.,* T cell therapy, such as CAR-T cell therapy) is associated with, or is likely to be associated with, a decreased functionality of the T cells compared to the functionality of the T cells at a time just prior to initiation of the immunotherapy (*e.g.,* T cell therapy, such as CAR-T cell therapy) or at a preceding time point after initiation of the T cell therapy. In some instances, the method involves, subsequent to administering the dose of cells of the T cell therapy, *e.g.,* adoptive T cell therapy, but prior to administering the immunomodulatory compound, assessing a sample from the subject for one or more function of T cells, such as expansion or persistence of the cells, e.g. as determined by level or amount in the blood, or other phenotypes or desired outcomes as described herein, *e.g.,* such as those described in Section III. Various parameters for determining or assessing the regimen of the combination therapy are described in Section III.

### B. ADMINISTRATION OF THE IMMUNOMODULATORY COMPOUND

The disclosed combination therapy methods, compositions, combinations, kits and uses involve administration of an immunomodulatory compound, such as a structural or functional analog or derivative of thalidomide and/or an inhibitor of E3 ubiquitin ligase, e.g. lenalidomide, which can be administered prior to, subsequently to, during, simultaneously or near simultaneously, sequentially and/or intermittently with administration of the T cell therapy, *e.g.,* administration of T cells expressing a chimeric antigen receptor (CAR).

In some instances, the immunomodulatory compound is one of a class of immunomodulatory compounds that is a structural or functional analog or derivative of thalidomide and/or an inhibitor of E3 ubiquitin ligase.

In some instances, the immunomodulatory compound binds to cereblon (CRBN). In some instances, the immunomodulatory compound binds to the CRBN E3 ubiquitin-ligase complex. In some instances, the immunomodulatory compound binds to CRBN and the CRBN E3 ubiquitin-ligase complex. In some instances, the immunomodulatory compound up-regulates the protein or gene expression of CRBN. In some aspects, CRBN is the substrate adaptor for the CRL4^{CRBN} E3 ubiquitin ligase, and modulates the specificity of the enzyme. In some instances, binding to CRB or the CRBN E3 ubiquitin ligase complex inhibits E3 ubiquitin ligase activity. In some instances, the immunomodulatory compound induces the ubiqutination of KZF1

(Ikaros) and IKZF3 (Aiolos) and/or induces degradation of IKZF 1 (Ikaros) and IKZF3 (Aiolos). In some instances, the immunomodulatory compound induces the ubiquitination of casein kinase 1A1 (CK1α) by the CRL4^{CRBN} E3 ubiquitin ligase. In some instances, the ubiquitination of CK1α results in CK1α degradation.

In some instances, the immunomodulatory compound is an inhibitor of the Ikaros (IKZF1) transcription factor. In some instances, the immunomodulatory compound enhances ubiquitination of Ikaros. In some instances, the immunomodulatory compound enhances the degradation of Ikaros. In some instances, the immunomodulatory compound down-regulates the protein or gene expression of Ikaros. In some instances, administration of the immunomodulatory compound causes a decrease in Ikaros protein levels.

In some instances, the immunomodulatory compound is an inhibitor of the Aiolos (IKZF3) transcription factor. In some instances, the immunomodulatory compound enhances ubiquitination of Aiolos. In some instances, the immunomodulatory compound enhances the degradation of Aiolos. In some instances, the immunomodulatory compound down-regulates the protein or gene expression of Aiolos. In some instances, administration of the immunomodulatory compound causes a decrease in Aiolos protein levels.

In some instances, the immunomodulatory compound is an inhibitor of both the Ikaros (IKZF1) and Aiolos (IKZF3) transcription factors. In some instances, the immunomodulatory compound enhances ubiquitination of both Ikaros and Aiolos. In some instances, the immunomodulatory compound enhances the degradation of both Ikaros and Aiolos. In some instances, the immunomodulatory compound enhances ubiquitination and degradation of both Ikaros and Aiolos. In some instances, administration of the immunomodulatory compound causes both Aiolos protein levels and Ikaros protein levels to decrease.

In some instances, the immunomodulatory compound is a Selective cytokine inhibitory drug (SelCID). In some instances, the immunomodulatory compound inhibit the activity of phosphodiesterase-4 (PDE4). In some instances, the immunomodulatory compound suppresses the enzymatic activity of the CDC25 phosphatases. In some instances, the immunomodulatory compound alters the intracellular trafficking of CDC25 phosphatases.

In some instances, the immunomodulatory compound in the combination therapy is thalidomide (2-(2,6-dioxopiperidin-3-yl)-1H-isoindole-1,3(2H)-dione) or an analog or derivative of thalidomide. In certain instances, a thalidomide derivative includes structural variants of thalidomide that have a similar biological activity. Exemplary thalidomide derivatives include, but are not limited to lenalidomide (REVLIMMUNOMODULATORY COMPOUND^{™}; Celgene Corporation), pomalidomide (also known as ACTIMMUNOMODULATORY COMPOUND^{™} or POMALYST^{™} (Celgene Corporation)), CC-1088, CDC-501, and CDC-801, and the compounds disclosed in U.S. Pat. Nos. 5,712,291; 7,320,991; and 8,716,315; U.S. Appl. No. 2016/0313300; and PCT Pub. Nos. WO 2002/068414 and WO 2008/154252.

In some instances, the immunomodulatory compound is 1-oxo- and 1,3 dioxo-2-(2,6-dioxopiperldin-3-yl) isoindolines substituted with amino in the benzo ring as described in U.S. Pat. No. 5,635,517.

In some instances, the immunomodulatory compound is a compound of the following formula: 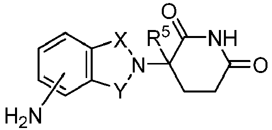 wherein one of X and Y is -C(O)- and the other of X and Y is -C(O)- or -CH₂-, and R⁵ is hydrogen or lower alkyl, or a pharmaceutically acceptable salt thereof. In some instances, X is - C(O)- and Y is -CH₂-. In some instances, both X and Y are -C(O)-. In some instances, R⁵ is hydrogen. In other instances, R⁵ is methyl.

In some instances, the immunomodulatory compound is a compound that belongs to a class of substituted 2-(2, 6-dioxopiperidin-3-yl)phthalimmunomodulatory compounds and substituted 2-(2,6-dioxopiperldin-3-yl)-1-oxoisoindoles, such as those described in U.S. Pat. Nos. 6,281,230; 6,316,471; 6,335,349; and 6,476,052, and International Patent Application No. PCT/US97/13375 (International Publication No. WO 98/03502).

In some instances, the immunomodulatory compound is a compound of the following formula: 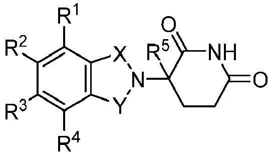 wherein
one of X and Y is -C(O)- and the other of X and Y is -C(O)- or -CH₂-;
   (1) each of R¹, R², R³, and R⁴ are independently halo, alkyl of 1 to 4 carbon atoms, or alkoxy or 1 to 4 carbon atoms, or
   (2) one of R¹, R³, R⁴ , and R⁵ is -NHR^{a} and the remaining of R¹, R², R³, and R⁴ is are hydrogen, wherein R^{a} is hydrogen or alkyl of 1 to 8 carbon atoms;
R⁵ is hydrogen or alkyl of 1 to 8 carbon atoms, benzyl, or halo;
provided that R⁵ is other than hydrogen if X and Y are -C(O)- and (i) each of R¹, R², R³, and R⁴ is fluoro; or (ii) one of R¹, R², R³, and R⁴ is amino;
or a pharmaceutically acceptable salt thereof.

In some instances, the immunomodulatory compound is a compound that belongs to a class of isoindole-immunomodulatory compounds disclosed in U.S. Pat. No. 7,091,353, U.S. Patent Publication No. 2003/0045552, and International Application No. PCT/USOI/50401 (International Publication No. WO02/059106). For example, in some instances, the immunomodulatory compound is [2-(2,6-dioxo-piperidin-3-yl)-1,3-dioxo-2,3-dihydro-1H-isoindol-4-ylmethyl]-amide; (2-(2,6-dioxo-piperidin-3-yl)-1,3-dioxo-2,3-dihydro-1H-isoindol-4-ylmethyl)-carbamic acid tert-butyl ester; 4-(aminomethyl)-2-(2,6-dioxo(3-piperidyl))-isoindoline-1,3-dione; N-(2-(2,6-dioxo-piperidin-3-yl)-1,3-dioxo-2,3-dihydro-1H-isoindol-4-ylmethyl)-acetamide; N-{(2-(2,6-dioxo(3-piperidyl)-1,3-dioxoisoindolin-4-yl)methyl}cyclopropyl-carboxamide; 2-chloro-N-{(2-(2,6-dioxo(3-piperidyl))-1,3-dioxoisoindolin-4-yl)methyl}acetamide; N-(2-(2,6-dioxo(3-piperidyl))-1,3-dioxoisoindolin-4-yl)-3-pyridylcarboxamide; 3-{ 1-oxo-4-(benzylamino)isoindolin-2-yl}piperidine-2,6-dione; 2-(2,6-dioxo(3-piperidyl))-4-(benzylamino)isoindoline-l,3-dione;N-{(2-(2,6-dioxo(3-piperidyl))-1,3-dioxoisoindolin-4-yl)methyl}propanamide; N- f (2-(2,6-dioxo(3-piperidyl))-1,3-dioxoisoindolin-4-yl)methyl}-3-pyridylcarboxamide; N-{(2-(2,6-dioxo(3-piperidyl))-1,3-dioxoisoindolin-4-yl)methyl}heptanamide; N-{(2-(2,6-dioxo(3-piperidyl))-1,3-dioxoisoindolin-4-yl)methyl}-2-furylcarboxamide; fN-(2-(2,6-dioxo(3-piperidyl))-1,3-dioxoisoindolin-4-yl)carbamoyl}methyl acetate; N-(2-(2,6-dioxo(3-piperidyl))-1,3-dioxoisoindolin-4-yl)pentanamide; N-(2-(2,6-dioxo(3-piperidyl))-1,3-dioxoisoindolin-4-yl)-2-thienylcarboxamide; N-{[2-(2,6-dioxo(3-piperidyl))-1,3-dioxoisoindolin-4-yl]methyl}(butylamino)carboxamide; N-{[2-(2,6-dioxo(3-piperidyl))-1,3-dioxoisoindolin-4-yl]methyl}(octylamino)carboxamide; or N-{[2-(2,6-dioxo(3-piperidyl))-1,3-dioxoisoindolin-4-yl]methyl}(benzylamino)carboxamide.

In some instances, the immunomodulatory compound is a compound that belongs to a class of isoindole-immunomodulatory compounds disclosed in U.S. Patent Application Publication Nos. 2002/0045643, International Publication No. WO 98/54170, and U.S. Pat. No. 6,395,754. In some instances, the immunomodulatory compound is a tetra substituted 2-(2,6-dioxopiperdin-3-yl)-1-oxoisoindolines described in U.S. Pat. No. 5,798,368. In some instances, the immunomodulatory compound is 1-oxo and 1,3-dioxo-2-(2,6-dioxopiperidin-3-yl) isoindolines disclosed in U.S. Pat. No. 6,403,613. In some instances the immunomodulatory compound is a 1-oxo or 1,3-dioxoisoindoline substituted in the 4- or 5-position of the indoline ring as described in U.S. Pat. No. 6,380,239 and U.S. Pat. No. 7,244,759.

In some instances, the immunomodulatory compound is 2-(4-amino-1-oxo-1,3-dihydro-isoindol-2-yl)-4-carbamoyl-butyric acid or 4-(4-amino-1-oxo-1,3-dihydro-isoindol-2-yl)-4-carbamoyl-butyric acid. In some instances, the immunomodulatory compound is 4-carbamoyl-4-{4-[(furan-2-yl-methyl)-amino]-1,3-dioxo-1,3-dihydro-isoindol-2-yl}-butyric acid, 4-carbamoyl-2-{4-[(furan-2-yl-methyl)-amino]-1,3-dioxo-1,3-dihydro-isoindol-2-yl}-butyric acid, 2-{4-[(furan-2-yl-methyl)-amino]-1,3-dioxo-1,3-dihydro-isoindol-2-yl}-4-phenylcarbamoyl-butyric acid, or 2-{4-[(furan-2-yl-methyl)-amino]-1,3-dioxo-1,3-dihydroisoindol-2-yl}-pentanedioic acid.

In some instances, the immunomodulatory compound is a isoindoline-1-one or isoindoline-1,3-dione substituted in the 2-position with 2,6-dioxo-3-hydroxypiperidin-5-yl as described in U.S. Pat. No. 6,458,810. In some instances, the immunomodulatory compound is 3-(5-amino-2-methyl-4-oxo-4H-quinazolin-3-yl)-piperidine-2,6-dione, or an enantiomer or a mixture of enantiomers thereof; or a pharmaceutically acceptable salt, solvate, hydrate, co-crystal, clathrate, or polymorph thereof. In some instances, the immunomodulatory compound is 3-[4-(4-morpholin-4-ylmethyl-benzyloxy)-1-oxo-1,3-dihydro-isoindol-2-yl]-piperidine-2,6-dione.

In some instances, the immunomodulatory compound is as described in Oshima, K. et al., Nihon Rinsho., 72(6):1130-5 (2014); Millrine, D. et al., Trends Mol Med., 23(4):348-364 (2017); and Collins, et al., Biochem J., 474(7):1127-1147 (2017).

In some instances, the immunomodulatory compound is an inhibitor of E3 ubiquitin ligase. In some instances, the immunomodulatory compound is a derivative of thalidomide. In some instances, the immunomodulatory compound is a structural and/or functional analogue of thalidomide. In some instances, the immunomodulatory compound is lenalidomide, pomalidomide, avadomide, or a pharmaceutically acceptable salt thereof.

In some instances, the immunomodulatory compound is lenalidomide, pomalidomide, avadomide, a stereoisomer of lenalidomide, pomalidomide, avadomide or a pharmaceutically acceptable salt, solvate, hydrate, co-crystal, clathrate, or polymorph thereof. In some instances, the immunomodulatory compound is lenalidomide, a stereoisomer of lenalidomide or a pharmaceutically acceptable salt, solvate, hydrate, co-crystal, clathrate, or polymorph thereof.

In some instances, the immunomodulatory compound is avadomide, which also is known as 3-(5-amino-2-methyl-4-oxo-4H-quinazolin-3-yl)-piperidine-2,6-dione having the following structure 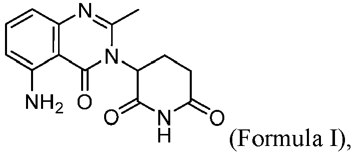 or is an enantiomer or a mixture of enantiomers thereof; or a pharmaceutically acceptable salt, solvate, hydrate, co-crystal, clathrate, or polymorph thereof (hereinafter **Compound 1**).

In some instances, the immunomodulatory compound is an enantiomer or a mixture of enantiomers of 3-(5-amino-2-methyl-4-oxo-4H-quinazolin-3-yl)-piperidine-2,6-dione, or a pharmaceutically acceptable salt, solvate, hydrate, co-crystal, clathrate, or polymorph of 3-(5-amino-2-methyl-4-oxo-4H-quinazolin-3-yl)-piperidine-2,6-dione. In some instances, the immunomodulatory compound is a solvate of 3-(5-amino-2-methyl-4-oxo-4H-quinazolin-3-yl)-piperidine-2,6-dione. In some instances, the immunomodulatory compound is a hydrate of 3-(5-amino-2-methyl-4-oxo-4H-quinazolin-3-yl)-piperidine-2,6-dione. In some instances, the immunomodulatory compound is a pharmaceutically acceptable salt of 3-(5-amino-2-methyl-4-oxo-4H-quinazolin-3-yl)-piperidine-2,6-dione. In some instances, the immunomodulatory compound is a polymorph of 3-(5-amino-2-methyl-4-oxo-4H-quinazolin-3-yl)-piperidine-2,6-dione. In some instances, the immunomodulatory compound is 3-(5-amino-2-methyl-4-oxo-4H-quinazolin-3-yl)-piperidine-2,6-dione. In some instances, the immunomodulatory compound has the structure of Formula I.

In some instances, the immunomodulatory compound is lenalidomide, which also is known as 3-(4-amino-1-oxo-1,3-dihydro-2H-isoindol-2-yl)piperidine-2,6-dione, or is an enantiomer or a mixture of enantiomers thereof; or a pharmaceutically acceptable salt, solvate, hydrate, co-crystal, clathrate, or polymorph thereof. In some instances, lenalidomide is 2,6-Piperidinedione, 3-(4-amino-1,3-dihydro-1-oxo-2H-isoindol-2-yl)-, 3-(4-Amino-1-oxo-1,3-dihydro-2H-isoindol-2-yl)-2,6-piperidinedione, 3-(4-Amino-1-oxo-1,3-dihydro-2H-isoindol-2-yl)-2,6-piperidinedione, 3-(4-Amino-1-oxo-1,3-dihydro-2H-isoindol-2-yl)piperidin-2,6-dion, 3-(4-amino-1-oxo-1,3-dihydro-2H-isoindol-2-yl)piperidine-2,6-dione, 3-(4-amino-1-oxo-1,3-dihydro-2H-isoindol-2-yl)piperidine-2,6-dione, all of which can be used interchangeably, or is an enantiomer or a mixture of enantiomers thereof; or a pharmaceutically acceptable salt, solvate, hydrate, co-crystal, clathrate, or polymorph thereof.

In some instances, the immunomodulatory compound is (A)-3-(4-amino-1-oxo-1,3-dihydro-2H-isoindol-2-yl)piperidine-2,6-dione. In some instances, the immunomodulatory compound is (*S*)-3-(4-amino-1-oxo-1,3-dihydro-2H-isoindol-2-yl)piperidine-2,6-dione. In some instances, the immunomodulatory compound is a mixture of (*R*)-3-(4-amino-1-oxo-1,3-dihydro-2H-isoindol-2-yl)piperidine-2,6-dione and (*S*)-3-(4-amino-1-oxo-1,3-dihydro-2H-isoindol-2-yl)piperidine-2,6-dione.

In some instances, the immunomodulatory compound is 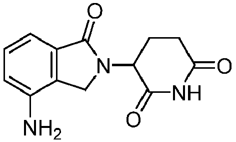 (Formula II), or an enantiomer or a mixture of enantiomers thereof; or a pharmaceutically acceptable salt, solvate, hydrate, co-crystal, clathrate, or polymorph thereof. In some instances, the immunomodulatory compound is 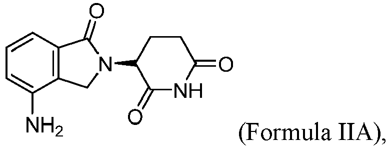 or a pharmaceutically acceptable salt, solvate, hydrate, co-crystal, clathrate, or polymorph thereof. In other instances, the immunomodulatory compound is 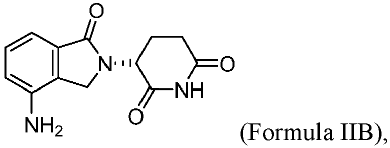 or a pharmaceutically acceptable salt, solvate, hydrate, co-crystal, clathrate, or polymorph thereof. In certain instances, the immunomodulatory compound comprises a mixture of 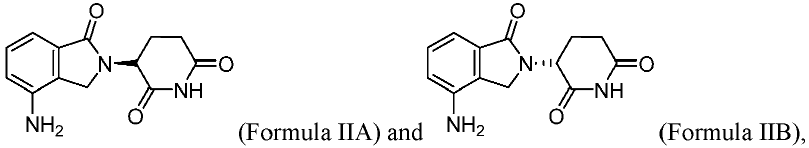 or pharmaceutically acceptable salts, solvates, hydrates, co-crystals, clathrates, or polymorphs thereof.

In some instances, the immunomodulatory compound is an enantiomer or a mixture of enantiomers of 3-(4-Amino-1-oxo-1,3-dihydro-2H-isoindol-2-yl)piperidine-2,6-dione, or a pharmaceutically acceptable salt, solvate, hydrate, co-crystal, clathrate, or polymorph of 3-(4-Amino-1-oxo-1,3-dihydro-2H-isoindol-2-yl)piperidine-2,6-dione. In some instances, the immunomodulatory compound is a solvate of (*R*)-3-(4-Amino-1-oxo-1,3-dihydro-2H-isoindol-2-yl)piperidine-2,6-dione and/or (*S*)-3-(4-Amino-1-oxo-1,3-dihydro-2H-isoindol-2-yl)piperidine-2,6-dione. In some instances, the immunomodulatory compound is a hydrate of (*R*S)-3-(4-Amino-1-oxo-11,3-dihydro-2H-isoindol-2-yl)piperidine-2,6-dione and/or (*S*)-3-(4-Amino-1-oxo-1,3-dihydro-2H-isoindol-2-yl)piperidine-2,6-dione. In some instances, the immunomodulatory compound is a pharmaceutically acceptable salt of (*R*)-3-(4-Amino-1-oxo-1,3-dihydro-2H-isoindol-2-yl)piperidine-2,6-dione and/or (*S*)-3-(4-Amino-1-oxo-1,3-dihydro-2H-isoindol-2-yl)piperidine-2,6-dione. In some instances, the immunomodulatory compound is lenalidomide, or 3-(4-amino-1-oxo-1,3-dihydro-2H-isoindol-2-yl)piperidine-2,6-dione. In some instances, the immunomodulatory compound has the structure of Formula II. In some instances, the immunomodulatory compound has the structure of Formula IIA or Formula IIB or a mixture thereof.

In some instances, the immunomodulatory compound is pomalidomide, which is also known as 4-amino-2-(2,6-dioxopiperidin-3-yl)isoindoline-1,3-dione, or is an enantiomer or a mixture of enantiomers thereof; or a pharmaceutically acceptable salt, solvate, hydrate, co-crystal, clathrate, or polymorph thereof. In some instances, the immunomodulatory compound is 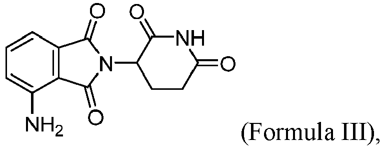 or an enantiomer or a mixture of enantiomers thereof; or a pharmaceutically acceptable salt, solvate, hydrate, co-crystal, clathrate, or polymorph thereof. In some instances, the immunomodulatory compound is 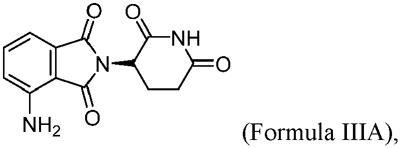 or a pharmaceutically acceptable salt, solvate, hydrate, co-crystal, clathrate, or polymorph thereof. In other instances, the immunomodulatory compound is 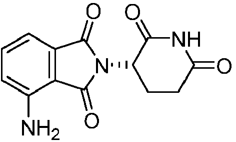 (Formula IIIB), or a pharmaceutically acceptable salt, solvate, hydrate, co-crystal, clathrate, or polymorph thereof. In certain instances, the immunomodulatory compound comprises a mixture of 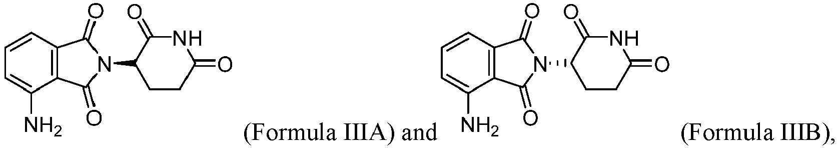 or pharmaceutically acceptable salts, solvates, hydrates, co-crystals, clathrates, or polymorphs thereof.

In some instances, the immunomodulatory compound is an enantiomer or a mixture of enantiomers of 4-amino-2-(2,6-dioxopiperidin-3-yl)isoindoline-1,3-dione, or a pharmaceutically acceptable salt, solvate, hydrate, co-crystal, clathrate, or polymorph of 4-amino-2-(2,6-dioxopiperidin-3-yl)isoindoline-1,3-dione. In some instances, the immunomodulatory compound is (*R*)-4-amino-2-(2,6-dioxopiperidin-3-yl)isoindoline-1,3-dione and/or (*S*)-4-amino-2-(2,6-dioxopiperidin-3-yl)isoindoline-1,3-dione,or a pharmaceutically acceptable salt, solvate, hydrate, co-crystal, clathrate, or polymorph of (*R*)-4-amino-2-(2,6-dioxopiperidin-3-yl)isoindoline-1,3-dione and/or (*S*)-4-amino-2-(2,6-dioxopiperidin-3-yl)isoindoline-1,3-dione. In some instances, the immunomodulatory compound is a solvate of (*R*)-4-amino-2-(2,6-dioxopiperidin-3-yl)isoindoline-1,3-dione and/or (*S*)-4-amino-2-(2,6-dioxopiperidin-3-yl)isoindoline-1,3-dione. In some instances, the immunomodulatory compound is a hydrate of (*R*)-4-amino-2-(2,6-dioxopiperidin-3-yl)isoindoline-1,3-dione and/or (*S*)-4-amino-2-(2,6-dioxopiperidin-3-yl)isoindoline-1,3-dione. In some instances, the immunomodulatory compound is a pharmaceutically acceptable salt of (*R*)-4-amino-2-(2,6-dioxopiperidin-3-yl)isoindoline-1,3-dione and/or (*S*)-4-amino-2-(2,6-dioxopiperidin-3-yl)isoindoline-1,3-dione. In some instances, the immunomodulatory compound is (*R*)-4-amino-2-(2,6-dioxopiperidin-3-yl)isoindoline-1,3-dione, (*S*)-4-amino-2-(2,6-dioxopiperidin-3-yl)isoindoline-1,3-dione, or a mixture thereof in any ratio. In some instances, the immunomodulatory compound has the structure of Formula III. In some instances, the immunomodulatory compound has the structure of Formula IIIA or Formula IIIB or a mixture thereof.

In some instances, the immunomodulatory compound is or comprises lenalidomide. Lenalidomide is FDA approved for the treatment of multiple myeloma, myelodysplastic syndrome associated with deletion 5q, and most recently in relapsed/refractory mantle-cell lymphoma (MCL). Lenalidomide is a synthetic derivative of thalidomide, and is currently understood to have multiple immunomodulatory effects, including enforcement of immune synapse formation between T cell and antigen presenting cells (APCs). For example, in some cases, lenalidomide modulates T cell responses and results in increased interleukin (IL)-2 production in CD4⁺ and CD8⁺ T cells, induces the shift of T helper (Th) responses from Th2 to Th1, inhibits expansion of regulatory subset of T cells (Tregs), and improves functioning of immunological synapses in follicular lymphoma (FL) and chronic lymphocytic leukemia (CLL) (Otahal et al., Oncoimmunology (2016) 5(4):e1115940). Lenalidomide also has direct tumoricidal activity in patients with multiple myeloma (MM) and directly and indirectly modulates survival of CLL tumor cells by affecting supportive cells, such as nurse-like cells found in the microenvironment of lymphoid tissues.

### 1. Compositions and formulations

In some instances of the combination therapy methods, compositions, combinations, kits and uses disclosed herein, the combination therapy can be administered in one or more compositions, e.g., a pharmaceutical composition containing an immunomodulatory compound, e.g., lenalidomide.

In some instances, the composition, *e.g.,* a pharmaceutical composition containing the immunomodulatory compound, *e.g.,* lenalidomide, can include carriers such as a diluent, adjuvant, excipient, or vehicle with which the immunomodulatory compound, *e.g.,* lenalidomide, and/or the cells are administered. Examples of suitable pharmaceutical carriers are described in "Remington's Pharmaceutical Sciences" by E. W. Martin. Such compositions will contain a therapeutically effective amount of the immunomodulatory compound, *e.g.* lenalidomide, generally in purified form, together with a suitable amount of carrier so as to provide the form for proper administration to the patient. Such pharmaceutical carriers can be sterile liquids, such as water and oils, including those of petroleum, animal, vegetable or synthetic origin, such as peanut oil, soybean oil, mineral oil, and sesame oil. Saline solutions and aqueous dextrose and glycerol solutions also can be employed as liquid carriers, particularly for injectable solutions. The pharmaceutical compositions can contain any one or more of a diluents(s), adjuvant(s), antiadherent(s), binder(s), coating(s), filler(s), flavor(s), color(s), lubricant(s), glidant(s), preservative(s), detergent(s), sorbent(s), emulsifying agent(s), pharmaceutical excipient(s), pH buffering agent(s), or sweetener(s) and a combination thereof. In some instances, the pharmaceutical composition can be liquid, solid, a lyophilized powder, in gel form, and/or combination thereof. In some aspects, the choice of carrier is determined in part by the particular inhibitor and/or by the method of administration.

Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.* Zn-protein complexes); and/or non-ionic surfactants such as polyethylene glycol (PEG), stabilizers and/or preservatives. The compositions containing the immunomodulatory compound, *e.g.,* lenalidomide can also be lyophilized.

In some instances, the pharmaceutical compositions can be formulated for administration by any known route including intramuscular, intravenous, intradermal, intralesional, intraperitoneal injection, subcutaneous, intratumoral, epidural, nasal, oral, vaginal, rectal, topical, local, otic, inhalational, buccal (*e.g.,* sublingual), and transdermal administration or any route. In some instances, other modes of administration also are contemplated. In some instances, the administration is by bolus infusion, by injection, *e.g.,* intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some instances, administration is by parenteral, intrapulmonary, and intranasal, and, if desired for local treatment, intralesional administration. Parenteral infusions include intramuscular, intravenous, intraarterial, intraperitoneal, or subcutaneous administration. In some instances, a given dose is administered by a single bolus administration. In some instances, it is administered by multiple bolus administrations, for example, over a period of no more than 3 days, or by continuous infusion administration.

In some instances, the administration can be local, topical or systemic depending upon the locus of treatment. In some instances local administration to an area in need of treatment can be achieved by, for example, but not limited to, local infusion during surgery, topical application, *e.g.,* in conjunction with a wound dressing after surgery, by injection, by means of a catheter, by means of a suppository, or by means of an implant. In some instances, compositions also can be administered with other biologically active agents, either sequentially, intermittently or in the same composition. In some instances, administration also can include controlled release systems including controlled release formulations and device controlled release, such as by means of a pump. In some instances, the administration is oral.

In some instances, pharmaceutically and therapeutically active compounds and derivatives thereof are typically formulated and administered in unit dosage forms or multiple dosage forms. Each unit dose contains a predetermined quantity of therapeutically active compound sufficient to produce the desired therapeutic effect, in association with the required pharmaceutical carrier, vehicle or diluent. In some instances, unit dosage forms, include, but are not limited to, tablets, capsules, pills, powders, granules, sterile parenteral solutions or suspensions, and oral solutions or suspensions, and oil water emulsions containing suitable quantities of the compounds or pharmaceutically acceptable derivatives thereof. Unit dose forms can be contained ampoules and syringes or individually packaged tablets or capsules. Unit dose forms can be administered in fractions or multiples thereof. In some instances, a multiple dose form is a plurality of identical unit dosage forms packaged in a single container to be administered in segregated unit dose form. Examples of multiple dose forms include vials, bottles of tablets or capsules or bottles of pints or gallons.

Active ingredients may be entrapped in microcapsules, in colloidal drug delivery systems (for example, liposomes, albumin microspheres, microemulsions, nano-particles and nanocapsules) or in macroemulsions. In certain instances, the pharmaceutical composition containing the immunomodulatory compound, *e.g.,* lenalidomide, is formulated as an inclusion complex, such as cyclodextrin inclusion complex, or as a liposome. Liposomes can serve to target the host cells (e.g., T-cells or NK cells) to a particular tissue. Many methods are available for preparing liposomes, such as those described in, for example, Szoka et al., Ann. Rev. Biophys. Bioeng., 9: 467 (1980), and U.S. Patents 4,235,871, 4,501,728, 4,837,028, and 5,019,369.

The pharmaceutical composition containing the immunomodulatory compound, *e.g.,* lenalidomide, in some aspects can employ time-released, delayed release, and sustained release delivery systems such that the delivery of the composition occurs prior to, and with sufficient time to cause, sensitization of the site to be treated. Many types of release delivery systems are available and known. Such systems can avoid repeated administrations of the composition, thereby increasing convenience to the subject and the physician.

The compositions containing the immunomodulatory compound, *e.g.,* lenalidomide, can also be lyophilized. The compositions can contain auxiliary substances such as wetting, dispersing, or emulsifying agents (e.g., methylcellulose), pH buffering agents, gelling or viscosity enhancing additives, preservatives, flavoring agents, colors, and the like, depending upon the route of administration and the preparation desired. Standard texts may in some aspects be consulted to prepare suitable preparations.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. Prolonged absorption of the injectable pharmaceutical form can be brought about by the use of agents delaying absorption, for example, aluminum monostearate and gelatin.

Sustained-release preparations may be prepared. Suitable examples of sustained-release preparations include semipermeable matrices of solid hydrophobic polymers containing the antibody, which matrices are in the form of shaped articles, e.g. films, or microcapsules.

In some instances, the composition containing the immunomodulatory compound, *e.g.,* lenalidomide, are administered in the form of a salt, e.g., a pharmaceutically acceptable salt. Suitable pharmaceutically acceptable acid addition salts include those derived from mineral acids, such as hydrochloric, hydrobromic, phosphoric, metaphosphoric, nitric, and sulphuric acids, and organic acids, such as tartaric, acetic, citric, malic, lactic, fumaric, benzoic, glycolic, gluconic, succinic, and arylsulphonic acids, for example, p-toluenesulphonic acid.

### 2. Immunomodulatory compound Dosage Schedule

In some instances, the provided combination therapy method involves administering to the subject a therapeutically effective amount of an immunomodulatory drug (immunomodulatory compound), *e.g.,* lenalidomide, and the cell therapy, such as a T cell therapy (*e.g.* CAR-expressing T cells).

In some instances, the administration of the immunomodulatory compound, *e.g.,* lenalidomide, is initiated prior to, subsequently to, during, during the course of, simultaneously, near simultaneously, sequentially and/or intermittently with the administration of the cell therapy, such as a T cell therapy (*e.g.* CAR-expressing T cells). In some instances, the method involves initiating the administration of the immunomodulatory compound, *e.g.,* lenalidomide, prior to administration of the T cell therapy. In other instances, the method involves initiating the administration of the immunomodulatory compound, *e.g.,* lenalidomide, after administration of the T cell therapy. In some instances, the dosage schedule comprises initiating the administration of the immunomodulatory compound, *e.g.,* lenalidomide, concurrently or simultaneously with the administration of the T cell therapy.

In some instances, the immunomodulatory compound, *e.g.,* lenalidomide, is administered in a cycle. In some instances, the cycle comprises an administration period in which the immunomodulatory compound, *e.g.,* lenalidomide, is administered followed by a rest period during which the immunomodulatory compound, *e.g.,* lenalidomide, is not administered. In some instances, the total number of days of the cycle, *e.g.* from the beginning of initiating administration of the immunomodulatory compound, is greater than or greater than about or is about 21 days, 28 days, 30 days, 40 days, 50 days, 60 days or more.

In some instances, the initiation of the administration of the immunomodulatory compound, *e.g.,* lenalidomide, is carried out in at least one cycle and initiation of administration of the T cell therapy are carried out on the same day, optionally concurrently. In some instances, the initiation of the administration of the immunomodulatory compound, *e.g.,* lenalidomide, in at least one cycle is prior to initiation of administration of the T cell therapy. In some instances, the initiation of the administration of the immunomodulatory compound, *e.g.,* lenalidomide, in at least one cycle is concurrent with or on the same day as initiation of administration of the T cell therapy. In some instances, the immunomodulatory compound, e.g., lenalidomide, is administered from or from about 0 to 30 days, such as 0 to 15 days, 0 to 6 days, 0 to 96 hours, 0 to 24 hours, 0 to 12 hours, 0 to 6 hours, or 0 to 2 hours, 2 hours to 15 days, 2 hours to 6 days, 2 hours to 96 hours, 2 hours to 24 hours, 2 hours to 12 hours, 2 hours to 6 hours, 6 hours to 30 days, 6 hours to 15 days, 6 hours to 6 days, 6 hours to 96 hours, 6 hours to 24 hours, 6 hours to 12 hours, 12 hours to 30 days, 12 hours to 15 days, 12 hours to 6 days, 12 hours to 96 hours, 12 hours to 24 hours, 24 hours to 30 days, 24 hours to 15 days, 24 hours to 6 days, 24 hours to 96 hours, 96 hours to 30 days, 96 hours to 15 days, 96 hours to 6 days, 6 days to 30 days, 6 days to 15 days, or 15 days to 30 days prior to initiation of the T cell therapy. In some aspects, the immunomodulatory compound, *e.g.,* lenalidomide, is administered no more than about 96 hours, 72 hours, 48 hours, 24 hours, 12 hours, 6 hours, 2 hours or 1 hour prior to initiation of the T cell therapy.

In some of any such instances in which the immunomodulatory compound, *e.g.,* lenalidomide, is given prior to the cell therapy (*e.g.* T cell therapy, such as CAR-T cell therapy), the administration of the immunomodulatory compound, *e.g.,* lenalidomide, continues at regular intervals until the initiation of the cell therapy and/or for a time after the initiation of the cell therapy.

In some instances, the immunomodulatory compound, *e.g.,* lenalidomide, is administered, or is further administered, after administration of the cell therapy (*e.g.* T cell therapy, such as CAR-T cell therapy). In some instances, the immunomodulatory compound, *e.g.,* lenalidomide, is administered within or within about 1 hours, 2 hours, 6 hours, 12 hours, 24 hours, 48 hours, 96 hours, 4 days, 5 days, 6 days or 7 days, 14 days, 15 days, 21 days, 24 days, 28 days, 30 days, 36 days, 42 days, 60 days, 72 days or 90 days after initiation of administration of the cell therapy (*e.g.* T cell therapy). In some instances, the disclosed methods involve continued administration, such as at regular intervals, of the immunomodulatory compound after initiation of administration of the cell therapy.

In some instances, the immunomodulatory compound, *e.g.,* lenalidomide, is administered up to or up to about 1 day, up to or up to about 2 days, up to or up to about 3 days, up to or up to about 4 days, up to or up to about 5 days, up to or up to about 6 days, up to or up to about 7 days, up to or up to about 12 days, up to or up to about 14 days, up to or up to about 21 days, up to or up to about 24 days, up to or up to about 28 days, up to or up to about 30 days, up to or up to about 35 days, up to or up to about 42 days, up to or up to about 60 days or up to or up to about 90 days, up to or up to about 120 days, up to or up to about 180 days, up to or up to about 240 days, up to or up about 360 days, or up to or up to about 720 days or more after the initiation of administration of the cell therapy (*e.g.* T cell therapy, such as CAR-T cell therapy).

In some of any such above instances, the immunomodulatory compound, *e.g.,* lenalidomide, is administered prior to and after initiation of administration of the cell therapy (*e.g.* T cell therapy, such as CAR-T cell therapy).

In some instances, the initiation of the administration of the immunomodulatory compound, *e.g.,* lenalidomide, is carried out at or after, optionally immediately after or within 1 to 3 days after: (i) peak or maximum level of the cells of the T cell therapy are detectable in the blood of the subject; (ii) the number of cells of the T cell therapy detectable in the blood, after having been detectable in the blood, is not detectable or is reduced, optionally reduced compared to a preceding time point after administration of the T cell therapy; (iii) the number of cells of the T cell therapy detectable in the blood is decreased by or more than 1.5-fold, 2.0-fold, 3.0-fold, 4.0-fold, 5.0-fold, 10-fold or more the peak or maximum number cells of the T cell therapy detectable in the blood of the subject after initiation of administration of the T cell therapy; (iv) at a time after a peak or maximum level of the cells of the T cell therapy are detectable in the blood of the subject, the number of cells of or derived from the T cells detectable in the blood from the subject is less than less than 10%, less than 5%, less than 1% or less than 0.1% of total peripheral blood mononuclear cells (PBMCs) in the blood of the subject; (v) the subject exhibits disease progression and/or has relapsed following remission after treatment with the T cell therapy; and/or (iv) the subject exhibits increased tumor burden as compared to tumor burden at a time prior to or after administration of the T cells and prior to initiation of administration of the immunomodulatory compound.

In some instances, the initiation of the administration of the immunomodulatory compound, *e.g.,* lenalidomide, in at least one cycle is after initiation of administration of the T cell therapy. In some instances, the initiation of the administration of the immunomodulatory compound, *e.g.,* lenalidomide, is at least or about at least 1 day, at least or about at least 2 days, at least or about at least 3 days, at least or about at least 4 days, at least or about at least 5 days, at least or about at least 6 days, at least or about at least 7 days, at least or about at least 8 days, at least or about at least 9 days, at least or about at least 10 days, at least or at least about 12 days, at least or about at least 14 days, at least or at least about 15 days, at least or about at least 21 days, at least or at least about 24 days, at least or about at least 28 days, at least or about at least 30 days, at least or about at least 35 days or at least or about at least 42 days, at least or about at least 60 days, or at least or about at least 90 days after initiation of the administration of the T cell therapy. In some instances, the initiation of the administration of the immunomodulatory compound, *e.g.,* lenalidomide, is carried out at least 2 days after, at least 1 week after, at least 2 weeks after, at least 3 weeks after, or at least 4 weeks after, the initiation of the administration of the T cell therapy. In some instances, the initiation of the administration of the immunomodulatory compound, *e.g.,* lenalidomide, is carried out 2 to 28 days or 7 to 21 days after initiation of administration of the T cell therapy. In some instances, the initiation of the administration of the immunomodulatory compound, *e.g.,* lenalidomide, is carried out at a time that is greater than or greater than about 14 days, 15 days, 16 days, 17 days, 18 days, 19, days, 20 days, 21 days, 24 days, or 28 days after initiation of the administration of the T cell therapy. In some instances, the immunomodulatory compound, *e.g.,* lenalidomide, is administered several times a day, twice a day, daily, every other day, three times a week, twice a week, or once a week after initiation of the cell therapy. In some instances, the immunomodulatory compound, *e.g.,* lenalidomide, is administered daily. In some instances the immunomodulatory compound, *e.g.,* lenalidomide, is administered twice a day. In some instances, the immunomodulatory compound, *e.g.,* lenalidomide, is administered three times a day. In other instances, the immunomodulatory compound, *e.g.,* lenalidomide, is administered every other day. In some instances, the immunomodulatory compound, *e.g.,* lenalidomide, is administered daily. In some instances, the immunomodulatory compound, *e.g.,* lenalidomide, is administered during the administration period for a plurality of consecutive days, such as for up to about 7, 8, 9, 10, 11, 12, 13, 14, 15, 16, 17, 18, 19, 20, 21, 22, 23, 24, 25, 26, 27, 28, 29, 30, or more than 30 consecutive days. In some instances, the immunomodulatory compound, *e.g.,* lenalidomide, is administered for greater than or greater than about 7 consecutive days, greater than or greater than about 14 consecutive days, greater than or greater than about 21 consecutive days, greater than or greater than about 21 consecutive days, or greater than or greater than about 28 consecutive days. In some instances, the immunomodulatory compound, *e.g.,* lenalidomide, is administered during the administration period for up to 21 consecutive days. In some instances, the immunomodulatory compound, *e.g.,* lenalidomide, is administered during the administration period for up to 21 consecutive days, wherein the cycle comprises greater than 30 days beginning upon initiation of the administration of the immunomodulatory compound.

In some instances, the immunomodulatory compound, *e.g.,* lenalidomide, is administered during the administration period for no more than about 7, 8, 9, 10, 11, 12, 13, 14, 15, 16, 17, 18, 19, 20, 21, 22, 23, 24, 25, 26, 27, 28, 29, 30, or no more than 30 consecutive days. In certain instances, the lenalidomide is administered once daily for 14 days over a 21 day treatment cycle. In certain instances, the lenalidomide is administered once daily for 21 days over a 28 day treatment cycle. In some instances, the immunomodulatory compound, *e.g.,* lenalidomide, is administered during the administration period for no more than 14 consecutive days.

In some instances, the immunomodulatory compound, *e.g.,* lenalidomide, is administered in a cycle, wherein the cycle comprises the administration of the immunomodulatory compound, *e.g.,* lenalidomide for a plurality of consecutive days followed by a rest period during which the immunomodulatory compound is not administered. In some instances, the rest period is greater than about 1 day, greater than about 3 consecutive days, greater than about 5 consecutive days, greater than about 7 consecutive days, greater than about 8 consecutive days, greater than about 9 consecutive days, greater than about 10 consecutive days, greater than about 11 consecutive days, greater than about 12 consecutive days, greater than about 13 consecutive days, greater than about 14 consecutive days, greater than about 15 consecutive days, greater than about 16 consecutive days, greater than about 17 consecutive days, greater than about 18 consecutive days, greater than about 19 consecutive days, greater than about 20 consecutive days, or greater than about 21 or more consecutive days. In some instances, the rest period is greater than 7 consecutive days, greater than 14 consecutive days, greater than 21 days, or greater than 28 days. In some instances, the rest period is greater than about 14 consecutive days. In some instances, the cycle of administration of the immunomodulatory compound does not contain a rest period.

In some instances, the immunomodulatory compound, *e.g.,* lenalidomide, is administered in a cycle, wherein the cycle is repeated at least one time. In some instances, the immunomodulatory compound, *e.g.,* lenalidomide, is administered for at least 2 cycles, at least 3 cycles, at least 4 cycles, at least 5 cycles, at least 6 cycles, at least 7 cycles, at least 8 cycles, at least 9 cycles, at least 10 cycles, at least 11 cycles, or at least 12 cycles. In some instances, the immunomodulatory compound, *e.g.,* lenalidomide, is administered for 1, 2, 3, 4, 5, 6, 7, 8, 9, 10, 11, 12, 13, 14, 15, 16, 17, 18, 19, 20, 21, 22, 23, or 24 cycles.

In some instances, the immunomodulatory compound, *e.g.,* lenalidomide, is administered six times daily, five times daily, four times daily, three times daily, twice daily, once daily, every other day, every three days, twice weekly, once weekly or only one time prior to or subsequently to initiation of administration of the T cell therapy. In some instances, the immunomodulatory compound, *e.g.,* lenalidomide, is administered in multiple doses in regular intervals prior to, during, during the course of, and/or after the period of administration of the T cell therapy. In some instances, the immunomodulatory compound, *e.g.,* lenalidomide, is administered in one or more doses in regular intervals prior to the administration of the T cell therapy. In some instances, the immunomodulatory compound, *e.g.,* lenalidomide, is administered in one or more doses in regular intervals after the administration of the T cell therapy. In some instances, one or more of the doses of the immunomodulatory compound, *e.g.,* lenalidomide, can occur simultaneously with the administration of a dose of the T cell therapy.

In some instances, the dose, frequency, duration, timing and/or order of administration of the immunomodulatory compound, *e.g.,* lenalidomide, is determined, based on particular thresholds or criteria of results of the screening step and/or assessment of treatment outcomes described herein, *e.g.,* those described in SectionIII herein.

In some instances, the method involves administering the cell therapy to a subject that has been previously administered a therapeutically effective amount of the immunomodulatory compound. In some instances, the immunomodulatory compound is administered to a subject before administering a dose of cells expressing a recombinant receptor to the subject. In some instances, the treatment with the immunomodulatory compound occurs at the same time as the administration of the dose of cells. In some instances, the immunomodulatory compound is administered after the administration of the dose of cells.

In some instances, the immunomodulatory compound, *e.g.,* lenalidomide, is administered daily for 7, 8, 9, 10, 11, 12, 13, 14, 15, 16, 17, 18, 19, 20, 21, or more than 21 days. In some instances, the immunomodulatory compound, *e.g.,* lenalidomide, is administered twice a day for 7, 8, 9, 10, 11, 12, 13, 14, 15, 16, 17, 18, 19, 20, 21, or more than 21 days. In some instances, the immunomodulatory compound, *e.g.,* lenalidomide, is administered three times a day for 7, 8, 9, 10, 11, 12, 13, 14, 15, 16, 17, 18, 19, 20, 21, or more than 21 days. In some instances, the immunomodulatory compound, *e.g.,* lenalidomide, is administered every other day for 7, 8, 9, 10, 11, 12, 13, 14, 15, 16, 17, 18, 19, 20, 21, or more than 21 days.

In some instances of the methods disclosed herein, the immunomodulatory compound, *e.g.,* lenalidomide, and the T cell therapy are administered simultaneously or near simultaneously.

In some instances, immunomodulatory compound, e.g. lenalidomide, is administered at a dose of from or from about 0.1 mg to about 100 mg, from or from about 0.1 mg to 50 mg, from or from about 0.1 mg to 25 mg, from or from about 0.1 mg to 10 mg, from or from about 0.1 mg to 5 mg, from or from about 0.1 mg to 1 mg, from or from about 1 mg to 100 mg, from or from about 1 mg to 50 mg, from or from about 1 mg to 25 mg, from or from about 1 mg to 10 mg, from or from about 1 mg to 5 mg, from or from about 5 mg to 100 mg, from or from about 5 mg to 50 mg, from or from about 5 mg to 25 mg, from or from about 5 mg to 10 mg, from or from about 10 mg to 100 mg, from or from about 10 mg to 50 mg, from or from 10 mg to 25 mg, from or from about 25 mg to 100 mg, from or from about 25 mg to 50 mg or from or from about 50 mg to 100 mg, each inclusive. In some instances, the amount is a once daily amount of the immunomodulatory compound, e.g. lenalidomide.

In some instances, the immunomodulatory compound, e.g. lenalidomide, is administered at a dosage of from about 1 mg to about 20 mg, e.g., from about 1 mg to about 10 mg, from about 2.5 mg to about 7.5 mg, from about 5 mg to about 15 mg, such as about 5 mg, 10 mg, 15 mg or 20 mg. In some instances, lenalidomide is administered at a dose of from about 10 µg/kg to 5 mg/kg, e.g., about 100 µg/kg to about 2 mg/kg, about 200 µg/kg to about 1 mg/kg, about 400 µg/kg to about 600 µg/kg, such as about 500 µg/kg. In some instances, the amount is a once daily amount of the immunomodulatory compound, e.g. lenalidomide.

In some instances, the immunomodulatory compound, e.g., lenalidomide, is administered at a total daily dosage amount of at least or at least about 0.1 mg per day, 0.5 mg per day, 1.0 mg per day, 2.5 mg per day, 5 mg per day, 10 mg per day, 25 mg per day, 50 mg per day or 100 mg per day. In some instances, the dose of lenalidomide is or is about 25 mg per day. In particular instances, the dose of lenalidomide is or is about10 mg per day.

In some instances, the immunomodulatory compound, e.g. lenalidomide, is administered in an amount greater than or greater than about 1 mg, 2.5 mg, 5 mg, 7.5 mg, 10 mg, 15 mg and less than 25 mg. In some instances, the immunomodulatory compound, e.g. lenalidomide, is administered in an amount greater than or greater than about 1 mg per day, 2.5 mg per day, 5 mg per day, 7.5 mg per day, 10 mg per day, 15 mg per day and less than 25 mg per day.

In any of the aforementioned instances, the immunomodulatory compound, e.g. lenalidomide, may be administered orally. In some instances, the immunomodulatory compound, e.g. lenalidomide, is administered as a tablet or capsule.

In some instances, dosages, such as daily dosages, are administered in one or more divided doses, such as 2, 3, or 4 doses, or in a single formulation. The immunomodulatory compound, *e.g.,* lenalidomide can be administered alone, in the presence of a pharmaceutically acceptable carrier, or in the presence of other therapeutic agents.

It is understood that higher or lower dosages of the immunomodulatory compound could be used, for example depending on the particular agent and the route of administration. In some instances, the immunomodulatory compound may be administered alone or in the form of a pharmaceutical composition wherein the compound is in admixture or mixture with one or more pharmaceutically acceptable carriers, excipients, or diluents. In some instances, the immunomodulatory compound may be administered either systemically or locally to the organ or tissue to be treated. Exemplary routes of administration include, but are not limited to, topical, injection (such as subcutaneous, intramuscular, intradermal, intraperitoneal, intratumoral, and intravenous), oral, sublingual, rectal, transdermal, intranasal, vaginal and inhalation routes. In some instances, the route of administration is oral, parenteral, rectal, nasal, topical, or ocular routes, or by inhalation. In some instances, the immunomodulatory compound is administered orally. In some instances, the immunomodulatory compound is administered orally in solid dosage forms, such as capsules, tablets and powders, or in liquid dosage forms, such as elixirs, syrups and suspensions.

Once improvement of the patient's disease has occurred, the dose may be adjusted for preventative or maintenance treatment. For example, the dosage or the frequency of administration, or both, may be reduced as a function of the symptoms, to a level at which the desired therapeutic or prophylactic effect is maintained. If symptoms have been alleviated to an appropriate level, treatment may cease. Patients may, however, require intermittent treatment on a long-term basis upon any recurrence of symptoms. Patients may also require chronic treatment on a long-term basis.

### C. LYMPHODEPLETING TREATMENT

In some aspects, the disclosed methods can further include administering one or more lymphodepleting therapies, such as prior to or simultaneous with initiation of administration of the T cell therapy. In some instances, the lymphodepleting therapy comprises administration of a phosphamide, such as cyclophosphamide. In some instances, the lymphodepleting therapy can include administration of fludarabine.

In some aspects, preconditioning subjects with immunodepleting (e.g., lymphodepleting) therapies can improve the effects of adoptive cell therapy (ACT). Preconditioning with lymphodepleting agents, including combinations of cyclosporine and fludarabine, have been effective in improving the efficacy of transferred tumor infiltrating lymphocyte (TIL) cells in cell therapy, including to improve response and/or persistence of the transferred cells. *See, e.g.,* Dudley et al., Science, 298, 850-54 (2002); Rosenberg et al., Clin Cancer Res, 17(13):4550-4557 (2011). Likewise, in the context of CAR+ T cells, several studies have incorporated lymphodepleting agents, most commonly cyclophosphamide, fludarabine, bendamustine, or combinations thereof, sometimes accompanied by low-dose irradiation. *See* Han et al. Journal of Hematology & Oncology, 6:47 (2013); Kochenderfer et al., Blood, 119: 2709-2720 (2012); Kalos et al., Sci Transl Med, 3(95):95ra73 (2011); Clinical Trial Study Record Nos.: NCT02315612; NCT01822652.

Such preconditioning can be carried out with the goal of reducing the risk of one or more of various outcomes that could dampen efficacy of the therapy. These include the phenomenon known as "cytokine sink," by which T cells, B cells, NK cells compete with TILs for homeostatic and activating cytokines, such as IL-2, IL-7, and/or IL-15; suppression of TILs by regulatory T cells, NK cells, or other cells of the immune system; impact of negative regulators in the tumor microenvironment. Muranski et al., Nat Clin Pract Oncol. December; 3(12): 668-681 (2006).

Thus in some instances, the disclosed method further involves administering a lymphodepleting therapy to the subject. In some instances, the method involves administering the lymphodepleting therapy to the subject prior to the administration of the dose of cells. In some instances, the lymphodepleting therapy contains a chemotherapeutic agent such as fludarabine and/or cyclophosphamide. In some instances, the administration of the cells and/or the lymphodepleting therapy is carried out via outpatient delivery.

In some instances, the methods include administering a preconditioning agent, such as a lymphodepleting or chemotherapeutic agent, such as cyclophosphamide, fludarabine, or combinations thereof, to a subject prior to the administration of the dose of cells. For example, the subject may be administered a preconditioning agent at least 2 days prior, such as at least 3, 4, 5, 6, or 7 days prior, to the first or subsequent dose. In some instances, the subject is administered a preconditioning agent no more than 7 days prior, such as no more than 6, 5, 4, 3, or 2 days prior, to the administration of the dose of cells.

In some instances, the subject is preconditioned with cyclophosphamide at a dose between or between about 20 mg/kg and 100 mg/kg, such as between or between about 40 mg/kg and 80 mg/kg. In some aspects, the subject is preconditioned with or with about 60 mg/kg of cyclophosphamide. In some instances, the fludarabine can be administered in a single dose or can be administered in a plurality of doses, such as given daily, every other day or every three days. In some instances, the cyclophosphamide is administered once daily for one or two days.

In some instances, where the lymphodepleting agent comprises fludarabine, the subject is administered fludarabine at a dose between or between about 1 mg/m² and 100 mg/m², such as between or between about 10 mg/m² and 75 mg/m², 15 mg/m² and 50 mg/m², 20 mg/m² and 30 mg/m², or 24 mg/m² and 26 mg/m². In some instances, the subject is administered 25 mg/m² of fludarabine. In some instances, the fludarabine can be administered in a single dose or can be administered in a plurality of doses, such as given daily, every other day or every three days. In some instances, fludarabine is administered daily, such as for 1-5 days, for example, for 3 to 5 days.

In some instances, the lymphodepleting agent comprises a combination of agents, such as a combination of cyclophosphamide and fludarabine. Thus, the combination of agents may include cyclophosphamide at any dose or administration schedule, such as those described above, and fludarabine at any dose or administration schedule, such as those described above. For example, in some aspects, the subject is administered 60 mg/kg (~2 g/m²) of cyclophosphamide and 3 to 5 doses of 25 mg/m² fludarabine prior to the dose of cells.

In one exemplary dosage regime, prior to receiving the first dose, subjects receive an immunomodulatory compound 1 day before the administration of cells and an lymphodepleting preconditioning chemotherapy of cyclophosphamide and fludarabine (CY/FLU), which is administered at least two days before the first dose of CAR-expressing cells and generally no more than 7 days before administration of cells. In another exemplary dosage regime, subjects receive the immunomodulatory compound concurrently with the administration of cells, such as on the same day. In yet another exemplary dosage regime, subjects receive the immunomodulatory compound several days after the administration of cells, such as 7, 8, 9, 10, 11, 12, 13, 14, or more than 14 days after. In some cases, for example, cyclophosphadmide is given from 24 to 27 days after the administration of the immunomodulatory compound, e.g., lenalidomide. After preconditioning treatment, subjects are administered the dose of CAR-expressing T cells as described above.

In some instances, the administration of the preconditioning agent prior to infusion of the dose of cells improves an outcome of the treatment. For example, in some aspects, preconditioning improves the efficacy of treatment with the dose or increases the persistence of the recombinant receptor-expressing cells (e.g., CAR-expressing cells, such as CAR-expressing T cells) in the subject. In some instances, preconditioning treatment increases disease-free survival, such as the percent of subjects that are alive and exhibit no minimal residual or molecularly detectable disease after a given period of time following the dose of cells. In some instances, the time to median disease-free survival is increased.

Once the cells are administered to the subject (e.g., human), the biological activity of the engineered cell populations in some aspects is measured by any of a number of known methods. Parameters to assess include specific binding of an engineered or natural T cell or other immune cell to antigen, *in vivo,* e.g., by imaging, or *ex vivo,* e.g., by ELISA or flow cytometry. In certain instances, the ability of the engineered cells to destroy target cells can be measured using any suitable method known in the art, such as cytotoxicity assays described in, for example, Kochenderfer et al., J. Immunotherapy, 32(7): 689-702 (2009) , and Herman et al. J. Immunological Methods, 285(1): 25-40 (2004). In certain instances, the biological activity of the cells also can be measured by assaying expression and/or secretion of certain cytokines, such as CD107a, IFNγ, IL-2, and TNF. In some aspects the biological activity is measured by assessing clinical outcome, such as reduction in tumor burden or load. In some aspects, toxic outcomes, persistence and/or expansion of the cells, and/or presence or absence of a host immune response, are assessed.

In some instances, the administration of the preconditioning agent prior to infusion of the dose of cells improves an outcome of the treatment such as by improving the efficacy of treatment with the dose or increases the persistence of the recombinant receptor-expressing cells (e.g., CAR-expressing cells, such as CAR-expressing T cells) in the subject. Therefore, in some instances, the dose of preconditioning agent given in the method which is a combination therapy with the immunomodulatory compound and cell therapy is higher than the dose given in the method without the immunomodulatory compound.

### II. T CELL THERAPY AND ENGINEERING CELLS

In some instances, the T cell therapy for use in accord with the disclosed combination therapy methods includes administering engineered cells expressing recombinant receptors designed to recognize and/or specifically bind to molecules associated with the disease or condition and result in a response, such as an immune response against such molecules upon binding to such molecules. The receptors may include chimeric receptors, *e.g.,* chimeric antigen receptors (CARs), and other transgenic antigen receptors including transgenic T cell receptors (TCRs).

In some instances, the cells contain or are engineered to contain an engineered receptor, *e.g.,* an engineered antigen receptor, such as a chimeric antigen receptor (CAR), or a T cell receptor (TCR). Also disclosed are populations of such cells, compositions containing such cells and/or enriched for such cells, such as in which cells of a certain type such as T cells or CD8⁺ or CD4⁺ cells are enriched or selected. Among the compositions are pharmaceutical compositions and formulations for administration, such as for adoptive cell therapy. Also disclosed are therapeutic methods for administering the cells and compositions to subjects, *e.g.,* patients.

Thus, in some instances, the cells include one or more nucleic acids introduced via genetic engineering, and thereby express recombinant or genetically engineered products of such nucleic acids. In some instances, gene transfer is accomplished by first stimulating the cells, such as by combining it with a stimulus that induces a response such as proliferation, survival, and/or activation, *e.g.,* as measured by expression of a cytokine or activation marker, followed by transduction of the activated cells, and expansion in culture to numbers sufficient for clinical applications.

### A. RECOMBINANT RECEPTORS

The cells generally express recombinant receptors, such as antigen receptors including functional non-TCR antigen receptors, *e.g.,* chimeric antigen receptors (CARs), and other antigen-binding receptors such as transgenic T cell receptors (TCRs). Also among the receptors are other chimeric receptors.

### 1. Chimeric Antigen Receptors (CARs)

In some instances, engineered cells, such as T cells, employed in the disclosed instances express a CAR with specificity for a particular antigen (or marker or ligand), such as an antigen expressed on the surface of a particular cell type. In some instances, the antigen is a polypeptide. In some instances, it is a carbohydrate or other molecule. In some instances, the antigen is selectively expressed or overexpressed on cells of the disease or condition, e.g., the tumor or pathogenic cells, as compared to normal or non-targeted cells or tissues. In other instances, the antigen is expressed on normal cells and/or is expressed on the engineered cells.

In particular instances, the recombinant receptor, such as chimeric receptor, contains an intracellular signaling region, which includes a cytoplasmic signaling domain or region (also interchangeably called an intracellular signaling domain or region), such as a cytoplasmic (intracellular) region capable of inducing a primary activation signal in a T cell, for example, a cytoplasmic signaling domain or region of a T cell receptor (TCR) component (e.g. a cytoplasmic signaling domain or region of a zeta chain of a CD3-zeta (CD3ζ) chain or a functional variant or signaling portion thereof) and/or that comprises an immunoreceptor tyrosine-based activation motif (ITAM).

In some instances, the chimeric receptor further contains an extracellular ligand-binding domain that specifically binds to a ligand (e.g. antigen) antigen. In some instances, the chimeric receptor is a CAR that contains an extracellular antigen-recognition domain that specifically binds to an antigen. In some instances, the ligand, such as an antigen, is a protein expressed on the surface of cells.

In some instances, the CAR is a TCR-like CAR and the antigen is a processed peptide antigen, such as a peptide antigen of an intracellular protein, which, like a TCR, is recognized on the cell surface in the context of a major histocompatibility complex (MHC) molecule. Generally, a CAR containing an antibody or antigen-binding fragment that exhibits TCR-like specificity directed against peptide-MHC complexes also may be referred to as a TCR-like CAR. In some instances, the extracellular antigen binding domain specific for an MHC-peptide complex of a TCR-like CAR is linked to one or more intracellular signaling components, in some aspects via linkers and/or transmembrane domain(s). In some instances, such molecules can typically mimic or approximate a signal through a natural antigen receptor, such as a TCR, and, optionally, a signal through such a receptor in combination with a costimulatory receptor.

Exemplary antigen receptors, including CARs, and methods for engineering and introducing such receptors into cells, include those described, for example, in international patent application publication numbers WO200014257, WO2013126726, WO2012/129514, WO2014031687, WO2013/166321, WO2013/071154, WO2013/123061, WO2016/0046724, WO2016/014789, WO2016/090320, WO2016/094304, WO2017/025038, WO2017/173256, U.S. patent application publication numbers US2002131960, US2013287748, US20130149337, U.S. Patent Nos. 6,451,995, 7,446,190, 8,252,592, 8,339,645, 8,398,282, 7,446,179, 6,410,319, 7,070,995, 7,265,209, 7,354,762, 7,446,191, 8,324,353, 8,479,118, and 9,765,342, and European patent application number EP2537416, and/or those described by Sadelain et al., Cancer Discov., 3(4): 388-398 (2013); Davila et al., PLoS ONE 8(4): e61338 (2013); Turtle et al., Curr. Opin. Immunol., 24(5): 633-39 (2012); Wu et al., Cancer, 18(2): 160-75 (2012). In some aspects, the antigen receptors include a CAR as described in U.S. Patent No. 7,446,190, and those described in International Patent Application Publication No. WO/2014055668 A1. Examples of the CARs include CARs as disclosed in any of the aforementioned publications, such as WO2014031687, US 8,339,645, US 7,446,179, US 2013/0149337, U.S. Patent No. 7,446,190, US Patent No. 8,389,282, Kochenderfer et al., Nature Reviews Clinical Oncology, 10, 267-276 (2013); Wang et al., J. Immunother. 35(9): 689-701 (2012); and Brentjens et al., Sci Transl Med. 5(177) (2013). See also WO2014031687, US 8,339,645, US 7,446,179, US 2013/0149337, U.S. Patent No. 7,446,190, and US Patent No. 8,389,282. The chimeric receptors, such as CARs, generally include an extracellular antigen binding domain, such as a portion of an antibody molecule, generally a variable heavy (V_{H}) chain region and/or variable light (V_{L}) chain region of the antibody, *e.g.,* an scFv antibody fragment.

In some instances, the CAR is constructed with a specificity for a particular antigen (or marker or ligand), such as an antigen expressed in a particular cell type to be targeted by adoptive therapy, *e.g.,* a cancer marker, and/or an antigen intended to induce a dampening response, such as an antigen expressed on a normal or non-diseased cell type. Thus, the CAR typically includes in its extracellular portion one or more antigen binding molecules, such as one or more antigen-binding fragment, domain, or portion, or one or more antibody variable domains, and/or antibody molecules. In some instances, the CAR includes an antigen-binding portion or portions of an antibody molecule, such as a single-chain antibody fragment (scFv) derived from the variable heavy (V_{H}) and variable light (V_{L}) chains of a monoclonal antibody (mAb), or a single domain antibody (sdAb), such as sdFv, nanobody, V_{H}H and V_{NAR}. In some instances, an antigen-binding fragment comprises antibody variable regions joined by a flexible linker.

Among the antigen binding domains included in the CARs are antibody fragments. An "antibody fragment" or "antigen-binding fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; heavy chain variable (V_{H}) regions, single-chain antibody molecules such as scFvs and single-domain antibodies comprising only the V_{H} region; and multispecific antibodies formed from antibody fragments. In particular instances, the antibodies are single-chain antibody fragments comprising a heavy chain variable (V_{H}) region and/or a light chain variable (V_{L}) region, such as scFvs.

In certain instances, multispecific binding molecules, e.g., multispecific chimeric receptors, such as multispecific CARs, can contain any of the multispecific antibodies, including, *e.g.* bispecific antibodies, multispecific single-chain antibodies, *e.g.,* diabodies, triabodies, and tetrabodies, tandem di-scFvs, and tandem tri-scFvs.

Single-domain antibodies (sdAbs) are antibody fragments comprising all or a portion of the heavy chain variable region or all or a portion of the light chain variable region of an antibody. In certain instances, a single-domain antibody is a human single-domain antibody.

Antibody fragments can be made by various techniques, including but not limited to proteolytic digestion of an intact antibody as well as production by recombinant host cells. In some instances, the antibodies are recombinantly-produced fragments, such as fragments comprising arrangements that do not occur naturally, such as those with two or more antibody regions or chains joined by synthetic linkers, *e.g.,* peptide linkers, and/or that are may not be produced by enzyme digestion of a naturally-occurring intact antibody. In some aspects, the antibody fragments are scFvs.

In some instances, the antibody or antigen-binding fragment thereof is a single-chain antibody fragment, such as a single chain variable fragment (scFv) or a diabody or a single domain antibody (sdAb). In some instances, the antibody or antigen-binding fragment is a single domain antibody comprising only the V_{H} region. In some instances, the antibody or antigen binding fragment is an scFv comprising a heavy chain variable (V_{H}) region and a light chain variable (V_{L}) region.

In some instances, the antigen targeted by the receptor is a polypeptide. In some instances, it is a carbohydrate or other molecule. In some instances, the antigen is selectively expressed or overexpressed on cells of the disease or condition, *e.g.,* the tumor or pathogenic cells, as compared to normal or non-targeted cells or tissues. In other instances, the antigen is expressed on normal cells and/or is expressed on the engineered cells.

In certain instances, the antigen or includes αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD133, CD138, CD171, chondroitin sulfate proteoglycan 4 (CSPG4), epidermal growth factor protein (EGFR), truncated epidermal growth factor protein (tEGFR), type III epidermal growth factor receptor mutation (EGFR vIII), epithelial glycoprotein 2 (EPG-2), epithelial glycoprotein 40 (EPG-40), ephrinB2, ephrine receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G Protein Coupled Receptor 5D (GPCR5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha(II,-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some instances include antigens associated with a B cell malignancy, such as any of a number of known B cell marker. In some instances, the antigen is or includes CD20, CD19, CD22, ROR1, CD45, CD21, CD5, CD33, Igkappa, Iglambda, CD79a, CD79b or CD30.

In some instances, the antigen is or includes a pathogen-specific or pathogen-expressed antigen. In some instances, the antigen is a viral antigen (such as a viral antigen from HIV, HCV, HBV, etc.), bacterial antigens, and/or parasitic antigens.

In some instances, the CAR is an anti-BCMA CAR that is specific for BCMA, e.g. human BCMA. Chimeric antigen receptors containing anti-BCMA antibodies, including mouse anti-human BCMA antibodies and human anti-human BCMA antibodies, and cells expressing such chimeric receptors have been previously described. See Carpenter et al., Clin Cancer Res., 2013, 19(8):2048-2060, US 9,765,342, WO 2016/090320, WO2016090327, WO2010104949A2, WO2016/0046724, WO2016/014789, WO2016/094304, WO2017/025038, and WO2017173256. In some instances, the anti-BCMA CAR contains an antigen-binding domain, such as an scFv, containing a variable heavy (V_{H}) and/or a variable light (V_{L}) region derived from an antibody described in WO 2016/090320 or WO2016090327. In some instances, the antigen-binding domain is an antibody fragment containing a variable heavy chain (V_{H}) and a variable light chain (V_{L}) region. In some aspects, the V_{H} region is or includes an amino acid sequence having at least 90%, 91%, 92%, 93%, 94%, 95%, 96%, 97%, 98% or 99% sequence identity to the V_{H} region amino acid sequence set forth in any of SEQ ID NOs: 30, 32, 34, 36, 38, 40, 42, 77, 79, 81, 83, 85, 87, 89, 91, 93, 95, 97, 99, 101, 103, 105, 107, 109, 111, 181, 183, 185 and 188; and/or the V_{L} region is or includes an amino acid sequence having at least 90%, 91%, 92%, 93%, 94%, 95%, 96%, 97%, 98%, or 99% sequence identity to the V_{L} region amino acid sequence set forth in any of SEQ ID NOs: 31, 33, 35, 37, 39, 41, 43, 78, 80, 82, 84, 86, 88, 90, 92, 94, 96, 98, 100, 102, 104, 106, 108, 110, 112, 182, 184, 186 and 189.

In some instances, the antigen-binding domain, such as an scFv, contains a V_{H} set forth in SEQ ID NO: 30 and a V_{L} set forth in SEQ ID NO:31. In some instances, the antigen-binding domain, such as an scFv, contains a V_{H} set forth in SEQ ID NO: 32 and a V_{L} set forth in SEQ ID NO:33. In some instances, the antigen-binding domain, such as an scFv, contains a V_{H} set forth in SEQ ID NO: 34 and a V_{L} set forth in SEQ ID NO: 35. In some instances, the antigen-binding domain, such as an scFv, contains a V_{H} set forth in SEQ ID NO: 36 and a V_{L} set forth in SEQ ID NO:37. In some instance the antigen-binding domain, such as an scFv, contains a V_{H} set forth in SEQ ID NO: 38 and a V_{L} set forth in SEQ ID NO: 39. In some instances, the antigen-binding domain, such as an scFv, contains a V_{H} set forth in SEQ ID NO: 40 and a V_{L} set forth in SEQ ID NO: 41. In some instances, the antigen-binding domain, such as an scFv, contains a V_{H} set forth in SEQ ID NO: 42 and a V_{L} set forth in SEQ ID NO: 43. In some instances, the antigen-binding domain, such as an scFv, contains a V_{H} set forth in SEQ ID NO: 77 and a V_{L} set forth in SEQ ID NO: 78. In some instances, the antigen-binding domain, such as an scFv, contains a V_{H} set forth in SEQ ID NO: 79 and a V_{L} set forth in SEQ ID NO: 80. In some instances, the antigen-binding domain, such as an scFv, contains a V_{H} set forth in SEQ ID NO: 81 and a V_{L} set forth in SEQ ID NO: 82. In some instances, the antigen-binding domain, such as an scFv, contains a V_{H} set forth in SEQ ID NO: 83 and a V_{L} set forth in SEQ ID NO: 84. In some instances, the antigen-binding domain, such as an scFv, contains a V_{H} set forth in SEQ ID NO: 85 and a V_{L} set forth in SEQ ID NO: 86. In some instances, the antigen-binding domain, such as an scFv, contains a V_{H} set forth in SEQ ID NO: 87 and a V_{L} set forth in SEQ ID NO: 88. In some instances, the antigen-binding domain, such as an scFv, contains a V_{H} set forth in SEQ ID NO: 89 and a V_{L} set forth in SEQ ID NO: 90. In some instances, the antigen-binding domain, such as an scFv, contains a V_{H} set forth in SEQ ID NO: 91 and a V_{L} set forth in SEQ ID NO: 92. In some instances, the antigen-binding domain, such as an scFv, contains a V_{H} set forth in SEQ ID NO: 93 and a V_{L} set forth in SEQ ID NO: 94. In some instances, the antigen-binding domain, such as an scFv, contains a V_{H} set forth in SEQ ID NO: 95 and a V_{L} set forth in SEQ ID NO: 96. In some instances, the antigen-binding domain, such as an scFv, contains a V_{H} set forth in SEQ ID NO: 97 and a V_{L} set forth in SEQ ID NO: 98. In some instances, the antigen-binding domain, such as an scFv, contains a V_{H} set forth in SEQ ID NO: 99 and a V_{L} set forth in SEQ ID NO: 100. In some instances, the antigen-binding domain, such as an scFv, contains a V_{H} set forth in SEQ ID NO: 101 and a V_{L} set forth in SEQ ID NO: 102. In some instances, the antigen-binding domain, such as an scFv, contains a V_{H} set forth in SEQ ID NO: 103 and a V_{L} set forth in SEQ ID NO: 104. In some instances, the antigen-binding domain, such as an scFv, contains a V_{H} set forth in SEQ ID NO: 105 and a V_{L} set forth in SEQ ID NO: 106. In some instances, the antigen-binding domain, such as an scFv, contains a V_{H} set forth in SEQ ID NO: 107 and a V_{L} set forth in SEQ ID NO: 106. In some instances, the antigen-binding domain, such as an scFv, contains a V_{H} set forth in SEQ ID NO: 30 and a V_{L} set forth in SEQ ID NO: 108. In some instances, the antigen-binding domain, such as an scFv, contains a V_{H} set forth in SEQ ID NO: 109 and a V_{L} set forth in SEQ ID NO: 110. In some instances, the antigen-binding domain, such as an scFv, contains a V_{H} set forth in SEQ ID NO: 111 and a V_{L} set forth in SEQ ID NO: 112. In some instances, the antigen-binding domain, such as an scFv, contains a V_{H} set forth in SEQ ID NO: 181 and a V_{L} set forth in SEQ ID NO: 182. In some instances, the antigen-binding domain, such as an scFv, contains a V_{H} set forth in SEQ ID NO: 183 and a V_{L} set forth in SEQ ID NO: 184.In some instances, the antigen-binding domain, such as an scFv, contains a V_{H} set forth in SEQ ID NO: 185 and a V_{L} set forth in SEQ ID NO: 186. In some instances, the antigen-binding domain, such as an scFv, contains a V_{H} set forth in SEQ ID NO: 187 and a V_{L} set forth in SEQ ID NO: 188. In some instances, the V_{H} or V_{L} has a sequence of amino acids that exhibits at least 85%, 86%, 87%, 88%, 89%, 90%, 91%, 92%, 93%, 94%, 95%, 96%, 97%, 98%, 99% or more sequence identity to any of the foregoing V_{H} or V_{L} sequences, and retains binding to BCMA. In some instances, the V_{H} region is amino-terminal to the V_{L} region. In some instances, the V_{H} region is carboxy-terminal to the V_{L} region. In some instances, the variable heavy and variable light chains are connected by a linker. In some instances, the linker is set forth in SEQ ID NO: 70, 72, 73, 74 or 189.

In some instances, the CAR is an anti-CD19 CAR that is specific for CD19, e.g. human CD19. In some instances the antigen-binding domain includes a V_{H} and/or V_{L} derived from FMC63, which, in some aspects, can be an scFv. In some instances the scFv and/or V_{H} domains is derived from FMC63. FMC63 generally refers to a mouse monoclonal IgG1 antibody raised against Nalm-1 and -16 cells expressing CD19 of human origin (Ling, N. R., *et al.* (1987). *Leucocyte typing III.* 302). The FMC63 antibody comprises CDRH1 and H2 set forth in SEQ ID NOS: 44, 45 respectively, and CDRH3 set forth in SEQ ID NOS: 46 or 47 and CDRL1 set forth in SEQ ID NOS: 48 and CDR L2 set forth in SEQ ID NO: 49 or 50 and CDR L3 sequences set forth in SEQ ID NO: 51 or 52. The FMC63 antibody comprises the heavy chain variable region (V_{H}) comprising the amino acid sequence of SEQ ID NO: 53 and the light chain variable region (V_{L}) comprising the amino acid sequence of SEQ ID NO: 54. In some instances, the svFv comprises a variable light chain containing the CDRL1 sequence of SEQ ID NO:48, a CDRL2 sequence of SEQ ID NO:49, and a CDRL3 sequence of SEQ ID NO:51 and/or a variable heavy chain containing a CDRH1 sequence of SEQ ID NO:44, a CDRH2 sequence of SEQ ID NO:45, and a CDRH3 sequence of SEQ ID NO:46. In some instances, the scFv comprises a variable heavy chain region of FMC63 set forth in SEQ ID NO:53 and a variable light chain region of FMC63 set forth in SEQ ID NO:54. In some instances, the variable heavy and variable light chains are connected by a linker. In some instances, the linker is set forth in SEQ ID NO: 70, 72, 73, 74 or 189. In some instances, the scFv comprises, in order, a V_{H}, a linker, and a V_{L}. In some instances, the scFv comprises, in order, a V_{L}, a linker, and a V_{H}. In some instances, the svFv is encoded by a sequence of nucleotides set forth in SEQ ID NO:69 or a sequence that exhibits at least 85%, 86%, 87%, 88%, 89%, 90%, 91%, 92%, 93%, 94%, 95%, 96%, 97%, 98%, or 99% sequence identity to SEQ ID NO:69. In some instances, the scFv comprises the sequence of amino acids set forth in SEQ ID NO:55 or a sequence that exhibits at least 85%, 86%, 87%, 88%, 89%, 90%, 91%, 92%, 93%, 94%, 95%, 96%, 97%, 98%, or 99% sequence identity to SEQ ID NO:55.

In some instances the antigen-binding domain includes a V_{H} and/or V_{L} derived from SJ25C1, which, in some aspects, can be an scFv. SJ25C1 is a mouse monoclonal IgG1 antibody raised against Nalm-1 and -16 cells expressing CD19 of human origin (Ling, N. R., *et al.* (1987). *Leucocyte typing III.* 302). The SJ25C1 antibody comprises CDRH1, H2 and H3 set forth in SEQ ID NOS: 59-61, respectively, and CDRL1, L2 and L3 sequences set forth in SEQ ID NOS: 56-58, respectively. The SJ25C1 antibody comprises the heavy chain variable region (V_{H}) comprising the amino acid sequence of SEQ ID NO: 62 and the light chain variable region (V_{L}) comprising the amino acid sequence of SEQ ID NO: 63. In some instances, the svFv comprises a variable light chain containing the CDRL1 sequence of SEQ ID NO:56, a CDRL2 sequence of SEQ ID NO: 57, and a CDRL3 sequence of SEQ ID NO:58 and/or a variable heavy chain containing a CDRH1 sequence of SEQ ID NO:59, a CDRH2 sequence of SEQ ID NO:60, and a CDRH3 sequence of SEQ ID NO:61. In some instances, the scFv comprises a variable heavy chain region of SJ25C1 set forth in SEQ ID NO:62 and a variable light chain region of SJ25C1 set forth in SEQ ID NO:63. In some instances, the variable heavy and variable light chain are connected by a linker. In some instances, the linker is set forth in SEQ ID NO:64. In some instances, the scFv comprises, in order, a V_{H}, a linker, and a V_{L}. In some instances, the scFv comprises, in order, a V_{L}, a linker, and a V_{H}. In some instances, the scFv comprises the sequence of amino acids set forth in SEQ ID NO:65 or a sequence that exhibits at least 85%, 86%, 87%, 88%, 89%, 90%, 91%, 92%, 93%, 94%, 95%, 96%, 97%, 98%, or 99% sequence identity to SEQ ID NO:65.

In some instances, the antibody is an antigen-binding fragment, such as an scFv, that includes one or more linkers joining two antibody domains or regions, such as a heavy chain variable (V_{H}) region and a light chain variable (V_{L}) region. Accordingly, the antibodies include single-chain antibody fragments, such as scFvs and diabodies, particularly human single-chain antibody fragments, typically comprising linker(s) joining two antibody domains or regions, such V_{H} and V_{L} regions. The linker typically is a peptide linker, *e.g.,* a flexible and/or soluble peptide linker, such as one rich in glycine and serine. Among the linkers are those rich in glycine and serine and/or in some cases threonine. In some instances, the linkers further include charged residues such as lysine and/or glutamate, which can improve solubility. In some instances, the linkers further include one or more proline.

In some aspects, the linkers rich in glycine and serine (and/or threonine) include at least 80%, 85%, 90%, 91%, 92%, 93%, 94%, 95%, 96%, 97%, 98%, or 99% such amino acid(s). In some instances, they include at least at or about 50%, 55%, 60%, 70%, or 75%, glycine, serine, and/or threonine. In some instances, the linker is comprised substantially entirely of glycine, serine, and/or threonine. The linkers generally are between about 5 and about 50 amino acids in length, typically between at or about 10 and at or about 30, e.g., 10, 11, 12, 13, 14, 15, 16, 17, 18, 19, 20, 21, 22, 23, 24, 25, 26, 27, 28, 29, or 30, and in some examples between 10 and 25 amino acids in length. Exemplary linkers include linkers having various numbers of repeats of the sequence GGGGS (4GS; SEQ ID NO:19) or GGGS (3GS; SEQ ID NO:71), such as between 2, 3, 4, and 5 repeats of such a sequence. Exemplary linkers include those having or consisting of an sequence set forth in SEQ ID NO:72 (GGGGSGGGGSGGGGS), SEQ ID NO:189 (ASGGGGSGGRASGGGGS), SEQ ID NO:73 (GSTSGSGKPGSGEGSTKG) or SEQ ID NO: 74 (SRGGGGSGGGGSGGGGSLEMA).

In some instances, the recombinant receptor such as the CAR, such as the antibody portion of the recombinant receptor, *e.g.,* CAR, further includes a spacer, which may be or include at least a portion of an immunoglobulin constant region or variant or modified version thereof, such as a hinge region, *e.g.,* an IgG4 hinge region, an IgG1 hinge region, a C_{H}1/C_{L}, and/or Fc region. In some instances, the recombinant receptor further comprises a spacer and/or a hinge region. In some instances, the constant region or portion is of a human IgG, such as IgG4 or IgG1. In some aspects, the portion of the constant region serves as a spacer region between the antigen-recognition component, *e.g.,* scFv, and transmembrane domain. The spacer can be of a length that provides for increased responsiveness of the cell following antigen binding, as compared to in the absence of the spacer.

Exemplary spacers, *e.g.,* hinge regions, include those described in international patent application publication number WO2014031687. In some examples, the spacer is or is about 12 amino acids in length or is no more than 12 amino acids in length. Exemplary spacers include those having at least about 10 to 229 amino acids, about 10 to 200 amino acids, about 10 to 175 amino acids, about 10 to 150 amino acids, about 10 to 125 amino acids, about 10 to 100 amino acids, about 10 to 75 amino acids, about 10 to 50 amino acids, about 10 to 40 amino acids, about 10 to 30 amino acids, about 10 to 20 amino acids, or about 10 to 15 amino acids, and including any integer between the endpoints of any of the listed ranges. In some instances, a spacer region has about 12 amino acids or less, about 119 amino acids or less, or about 229 amino acids or less. In some instances, the spacer is a spacer having at least a particular length, such as having a length that is at least 100 amino acids, such as at least 110, 125, 130, 135, 140, 145, 150, 160, 170, 180, 190, 200, 210, 220, 230, 240, or 250 amino acids in length. Exemplary spacers include IgG4 hinge alone, IgG4 hinge linked to C_{H}2 and C_{H}3 domains, or IgG4 hinge linked to the C_{H}3 domain. Exemplary spacers include IgG4 hinge alone, IgG4 hinge linked to C_{H}2 and C_{H}3 domains, or IgG4 hinge linked to the C_{H}3 domain. Exemplary spacers include, but are not limited to, those described in Hudecek et al., Clin. Cancer Res., 19:3153 (2013), Hudecek et al. (2015) Cancer Immunol Res. 3(2): 125-135, international patent application publication number WO2014031687, U.S. Patent No. 8,822,647 or published app. No. US2014/0271635. In some instances, the spacer includes a sequence of an immunoglobulin hinge region, a C_{H}2 and C_{H}3 region. In some instances, one of more of the hinge, C_{H}2 and C_{H}3 is derived all or in part from IgG4 or IgG2. In some cases, the hinge, C_{H}2 and C_{H}3 is derived from IgG4. In some aspects, one or more of the hinge, C_{H}2 and C_{H}3 is chimeric and contains sequence derived from IgG4 and IgG2. In some examples, the spacer contains an IgG4/2 chimeric hinge, an IgG2/4 C_{H}2, and an IgG4 C_{H}3 region.

In some instances, the spacer, which can be a constant region or portion thereof of an immunoglobulin, is of a human IgG, such as IgG4 or IgG1. In some instances, the spacer has the sequence ESKYGPPCPPCP (set forth in SEQ ID NO: 1). In some instances, the spacer has the sequence set forth in SEQ ID NO: 3. In some instances, the spacer has the sequence set forth in SEQ ID NO: 4. In some instances, the encoded spacer is or contains the sequence set forth in SEQ ID NO: 29. In some instances, the constant region or portion is of IgD. In some instances, the spacer has the sequence set forth in SEQ ID NO: 5. In some instances, the spacer has the sequence set forth in SEQ ID NO: 125.

In some instances, the spacer can be derived all or in part from IgG4 and/or IgG2 and can contain mutations, such as one or more single amino acid mutations in one or more domains. In some examples, the amino acid modification is a substitution of a proline (P) for a serine (S) in the hinge region of an IgG4. In some instances, the amino acid modification is a substitution of a glutamine (Q) for an asparagine (N) to reduce glycosylation heterogeneity, such as an N177Q mutation at position 177, in the C_{H}2 region, of the full-length IgG4 Fc sequence or an N176Q. at position 176, in the CH2 region, of the full-length IgG4 Fc sequence.

Other exemplary spacer regions include hinge regions derived from CD8a, CD28, CTLA4, PD-1, or FcyRIIIa. In some instances, the spacer contains a truncated extracellular domain or hinge region of a CD8a, CD28, CTLA4, PD-1, or FcyRIIIa. In some instances, the spacer is a truncated CD28 hinge region. In some instances, a short oligo- or polypeptide linker, for example, a linker of between 2 and 10 amino acids in length, such as one containing alanines or alanine and arginine, e.g., alanine triplet (AAA) or RAAA (SEQ ID NO: 180), is present and forms a linkage between the scFv and the spacer region of the CAR. In some instances, the spacer has the sequence set forth in SEQ ID NO: 114. In some instances, the spacer has the sequence set forth in SEQ ID NO: 116. In some instances, the spacer has the sequence set forth in any of SEQ ID NOs: 117-119, In some instances, the spacer has the sequence set forth in SEQ ID NO: 120. In some instances, the spacer has the sequence set forth in SEQ ID NO: 122. In some instances, the spacer has the sequence set forth in SEQ ID NO: 124.

In some instances, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 1, 3, 4, 5 or 29, 114, 116, 117, 118, 119, 120, 122, 124, or 125.

This antigen recognition domain generally is linked to one or more intracellular signaling components, such as signaling components that mimic stimulation and/or activation through an antigen receptor complex, such as a TCR complex, in the case of a CAR, and/or signal via another cell surface receptor. Thus, in some instances, the antigen-binding component (*e.g.,* antibody) is linked to one or more transmembrane and intracellular signaling domains. In some instances, the transmembrane domain is fused to the extracellular domain. In one instance, a transmembrane domain that naturally is associated with one of the domains in the receptor, *e.g.,* CAR, is used. In some instances, the transmembrane domain is selected or modified by amino acid substitution to avoid binding of such domains to the transmembrane domains of the same or different surface membrane proteins to minimize interactions with other members of the receptor complex.

The transmembrane domain in some instances is derived either from a natural or from a synthetic source. Where the source is natural, the domain in some aspects is derived from any membrane-bound or transmembrane protein. Transmembrane regions include those derived from (*i.e.* comprise at least the transmembrane region(s) of) the alpha, beta or zeta chain of the T-cell receptor, CD28, CD3 epsilon, CD45, CD4, CD5, CD8, CD8a, CD9, CD16, CD22, CD33, CD37, CD64, CD80, CD86, CD 134, CD137 (4-1BB), CD154, CTLA-4, or PD-1. Alternatively the transmembrane domain in some instances is synthetic. In some aspects, the synthetic transmembrane domain comprises predominantly hydrophobic residues such as leucine and valine. In some aspects, a triplet of phenylalanine, tryptophan and valine will be found at each end of a synthetic transmembrane domain. In some instances, the linkage is by linkers, spacers, and/or transmembrane domain(s). Exemplary sequences of transmembrane domains are or comprise the sequences set forth in SEQ ID NOs: 8, 115, 121, 123, 178, or 179.

Among the intracellular signaling domains are those that mimic or approximate a signal through a natural antigen receptor, a signal through such a receptor in combination with a costimulatory receptor, and/or a signal through a costimulatory receptor alone. In some instances, a short oligo- or polypeptide linker, for example, a linker of between 2 and 10 amino acids in length, such as one containing glycines and serines, *e.g.,* glycine-serine doublet, is present and forms a linkage between the transmembrane domain and the cytoplasmic signaling domain of the CAR.

The receptor, *e.g.,* the CAR, generally includes at least one intracellular signaling component or components. In some instances, the receptor includes an intracellular component of a TCR complex, such as a TCR CD3 chain that mediates T-cell stimulation and/or activation and cytotoxicity, *e.g.,* CD3 zeta chain. Thus, in some aspects, the antigen-binding portion is linked to one or more cell signaling modules. In some instances, cell signaling modules include CD3 transmembrane domain, CD3 intracellular signaling domains, and/or other CD transmembrane domains. In some instances, the receptor, *e.g.,* CAR, further includes a portion of one or more additional molecules such as Fc receptor γ, CD8, CD4, CD25 or CD16. For example, in some aspects, the CAR or other chimeric receptor includes a chimeric molecule between CD3-zeta (CD3-ζ) or Fc receptor γ and CD8, CD4, CD25 or CD16.

In some instances, upon ligation of the CAR or other chimeric receptor, the cytoplasmic domain or intracellular signaling domain of the receptor stimulates and/or activates at least one of the normal effector functions or responses of the immune cell, *e.g.,* T cell engineered to express the CAR. For example, in some contexts, the CAR induces a function of a T cell such as cytolytic activity or T-helper activity, such as secretion of cytokines or other factors. In some instances, a truncated portion of an intracellular signaling domain of an antigen receptor component or costimulatory molecule is used in place of an intact immunostimulatory chain, for example, if it transduces the effector function signal. In some instances, the intracellular signaling domain or domains include the cytoplasmic sequences of the T cell receptor (TCR), and in some aspects also those of co-receptors that in the natural context act in concert with such receptors to initiate signal transduction following antigen receptor engagement, and/or any derivative or variant of such molecules, and/or any synthetic sequence that has the same functional capability.

In the context of a natural TCR, full activation generally requires not only signaling through the TCR, but also a costimulatory signal. Thus, in some instances, to promote full activation, a component for generating secondary or co-stimulatory signal is also included in the CAR. In other instances, the CAR does not include a component for generating a costimulatory signal. In some aspects, an additional CAR is expressed in the same cell and provides the component for generating the secondary or costimulatory signal.

T cell stimulation and/or activation is in some aspects described as being mediated by two classes of cytoplasmic signaling sequences: those that initiate antigen-dependent primary stimulation and/or activation through the TCR (primary cytoplasmic signaling regions, domains or sequences), and those that act in an antigen-independent manner to provide a secondary or co-stimulatory signal (secondary cytoplasmic signaling regions, domains or sequences). In some aspects, the CAR includes one or both of such signaling components.

In some aspects, the CAR includes a primary cytoplasmic signaling regions, domains or sequence that regulates primary activation of the TCR complex. Primary cytoplasmic signaling regions, domains or sequences that act in a stimulatory manner may contain signaling motifs which are known as immunoreceptor tyrosine-based activation motifs or ITAMs. Examples of ITAM containing primary cytoplasmic signaling sequences include those derived from TCR zeta, FcR gamma, FcR beta, CD3 gamma, CD3 delta, CD3 epsilon, CD8, CD22, CD79a, CD79b and CD66d. In some instances, cytoplasmic signaling molecule(s) in the CAR contain(s) a cytoplasmic signaling domain, portion thereof, or sequence derived from CD3 zeta. In some instances, the CAR includes a signaling region and/or transmembrane portion of a costimulatory receptor, such as CD28, 4-1BB, OX40 (CD134), CD27, DAP10, DAP12, ICOS and/or other costimulatory receptors. In some aspects, the same CAR includes both the primary cytoplasmic signaling region and costimulatory signaling components.

In some instances, one or more different recombinant receptors can contain one or more different intracellular signaling region(s) or domain(s). In some instances, the primary cytoplasmic signaling region is included within one CAR, whereas the costimulatory component is provided by another receptor, e.g., another CAR recognizing another antigen. In some instances, the CARs include activating or stimulatory CARs, and costimulatory CARs, both expressed on the same cell (*see* WO2014/055668).

In some aspects, the cells include one or more stimulatory or activating CAR and/or a costimulatory CAR. In some instances, the cells further include inhibitory CARs (iCARs, see Fedorov et al., Sci. Transl. Medicine, 5(215) (2013), such as a CAR recognizing an antigen other than the one associated with and/or specific for the disease or condition whereby an activating signal delivered through the disease-targeting CAR is diminished or inhibited by binding of the inhibitory CAR to its ligand, *e.g.,* to reduce off-target effects.

In certain instances, the intracellular signaling domain comprises a CD28 transmembrane and signaling domain linked to a CD3 (*e.g.,* CD3-zeta) intracellular domain. In some instances, the intracellular signaling domain comprises a chimeric CD28 and CD137 (4-1BB, TNFRSF9) co-stimulatory domains, linked to a CD3 zeta intracellular domain.

In some instances, the CAR encompasses one or more, e.g., two or more, costimulatory domains and primary cytoplasmic signaling region, in the cytoplasmic portion. Exemplary CARs include intracellular components, such as intracellular signaling region(s) or domain(s), of CD3-zeta, CD28, CD137 (4-1BB), OX40 (CD134), CD27, DAP10, DAP12, NKG2D and/or ICOS. In some instances, the chimeric antigen receptor contains an intracellular signaling region or domain of a T cell costimulatory molecule, e.g., from CD28, CD137 (4-1BB), OX40 (CD134), CD27, DAP10, DAP12, NKG2D and/or ICOS, in some cases, between the transmembrane domain and intracellular signaling region or domain. In some aspects, the T cell costimulatory molecule is one or more of CD28, CD137 (4-1BB), OX40 (CD134), CD27, DAP10, DAP12, NKG2D and/or ICOS.

In some cases, CARs are referred to as first, second, and/or third generation CARs. In some aspects, a first generation CAR is one that solely provides a CD3-chain induced signal upon antigen binding; in some aspects, a second-generation CARs is one that provides such a signal and costimulatory signal, such as one including an intracellular signaling domain from a costimulatory receptor such as CD28 or CD137; in some aspects, a third generation CAR is one that includes multiple costimulatory domains of different costimulatory receptors.

In some instances, the chimeric antigen receptor includes an extracellular portion containing an antibody or antibody fragment. In some aspects, the chimeric antigen receptor includes an extracellular portion containing the antibody or fragment and an intracellular signaling domain. In some instances, the antibody or fragment includes an scFv and the intracellular domain contains an ITAM. In some aspects, the intracellular signaling domain includes a signaling domain of a zeta chain of a CD3-zeta (CD3ζ) chain. In some instances, the chimeric antigen receptor includes a transmembrane domain linking the extracellular domain and the intracellular signaling domain. In some aspects, the transmembrane domain contains a transmembrane portion of CD28. In some instances, the chimeric antigen receptor contains an intracellular domain of a T cell costimulatory molecule. The extracellular domain and transmembrane domain can be linked directly or indirectly. In some instances, the extracellular domain and transmembrane are linked by a spacer, such as any described herein. In some instances, the receptor contains extracellular portion of the molecule from which the transmembrane domain is derived, such as a CD28 extracellular portion. In some instances, the chimeric antigen receptor contains an intracellular domain derived from a T cell costimulatory molecule or a functional variant thereof, such as between the transmembrane domain and intracellular signaling domain. In some aspects, the T cell costimulatory molecule is CD28 or 4-1BB.

For example, in some instances, the CAR contains an antibody, *e.g.,* an antibody fragment, a transmembrane domain that is or contains a transmembrane portion of CD28 or a functional variant thereof, and an intracellular signaling domain containing a signaling portion of CD28 or functional variant thereof and a signaling portion of CD3 zeta or functional variant thereof. In some instances, the CAR contains an antibody, *e.g.,* antibody fragment, a transmembrane domain that is or contains a transmembrane portion of CD28 or a functional variant thereof, and an intracellular signaling domain containing a signaling portion of a 4-1BB or functional variant thereof and a signaling portion of CD3 zeta or functional variant thereof. In some such instances, the receptor further includes a spacer containing a portion of an Ig molecule, such as a human Ig molecule, such as an Ig hinge, *e.g.* an IgG4 hinge, such as a hinge-only spacer.

In some instances, the transmembrane domain of the recombinant receptor, *e.g.,* the CAR, is or includes a transmembrane domain of human CD28 (*e.g.* Accession No. P10747.1) or CD8a (Accession No. P01732.1) or variant thereof, such as a transmembrane domain that comprises the sequence of amino acids set forth in SEQ ID NO: 8, 115, 178, or 179 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 8, 115, 178, or 179; in some instances, the transmembrane-domain containing portion of the recombinant receptor comprises the sequence of amino acids set forth in SEQ ID NO: 9 or a sequence of amino acids having at least at or about 85%, 86%, 87%, 88%, 89%, 90%, 91%, 92%, 93%, 94%, 95%, 96%, 97%, 98%, 99% or more sequence identity thereto.

In some instances, the intracellular signaling component(s) of the recombinant receptor, *e.g.* the CAR, contains an intracellular costimulatory signaling domain of human CD28 or a functional variant or portion thereof, such as a domain with an LL to GG substitution at positions 186-187 of a native CD28 protein. For example, the intracellular signaling domain can comprise the sequence of amino acids set forth in SEQ ID NO: 10 or 11 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 10 or 11. In some instances, the intracellular domain comprises an intracellular costimulatory signaling domain of 4-1BB (*e.g.* Accession No. Q07011.1) or functional variant or portion thereof, such as the sequence of amino acids set forth in SEQ ID NO: 12 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 12.

In some instances, the intracellular signaling domain of the recombinant receptor, *e.g.* the CAR, comprises a human CD3 zeta stimulatory signaling domain or functional variant thereof, such as an 112 AA cytoplasmic domain of isoform 3 of human CD3ζ (Accession No. P20963.2) or a CD3 zeta signaling domain as described in U.S. Patent No. 7,446,190 or U.S. Patent No. 8,911,993. For example, in some instances, the intracellular signaling domain comprises the sequence of amino acids as set forth in SEQ ID NO: 13, 14 or 15 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 13, 14 or 15.

In some aspects, the spacer contains only a hinge region of an IgG, such as only a hinge of IgG4 or IgG1, such as the hinge only spacer set forth in SEQ ID NO: 1 or SEQ ID NO: 125. In other instances, the spacer is or contains an Ig hinge, *e.g.,* an IgG4-derived hinge, optionally linked to a CH2 and/or CH3 domains. In some instances, the spacer is an Ig hinge, *e.g.,* an IgG4 hinge, linked to CH2 and CH3 domains, such as set forth in SEQ ID NO: 4. In some instances, the spacer is an Ig hinge, *e.g.,* an IgG4 hinge, linked to a CH3 domain only, such as set forth in SEQ ID NO: 3. In some instances, the spacer is or comprises a glycine-serine rich sequence or other flexible linker such as known flexible linkers. In some instances, the spacer is a CD8a hinge, such as set forth in any of SEQ ID NOs: 117-119, an FcyRIIIa hinge, such as set forth in SEQ ID NO: 124, a CTLA4 hinge, such as set forth in SEQ ID NO: 120, or a PD-1 hinge, such as set forth in SEQ ID NO: 122.

For example, in some instances, the CAR includes an antibody such as an antibody fragment, including scFvs, a spacer, such as a spacer containing a portion of an immunoglobulin molecule, such as a hinge region and/or one or more constant regions of a heavy chain molecule, such as an Ig-hinge containing spacer, a transmembrane domain containing all or a portion of a CD28-derived transmembrane domain, a CD28-derived intracellular signaling domain, and a CD3 zeta signaling domain. In some instances, the CAR includes an antibody or fragment, such as scFv, a spacer such as any of the Ig-hinge containing spacers, a CD28-derived transmembrane domain, a 4-1BB-derived intracellular signaling domain, and a CD3 zeta-derived signaling domain.

The recombinant receptors, such as CARs, expressed by the cells administered to the subject generally recognize or specifically bind to a molecule that is expressed in, associated with, and/or specific for the disease or condition or cells thereof being treated. Upon specific binding to the molecule, *e.g.,* antigen, the receptor generally delivers an immunostimulatory signal, such as an ITAM-transduced signal, into the cell, thereby promoting an immune response targeted to the disease or condition. For example, in some instances, the cells express a CAR that specifically binds to an antigen expressed by a cell or tissue of the disease or condition or associated with the disease or condition. Non-limiting exemplary CAR sequences are set forth in SEQ ID NOs: 126-177.

In some instances, the encoded CAR can sequence can further include a signal sequence or signal peptide that directs or delivers the CAR to the surface of the cell in which the CAR is expressed. In some instances, the signal peptide is derived from a transmembrane protein. In some examples the signal peptide is derived from CD8a, CD33, or an IgG. Exemplary signal peptides include the sequences set forth in SEQ ID NOs: 21, 75 and 76 or variant thereof.

In some instances, the CAR includes an anti-CD19 antibody such as an antibody fragment, including scFvs, a spacer, such as a spacer containing a portion of an immunoglobulin molecule, such as a hinge region and/or one or more constant regions of a heavy chain molecule, such as any of the Ig-hinge containing spacers or other spacers described herein, a transmembrane domain containing all or a portion of a CD28-derived transmembrane domain, a CD28-derived intracellular signaling domain, and a CD3 zeta signaling domain. In some instances, the CAR includes an anti-CD19 antibody or fragment, such as scFv, a spacer such as any of the Ig-hinge containing spacers or other spacers described herein, a CD28-derived transmembrane domain, a 4-1BB-derived intracellular signaling domain, and a CD3 zeta-derived signaling domain. In some instances, such CAR constructs further includes a T2A ribosomal skip element and/or a tEGFR sequence, *e.g.,* downstream of the CAR.

In some instances, the CAR includes an anti-BCMA antibody or fragment, such as any of the anti-human BCMA antibodies, including sdAbs and scFvs, described herein, a spacer such as any of the Ig-hinge containing spacers or other spacers described herein, a CD28 transmembrane domain, a CD28 intracellular signaling domain, and a CD3 zeta signaling domain. In some instances, the CAR includes an anti-BCMA antibody or fragment, such as any of the anti-human BCMA antibodies, including sdAbs and scFvs described herein, a spacer such as any of the Ig-hinge containing spacers or other spacers described herein, a CD28 transmembrane domain, a 4-1BB intracellular signaling domain, and a CD3 zeta signaling domain. In some instances, such CAR constructs further includes a T2A ribosomal skip element and/or a tEGFR sequence, *e.g.,* downstream of the CAR.

### 2. Chimeric A uto-Antibody Receptor (CAAR)

In some instances, the recombinant receptor is a chimeric autoantibody receptor (CAAR). In some instances, the CAAR binds, e.g., specifically binds, or recognizes, an autoantibody. In some instances, a cell expressing the CAAR, such as a T cell engineered to express a CAAR, can be used to bind to and kill autoantibody-expressing cells, but not normal antibody expressing cells. In some instances, CAAR-expressing cells can be used to treat an autoimmune disease associated with expression of self-antigens, such as autoimmune diseases. In some instances, CAAR-expressing cells can target B cells that ultimately produce the autoantibodies and display the autoantibodies on their cell surfaces, mark these B cells as disease-specific targets for therapeutic intervention. In some instances, CAAR-expressing cells can be used to efficiently targeting and killing the pathogenic B cells in autoimmune diseases by targeting the disease-causing B cells using an antigen-specific chimeric autoantibody receptor. In some instances, the recombinant receptor is a CAAR, such as any described in U.S. Patent Application Pub. No. US 2017/0051035.

In some instances, the CAAR comprises an autoantibody binding domain, a transmembrane domain, and one or more intracellular signaling region or domain (also interchangeably called a cytoplasmic signaling domain or region). In some instances, the intracellular signaling region comprises an intracellular signaling domain. In some instances, the intracellular signaling domain is or comprises a primary signaling region, a signaling domain that is capable of stimulating and/or inducing a primary activation signal in a T cell, a signaling domain of a T cell receptor (TCR) component (e.g. an intracellular signaling domain or region of a zeta chain of a CD3-zeta (CD3ζ) chain or a functional variant or signaling portion thereof), and/or a signaling domain comprising an immunoreceptor tyrosine-based activation motif (ITAM).

In some instances, the autoantibody binding domain comprises an autoantigen or a fragment thereof. The choice of autoantigen can depend upon the type of autoantibody being targeted. For example, the autoantigen may be chosen because it recognizes an autoantibody on a target cell, such as a B cell, associated with a particular disease state, e.g. an autoimmune disease, such as an autoantibody-mediated autoimmune disease. In some instances, the autoimmune disease includes pemphigus vulgaris (PV). Exemplary autoantigens include desmoglein 1 (Dsg1) and Dsg3.

### 3. TCRs

In some instances, engineered cells, such as T cells, are provided that express a T cell receptor (TCR) or antigen-binding portion thereof that recognizes an peptide epitope or T cell epitope of a target polypeptide, such as an antigen of a tumor, viral or autoimmune protein. In some aspects, the TCR is or includes a recombinant TCR.

In some instances, a "T cell receptor" or "TCR" is a molecule that contains a variable α and β chains (also known as TCRα and TCRβ, respectively) or a variable γ and δ chains (also known as TCRα and TCRβ, respectively), or antigen-binding portions thereof, and which is capable of specifically binding to a peptide bound to an MHC molecule. In some instances, the TCR is in the αβ form. Typically, TCRs that exist in αβ and γδ forms are generally structurally similar, but T cells expressing them may have distinct anatomical locations or functions. A TCR can be found on the surface of a cell or in soluble form. Generally, a TCR is found on the surface of T cells (or T lymphocytes) where it is generally responsible for recognizing antigens bound to major histocompatibility complex (MHC) molecules.

Unless otherwise stated, the term "TCR" should be understood to encompass full TCRs as well as antigen-binding portions or antigen-binding fragments thereof. In some instances, the TCR is an intact or full-length TCR, including TCRs in the αβ form or γδ form. In some instances, the TCR is an antigen-binding portion that is less than a full-length TCR but that binds to a specific peptide bound in an MHC molecule, such as binds to an MHC-peptide complex. In some cases, an antigen-binding portion or fragment of a TCR can contain only a portion of the structural domains of a full-length or intact TCR, but yet is able to bind the peptide epitope, such as MHC-peptide complex, to which the full TCR binds. In some cases, an antigen-binding portion contains the variable domains of a TCR, such as variable α chain and variable β chain of a TCR, sufficient to form a binding site for binding to a specific MHC-peptide complex. Generally, the variable chains of a TCR contain complementarity determining regions involved in recognition of the peptide, MHC and/or MHC-peptide complex.

In some instances, the variable domains of the TCR contain hypervariable loops, or complementarity determining regions (CDRs), which generally are the primary contributors to antigen recognition and binding capabilities and specificity. In some instances, a CDR of a TCR or combination thereof forms all or substantially all of the antigen-binding site of a given TCR molecule. The various CDRs within a variable region of a TCR chain generally are separated by framework regions (FRs), which generally display less variability among TCR molecules as compared to the CDRs (see, *e.g.,* Jores et al., Proc. Nat'l Acad. Sci. U.S.A. 87:9138, 1990; Chothia et al., EMBO J. 7:3745, 1988; see also Lefranc et al., Dev. Comp. Immunol. 27:55, 2003). In some instances, CDR3 is the main CDR responsible for antigen binding or specificity, or is the most important among the three CDRs on a given TCR variable region for antigen recognition, and/or for interaction with the processed peptide portion of the peptide-MHC complex. In some contexts, the CDR1 of the alpha chain can interact with the N-terminal part of certain antigenic peptides. In some contexts, CDR1 of the beta chain can interact with the C-terminal part of the peptide. In some contexts, CDR2 contributes most strongly to or is the primary CDR responsible for the interaction with or recognition of the MHC portion of the MHC-peptide complex. In some instances, the variable region of the β-chain can contain a further hypervariable region (CDR4 or HVR4), which generally is involved in superantigen binding and not antigen recognition (Kotb (1995) Clinical Microbiology Reviews, 8:411-426).

In some instances, a TCR also can contain a constant domain, a transmembrane domain and/or a short cytoplasmic tail (see, *e.g.,* Janeway et al., Immunobiology: The Immune System in Health and Disease, 3rd Ed., Current Biology Publications, p. 4:33, 1997). In some aspects, each chain of the TCR can possess one N-terminal immunoglobulin variable domain, one immunoglobulin constant domain, a transmembrane region, and a short cytoplasmic tail at the C-terminal end. In some instances, a TCR is associated with invariant proteins of the CD3 complex involved in mediating signal transduction.

In some instances, a TCR chain contains one or more constant domain. For example, the extracellular portion of a given TCR chain (*e.g.,* α-chain or β-chain) can contain two immunoglobulin-like domains, such as a variable domain (*e.g.,* Vα or Vβ; typically amino acids 1 to 116 based on Kabat numbering Kabat et al., "Sequences of Proteins of Immunological Interest, US Dept. Health and Human Services, Public Health Service National Institutes of Health, 1991, 5th ed.) and a constant domain (*e.g.,* α-chain constant domain or Cα, typically positions 117 to 259 of the chain based on Kabat numbering or β chain constant domain or Cβ, typically positions 117 to 295 of the chain based on Kabat) adjacent to the cell membrane. For example, in some cases, the extracellular portion of the TCR formed by the two chains contains two membrane-proximal constant domains, and two membrane-distal variable domains, which variable domains each contain CDRs. The constant domain of the TCR may contain short connecting sequences in which a cysteine residue forms a disulfide bond, thereby linking the two chains of the TCR. In some instances, a TCR may have an additional cysteine residue in each of the α and β chains, such that the TCR contains two disulfide bonds in the constant domains.

In some instances, the TCR chains contain a transmembrane domain. In some instances, the transmembrane domain is positively charged. In some cases, the TCR chain contains a cytoplasmic tail. In some cases, the structure allows the TCR to associate with other molecules like CD3 and subunits thereof. For example, a TCR containing constant domains with a transmembrane region may anchor the protein in the cell membrane and associate with invariant subunits of the CD3 signaling apparatus or complex. The intracellular tails of CD3 signaling subunits (*e.g.* CD3γ, CD36, CD3ε and CD3ζ chains) contain one or more immunoreceptor tyrosine-based activation motif or ITAM that are involved in the signaling capacity of the TCR complex.

In some instances, the TCR may be a heterodimer of two chains α and β (or optionally γ and δ) or it may be a single chain TCR construct. In some instances, the TCR is a heterodimer containing two separate chains (α and β chains or γ and δ chains) that are linked, such as by a disulfide bond or disulfide bonds.

In some instances, the TCR can be generated from a known TCR sequence(s), such as sequences of Vα,β chains, for which a substantially full-length coding sequence is readily available. Methods for obtaining full-length TCR sequences, including V chain sequences, from cell sources are well known. In some instances, nucleic acids encoding the TCR can be obtained from a variety of sources, such as by polymerase chain reaction (PCR) amplification of TCR-encoding nucleic acids within or isolated from a given cell or cells, or synthesis of publicly available TCR DNA sequences.

In some instances, the recombinant receptors include recombinant TCRs and/or TCRs cloned from naturally occurring T cells. In some instances, a high-affinity T cell clone for a target antigen (e.g., a cancer antigen) is identified, isolated from a patient, and introduced into the cells. In some instances, the TCR clone for a target antigen has been generated in transgenic mice engineered with human immune system genes (e.g., the human leukocyte antigen system, or HLA). See, e.g., tumor antigens (see, e.g., Parkhurst et al. (2009) Clin Cancer Res. 15:169-180 and Cohen et al. (2005) J Immunol. 175:5799-5808. In some instances, phage display is used to isolate TCRs against a target antigen (see, e.g., Varela-Rohena et al. (2008) Nat Med. 14:1390-1395 and Li (2005) Nat Biotechnol. 23:349-354.

In some instances, the TCR is obtained from a biological source, such as from cells such as from a T cell (*e.g.* cytotoxic T cell), T-cell hybridomas or other publicly available source. In some instances, the T-cells can be obtained from *in vivo* isolated cells. In some instances, the TCR is a thymically selected TCR. In some instances, the TCR is a neoepitope-restricted TCR. In some instances, the T- cells can be a cultured T-cell hybridoma or clone. In some instances, the TCR or antigen-binding portion thereof can be synthetically generated from knowledge of the sequence of the TCR.

In some instances, the TCR is generated from a TCR identified or selected from screening a library of candidate TCRs against a target polypeptide antigen, or target T cell epitope thereof. TCR libraries can be generated by amplification of the repertoire of Vα and Vβ from T cells isolated from a subject, including cells present in PBMCs, spleen or other lymphoid organ. In some cases, T cells can be amplified from tumor-infiltrating lymphocytes (TILs). In some instances, TCR libraries can be generated from CD4⁺ or CD8⁺ cells. In some instances, the TCRs can be amplified from a T cell source of a normal of healthy subject, *i.e.* normal TCR libraries. In some instances, the TCRs can be amplified from a T cell source of a diseased subject, *i.e.* diseased TCR libraries. In some instances, degenerate primers are used to amplify the gene repertoire of Vα and Vβ, such as by RT-PCR in samples, such as T cells, obtained from humans. In some instances, scTv libraries can be assembled from naive Vα and Vβ libraries in which the amplified products are cloned or assembled to be separated by a linker. Depending on the source of the subject and cells, the libraries can be HLA allele-specific. Alternatively, in some instances, TCR libraries can be generated by mutagenesis or diversification of a parent or scaffold TCR molecule. In some aspects, the TCRs are subjected to directed evolution, such as by mutagenesis, *e.g.,* of the α or β chain. In some aspects, particular residues within CDRs of the TCR are altered. In some instances, selected TCRs can be modified by affinity maturation. In some instances, antigen-specific T cells may be selected, such as by screening to assess CTL activity against the peptide. In some aspects, TCRs, *e.g.* present on the antigen-specific T cells, may be selected, such as by binding activity, *e.g.,* particular affinity or avidity for the antigen.

In some instances, the TCR or antigen-binding portion thereof is one that has been modified or engineered. In some instances, directed evolution methods are used to generate TCRs with altered properties, such as with higher affinity for a specific MHC-peptide complex. In some instances, directed evolution is achieved by display methods including, but not limited to, yeast display (Holler et al., (2003) Nat Immunol, 4, 55-62; Holler et al., (2000) Proc Natl Acad Sci USA, 97, 5387-92), phage display (Li et al., (2005) Nat Biotechnol, 23, 349-54), or T cell display (Chervin et al., (2008) J Immunol Methods, 339, 175-84). In some instances, display approaches involve engineering, or modifying, a known, parent or reference TCR. For example, in some cases, a wild-type TCR can be used as a template for producing mutagenized TCRs in which in one or more residues of the CDRs are mutated, and mutants with an desired altered property, such as higher affinity for a desired target antigen, are selected.

In some instances, peptides of a target polypeptide for use in producing or generating a TCR of interest are known or can be readily identified as a matter of routine. In some instances, peptides suitable for use in generating TCRs or antigen-binding portions can be determined based on the presence of an HLA-restricted motif in a target polypeptide of interest, such as a target polypeptide described below. In some instances, peptides are identified using computer prediction models as a matter of routine. In some instances, for predicting MHC class I binding sites, such models include, but are not limited to, ProPred1 (Singh and Raghava (2001) Bioinformatics 17(12): 1236-1237, and SYFPEITHI (see Schuler et al., (2007) Immunoinformatics Methods in Molecular Biology, 409(1): 75-93 2007). In some instances, the MHC-restricted epitope is HLA-A0201, which is expressed in approximately 39-46% of all Caucasians and therefore, represents a suitable choice of MHC antigen for use preparing a TCR or other MHC-peptide binding molecule.

HLA-A0201-binding motifs and the cleavage sites for proteasomes and immune-proteasomes using computer prediction models are known. For predicting MHC class I binding sites, such models include, but are not limited to, ProPred1 (described in more detail in Singh and Raghava, ProPred: prediction of HLA-DR binding sites. BIOINFORMATICS 17(12): 1236-1237 2001), and SYFPEITHI (see Schuler et al., SYFPEITHI, Database for Searching and T-Cell Epitope Prediction. in Immunoinformatics Methods in Molecular Biology, vol 409(1): 75-93 2007)

In some instances, the TCR or antigen binding portion thereof may be a recombinantly produced natural protein or mutated form thereof in which one or more property, such as binding characteristic, has been altered. In some instances, a TCR may be derived from one of various animal species, such as human, mouse, rat, or other mammal. A TCR may be cell-bound or in soluble form. In some instances, for purposes of the disclosed methods, the TCR is in cell-bound form expressed on the surface of a cell.

In some instances, the TCR is a full-length TCR. In some instances, the TCR is an antigen-binding portion. In some instances, the TCR is a dimeric TCR (dTCR). In some instances, the TCR is a single-chain TCR (sc-TCR). In some instances, a dTCR or scTCR have the structures as described in WO 03/020763, WO 04/033685, WO2011/044186.

In some instances, the TCR contains a sequence corresponding to the transmembrane sequence. In some instances, the TCR does contain a sequence corresponding to cytoplasmic sequences. In some instances, the TCR is capable of forming a TCR complex with CD3. In some instances, any of the TCRs, including a dTCR or scTCR, can be linked to signaling domains that yield an active TCR on the surface of a T cell. In some instances, the TCR is expressed on the surface of cells.

In some instances a dTCR contains a first polypeptide wherein a sequence corresponding to a TCR α chain variable region sequence is fused to the N terminus of a sequence corresponding to a TCR α chain constant region extracellular sequence, and a second polypeptide wherein a sequence corresponding to a TCR β chain variable region sequence is fused to the N terminus a sequence corresponding to a TCR β chain constant region extracellular sequence, the first and second polypeptides being linked by a disulfide bond. In some instances, the bond can correspond to the native inter-chain disulfide bond present in native dimeric αβ TCRs. In some instances, the interchain disulfide bonds are not present in a native TCR. For example, in some instances, one or more cysteines can be incorporated into the constant region extracellular sequences of dTCR polypeptide pair. In some cases, both a native and a non-native disulfide bond may be desirable. In some instances, the TCR contains a transmembrane sequence to anchor to the membrane.

In some instances, a dTCR contains a TCR α chain containing a variable α domain, a constant α domain and a first dimerization motif attached to the C-terminus of the constant α domain, and a TCR β chain comprising a variable β domain, a constant β domain and a first dimerization motif attached to the C-terminus of the constant β domain, wherein the first and second dimerization motifs easily interact to form a covalent bond between an amino acid in the first dimerization motif and an amino acid in the second dimerization motif linking the TCR α chain and TCR β chain together.

In some instances, the TCR is a scTCR. Typically, a scTCR can be generated using suitable known methods, See *e.g.,* Soo Hoo, W. F. et al., PNAS (USA) 89, 4759 (1992); Wülfing, C. and Plückthun, A., J. Mol. Biol. 242, 655 (1994); Kurucz, I. et al., PNAS (USA) 90 3830 (1993); International published PCT Nos. WO 96/13593, WO 96/18105, WO99/60120, WO99/18129, WO 03/020763, WO2011/044186; and Schlueter, C. J. et al., J. Mol. Biol. 256, 859 (1996). In some instances, a scTCR contains an introduced non-native disulfide interchain bond to facilitate the association of the TCR chains (see e.g. International published PCT No. WO 03/020763). In some instances, a scTCR is a non-disulfide linked truncated TCR in which heterologous leucine zippers fused to the C-termini thereof facilitate chain association (see *e.g.* International published PCT No. WO99/60120). In some instances, a scTCR contain a TCRα variable domain covalently linked to a TCRβ variable domain via a peptide linker (see *e.g.,* International published PCT No. WO99/18129).

In some instances, a scTCR contains a first segment constituted by an amino acid sequence corresponding to a TCR α chain variable region, a second segment constituted by an amino acid sequence corresponding to a TCR β chain variable region sequence fused to the N terminus of an amino acid sequence corresponding to a TCR β chain constant domain extracellular sequence, and a linker sequence linking the C terminus of the first segment to the N terminus of the second segment.

In some instances, a scTCR contains a first segment constituted by an α chain variable region sequence fused to the N terminus of an α chain extracellular constant domain sequence, and a second segment constituted by a β chain variable region sequence fused to the N terminus of a sequence β chain extracellular constant and transmembrane sequence, and, optionally, a linker sequence linking the C terminus of the first segment to the N terminus of the second segment.

In some instances, a scTCR contains a first segment constituted by a TCR β chain variable region sequence fused to the N terminus of a β chain extracellular constant domain sequence, and a second segment constituted by an α chain variable region sequence fused to the N terminus of a sequence α chain extracellular constant and transmembrane sequence, and, optionally, a linker sequence linking the C terminus of the first segment to the N terminus of the second segment.

In some instances, the linker of a scTCRs that links the first and second TCR segments can be any linker capable of forming a single polypeptide strand, while retaining TCR binding specificity. In some instances, the linker sequence may, for example, have the formula - P-AA-P- wherein P is proline and AA represents an amino acid sequence wherein the amino acids are glycine and serine. In some instances, the first and second segments are paired so that the variable region sequences thereof are orientated for such binding. Hence, in some cases, the linker has a sufficient length to span the distance between the C terminus of the first segment and the N terminus of the second segment, or vice versa, but is not too long to block or reduces bonding of the scTCR to the target ligand. In some instances, the linker can contain from or from about 10 to 45 amino acids, such as 10 to 30 amino acids or 26 to 41 amino acids residues, for example 29, 30, 31 or 32 amino acids. In some instances, the linker has the formula -PGGG-(SGGGG)5-P- wherein P is proline, G is glycine and S is serine (SEQ ID NO: 16). In some instances, the linker has the sequence GSADDAKKDAAKKDGKS (SEQ ID NO: 17)

In some instances, the scTCR contains a covalent disulfide bond linking a residue of the immunoglobulin region of the constant domain of the α chain to a residue of the immunoglobulin region of the constant domain of the β chain. In some instances, the interchain disulfide bond in a native TCR is not present. For example, in some instances, one or more cysteines can be incorporated into the constant region extracellular sequences of the first and second segments of the scTCR polypeptide. In some cases, both a native and a non-native disulfide bond may be desirable.

In some instances of a dTCR or scTCR containing introduced interchain disulfide bonds, the native disulfide bonds are not present. In some instances, the one or more of the native cysteines forming a native interchain disulfide bonds are substituted to another residue, such as to a serine or alanine. In some instances, an introduced disulfide bond can be formed by mutating non-cysteine residues on the first and second segments to cysteine. Exemplary non-native disulfide bonds of a TCR are described in published International PCT No. WO2006/000830.

In some instances, the TCR or antigen-binding fragment thereof exhibits an affinity with an equilibrium binding constant for a target antigen of between or between about 10⁻⁵ and 10⁻¹² M and all individual values and ranges therein. In some instances, the target antigen is an MHC-peptide complex or ligand.

In some instances, nucleic acid or nucleic acids encoding a TCR, such as α and β chains, can be amplified by PCR, cloning or other suitable means and cloned into a suitable expression vector or vectors. The expression vector can be any suitable recombinant expression vector, and can be used to transform or transfect any suitable host. Suitable vectors include those designed for propagation and expansion or for expression or both, such as plasmids and viruses.

In some instances, the vector can a vector of the pUC series (Fermentas Life Sciences), the pBluescript series (Stratagene, La Jolla, Calif.), the pET series (Novagen, Madison, Wis.), the pGEX series (Pharmacia Biotech, Uppsala, Sweden), or the pEX series (Clontech, Palo Alto, Calif.). In some cases, bacteriophage vectors, such as λG10, λGT11, λZapII (Stratagene), λEMBL4, and λNM1149, also can be used. In some instances, plant expression vectors can be used and include pBI01, pBI101.2, pBI101.3, pBI121 and pBIN19 (Clontech). In some instances, animal expression vectors include pEUK-Cl, pMAM and pMAMneo (Clontech). In some instances, a viral vector is used, such as a retroviral vector.

In some instances, the recombinant expression vectors can be prepared using standard recombinant DNA techniques. In some instances, vectors can contain regulatory sequences, such as transcription and translation initiation and termination codons, which are specific to the type of host (e.g., bacterium, fungus, plant, or animal) into which the vector is to be introduced, as appropriate and taking into consideration whether the vector is DNA- or RNA-based. In some instances, the vector can contain a nonnative promoter operably linked to the nucleotide sequence encoding the TCR or antigen-binding portion (or other MHC-peptide binding molecule). In some instances, the promoter can be a non-viral promoter or a viral promoter, such as a cytomegalovirus (CMV) promoter, an SV40 promoter, an RSV promoter, and a promoter found in the long-terminal repeat of the murine stem cell virus. Other known promoters also are contemplated.

In some instances, to generate a vector encoding a TCR, the α and β chains are PCR amplified from total cDNA isolated from a T cell clone expressing the TCR of interest and cloned into an expression vector. In some instances, the α and β chains are cloned into the same vector. In some instances, the α and β chains are cloned into different vectors. In some instances, the generated α and β chains are incorporated into a retroviral, *e*.*g*. lentiviral, vector.

### 4. Multi-targeting

In some instances, the cells and methods include multi-targeting strategies, such as expression of two or more genetically engineered receptors on the cell, each recognizing the same of a different antigen and typically each including a different intracellular signaling component. Such multi-targeting strategies are described, for example, in PCT Pub. No. WO 2014055668 A1 (describing combinations of activating and costimulatory CARs, *e*.*g*., targeting two different antigens present individually on off-target, *e*.*g*., normal cells, but present together only on cells of the disease or condition to be treated) and Fedorov et al., Sci. Transl. Medicine, 5(215) (2013) (describing cells expressing an activating and an inhibitory CAR, such as those in which the activating CAR binds to one antigen expressed on both normal or non-diseased cells and cells of the disease or condition to be treated, and the inhibitory CAR binds to another antigen expressed only on the normal cells or cells which it is not desired to treat).

For example, in some instances, the cells include a receptor expressing a first genetically engineered antigen receptor (*e*.*g*., CAR or TCR) which is capable of inducing an activating signal to the cell, generally upon specific binding to the antigen recognized by the first receptor, *e.g.,* the first antigen. In some instances, the cell further includes a second genetically engineered antigen receptor (*e.g.,* CAR or TCR), *e.g.,* a chimeric costimulatory receptor, which is capable of inducing a costimulatory signal to the immune cell, generally upon specific binding to a second antigen recognized by the second receptor. In some instances, the first antigen and second antigen are the same. In some instances, the first antigen and second antigen are different.

In some instances, the first and/or second genetically engineered antigen receptor (*e.g.* CAR or TCR) is capable of inducing an activating signal to the cell. In some instances, the receptor includes an intracellular signaling component containing ITAM or ITAM-like motifs. In some instances, the activation induced by the first receptor involves a signal transduction or change in protein expression in the cell resulting in initiation of an immune response, such as ITAM phosphorylation and/or initiation of ITAM-mediated signal transduction cascade, formation of an immunological synapse and/or clustering of molecules near the bound receptor (*e.g.* CD4 or CD8, etc.), activation of one or more transcription factors, such as NF-κB and/or AP-1, and/or induction of gene expression of factors such as cytokines, proliferation, and/or survival.

In some instances, the first and/or second receptor includes intracellular signaling domains of costimulatory receptors such as CD28, CD137 (4-1 BB), OX40, and/or ICOS. In some instances, the first and second receptors include an intracellular signaling domain of a costimulatory receptor that are different. In one instance, the first receptor contains a CD28 costimulatory signaling region and the second receptor contain a 4-1BB co-stimulatory signaling region or vice versa.

In some instances, the first and/or second receptor includes both an intracellular signaling domain containing ITAM or ITAM-like motifs and an intracellular signaling domain of a costimulatory receptor.

In some instances, the first receptor contains an intracellular signaling domain containing ITAM or ITAM-like motifs and the second receptor contains an intracellular signaling domain of a costimulatory receptor. The costimulatory signal in combination with the activating signal induced in the same cell is one that results in an immune response, such as a robust and sustained immune response, such as increased gene expression, secretion of cytokines and other factors, and T cell mediated effector functions such as cell killing.

In some instances, neither ligation of the first receptor alone nor ligation of the second receptor alone induces a robust immune response. In some aspects, if only one receptor is ligated, the cell becomes tolerized or unresponsive to antigen, or inhibited, and/or is not induced to proliferate or secrete factors or carry out effector functions. In some such instances, however, when the plurality of receptors are ligated, such as upon encounter of a cell expressing the first and second antigens, a desired response is achieved, such as full immune activation or stimulation, e.g., as indicated by secretion of one or more cytokine, proliferation, persistence, and/or carrying out an immune effector function such as cytotoxic killing of a target cell.

In some instances, the cells expressing the recombinant receptor further include inhibitory CARs (iCARs, see Fedorov et al., Sci. Transl. Medicine, 5(215) (2013), such as a CAR recognizing an antigen other than the one associated with and/or specific for the disease or condition whereby an activating signal delivered through the disease-targeting CAR is diminished or inhibited by binding of the inhibitory CAR to its ligand, *e.g.,* to reduce off-target effects.

In some instances, the two receptors induce, respectively, an activating and an inhibitory signal to the cell, such that binding by one of the receptor to its antigen activates the cell or induces a response, but binding by the second inhibitory receptor to its antigen induces a signal that suppresses or dampens that response. Examples are combinations of activating CARs and inhibitory CARs or iCARs. Such a strategy may be used, for example, in which the activating CAR binds an antigen expressed in a disease or condition but which is also expressed on normal cells, and the inhibitory receptor binds to a separate antigen which is expressed on the normal cells but not cells of the disease or condition.

In some aspects, the chimeric receptor is or includes an inhibitory CAR (e.g. iCAR) and includes intracellular components that dampen or suppress an immune response, such as an ITAM- and/or co stimulatory-promoted response in the cell. Exemplary of such intracellular signaling components are those found on immune checkpoint molecules, including PD-1, CTLA4, LAG3, BTLA, OX2R, TIM-3, TIGIT, LAIR-1, PGE2 receptors, EP2/4 Adenosine receptors including A2AR. In some aspects, the engineered cell includes an inhibitory CAR including a signaling domain of or derived from such an inhibitory molecule, such that it serves to dampen the response of the cell, for example, that induced by an activating and/or costimulatory CAR.

In some instances, the multi-targeting strategy is employed in a case where an antigen associated with a particular disease or condition is expressed on a non-diseased cell and/or is expressed on the engineered cell itself, either transiently (*e*.*g*., upon stimulation in association with genetic engineering) or permanently. In such cases, by requiring ligation of two separate and individually specific antigen receptors, specificity, selectivity, and/or efficacy may be improved.

In some instances, the plurality of antigens, *e*.*g*., the first and second antigens, are expressed on the cell, tissue, or disease or condition being targeted, such as on the cancer cell. In some aspects, the cell, tissue, disease or condition is multiple myeloma or a multiple myeloma cell. In some instances, one or more of the plurality of antigens generally also is expressed on a cell which it is not desired to target with the cell therapy, such as a normal or non-diseased cell or tissue, and/or the engineered cells themselves. In such instances, by requiring ligation of multiple receptors to achieve a response of the cell, specificity and/or efficacy is achieved.

### B. CELLS AND PREPARATION OF CELLS FOR GENETIC ENGINEERING

Among the cells expressing the receptors and administered by the disclosed methods are engineered cells. The genetic engineering generally involves introduction of a nucleic acid encoding the recombinant or engineered component into a composition containing the cells, such as by retroviral transduction, transfection, or transformation.

In some instances, the nucleic acids are heterologous, *i.e.,* normally not present in a cell or sample obtained from the cell, such as one obtained from another organism or cell, which for example, is not ordinarily found in the cell being engineered and/or an organism from which such cell is derived. In some instances, the nucleic acids are not naturally occurring, such as a nucleic acid not found in nature, including one comprising chimeric combinations of nucleic acids encoding various domains from multiple different cell types.

The cells generally are eukaryotic cells, such as mammalian cells, and typically are human cells. In some instances, the cells are derived from the blood, bone marrow, lymph, or lymphoid organs, are cells of the immune system, such as cells of the innate or adaptive immunity, *e*.*g*., myeloid or lymphoid cells, including lymphocytes, typically T cells and/or NK cells. Other exemplary cells include stem cells, such as multipotent and pluripotent stem cells, including induced pluripotent stem cells (iPSCs). The cells typically are primary cells, such as those isolated directly from a subject and/or isolated from a subject and frozen. In some instances, the cells include one or more subsets of T cells or other cell types, such as whole T cell populations, CD4⁺ cells, CD8⁺ cells, and subpopulations thereof, such as those defined by function, activation state, maturity, potential for differentiation, expansion, recirculation, localization, and/or persistence capacities, antigen-specificity, type of antigen receptor, presence in a particular organ or compartment, marker or cytokine secretion profile, and/or degree of differentiation. With reference to the subject to be treated, the cells may be allogeneic and/or autologous. Among the methods include off-the-shelf methods. In some aspects, such as for off-the-shelf technologies, the cells are pluripotent and/or multipotent, such as stem cells, such as induced pluripotent stem cells (iPSCs). In some instances, the methods include isolating cells from the subject, preparing, processing, culturing, and/or engineering them, and re-introducing them into the same subject, before or after cryopreservation.

Among the sub-types and subpopulations of T cells and/or of CD4⁺ and/or of CD8⁺ T cells are naive T (T_{N}) cells, effector T cells (T_{EFF}), memory T cells and sub-types thereof, such as stem cell memory T (T_{SCM}), central memory T (T_{CM}), effector memory T (T_{EM}), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (Treg) cells, helper T cells, such as TH1 cells, TH2 cells, TH3 cells, TH17 cells, TH9 cells, TH22 cells, follicular helper T cells, alpha/beta T cells, and delta/gamma T cells.

In some instances, the cells are natural killer (NK) cells. In some instances, the cells are monocytes or granulocytes, *e*.*g*., myeloid cells, macrophages, neutrophils, dendritic cells, mast cells, eosinophils, and/or basophils.

In some instances, the cells include one or more nucleic acids introduced via genetic engineering, and thereby express recombinant or genetically engineered products of such nucleic acids. In some instances, the nucleic acids are heterologous, *i*.*e*., normally not present in a cell or sample obtained from the cell, such as one obtained from another organism or cell, which for example, is not ordinarily found in the cell being engineered and/or an organism from which such cell is derived. In some instances, the nucleic acids are not naturally occurring, such as a nucleic acid not found in nature, including one comprising chimeric combinations of nucleic acids encoding various domains from multiple different cell types.

In some instances, preparation of the engineered cells includes one or more culture and/or preparation steps. The cells for introduction of the nucleic acid encoding the transgenic receptor such as the CAR, may be isolated from a sample, such as a biological sample, *e*.*g*., one obtained from or derived from a subject. In some instances, the subject from which the cell is isolated is one having the disease or condition or in need of a cell therapy or to which cell therapy will be administered. The subject in some instances is a human in need of a particular therapeutic intervention, such as the adoptive cell therapy for which cells are being isolated, processed, and/or engineered.

Accordingly, the cells in some instances are primary cells, *e*.*g*., primary human cells. The samples include tissue, fluid, and other samples taken directly from the subject, as well as samples resulting from one or more processing steps, such as separation, centrifugation, genetic engineering (*e*.*g*. transduction with viral vector), washing, and/or incubation. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some aspects, the sample from which the cells are derived or isolated is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, *e*.*g*., adoptive cell therapy, samples from autologous and allogeneic sources.

In some instances, the cells are derived from cell lines, *e.g.,* T cell lines. The cells in some instances are obtained from a xenogeneic source, for example, from mouse, rat, non-human primate, and pig.

In some instances, isolation of the cells includes one or more preparation and/or non-affinity based cell separation steps. In some examples, cells are washed, centrifuged, and/or incubated in the presence of one or more reagents, for example, to remove unwanted components, enrich for desired components, lyse or remove cells sensitive to particular reagents. In some examples, cells are separated based on one or more property, such as density, adherent properties, size, sensitivity and/or resistance to particular components.

In some examples, cells from the circulating blood of a subject are obtained, *e*.*g*., by apheresis or leukapheresis. The samples, in some aspects, contain lymphocytes, including T cells, monocytes, granulocytes, B cells, other nucleated white blood cells, red blood cells, and/or platelets, and in some aspects contain cells other than red blood cells and platelets.

In some instances, the blood cells collected from the subject are washed, *e*.*g*., to remove the plasma fraction and to place the cells in an appropriate buffer or media for subsequent processing steps. In some instances, the cells are washed with phosphate buffered saline (PBS). In some instances, the wash solution lacks calcium and/or magnesium and/or many or all divalent cations. In some aspects, a washing step is accomplished a semi-automated "flow-through" centrifuge (for example, the Cobe 2991 cell processor, Baxter) according to the manufacturer's instructions. In some aspects, a washing step is accomplished by tangential flow filtration (TFF) according to the manufacturer's instructions. In some instances, the cells are resuspended in a variety of biocompatible buffers after washing, such as, for example, Ca⁺⁺/Mg⁺⁺ free PBS. In certain instances, components of a blood cell sample are removed and the cells directly resuspended in culture media.

In some instances, the methods include density-based cell separation methods, such as the preparation of white blood cells from peripheral blood by lysing the red blood cells and centrifugation through a Percoll or Ficoll gradient.

In some instances, the isolation methods include the separation of different cell types based on the expression or presence in the cell of one or more specific molecules, such as surface markers, *e*.*g*., surface proteins, intracellular markers, or nucleic acid. In some instances, any known method for separation based on such markers may be used. In some instances, the separation is affinity- or immunoaffinity-based separation. For example, the isolation in some aspects includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

Such separation steps can be based on positive selection, in which the cells having bound the reagents are retained for further use, and/or negative selection, in which the cells having not bound to the antibody or binding partner are retained. In some examples, both fractions are retained for further use. In some aspects, negative selection can be particularly useful where no antibody is available that specifically identifies a cell type in a heterogeneous population, such that separation is best carried out based on markers expressed by cells other than the desired population.

The separation need not result in 100% enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells.

In some examples, multiple rounds of separation steps are carried out, where the positively or negatively selected fraction from one step is subjected to another separation step, such as a subsequent positive or negative selection. In some examples, a single separation step can deplete cells expressing multiple markers simultaneously, such as by incubating cells with a plurality of antibodies or binding partners, each specific for a marker targeted for negative selection. Likewise, multiple cell types can simultaneously be positively selected by incubating cells with a plurality of antibodies or binding partners expressed on the various cell types.

For example, in some aspects, specific subpopulations of T cells, such as cells positive or expressing high levels of one or more surface markers, *e*.*g*., CD28⁺, CD62L⁺, CCR7⁺, CD27⁺, CD127⁺, CD4⁺, CD8⁺, CD45RA⁺, and/or CD45RO⁺ T cells, are isolated by positive or negative selection techniques.

For example, CD3⁺, CD28⁺ T cells can be positively selected using anti-CD3/anti-CD28 conjugated magnetic beads (*e*.*g*., DYNABEADS^{®} M-450 CD3/CD28 T Cell Expander).

In some instances, isolation is carried out by enrichment for a particular cell population by positive selection, or depletion of a particular cell population, by negative selection. In some instances, positive or negative selection is accomplished by incubating cells with one or more antibodies or other binding agent that specifically bind to one or more surface markers expressed or expressed (marker⁺) at a relatively higher level (marker^{high}) on the positively or negatively selected cells, respectively.

In some instances, T cells are separated from a PBMC sample by negative selection of markers expressed on non-T cells, such as B cells, monocytes, or other white blood cells, such as CD14. In some aspects, a CD4⁺ or CD8⁺ selection step is used to separate CD4⁺ helper and CD8⁺ cytotoxic T cells. Such CD4⁺ and CD8⁺ populations can be further sorted into sub-populations by positive or negative selection for markers expressed or expressed to a relatively higher degree on one or more naive, memory, and/or effector T cell subpopulations.

In some instances, CD8⁺ cells are further enriched for or depleted of naive, central memory, effector memory, and/or central memory stem cells, such as by positive or negative selection based on surface antigens associated with the respective subpopulation. In some instances, enrichment for central memory T (T_{CM}) cells is carried out to increase efficacy, such as to improve long-term survival, expansion, and/or engraftment following administration, which in some aspects is particularly robust in such sub-populations. See Terakura et al., Blood.1:72-82 (2012); Wang et al., J Immunother. 35(9):689-701 (2012). In some instances, combining TCM-enriched CD8⁺ T cells and CD4⁺ T cells further enhances efficacy.

In instances, memory T cells are present in both CD62L⁺ and CD62L- subsets of CD8⁺ peripheral blood lymphocytes. PBMC can be enriched for or depleted of CD62L-CD8⁺ and/or CD62L⁺CD8⁺ fractions, such as using anti-CD8 and anti-CD62L antibodies.

In some instances, the enrichment for central memory T (T_{CM}) cells is based on positive or high surface expression of CD45RO, CD62L, CCR7, CD28, CD3, and/or CD 127; in some aspects, it is based on negative selection for cells expressing or highly expressing CD45RA and/or granzyme B. In some aspects, isolation of a CD8⁺ population enriched for T_{CM} cells is carried out by depletion of cells expressing CD4, CD14, CD45RA, and positive selection or enrichment for cells expressing CD62L. In one aspect, enrichment for central memory T (T_{CM}) cells is carried out starting with a negative fraction of cells selected based on CD4 expression, which is subjected to a negative selection based on expression of CD14 and CD45RA, and a positive selection based on CD62L. Such selections in some aspects are carried out simultaneously and in other aspects are carried out sequentially, in either order. In some aspects, the same CD4 expression-based selection step used in preparing the CD8⁺ cell population or subpopulation, also is used to generate the CD4⁺ cell population or subpopulation, such that both the positive and negative fractions from the CD4-based separation are retained and used in subsequent steps of the methods, optionally following one or more further positive or negative selection steps.

In a particular example, a sample of PBMCs or other white blood cell sample is subjected to selection of CD4⁺ cells, where both the negative and positive fractions are retained. The negative fraction then is subjected to negative selection based on expression of CD14 and CD45RA or CD19, and positive selection based on a marker characteristic of central memory T cells, such as CD62L or CCR7, where the positive and negative selections are carried out in either order.

CD4⁺ T helper cells are sorted into naive, central memory, and effector cells by identifying cell populations that have cell surface antigens. CD4⁺ lymphocytes can be obtained by standard methods. In some instances, naive CD4⁺ T lymphocytes are CD45RO-, CD45RA⁺, CD62L⁺, CD4⁺ T cells. In some instances, central memory CD4⁺ cells are CD62L⁺ and CD45RO⁺. In some instances, effector CD4⁺ cells are CD62L- and CD45RO-.

In one example, to enrich for CD4⁺ cells by negative selection, a monoclonal antibody cocktail typically includes antibodies to CD14, CD20, CD11b, CD16, HLA-DR, and CD8. In some instances, the antibody or binding partner is bound to a solid support or matrix, such as a magnetic bead or paramagnetic bead, to allow for separation of cells for positive and/or negative selection. For example, in some instances, the cells and cell populations are separated or isolated using immunomagnetic (or affinitymagnetic) separation techniques (reviewed in Methods in Molecular Medicine, vol. 58: Metastasis Research Protocols, Vol. 2: Cell Behavior In vitro and In vivo, p 17-25 Edited by: S. A. Brooks and U. Schumacher © Humana Press Inc., Totowa, NJ).

In some aspects, the sample or composition of cells to be separated is incubated with small, magnetizable or magnetically responsive material, such as magnetically responsive particles or microparticles, such as paramagnetic beads (*e*.*g*., such as Dynalbeads or MACS beads). The magnetically responsive material, *e*.*g*., particle, generally is directly or indirectly attached to a binding partner, *e.g.,* an antibody, that specifically binds to a molecule, *e.g.,* surface marker, present on the cell, cells, or population of cells that it is desired to separate, *e*.*g*., that it is desired to negatively or positively select.

In some instances, the magnetic particle or bead comprises a magnetically responsive material bound to a specific binding member, such as an antibody or other binding partner. There are many well-known magnetically responsive materials used in magnetic separation methods. Suitable magnetic particles include those described in Molday, U.S. Pat. No. 4,452,773, and in European Patent Specification EP 452342 B. Colloidal sized particles, such as those described in Owen U.S. Pat. No. 4,795,698, and Liberti et al., U.S. Pat. No. 5,200,084 are other examples.

The incubation generally is carried out under conditions whereby the antibodies or binding partners, or molecules, such as secondary antibodies or other reagents, which specifically bind to such antibodies or binding partners, which are attached to the magnetic particle or bead, specifically bind to cell surface molecules if present on cells within the sample.

In some aspects, the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained. In some aspects, a combination of positive and negative selection is performed during the same selection step, where the positive and negative fractions are retained and further processed or subject to further separation steps.

In certain instances, the magnetically responsive particles are coated in primary antibodies or other binding partners, secondary antibodies, lectins, enzymes, or streptavidin. In certain instances, the magnetic particles are attached to cells via a coating of primary antibodies specific for one or more markers. In certain instances, the cells, rather than the beads, are labeled with a primary antibody or binding partner, and then cell-type specific secondary antibody- or other binding partner (*e*.*g*., streptavidin)-coated magnetic particles, are added. In certain instances, streptavidin-coated magnetic particles are used in conjunction with biotinylated primary or secondary antibodies.

In some instances, the magnetically responsive particles are left attached to the cells that are to be subsequently incubated, cultured and/or engineered; in some aspects, the particles are left attached to the cells for administration to a patient. In some instances, the magnetizable or magnetically responsive particles are removed from the cells. Methods for removing magnetizable particles from cells are known and include, *e.g.,* the use of competing non-labeled antibodies, and magnetizable particles or antibodies conjugated to cleavable linkers. In some instances, the magnetizable particles are biodegradable.

In some instances, the affinity-based selection is via magnetic-activated cell sorting (MACS) (Miltenyi Biotec, Auburn, CA). Magnetic Activated Cell Sorting (MACS) systems are capable of high-purity selection of cells having magnetized particles attached thereto. In certain instances, MACS operates in a mode wherein the non-target and target species are sequentially eluted after the application of the external magnetic field. That is, the cells attached to magnetized particles are held in place while the unattached species are eluted. Then, after this first elution step is completed, the species that were trapped in the magnetic field and were prevented from being eluted are freed in some manner such that they can be eluted and recovered. In certain instances, the non-target cells are labelled and depleted from the heterogeneous population of cells.

In certain instances, the isolation or separation is carried out using a system, device, or apparatus that carries out one or more of the isolation, cell preparation, separation, processing, incubation, culture, and/or formulation steps of the methods. In some aspects, the system is used to carry out each of these steps in a closed or sterile environment, for example, to minimize error, user handling and/or contamination. In one example, the system is a system as described in PCT Pub. Number WO2009/072003, or US 20110003380 A1.

In some instances, the system or apparatus carries out one or more, *e.g.,* all, of the isolation, processing, engineering, and formulation steps in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps.

In some aspects, the separation and/or other steps is carried out using CliniMACS system (Miltenyi Biotec), for example, for automated separation of cells on a clinical-scale level in a closed and sterile system. Components can include an integrated microcomputer, magnetic separation unit, peristaltic pump, and various pinch valves. The integrated computer in some aspects controls all components of the instrument and directs the system to perform repeated procedures in a standardized sequence. The magnetic separation unit in some aspects includes a movable permanent magnet and a holder for the selection column. The peristaltic pump controls the flow rate throughout the tubing set and, together with the pinch valves, ensures the controlled flow of buffer through the system and continual suspension of cells.

The CliniMACS system in some aspects uses antibody-coupled magnetizable particles that are supplied in a sterile, non-pyrogenic solution. In some instances, after labelling of cells with magnetic particles the cells are washed to remove excess particles. A cell preparation bag is then connected to the tubing set, which in turn is connected to a bag containing buffer and a cell collection bag. The tubing set consists of pre-assembled sterile tubing, including a pre-column and a separation column, and are for single use only. After initiation of the separation program, the system automatically applies the cell sample onto the separation column. Labelled cells are retained within the column, while unlabeled cells are removed by a series of washing steps. In some instances, the cell populations for use with the methods described herein are unlabeled and are not retained in the column. In some instances, the cell populations for use with the methods described herein are labeled and are retained in the column. In some instances, the cell populations for use with the methods described herein are eluted from the column after removal of the magnetic field, and are collected within the cell collection bag.

In certain instances, separation and/or other steps are carried out using the CliniMACS Prodigy system (Miltenyi Biotec). The CliniMACS Prodigy system in some aspects is equipped with a cell processing unity that permits automated washing and fractionation of cells by centrifugation. The CliniMACS Prodigy system can also include an onboard camera and image recognition software that determines the optimal cell fractionation endpoint by discerning the macroscopic layers of the source cell product. For example, peripheral blood is automatically separated into erythrocytes, white blood cells and plasma layers. The CliniMACS Prodigy system can also include an integrated cell cultivation chamber which accomplishes cell culture protocols such as, *e*.*g*., cell differentiation and expansion, antigen loading, and long-term cell culture. Input ports can allow for the sterile removal and replenishment of media and cells can be monitored using an integrated microscope. See, *e.g.,* Klebanoff et al., J Immunother. 35(9): 651-660 (2012), Terakura et al., Blood. 1:72-82 (2012), and Wang et al., J Immunother. 35(9):689-701 (2012).

In some instances, a cell population described herein is collected and enriched (or depleted) via flow cytometry, in which cells stained for multiple cell surface markers are carried in a fluidic stream. In some instances, a cell population described herein is collected and enriched (or depleted) via preparative scale (FACS)-sorting. In certain instances, a cell population described herein is collected and enriched (or depleted) by use of microelectromechanical systems (MEMS) chips in combination with a FACS-based detection system (see, *e.g.,* WO 2010/033140, Cho et al., Lab Chip 10, 1567-1573 (2010); and Godin et al., J Biophoton. 1(5):355-376 (2008). In both cases, cells can be labeled with multiple markers, allowing for the isolation of well-defined T cell subsets at high purity.

In some instances, the antibodies or binding partners are labeled with one or more detectable marker, to facilitate separation for positive and/or negative selection. For example, separation may be based on binding to fluorescently labeled antibodies. In some examples, separation of cells based on binding of antibodies or other binding partners specific for one or more cell surface markers are carried in a fluidic stream, such as by fluorescence-activated cell sorting (FACS), including preparative scale (FACS) and/or microelectromechanical systems (MEMS) chips, *e.g.,* in combination with a flow-cytometric detection system. Such methods allow for positive and negative selection based on multiple markers simultaneously.

In some instances, the preparation methods include steps for freezing, *e.g.,* cryopreserving, the cells, either before or after isolation, incubation, and/or engineering. In some instances, the freeze and subsequent thaw step removes granulocytes and, to some extent, monocytes in the cell population. In some instances, the cells are suspended in a freezing solution, *e*.*g*., following a washing step to remove plasma and platelets. Any of a variety of known freezing solutions and parameters in some aspects may be used. One example involves using PBS containing 20% DMSO and 8% human serum albumin (HSA), or other suitable cell freezing media. This is then diluted 1:1 with media so that the final concentration of DMSO and HSA are 10% and 4%, respectively. The cells are generally then frozen to -80° C. at a rate of 1° per minute and stored in the vapor phase of a liquid nitrogen storage tank.

In some instances, the cells are incubated and/or cultured prior to or in connection with genetic engineering. The incubation steps can include culture, cultivation, stimulation, activation, and/or propagation. The incubation and/or engineering may be carried out in a culture vessel, such as a unit, chamber, well, column, tube, tubing set, valve, vial, culture dish, bag, or other container for culture or cultivating cells. In some instances, the compositions or cells are incubated in the presence of stimulating conditions or a stimulatory agent. Such conditions include those designed to induce proliferation, expansion, activation, and/or survival of cells in the population, to mimic antigen exposure, and/or to prime the cells for genetic engineering, such as for the introduction of a recombinant antigen receptor.

The conditions can include one or more of particular media, temperature, oxygen content, carbon dioxide content, time, agents, *e*.*g*., nutrients, amino acids, antibiotics, ions, and/or stimulatory factors, such as cytokines, chemokines, antigens, binding partners, fusion proteins, recombinant soluble receptors, and any other agents designed to activate the cells.

In some instances, the stimulating conditions or agents include one or more agent, e.g., ligand, which is capable of activating an intracellular signaling domain of a TCR complex. In some aspects, the agent turns on or initiates TCR/CD3 intracellular signaling cascade in a T cell. Such agents can include antibodies, such as those specific for a TCR, e.g. anti-CD3. In some instances, the stimulating conditions include one or more agent, e.g. ligand, which is capable of stimulating a costimulatory receptor, e.g., anti-CD28. In some instances, such agents and/or ligands may be, bound to solid support such as a bead, and/or one or more cytokines. Optionally, the expansion method may further comprise the step of adding anti-CD3 and/or anti CD28 antibody to the culture medium (e.g., at a concentration of at least about 0.5 ng/ml). In some instances, the stimulating agents include IL-2, IL-15 and/or IL-7. In some aspects, the IL-2 concentration is at least about 10 units/mL.

In some aspects, incubation is carried out in accordance with techniques such as those described in US Patent No. 6,040,177 to Riddell et al.*,* Klebanoff et al., J Immunother. 35(9): 651-660 (2012), Terakura et al., Blood.1:72-82 (2012), and/or Wang et al., J Immunother. 35(9):689-701 (2012).

In some instances, the T cells are expanded by adding to a culture-initiating composition feeder cells, such as non-dividing peripheral blood mononuclear cells (PBMC), (*e*.*g*., such that the resulting population of cells contains at least about 5, 10, 20, or 40 or more PBMC feeder cells for each T lymphocyte in the initial population to be expanded); and incubating the culture (*e.g.* for a time sufficient to expand the numbers of T cells). In some aspects, the non-dividing feeder cells can comprise gamma-irradiated PBMC feeder cells. In some instances, the PBMC are irradiated with gamma rays in the range of about 3000 to 3600 rads to prevent cell division. In some aspects, the feeder cells are added to culture medium prior to the addition of the populations of T cells.

In some instances, the stimulating conditions include temperature suitable for the growth of human T lymphocytes, for example, at least about 25 degrees Celsius, generally at least about 30 degrees, and generally at or about 37 degrees Celsius. Optionally, the incubation may further comprise adding non-dividing EBV-transformed lymphoblastoid cells (LCL) as feeder cells. LCL can be irradiated with gamma rays in the range of about 6000 to 10,000 rads. The LCL feeder cells in some aspects is provided in any suitable amount, such as a ratio of LCL feeder cells to initial T lymphocytes of at least about 10:1.

In instances, antigen-specific T cells, such as antigen-specific CD4⁺ and/or CD8⁺ T cells, are obtained by stimulating naive or antigen specific T lymphocytes with antigen. For example, antigen-specific T cell lines or clones can be generated to cytomegalovirus antigens by isolating T cells from infected subjects and stimulating the cells *in vitro* with the same antigen.

### C. NUCLEIC ACIDS, VECTORS AND METHODS FOR GENETIC ENGINEERING

In some instances, the cells, e.g., T cells, are genetically engineered to express a recombinant receptor. In some instances, the engineering is carried out by introducing nucleic acid molecules that encode the recombinant receptor. Also disclosed are nucleic acid molecules encoding a recombinant receptor, and vectors or constructs containing such nucleic acids and/or nucleic acid molecules.

In some cases, the nucleic acid sequence encoding the recombinant receptor, e.g., chimeric antigen receptor (CAR), contains a signal sequence that encodes a signal peptide. In some aspects, the signal sequence may encode a signal peptide derived from a native polypeptide. In other aspects, the signal sequence may encode a heterologous or non-native signal peptide. In some instances, the signal peptide is derived from a transmembrane protein. In some examples the signal peptide is derived from CD8a, CD33, or an IgG. Non-limiting exemplary examples of signal peptides include, for example, the CD33 signal peptide set forth in SEQ ID NO:21, CD8a signal peptide set forth in SEQ ID NO:75, or the signal peptide set forth in SEQ ID NO:76 or modified variant thereof.

In some instances, the nucleic acid molecule encoding the recombinant receptor contains at least one promoter that is operatively linked to control expression of the recombinant receptor. In some examples, the nucleic acid molecule contains two, three, or more promoters operatively linked to control expression of the recombinant receptor. In some instances, nucleic acid molecule can contain regulatory sequences, such as transcription and translation initiation and termination codons, which are specific to the type of host (e.g., bacterium, fungus, plant, or animal) into which the nucleic acid molecule is to be introduced, as appropriate and taking into consideration whether the nucleic acid molecule is DNA- or RNA-based. In some instances, the nucleic acid molecule can contain regulatory/control elements, such as a promoter, an enhancer, an intron, a polyadenylation signal, a Kozak consensus sequence, and splice acceptor or donor. In some instances, the nucleic acid molecule can contain a nonnative promoter operably linked to the nucleotide sequence encoding the recombinant receptor and/or one or more additional polypeptide(s). In some instances, the promoter is selected from among an RNA pol I, pol II or pol III promoter. In some instances, the promoter is recognized by RNA polymerase II (e.g., a CMV, SV40 early region or adenovirus major late promoter). In another instance, the promoter is recognized by RNA polymerase III (e.g., a U6 or H1 promoter). In some instances, the promoter can be a non-viral promoter or a viral promoter, such as a cytomegalovirus (CMV) promoter, an SV40 promoter, an RSV promoter, and a promoter found in the long-terminal repeat of the murine stem cell virus. Other known promoters also are contemplated.

In some instances, the promoter is or comprises a constitutive promoter. Exemplary constitutive promoters include, e.g., simian virus 40 early promoter (SV40), cytomegalovirus immediate-early promoter (CMV), human Ubiquitin C promoter (UBC), human elongation factor 1α promoter (EF1α), mouse phosphoglycerate kinase 1 promoter (PGK), and chicken β-Actin promoter coupled with CMV early enhancer (CAGG). In some instances, the constitutive promoter is a synthetic or modified promoter. In some instances, the promoter is or comprises an MND promoter, a synthetic promoter that contains the U3 region of a modified MoMuLV LTR with myeloproliferative sarcoma virus enhancer (see Challita et al. (1995) J. Virol. 69(2):748-755). In some instances, the promoter is a tissue-specific promoter. In another instance, the promoter is a viral promoter. In another instance, the promoter is a non-viral promoter.

In another instance, the promoter is a regulated promoter (e.g., inducible promoter). In some instances, the promoter is an inducible promoter or a repressible promoter. In some instances, the promoter comprises a Lac operator sequence, a tetracycline operator sequence, a galactose operator sequence or a doxycycline operator sequence, or is an analog thereof or is capable of being bound by or recognized by a Lac repressor or a tetracycline repressor, or an analog thereof. In some instances, the nucleic acid molecule does not include a regulatory element, e.g. promoter.

In some instances, the nucleic acid molecule encoding the recombinant receptor, e.g., CAR or other antigen receptor, further includes nucleic acid sequences encoding a marker and/or cells expressing the CAR or other antigen receptor further includes a marker, e.g., a surrogate marker, such as a cell surface marker, which may be used to confirm transduction or engineering of the cell to express the receptor, such as a truncated version of a cell surface receptor, such as truncated EGFR (tEGFR). In some instances, the one or more marker(s) is a transduction marker, surrogate marker and/or a selection marker.

In some instances, the marker is a transduction marker or a surrogate marker. A transduction marker or a surrogate marker can be used to detect cells that have been introduced with the nucleic acid molecule, e.g., a nucleic acid molecule encoding a recombinant receptor. In some instances, the transduction marker can indicate or confirm modification of a cell. In some instances, the surrogate marker is a protein that is made to be co-expressed on the cell surface with the recombinant receptor, e.g. CAR. In particular instances, such a surrogate marker is a surface protein that has been modified to have little or no activity. In certain instances, the surrogate marker is encoded on the same nucleic acid molecule that encodes the recombinant receptor. In some instances, the nucleic acid sequence encoding the recombinant receptor is operably linked to a nucleic acid sequence encoding a marker, optionally separated by an internal ribosome entry site (IRES), or a nucleic acid encoding a self-cleaving peptide or a peptide that causes ribosome skipping, such as a 2A sequence, such as a T2A, a P2A, an E2A or an F2A. Extrinsic marker genes may in some cases be utilized in connection with engineered cell to permit detection or selection of cells and, in some cases, also to promote cell suicide.

Exemplary surrogate markers can include truncated forms of cell surface polypeptides, such as truncated forms that are non-functional and to not transduce or are not capable of transducing a signal or a signal ordinarily transduced by the full-length form of the cell surface polypeptide, and/or do not or are not capable of internalizing. Exemplary truncated cell surface polypeptides including truncated forms of growth factors or other receptors such as a truncated human epidermal growth factor receptor 2 (tHER2), a truncated epidermal growth factor receptor (tEGFR, exemplary tEGFR sequence set forth in SEQ ID NO: 11 or 76) or a prostate-specific membrane antigen (PSMA) or modified form thereof. tEGFR may contain an epitope recognized by the antibody cetuximab (Erbitux^{®}) or other therapeutic anti-EGFR antibody or binding molecule, which can be used to identify or select cells that have been engineered with the tEGFR construct and an encoded exogenous protein, and/or to eliminate or separate cells expressing the encoded exogenous protein. See U.S. Patent No. 8,802,374 and Liu et al., Nature Biotech. 2016 April; 34(4): 430-434). In some aspects, the marker, *e*.*g*. surrogate marker, includes all or part (*e*.*g*., truncated form) of CD34, a NGFR, a CD19 or a truncated CD19, e.g., a truncated non-human CD19, or epidermal growth factor receptor *(e.g.,* tEGFR). In some instances, the marker is or comprises a fluorescent protein, such as green fluorescent protein (GFP), enhanced green fluorescent protein (EGFP), such as super-fold GFP (sfGFP), red fluorescent protein (RFP), such as tdTomato, mCherry, mStrawberry, AsRed2, DsRed or DsRed2, cyan fluorescent protein (CFP), blue green fluorescent protein (BFP), enhanced blue fluorescent protein (EBFP), and yellow fluorescent protein (YFP), and variants thereof, including species variants, monomeric variants, and codon-optimized and/or enhanced variants of the fluorescent proteins. In some instances, the marker is or comprises an enzyme, such as a luciferase, the lacZ gene from *E. coli,* alkaline phosphatase, secreted embryonic alkaline phosphatase (SEAP), chloramphenicol acetyl transferase (CAT). Exemplary light-emitting reporter genes include luciferase (luc), β-galactosidase, chloramphenicol acetyltransferase (CAT), β-glucuronidase (GUS) or variants thereof.

In some instances, the marker is a selection marker. In some instances, the selection marker is or comprises a polypeptide that confers resistance to exogenous agents or drugs. In some instances, the selection marker is an antibiotic resistance gene. In some instances, the selection marker is an antibiotic resistance gene confers antibiotic resistance to a mammalian cell. In some instances, the selection marker is or comprises a Puromycin resistance gene, a Hygromycin resistance gene, a Blasticidin resistance gene, a Neomycin resistance gene, a Geneticin resistance gene or a Zeocin resistance gene or a modified form thereof.

In some aspects, the marker, *e.g.* surrogate marker, includes all or part *(e.g.,* truncated form) of CD34, a NGFR, or epidermal growth factor receptor (*e*.*g*., tEGFR). In some instances, the nucleic acid encoding the marker is operably linked to a polynucleotide encoding for a linker sequence, such as a cleavable linker sequence, *e*.*g*., T2A. For example, a marker, and optionally a linker sequence, can be any as disclosed in PCT Pub. No. WO2014031687. For example, the marker can be a truncated EGFR (tEGFR) that is, optionally, linked to a linker sequence, such as a T2A cleavable linker sequence. An exemplary polypeptide for a truncated EGFR *(e.g.* tEGFR) comprises the sequence of amino acids set forth in SEQ ID NO: 7 or 28, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 7 or 28. An exemplary T2A linker sequence comprises the sequence of amino acids set forth in SEQ ID NO: 6 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 6.

In some instances, nucleic acid molecules encoding such CAR constructs further includes a sequence encoding a T2A ribosomal skip element and/or a tEGFR sequence, *e*.*g*., downstream of the sequence encoding the CAR. In some instances, the sequence encodes a T2A ribosomal skip element set forth in SEQ ID NO: 6, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 6. In some instances, T cells expressing an antigen receptor (*e.g.* CAR) can also be generated to express a truncated EGFR (EGFRt) as a non-immunogenic selection epitope (*e.g.* by introduction of a construct encoding the CAR and EGFRt separated by a T2A ribosome switch to express two proteins from the same construct), which then can be used as a marker to detect such cells (see *e.g.* U.S. Patent No. 8,802,374). In some instances, the sequence encodes an tEGFR sequence set forth in SEQ ID NO: 7 or 28, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 7 or 28.

In some instances, a single promoter may direct expression of an RNA that contains, in a single open reading frame (ORF), two or three genes (*e*.*g*. encoding the molecule involved in modulating a metabolic pathway and encoding the recombinant receptor) separated from one another by sequences encoding a self-cleavage peptide (*e.g.,* 2A sequences) or a protease recognition site (*e.g.,* furin). The ORF thus encodes a single polypeptide, which, either during (in the case of 2A) or after translation, is processed into the individual proteins. In some cases, the peptide, such as T2A, can cause the ribosome to skip (ribosome skipping) synthesis of a peptide bond at the C-terminus of a 2A element, leading to separation between the end of the 2A sequence and the next peptide downstream (*see,* for example, de Felipe. Genetic Vaccines and Ther. 2:13 (2004) and deFelipe et al. Traffic 5:616-626 (2004)). Many 2A elements are known in the art. Examples of 2A sequences that can be used in the methods and nucleic acids disclosed herein, without limitation, 2A sequences from the foot-and-mouth disease virus (F2A, *e.g.,* SEQ ID NO: 27), equine rhinitis A virus (E2A, *e.g.,* SEQ ID NO: 26), Thosea asigna virus (T2A, *e.g.,* SEQ ID NO: 6 or 23), and porcine teschovirus-1 (P2A, *e.g.,* SEQ ID NO: 24 or 25) as described in U.S. Patent Publication No. 20070116690.

In some instances, the marker is a molecule, *e.g.,* cell surface protein, not naturally found on T cells or not naturally found on the surface of T cells, or a portion thereof. In some instances, the molecule is a non-self molecule, *e.g.,* non-self protein, *i.e.,* one that is not recognized as "self' by the immune system of the host into which the cells will be adoptively transferred.

In some instances, the marker serves no therapeutic function and/or produces no effect other than to be used as a marker for genetic engineering, *e*.*g*., for selecting cells successfully engineered. In other instances, the marker may be a therapeutic molecule or molecule otherwise exerting some desired effect, such as a ligand for a cell to be encountered *in vivo,* such as a costimulatory or immune checkpoint molecule to enhance and/or dampen responses of the cells upon adoptive transfer and encounter with ligand.

Introduction of the nucleic acid molecules encoding the recombinant receptor in the cell may be carried out using any of a number of known vectors. Such vectors include viral and non-viral systems, including lentiviral and gammaretroviral systems, as well as transposon-based systems such as PiggyBac or Sleeping Beauty-based gene transfer systems. Exemplary methods include those for transfer of nucleic acids encoding the receptors, including via viral, *e*.*g*., retroviral or lentiviral, transduction, transposons, and electroporation.

In some instances, gene transfer is accomplished by first stimulating the cell, such as by combining it with a stimulus that induces a response such as proliferation, survival, and/or activation, e.g., as measured by expression of a cytokine or activation marker, followed by transduction of the activated cells, and expansion in culture to numbers sufficient for clinical applications.

In some instances, prior to or during gene transfer, the cells are incubated or cultured in the presence of an immunomodulatory compound, *e*.*g*., lenalidomide, including any as described herein. In some instances, the immunomodulatory compound, *e*.*g*., lenalidomide is added during the cell manufacturing process, for example, during the process of engineering CAR-T cells. In some aspects, the presence of the immunomodulatory compound can improve the quality of the population of cells produced. In some aspects, the immunomodulatory compound, *e*.*g*., lenalidomide may increase the proliferation or expansion of cells or may alter one or more signaling pathways thereby resulting in cells with a less-differentiated or less activated surface phenotype, despite exhibiting substantial expansion and/or effector function.

In some contexts, overexpression of a stimulatory factor (for example, a lymphokine or a cytokine) may be toxic to a subject. Thus, in some contexts, the engineered cells include gene segments that cause the cells to be susceptible to negative selection in vivo, such as upon administration in adoptive immunotherapy. For example in some aspects, the cells are engineered so that they can be eliminated as a result of a change in the in vivo condition of the patient to which they are administered. The negative selectable phenotype may result from the insertion of a gene that confers sensitivity to an administered agent, for example, a compound. Negative selectable genes include the Herpes simplex virus type I thymidine kinase (HSV-I TK) gene (Wigler et al., Cell 2:223, 1977) which confers ganciclovir sensitivity; the cellular hypoxanthine phosphribosyltransferase (HPRT) gene, the cellular adenine phosphoribosyltransferase (APRT) gene, bacterial cytosine deaminase, (Mullen et al., Proc. Natl. Acad. Sci. USA. 89:33 (1992)).

In some instances, recombinant nucleic acids are transferred into cells using recombinant infectious virus particles, such as, e.g., vectors derived from simian virus 40 (SV40), adenoviruses, adeno-associated virus (AAV). In some instances, recombinant nucleic acids are transferred into T cells using recombinant lentiviral vectors or retroviral vectors, such as gamma-retroviral vectors (see, e.g., Koste et al. (2014) Gene Therapy 2014 Apr 3. doi: 10.1038/gt.2014.25; Carlens et al. (2000) Exp Hematol 28(10): 1137-46; Alonso-Camino et al. (2013) Mol Ther Nucl Acids 2, e93; Park et al., Trends Biotechnol. 2011 November 29(11): 550-557.

In some instances, the retroviral vector has a long terminal repeat sequence (LTR), e.g., a retroviral vector derived from the Moloney murine leukemia virus (MoMLV), myeloproliferative sarcoma virus (MPSV), murine embryonic stem cell virus (MESV), murine stem cell virus (MSCV), spleen focus forming virus (SFFV), or adeno-associated virus (AAV). Most retroviral vectors are derived from murine retroviruses. In some instances, the retroviruses include those derived from any avian or mammalian cell source. The retroviruses typically are amphotropic, meaning that they are capable of infecting host cells of several species, including humans. In one instance, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman (1989) BioTechniques 7:980-990; Miller, A. D. (1990) Human Gene Therapy 1:5-14; Scarpa et al. (1991) Virology 180:849-852; Burns et al. (1993) Proc. Natl. Acad. Sci. USA 90:8033-8037; and Boris-Lawrie and Temin (1993) Cur. Opin. Genet. Develop. 3:102-109.

Methods of lentiviral transduction are known. Exemplary methods are described in, e.g., Wang et al. (2012) J. Immunother. 35(9): 689-701; Cooper et al. (2003) Blood. 101:1637-1644; Verhoeyen et al. (2009) Methods Mol Biol. 506: 97-114; and Cavalieri et al. (2003) Blood. 102(2): 497-505.

In some instances, recombinant nucleic acids are transferred into T cells via electroporation (*see,* e.g., Chicaybam et al, (2013) PLoS ONE 8(3): e60298 and Van Tedeloo et al. (2000) Gene Therapy 7(16): 1431-1437). In some instances, recombinant nucleic acids are transferred into T cells via transposition (see, e.g., Manuri et al. (2010) Hum Gene Ther 21(4): 427-437; Sharma et al. (2013) Molec Ther Nucl Acids 2, e74; and Huang et al. (2009) Methods Mol Biol 506: 115-126). Other methods of introducing and expressing genetic material in immune cells include calcium phosphate transfection (e.g., as described in Current Protocols in Molecular Biology, John Wiley & Sons, New York. N.Y.), protoplast fusion, cationic liposome-mediated transfection; tungsten particle-facilitated microparticle bombardment (Johnston, Nature, 346: 776-777 (1990)); and strontium phosphate DNA co-precipitation (Brash et al., Mol. Cell Biol., 7: 2031-2034 (1987)).

Other approaches and vectors for transfer of the nucleic acids encoding the recombinant products are those described, e.g., in international patent application, Publication No.: WO2014055668, and U.S. Patent No. 7,446,190.

In some instances, the cells, e.g., T cells, may be transfected either during or after expansion e.g. with a T cell receptor (TCR) or a chimeric antigen receptor (CAR). This transfection for the introduction of the gene of the desired receptor can be carried out with any suitable retroviral vector, for example. The genetically modified cell population can then be liberated from the initial stimulus (the CD3/CD28 stimulus, for example) and subsequently be stimulated with a second type of stimulus e.g. via a de novo introduced receptor). This second type of stimulus may include an antigenic stimulus in form of a peptide/MHC molecule, the cognate (cross-linking) ligand of the genetically introduced receptor (e.g. natural ligand of a CAR) or any ligand (such as an antibody) that directly binds within the framework of the new receptor (e.g. by recognizing constant regions within the receptor). See, for example, Cheadle et al, "Chimeric antigen receptors for T-cell based therapy" Methods Mol Biol. 2012; 907:645-66 or Barrett et al., Chimeric Antigen Receptor Therapy for Cancer Annual Review of Medicine Vol. 65: 333-347 (2014).

In some cases, a vector may be used that does not require that the cells, *e*.*g*., T cells, are activated. In some such instances, the cells may be selected and/or transduced prior to activation. Thus, the cells may be engineered prior to, or subsequent to culturing of the cells, and in some cases at the same time as or during at least a portion of the culturing.

In some aspects, the cells further are engineered to promote expression of cytokines or other factors. Among additional nucleic acids, e.g., genes for introduction are those to improve the efficacy of therapy, such as by promoting viability and/or function of transferred cells; genes to provide a genetic marker for selection and/or evaluation of the cells, such as to assess *in vivo* survival or localization; genes to improve safety, for example, by making the cell susceptible to negative selection in vivo as described by Lupton S. D. et al., Mol. and Cell Biol., 11:6 (1991); and Riddell et al., Human Gene Therapy 3:319-338 (1992); see also the publications of PCT/US91/08442 and PCT/US94/05601 by Lupton et al. describing the use of bifunctional selectable fusion genes derived from fusing a dominant positive selectable marker with a negative selectable marker. *See,* e.g., Riddell et al., US Patent No. 6,040,177, at columns 14-17.

### III.EXEMPLARY TREATMENT OUTCOMES AND METHODS FOR ASSESSING SAME

In some instances of the methods, compositions, combinations, kits and uses disclosed herein, the provided combination therapy results in one or more treatment outcomes, such as a feature associated with any one or more of the parameters associated with the therapy or treatment, as described below. In some instances, the method includes assessment of the exposure, persistence and proliferation of the T cells, *e.g.,* T cells administered for the T cell based therapy. In some instances, the exposure, or prolonged expansion and/or persistence of the cells, and/or changes in cell phenotypes or functional activity of the cells, *e.g.,* cells administered for immunotherapy, *e*.*g*. T cell therapy, in the methods disclosed herein, can be measured by assessing the characteristics of the T cells *in vitro* or *ex vivo.* In some instances, such assays can be used to determine or confirm the function of the T cells, *e.g.* T cell therapy, before or after administering the combination therapy disclosed herein.

In some instances, the combination therapy can further include one or more screening steps to identify subjects for treatment with the combination therapy and/or continuing the combination therapy, and/or a step for assessment of treatment outcomes and/or monitoring treatment outcomes. In some instances, the step for assessment of treatment outcomes can include steps to evaluate and/or to monitor treatment and/or to identify subjects for administration of further or remaining steps of the therapy and/or for repeat therapy. In some instances, the screening step and/or assessment of treatment outcomes can be used to determine the dose, frequency, duration, timing and/or order of the combination therapy disclosed herein.

In some instances, any of the screening steps and/or assessment of treatment of outcomes described herein can be used prior to, during, during the course of, or subsequent to administration of one or more steps of the provided combination therapy, *e*.*g*., administration of the T cell therapy *(e.g.* CAR-expressing T cells), and/or an immunomodulatory compound, *e.g.,* lenalidomide. In some instances, assessment is made prior to, during, during the course of, or after performing any of the methods disclosed herein. In some instances, the assessment is made prior to performing the methods disclosed herein. In some instances, assessment is made after performing one or more steps of the methods disclosed herein. In some instances, the assessment is performed prior to administration of administration of one or more steps of the provided combination therapy, for example, to screen and identify patients suitable and/or susceptible to receive the combination therapy. In some instances, the assessment is performed during, during the course of, or subsequent to administration of one or more steps of the provided combination therapy, for example, to assess the intermediate or final treatment outcome, *e.g.,* to determine the efficacy of the treatment and/or to determine whether to continue or repeat the treatments and/or to determine whether to administer the remaining steps of the combination therapy.

In some instances, treatment of outcomes includes improved immune function, *e.g.,* immune function of the T cells administered for cell based therapy and/or of the endogenous T cells in the body. In some instances, exemplary treatment outcomes include, but are not limited to, enhanced T cell proliferation, enhanced T cell functional activity, changes in immune cell phenotypic marker expression, such as such features being associated with the engineered T cells, e.g. CAR-T cells, administered to the subject. In some instances, exemplary treatment outcomes include decreased disease burden, *e*.*g*., tumor burden, improved clinical outcomes and/or enhanced efficacy of therapy.

In some instances, the screening step and/or assessment of treatment of outcomes includes assessing the survival and/or function of the T cells administered for cell based therapy. In some instances, the screening step and/or assessment of treatment of outcomes includes assessing the levels of cytokines or growth factors. In some instances, the screening step and/or assessment of treatment of outcomes includes assessing disease burden and/or improvements, *e*.*g*., assessing tumor burden and/or clinical outcomes. In some instances, either of the screening step and/or assessment of treatment of outcomes can include any of the assessment methods and/or assays described herein and/or known in the art, and can be performed one or more times, *e*.*g*., prior to, during, during the course of, or subsequently to administration of one or more steps of the combination therapy. Exemplary sets of parameters associated with a treatment outcome, which can be assessed in some instances of the methods disclosed herein, include peripheral blood immune cell population profile and/or tumor burden.

In some instances, the methods affect efficacy of the cell therapy in the subject. In some instances, the persistence, expansion, and/or presence of recombinant receptor-expressing, *e*.*g*., CAR-expressing, cells in the subject following administration of the dose of cells in the method with the immunomodulatory compound is greater as compared to that achieved via a method without the administration of the immunomodulatory compound. In some instances of the immunotherapy methods disclosed herein, such as a T cell therapy (*e*.*g*. CAR-expressing T cells), assessment of the parameter includes assessing the expansion and/or persistence in the subject of the administered T cells for the immunotherapy, *e*.*g*., T cell therapy, as compared to a method in which the immunotherapy is administered to the subject in the absence of the immunomodulatory compound. In some instances, the methods result in the administered T cells exhibiting increased or prolonged expansion and/or persistence in the subject as compared to a method in which the T cell therapy is administered to the subject in the absence of the immunomodulatory compound.

In some instances, the administration of the immunomodulatory compound, *e.g.,* lenalidomide decreases disease burden, *e*.*g*., tumor burden, in the subject as compared to a method in which the dose of cells expressing the recombinant receptor is administered to the subject in the absence of the immunomodulatory compound. In some instances, the administration of the immunomodulatory compound, *e*.*g*., lenalidomide decreases blast marrow in the subject as compared to a method in which the dose of cells expressing the recombinant receptor is administered to the subject in the absence of the immunomodulatory compound. In some instances, the administration of the immunomodulatory compound, *e*.*g*., lenalidomide results in improved clinical outcomes, *e*.*g*., objective response rate (ORR), progression-free survival (PFS) and overall survival (OS), compared to a method in which the dose of cells expressing the recombinant receptor is administered to the subject in the absence of the immunomodulatory compound.

In some instances, the subject can be screened prior to the administration of one or more steps of the combination therapy. For example, the subject can be screened for characteristics of the disease and/or disease burden, *e*.*g*., tumor burden, prior to administration of the combination therapy, to determine suitability, responsiveness and/or susceptibility to administering the combination therapy. In some instances, the screening step and/or assessment of treatment outcomes can be used to determine the dose, frequency, duration, timing and/or order of the combination therapy disclosed herein.

In some instances, the subject can be screened after administration of one of the steps of the combination therapy, to determine and identify subjects to receive the remaining steps of the combination therapy and/or to monitor efficacy of the therapy. In some instances, the number, level or amount of administered T cells and/or proliferation and/or activity of the administered T cells is assessed prior to administration and/or after administration of the immunomodulatory compound, *e*.*g*., lenalidomide.

In some instances, the immunomodulatory compound, *e.g.,* lenalidomide is administered until the concentration or number of engineered cells in the blood of the subject is (i) at least at or about 10 engineered cells per microliter, (ii) at least 20%, 30%, 40% or 50% of the total number of peripheral blood mononuclear cells (PBMCs), (iii) at least or at least about 1 × 10⁵ engineered cells; or (iv) at least 5,000 copies of recombinant receptor-encoding DNA per micrograms DNA; and/or at day 90 following the initiation of the administration in (a), CAR-expressing cells are detectable in the blood or serum of the subject; and/or at day 90 following the initiation of the administration in (a), the blood of the subject contains at least 20% CAR-expressing cells, at least 10 CAR-expressing cells per microliter or at least 1 × 10⁴ CAR-expressing cells.

In some instances, the immunomodulatory compound, *e*.*g*., lenalidomide is administered until there is a clinical benefit to the treatment, such as at least or greater than a 50% decrease in the total tumor volume or a complete response (CR) in which detectable tumor has disappeared, progression free survival or disease free survival for greater than 6 months or greater than 1 year or more.

In some instances, a change and/or an alteration, *e*.*g*., an increase, an elevation, a decrease or a reduction, in levels, values or measurements of a parameter or outcome compared to the levels, values or measurements of the same parameter or outcome in a different time point of assessment, a different condition, a reference point and/or a different subject is determined or assessed. For example, in some instances, a fold change, *e*.*g*., an increase or decrease, in particular parameters, e.g., number of engineered T cells in a sample, compared to the same parameter in a different condition, e.g., before or after administration of the immunomodulatory compound, *e*.*g*., lenalidomide can be determined. In some instances, the levels, values or measurements of two or more parameters are determined, and relative levels are compared. In some instances, the determined levels, values or measurements of parameters are compared to the levels, values or measurements from a control sample or an untreated sample. In some instances, the determined levels, values or measurements of parameters are compared to the levels from a sample from the same subject but at a different time point. The values obtained in the quantification of individual parameter can be combined for the purpose of disease assessment, *e*.*g*., by forming an arithmetical or logical operation on the levels, values or measurements of parameters by using multi-parametric analysis. In some instances, a ratio of two or more specific parameters can be calculated.

### A. T CELL EXPOSURE, PERSISTENCE AND PROLIFERATION

In some instances, the parameter associated with therapy or a treatment outcome, which include parameters that can be assessed for the screening steps and/or assessment of treatment of outcomes and/or monitoring treatment outcomes, is or includes assessment of the exposure, persistence and proliferation of the T cells, *e*.*g*., T cells administered for the T cell based therapy. In some instances, the increased exposure, or prolonged expansion and/or persistence of the cells, and/or changes in cell phenotypes or functional activity of the cells, *e.g.,* cells administered for immunotherapy, *e*.*g*. T cell therapy, in the methods disclosed herein, can be measured by assessing the characteristics of the T cells *in vitro* or *ex vivo.* In some instances, such assays can be used to determine or confirm the function of the T cells used for the immunotherapy, *e*.*g*. T cell therapy, before or after administering one or more steps of the combination therapy disclosed herein.

In some instances, the administration of the immunomodulatory compound, e.g., lenalidomide are designed to promote exposure of the subject to the cells, *e.g.,* T cells administered for T cell based therapy, such as by promoting their expansion and/or persistence over time. In some instances, the T cell therapy exhibits increased or prolonged expansion and/or persistence in the subject as compared to a method in which the T cell therapy is administered to the subject in the absence of the immunomodulatory compound, *e*.*g*., lenalidomide.

In some instances, the disclosed methods increase exposure of the subject to the administered cells (*e*.*g*., increased number of cells or duration over time) and/or improve efficacy and therapeutic outcomes of the immunotherapy, *e*.*g*. T cell therapy. In some aspects, the methods are advantageous in that a greater and/or longer degree of exposure to the cells expressing the recombinant receptors, *e*.*g*., CAR-expressing cells, improves treatment outcomes as compared with other methods. Such outcomes may include patient survival and remission, even in individuals with severe tumor burden.

In some instances, the administration of the immunomodulatory compound, *e*.*g*., lenalidomide can increase the maximum, total, and/or duration of exposure to the cells, *e.g.* T cells administered for the T cell based therapy, in the subject as compared to administration of the T cells alone in the absence of the immunomodulatory compound. In some aspects, administration of the immunomodulatory compound, *e*.*g*., lenalidomide, in the context of high disease burden (and thus higher amounts of antigen) and/or a more aggressive or resistant cancer enhances efficacy as compared with administration of the T cells alone in the absence of the immunomodulatory compound in the same context, which may result in immunosuppression, anergy and/or exhaustion which may prevent expansion and/or persistence of the cells.

In some instances, the presence and/or amount of cells expressing the recombinant receptor (*e*.*g*., CAR-expressing cells administered for T cell based therapy) in the subject following the administration of the T cells and before, during and/or after the administration of the immunomodulatory compound, *e*.*g*., lenalidomide is detected. In some aspects, quantitative PCR (qPCR) is used to assess the quantity of cells expressing the recombinant receptor (*e.g.,* CAR-expressing cells administered for T cell based therapy) in the blood or serum or organ or tissue sample *(e.g.,* disease site, *e.g.,* tumor sample) of the subject. In some aspects, persistence is quantified as copies of DNA or plasmid encoding the receptor, *e.g.,* CAR, per microgram of DNA, or as the number of receptor-expressing, e.g., CAR-expressing, cells per microliter of the sample, *e.g.,* of blood or serum, or per total number of peripheral blood mononuclear cells (PBMCs) or white blood cells or T cells per microliter of the sample.

In some instances, the cells are detected in the subject at or at least at 4, 14, 15, 27, or 28 days following the administration of the T cells, *e*.*g*., CAR-expressing T cells. In some aspects, the cells are detected at or at least at 2, 4, or 6 weeks following, or 3, 6, or 12, 18, or 24, or 30 or 36 months, or 1, 2, 3, 4, 5, or more years, following the administration of the T cells, *e.g.,* CAR-expressing T cells and/or the immunomodulatory compound, *e.g.,* lenalidomide.

In some instances, the persistence of receptor-expressing cells (*e*.*g*. CAR-expressing cells) in the subject by the methods, following the administration of the T cells, *e.g.,* CAR-expressing T cells and/or the immunomodulatory compound, *e*.*g*., lenalidomide, is greater as compared to that which would be achieved by alternative methods such as those involving the administration of the immunotherapy alone, *e.g.,* administration the T cells, *e.g.,* CAR-expressing T cells, in the absence of the immunomodulatory compound.

The exposure, *e.g.,* number of cells, *e.g.* T cells administered for T cell therapy, indicative of expansion and/or persistence, may be stated in terms of maximum numbers of the cells to which the subject is exposed, duration of detectable cells or cells above a certain number or percentage, area under the curve for number of cells over time, and/or combinations thereof and indicators thereof. Such outcomes may be assessed using known methods, such as qPCR to detect copy number of nucleic acid encoding the recombinant receptor compared to total amount of nucleic acid or DNA in the particular sample, *e*.*g*., blood, serum, plasma or tissue, such as a tumor sample, and/or flow cytometric assays detecting cells expressing the receptor generally using antibodies specific for the receptors. Cell-based assays may also be used to detect the number or percentage of functional cells, such as cells capable of binding to and/or neutralizing and/or inducing responses, *e*.*g*., cytotoxic responses, against cells of the disease or condition or expressing the antigen recognized by the receptor.

In some aspects, increased exposure of the subject to the cells includes increased expansion of the cells. In some instances, the receptor expressing cells, *e*.*g*. CAR-expressing cells, expand in the subject following administration of the T cells, *e*.*g*., CAR-expressing T cells, and/or following administration of immunomodulatory compound, *e*.*g*., lenalidomide. In some aspects, the methods result in greater expansion of the cells compared with other methods, such as those involving the administration of the T cells, *e*.*g*., CAR-expressing T cells, in the absence of administering the immunomodulatory compound, *e*.*g*., lenalidomide.

In some aspects, the method results in high *in vivo* proliferation of the administered cells, for example, as measured by flow cytometry. In some aspects, high peak proportions of the cells are detected. For example, in some instances, at a peak or maximum level following the administration of the T cells, *e*.*g*., CAR-expressing T cells and/or the immunomodulatory compound, *e*.*g*., lenalidomide, in the blood or disease-site of the subject or white blood cell fraction thereof, *e.g.,* PBMC fraction or T cell fraction, at least about 10%, at least about 20%, at least about 30%, at least about 40%, at least about 50%, at least about 60%, at least about 70%, at least about 80%, or at least about 90% of the cells express the recombinant receptor, *e.g.,* the CAR.

In some instances, the method results in a maximum concentration, in the blood or serum or other bodily fluid or organ or tissue of the subject, of at least 100, 500, 1000, 1500, 2000, 5000, 10,000 or 15,000 copies of or nucleic acid encoding the receptor, *e.g.,* the CAR, per microgram of DNA, or at least 0.1, 0.2, 0.3, 0.4, 0.5, 0.6, 0.7, 0.8, or 0.9 receptor-expressing, e.g., CAR,-expressing cells per total number of peripheral blood mononuclear cells (PBMCs), total number of mononuclear cells, total number of T cells, or total number of microliters. In some instances, the cells expressing the receptor are detected as at least 10, 20, 30, 40, 50, or 60 % of total PBMCs in the blood of the subject, and/or at such a level for at least 1, 2, 3, 4, 5, 6, 7, 8, 9, 10, 11, 12, 24, 36, 48, or 52 weeks following the T cells, *e*.*g*., CAR-expressing T cells and/or the immunomodulatory compound, *e*.*g*., lenalidomide, or for 1, 2, 3, 4, or 5, or more years following such administration.

In some aspects, the method results in at least a 2-fold, at least a 4-fold, at least a 10-fold, or at least a 20-fold increase in copies of nucleic acid encoding the recombinant receptor, *e.g.,* CAR, per microgram of DNA, *e.g.,* in the serum, plasma, blood or tissue, *e.g.,* tumor sample, of the subject.

In some instances, cells expressing the receptor are detectable in the serum, plasma, blood or tissue, *e.g.,* tumor sample, of the subject, *e.g.,* by a specified method, such as qPCR or flow cytometry-based detection method, at least 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 or more days following administration of the T cells, *e*.*g*., CAR-expressing T cells, or after administration of the immunomodulatory compound, *e*.*g*., lenalidomide, for at least at or about 2, 3, 4, 5, 6, 7, 8, 9, 10, 11, 12, 13, 14, 15, 16, 17, 18, 19, 20, 21, 22, 23, or 24 or more weeks following the administration of the T cells, *e*.*g*., CAR-expressing T cells, and/or the immunomodulatory compound, *e*.*g*., lenalidomide.

In some aspects, at least about 1 × 10², at least about 1 × 10³, at least about 1 × 10⁴, at least about 1 × 10⁵, or at least about 1 × 10⁶ or at least about 5 × 10⁶ or at least about 1 × 10⁷ or at least about 5 × 10⁷ or at least about 1 × 10⁸ recombinant receptor-expressing, *e.g.,* CAR-expressing cells, and/or at least 10, 25, 50, 100, 200, 300, 400, or 500, or 1000 receptor-expressing cells per microliter, *e*.*g*., at least 10 per microliter, are detectable or are present in the subject or fluid, plasma, serum, tissue, or compartment thereof, such as in the blood, *e.g.,* peripheral blood, or disease site, *e*.*g*., tumor, thereof. In some instances, such a number or concentration of cells is detectable in the subject for at least about 20 days, at least about 40 days, or at least about 60 days, or at least about 3, 4, 5, 6, 7, 8, 9, 10, 11, or 12 months, or at least 2 or 3 years, following administration of the T cells, *e*.*g*., CAR-expressing T cells, and/or following the administration of the immunomodulatory compound, *e*.*g*., lenalidomide. Such cell numbers may be as detected by flow cytometry-based or quantitative PCR-based methods and extrapolation to total cell numbers using known methods. *See, e.g.,* Brentjens et al., Sci Transl Med. 2013 5(177), Park et al, Molecular Therapy 15(4):825-833 (2007), Savoldo et al., JCI 121(5):1822-1826 (2011), Davila et al., (2013) PLoS ONE 8(4):e61338, Davila et al., Oncoimmunology 1(9):1577-1583 (2012), Lamers, Blood 2011 117:72-82, Jensen et al., Biol Blood Marrow Transplant 2010 September; 16(9): 1245-1256, Brentjens et al., Blood 2011 118(18):4817-4828.

In some aspects, the copy number of nucleic acid encoding the recombinant receptor, *e.g.,* vector copy number, per 100 cells, for example in the peripheral blood or bone marrow or other compartment, as measured by immunohistochemistry, PCR, and/or flow cytometry, is at least 0.01, at least 0.1, at least 1, or at least 10, at about 1 week, about 2 weeks, about 3 weeks, about 4 weeks, about 5 weeks, or at least about 6 weeks, or at least about 2, 3, 4, 5, 6, 7, 8. 9, 10, 11, or 12 months or at least 2 or 3 years following administration of the cells, *e.g.,* CAR-expressing T cells, and/or the immunomodulatory compound, *e*.*g*., lenalidomide. In some instances, the copy number of the vector expressing the receptor, *e.g.* CAR, per microgram of genomic DNA is at least 100, at least 1000, at least 5000, or at least 10,000, or at least 15,000 or at least 20,000 at a time about 1 week, about 2 weeks, about 3 weeks, or at least about 4 weeks following administration of the T cells, *e*.*g*., CAR-expressing T cells, or immunomodulatory compound, *e*.*g*., lenalidomide, or at least 2, 3, 4, 5, 6, 7, 8, 9, 10, 11, or 12 months or at least 2 or 3 years following such administration.

In some aspects, the receptor, *e.g.* CAR, expressed by the cells, is detectable by quantitative PCR (qPCR) or by flow cytometry in the subject, plasma, serum, blood, tissue and/or disease site thereof, *e*.*g*., tumor site, at a time that is at least about 3 months, at least about 6 months, at least about 12 months, at least about 1 year, at least about 2 years, at least about 3 years, or more than 3 years, following the administration of the cells, *e*.*g*., following the initiation of the administration of the T cells, *e*.*g*., CAR-expressing T cells, and/or the immunomodulatory compound, *e*.*g*., lenalidomide.

In some instances, the area under the curve (AUC) for concentration of receptor-(*e.g.,* CAR-) expressing cells in a fluid, plasma, serum, blood, tissue, organ and/or disease site, *e.g.* tumor site, of the subject over time following the administration of the T cells, *e.g.,* CAR-expressing T cells and/or immunomodulatory compound, *e*.*g*., lenalidomide, is greater as compared to that achieved via an alternative dosing regimen where the subject is administered the T cells, *e*.*g*., CAR-expressing T cells, in the absence of administering the immunomodulatory compound.

In some aspects, the method results in high *in vivo* proliferation of the administered cells, for example, as measured by flow cytometry. In some aspects, high peak proportions of the cells are detected. For example, in some instances, at a peak or maximum level following the T cells, *e.g.,* CAR-expressing T cells and/or immunomodulatory compound, *e.g.,* lenalidomide, in the blood, plasma, serum, tissue or disease site of the subject or white blood cell fraction thereof, *e.g.,* PBMC fraction or T cell fraction, at least about 10%, at least about 20%, at least about 30%, at least about 40%, at least about 50%, at least about 60%, at least about 70%, at least about 80%, or at least about 90% of the cells express the recombinant receptor, *e.g.,* the CAR.

In some aspects, the increased or prolonged expansion and/or persistence of the dose of cells in the subject administered with the immunomodulatory compound, *e*.*g*., lenalidomide is associated with a benefit in tumor related outcomes in the subject. In some instances, the tumor related outcome includes a decrease in tumor burden or a decrease in blast marrow in the subject. In some instances, the tumor burden is decreased by or by at least at or about 10, 20, 30, 40, 50, 60, 70, 80, 90, or 100 percent after administration of the method. In some instances, disease burden, tumor size, tumor volume, tumor mass, and/or tumor load or bulk is reduced following the dose of cells by at least at or about 50%, 60%, 70%, 80%, 90% or more compared a subject that has been treated with a method that does not involve the administration of an immunomodulatory compound, *e*.*g*., lenalidomide.

### B. T CELL FUNCTIONAL ACTIVITY

In some instances, parameters associated with therapy or a treatment outcome, which include parameters that can be assessed for the screening steps and/or assessment of treatment of outcomes and/or monitoring treatment outcomes, includes one or more of activity, phenotype, proliferation or function of T cells. In some instances, any of the known assays in the art for assessing the activity, phenotypes, proliferation and/or function of the T cells, *e.g.,* T cells administered for T cell therapy, can be used. Prior to and/or subsequent to administration of the cells and/or immunomodulatory compound, *e*.*g*., lenalidomide, the biological activity of the engineered cell populations in some instances is measured, *e*.*g*., by any of a number of known methods. Parameters to assess include specific binding of an engineered or natural T cell or other immune cell to antigen, *in vivo, e.g.,* by imaging, or *ex vivo, e.g.,* by ELISA or flow cytometry. In certain instances, the ability of the engineered cells to destroy target cells can be measured using any suitable method known in the art, such as cytotoxicity assays described in, for example, Kochenderfer et al., J. Immunotherapy, 32(7): 689-702 (2009), and Herman et al., J. Immunological Methods, 285(1): 25-40 (2004). In certain instances, the biological activity of the cells is measured by assaying expression and/or secretion of one or more cytokines, such as CD107a, IFNγ, IL-2, GM-CSF and TNFα, and/or by assessing cytolytic activity.

In some instances, assays for the activity, phenotypes, proliferation and/or function of the T cells, *e.g.,* T cells administered for T cell therapy include, but are not limited to, ELISPOT, ELISA, cellular proliferation, cytotoxic lymphocyte (CTL) assay, binding to the T cell epitope, antigen or ligand, or intracellular cytokine staining, proliferation assays, lymphokine secretion assays, direct cytotoxicity assays, and limiting dilution assays. In some instances, proliferative responses of the T cells can be measured, *e*.*g*. by incorporation of ³H-thymidine, BrdU (5-Bromo-2'-Deoxyuridine) or 2'-deoxy-5-ethynyluridine (EdU) into their DNA or dye dilution assays, using dyes such as carboxyfluorescein diacetate succinimmunomodulatory compoundyl ester (CFSE), CellTrace Violet, or membrane dye PKH26.

In some instances, assessing the activity, phenotypes, proliferation and/or function of the T cells, *e*.*g*., T cells administered for T cell therapy, include measuring cytokine production from T cells, and/or measuring cytokine production in a biological sample from the subject, *e*.*g*., plasma, serum, blood, and/or tissue samples, *e*.*g*., tumor samples. In some cases, such measured cytokines can include, without limitation, interlekukin-2 (IL-2), interferon-gamma (IFNγ), interleukin-4 (IL-4), TNF-alpha (TNFα), interleukin-6 (IL-6), interleukin-10 (IL-10), interleukin-12 (IL-12), granulocyte-macrophage colony-stimulating factor (GM-CSF), CD107a, and/or TGF-beta (TGFβ). Assays to measure cytokines are well known in the art, and include but are not limited to, ELISA, intracellular cytokine staining, cytometric bead array, RT-PCR, ELISPOT, flow cytometry and bio-assays in which cells responsive to the relevant cytokine are tested for responsiveness (*e*.*g*. proliferation) in the presence of a test sample.

In some instances, assessing the activity, phenotypes, proliferation and/or function of the T cells, *e.g.,* T cells administered for T cell therapy, include assessing cell phenotypes, *e.g.,* expression of particular cell surface markers. In some instances, the T cells, *e.g.,* T cells administered for T cell therapy, are assessed for expression of T cell activation markers, T cell exhaustion markers, and/or T cell differentiation markers. In some instances, the cell phenotype is assessed before administration. In some instances, the cell phenotype is assessed after administration. T cell activation markers, T cell exhaustion markers, and/or T cell differentiation markers for assessment include any markers known in the art for particular subsets of T cells, *e.g.,* CD25, CD38, human leukocyte antigen-DR (HLA-DR), CD69, CD44, CD137, KLRG1, CD62L^{low}, CCR7^{low}, CD71, CD2, CD54, CD58, CD244, CD160, programmed cell death protein 1 (PD-1), lymphocyte activation gene 3 protein (LAG-3), T-cell immunoglobulin domain and mucin domain protein 3 (TIM-3), cytotoxic T lymphocyte antigen-4 (CTLA-4), band T lymphocyte attenuator (BTLA) and/or T-cell immunoglobulin and immunoreceptor tyrosine-based inhibitory motif domain (TIGIT) (see, *e.g.,* Liu et al., Cell Death and Disease (2015) 6, e1792). In some instances, the assessed cell surface marker is CD25, PD-1 and/or TIM-3. In some instances, the assessed cell surface marker is CD25.

In some aspects, detecting the expression levels includes performing an *in vitro* assay. In some instances, the *in vitro* assay is an immunoassay, an aptamer-based assay, a histological or cytological assay, or an mRNA expression level assay. In some instances, the parameter or parameters for one or more of each of the one or more factors, effectors, enzymes and/or surface markers are detected by an enzyme linked immunosorbent assay (ELISA), immunoblotting, immunoprecipitation, radioimmunoassay (RIA), immunostaining, flow cytometry assay, surface plasmon resonance (SPR), chemiluminescence assay, lateral flow immunoassay, inhibition assay or avidity assay. In some instances, detection of cytokines and/or surface markers is determined using a binding reagent that specifically binds to at least one biomarker. In some cases, the binding reagent is an antibody or antigen-binding fragment thereof, an aptamer or a nucleic acid probe.

In some instances, the administration of the immunomodulatory compound, *e.g.,* lenalidomide increases the level of circulating CAR T cells.

### C. DISEASE BURDEN

In some instances, parameters associated with therapy or a treatment outcome, which include parameters that can be assessed for the screening steps and/or assessment of treatment of outcomes and/or monitoring treatment outcomes, includes tumor or disease burden. The administration of the immunotherapy, such as a T cell therapy (*e*.*g*. CAR-expressing T cells) and/or the immunomodulatory compound, *e*.*g*., lenalidomide, can reduce or prevent the expansion or burden of the disease or condition in the subject. For example, where the disease or condition is a tumor, the methods generally reduce tumor size, bulk, metastasis, percentage of blasts in the bone marrow or molecularly detectable cancer and/or improve prognosis or survival or other symptom associated with tumor burden.

In some instances, the disclosed methods result in a decreased tumor burden in treated subjects compared to alternative methods in which the immunotherapy, such as a T cell therapy (*e*.*g*. CAR-expressing T cells) is given without administration of the immunomodulatory compound, *e*.*g*., lenalidomide. It is not necessary that the tumor burden actually be reduced in all subjects receiving the combination therapy, but that tumor burden is reduced on average in subjects treated, such as based on clinical data, in which a majority of subjects treated with such a combination therapy exhibit a reduced tumor burden, such as at least 50%, 60%, 70%, 80%, 90%, 95% or more of subjects treated with the combination therapy, exhibit a reduced tumor burden.

Disease burden can encompass a total number of cells of the disease in the subject or in an organ, tissue, or bodily fluid of the subject, such as the organ or tissue of the tumor or another location, *e*.*g*., which would indicate metastasis. For example, tumor cells may be detected and/or quantified in the blood, lymph or bone marrow in the context of certain hematological malignancies. Disease burden can include, in some instances, the mass of a tumor, the number or extent of metastases and/or the percentage of blast cells present in the bone marrow.

In some instances, the subject has a myeloma, a lymphoma or a leukemia. In some instances, the subject has a non-Hodgkin lymphoma (NHL), an acute lymphoblastic leukemia (ALL), a chronic lymphocytic leukemia (CLL), a diffuse large B-cell lymphoma (DLBCL) or a myeloma, *e*.*g*., a multiple myeloma (MM). In some instances, the subject has a MM or a DBCBL. In some instances, the subject has a follicular lymphoma (FL).

In some instances, the subject has a solid tumor.

In the case of MM, exemplary parameters to assess the extent of disease burden include such parameters as number of clonal plasma cells (*e*.*g*., >10% on bone marrow biopsy or in any quantity in a biopsy from other tissues; plasmacytoma), presence of monoclonal protein (paraprotein) in either serum or urine, evidence of end-organ damage felt related to the plasma cell disorder (*e*.*g*., hypercalcemia (corrected calcium >2.75 mmol/l); renal insufficiency attributable to myeloma; anemia (hemoglobin <10 g/dl); and/or bone lesions (lytic lesions or osteoporosis with compression fractures)).

In the case of DLBCL, exemplary parameters to assess the extent of disease burden include such parameters as cellular morphology (*e*.*g*., centroblastic, immunoblastic, and anaplastic cells), gene expression, miRNA expression and protein expression (*e*.*g*., expression of BCL2, BCL6, MUM1, LMO2, MYC, and p21).

In the case of leukemia, the extent of disease burden can be determined by assessment of residual leukemia in blood or bone marrow. In some instances, a subject exhibits morphologic disease if there are greater than or equal to 5% blasts in the bone marrow, for example, as detected by light microscopy. In some instances, a subject exhibits complete or clinical remission if there are less than 5% blasts in the bone marrow.

In some instances, for leukemia, a subject may exhibit complete remission, but a small proportion of morphologically undetectable (by light microscopy techniques) residual leukemic cells are present. A subject is said to exhibit minimum residual disease (MRD) if the subject exhibits less than 5% blasts in the bone marrow and exhibits molecularly detectable cancer. In some instances, molecularly detectable cancer can be assessed using any of a variety of molecular techniques that permit sensitive detection of a small number of cells. In some aspects, such techniques include PCR assays, which can determine unique Ig/T-cell receptor gene rearrangements or fusion transcripts produced by chromosome translocations. In some instances, flow cytometry can be used to identify cancer cell based on leukemia-specific immunophenotypes. In some instances, molecular detection of cancer can detect as few as 1 leukemia cell in 100,000 normal cells. In some instances, a subject exhibits MRD that is molecularly detectable if at least or greater than 1 leukemia cell in 100,000 cells is detected, such as by PCR or flow cytometry. In some instances, the disease burden of a subject is molecularly undetectable or MRD⁻, such that, in some cases, no leukemia cells are able to be detected in the subject using PCR or flow cytometry techniques.

In some instances, the methods and/or administration of an immunotherapy, such as a T cell therapy (*e.g.* CAR-expressing T cells) and/or immunomodulatory compound, *e.g.,* lenalidomide decrease(s) disease burden as compared with disease burden at a time immediately prior to the administration of the immunotherapy, *e.g*., T cell therapy and/or immunomodulatory compound.

In some aspects, administration of the immunotherapy, *e.g*. T cell therapy and/or immunomodulatory compound, *e.g*., lenalidomide, may prevent an increase in disease burden, and this may be evidenced by no change in disease burden.

In some instances, the method reduces the burden of the disease or condition, *e.g.*, number of tumor cells, size of tumor, duration of patient survival or event-free survival, to a greater degree and/or for a greater period of time as compared to the reduction that would be observed with a comparable method using an alternative therapy, such as one in which the subject receives immunotherapy, *e.g*. T cell therapy alone, in the absence of administration of the immunomodulatory compound, *e.g*., lenalidomide In some instances, disease burden is reduced to a greater extent or for a greater duration following the combination therapy of administration of the immunotherapy, *e.g*., T cell therapy, and the immunomodulatory compound, *e.g*., lenalidomide, compared to the reduction that would be effected by administering each of the agent alone, *e.g*., administering the immunomodulatory compound to a subject having not received the immunotherapy, *e.g*. T cell therapy; or administering the immunotherapy, *e.g*. T cell therapy, to a subject having not received the immunomodulatory compound.

In some instances, the burden of a disease or condition in the subject is detected, assessed, or measured. Disease burden may be detected in some aspects by detecting the total number of disease or disease-associated cells, *e.g.,* tumor cells, in the subject, or in an organ, tissue, or bodily fluid of the subject, such as blood or serum. In some instances, disease burden, *e.g.* tumor burden, is assessed by measuring the mass of a solid tumor and/or the number or extent of metastases. In some aspects, survival of the subject, survival within a certain time period, extent of survival, presence or duration of event-free or symptom-free survival, or relapse-free survival, is assessed. In some instances, any symptom of the disease or condition is assessed. In some instances, the measure of disease or condition burden is specified. In some instances, exemplary parameters for determination include particular clinical outcomes indicative of amelioration or improvement in the disease or condition, *e.g*., tumor. Such parameters include: duration of disease control, including complete response (CR), partial response (PR) or stable disease (SD) (see, *e.g*., Response Evaluation Criteria In Solid Tumors (RECIST) guidelines), objective response rate (ORR), progression-free survival (PFS) and overall survival (OS). Specific thresholds for the parameters can be set to determine the efficacy of the method of combination therapy disclosed herein.

In some instances, the subjects treated according to the method achieve a more durable response. In some cases, a measure of duration of response (DOR) includes the time from documentation of tumor response to disease progression. In some instances, the parameter for assessing response can include durable response, e.g., response that persists after a period of time from initiation of therapy. In some instances, durable response is indicated by the response rate at approximately 1, 2, 3, 4, 5, 6, 7, 8, 9, 10, 11, 12, 18 or 24 months after initiation of therapy. In some instances, the response is durable for greater than 3 months, greater than 6 months, or great than 12 months. In some particular instances, the subjects treated according to the method achieve a more durable response after the subject previously relapsed following remission in response to the administration of the genetically engineered cells.

In some aspects, disease burden is measured or detected prior to administration of the immunotherapy, *e.g.* T cell therapy, following the administration of the immunotherapy, *e.g.* T cell therapy but prior to administration of the immunomodulatory compound, *e.g*., lenalidomide, following administration of the immunomodulatory compound but prior to the administration of the immunotherapy, *e.g*., T cell therapy, and/or following the administration of both the immunotherapy, *e.g*. T cell therapy and the immunomodulatory compound. In the context of multiple administration of one or more steps of the combination therapy, disease burden in some instances may be measured prior to or following administration of any of the steps, doses and/or cycles of administration, or at a time between administration of any of the steps, doses and/or cycles of administration.

In some instances, the burden is decreased by or by at least at or about 10, 20, 30, 40, 50, 60, 70, 80, 90, or 100 percent by the disclosed methods compared to immediately prior to the administration of the immunomodulatory compound, *e.g*., lenalidomide and the immunotherapy, *e.g.* T cell therapy. In some instances, disease burden, tumor size, tumor volume, tumor mass, and/or tumor load or bulk is reduced following administration of the immunotherapy, *e.g*. T cell therapy and the immunomodulatory compound, by at least at or about 10, 20, 30, 40, 50, 60, 70, 80, 90 % or more compared to that immediately prior to the administration of the immunotherapy, *e.g*. T cell therapy and/or the immunomodulatory compound.

In some instances, reduction of disease burden by the method comprises an induction in morphologic complete remission, for example, as assessed at 1 month, 2 months, 3 months, or more than 3 months, after administration of, *e.g*., initiation of, the combination therapy.

In some aspects, an assay for minimal residual disease, for example, as measured by multiparametric flow cytometry, is negative, or the level of minimal residual disease is less than about 0.3%, less than about 0.2%, less than about 0.1%, or less than about 0.05%.

In some instances, the event-free survival rate or overall survival rate of the subject is improved by the methods, as compared with other methods. For example, in some instances, event-free survival rate or probability for subjects treated by the methods at 6 months following the method of combination therapy disclosed herein, is greater than about 40%, greater than about 50%, greater than about 60%, greater than about 70%, greater than about 80%, greater than about 90%, or greater than about 95%. In some aspects, overall survival rate is greater than about 40%, greater than about 50%, greater than about 60%, greater than about 70%, greater than about 80%, greater than about 90%, or greater than about 95%. In some instances, the subject treated with the methods exhibits event-free survival, relapse-free survival, or survival to at least 6 months, or at least 1, 2, 3, 4, 5, 6, 7, 8, 9, or 10 years. In some instances, the time to progression is improved, such as a time to progression of greater than at or about 6 months, or at least 1, 2, 3, 4, 5, 6, 7, 8, 9, or 10 years.

In some instances, following treatment by the method, the probability of relapse is reduced as compared to other methods. For example, in some instances, the probability of relapse at 6 months following the method of combination therapy, is less than about 80%, less than about 70%, less than about 60%, less than about 50%, less than about 40%, less than about 30%, less than about 20%, or less than about 10%.

### IV. ARTICLES OF MANUFACTURE AND KITS

Also disclosed are articles of manufacture containing an immunomodulatory drug (immunomodulatory compound), such as lenalidomide, and components for the immunotherapy, *e.g*., antibody or antigen binding fragment thereof or T cell therapy, *e.g.* engineered cells, and/or compositions thereof. The articles of manufacture may include a container and a label or package insert on or associated with the container. Suitable containers include, for example, bottles, vials, syringes, IV solution bags, etc. The containers may be formed from a variety of materials such as glass or plastic. The container in some instances holds a composition which is by itself or combined with another composition effective for treating, preventing and/or diagnosing the condition. In some instances, the container has a sterile access port. Exemplary containers include an intravenous solution bags, vials, including those with stoppers pierceable by a needle for injection, or bottles or vials for orally administered agents. The label or package insert may indicate that the composition is used for treating a disease or condition.

The article of manufacture may include (a) a first container with a composition contained therein, wherein the composition includes the antibody or engineered cells used for the immunotherapy, *e.g*. T cell therapy; and (b) a second container with a composition contained therein, wherein the composition includes the second agent, such as an immunomodulatory compound, *e.g*., lenalidomide. The article of manufacture may further include a package insert indicating that the compositions can be used to treat a particular condition. Alternatively, or additionally, the article of manufacture may further include another or the same container comprising a pharmaceutically-acceptable buffer. It may further include other materials such as other buffers, diluents, filters, needles, and/or syringes.

### V. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

As used herein, a "subject" is a mammal, such as a human or other animal, and typically is human. In some instances, the subject, e.g., patient, to whom the immunomodulatory polypeptides, engineered cells, or compositions are administered, is a mammal, typically a primate, such as a human. In some instances, the primate is a monkey or an ape. The subject can be male or female and can be any suitable age, including infant, juvenile, adolescent, adult, and geriatric subjects. In some instances, the subject is a non-primate mammal, such as a rodent.

As used herein, "treatment" (and grammatical variations thereof such as "treat" or "treating") refers to complete or partial amelioration or reduction of a disease or condition or disorder, or a symptom, adverse effect or outcome, or phenotype associated therewith. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. The terms do not imply complete curing of a disease or complete elimination of any symptom or effect(s) on all symptoms or outcomes.

As used herein, "delaying development of a disease" means to defer, hinder, slow, retard, stabilize, suppress and/or postpone development of the disease (such as cancer). This delay can be of varying lengths of time, depending on the history of the disease and/or individual being treated. As is evident, a sufficient or significant delay can, in effect, encompass prevention, in that the individual does not develop the disease. For example, a late stage cancer, such as development of metastasis, may be delayed.

"Preventing," as used herein, includes providing prophylaxis with respect to the occurrence or recurrence of a disease in a subject that may be predisposed to the disease but has not yet been diagnosed with the disease. In some instances, the provided cells and compositions are used to delay development of a disease or to slow the progression of a disease.

As used herein, to "suppress" a function or activity is to reduce the function or activity when compared to otherwise same conditions except for a condition or parameter of interest, or alternatively, as compared to another condition. For example, cells that suppress tumor growth reduce the rate of growth of the tumor compared to the rate of growth of the tumor in the absence of the cells.

An "effective amount" of an agent, e.g., a pharmaceutical formulation, cells, or composition, in the context of administration, refers to an amount effective, at dosages/amounts and for periods of time necessary, to achieve a desired result, such as a therapeutic or prophylactic result.

A "therapeutically effective amount" of an agent, e.g., a pharmaceutical formulation or engineered cells, refers to an amount effective, at dosages and for periods of time necessary, to achieve a desired therapeutic result, such as for treatment of a disease, condition, or disorder, and/or pharmacokinetic or pharmacodynamic effect of the treatment. The therapeutically effective amount may vary according to factors such as the disease state, age, sex, and weight of the subject, and the immunomodulatory polypeptides or engineered cells administered. In some instances, the disclosed methods involve administering the immunomodulatory polypeptides, engineered cells, or compositions at effective amounts, e.g., therapeutically effective amounts.

A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

As used herein, recitation that nucleotides or amino acid positions "correspond to" nucleotides or amino acid positions in a disclosed sequence, such as set forth in the Sequence listing, refers to nucleotides or amino acid positions identified upon alignment with the disclosed sequence to maximize identity using a standard alignment algorithm, such as the GAP algorithm. By aligning the sequences, one can identify corresponding residues, for example, using conserved and identical amino acid residues as guides. In general, to identify corresponding positions, the sequences of amino acids are aligned so that the highest order match is obtained (see, e.g. : Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New. Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; Carrillo et al. (1988) SIAM J Applied Math 48: 1073).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors." Among the vectors are viral vectors, such as retroviral, e.g., gammaretroviral and lentiviral vectors.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control under otherwise identical conditions, and/or at a level substantially lower than that for cell known to be positive for the marker, and/or at a level substantially similar as compared to that for a cell known to be negative for the marker.

An amino acid substitution may include replacement of one amino acid in a polypeptide with another amino acid. The substitution may be a conservative amino acid substitution or a non-conservative amino acid substitution. Amino acid substitutions may be introduced into a binding molecule, e.g., antibody, of interest and the products screened for a desired activity, *e*.*g*., retained/improved antigen binding, decreased immunogenicity, or improved ADCC or CDC.

Amino acids generally can be grouped according to the following common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

In some instances, conservative substitutions can involve the exchange of a member of one of these classes for another member of the same class. In some instances, non-conservative amino acid substitutions can involve exchanging a member of one of these classes for another class.

As used herein, "percent (%) amino acid sequence identity" and "percent identity" when used with respect to an amino acid sequence (reference polypeptide sequence) is defined as the percentage of amino acid residues in a candidate sequence (e.g., the subject antibody or fragment) that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways, for instance, using publicly available computer software such as BLAST, BLAST-2, ALIGN or Megalign (DNASTAR) software. Appropriate parameters for aligning sequences, including any algorithms needed to achieve maximal alignment over the full length of the sequences being compared, can be determined.

As used herein, the singular forms "a," "an," and "the" include plural referents unless the context clearly dictates otherwise. For example, "a" or "an" means "at least one" or "one or more." It is understood that aspects and variations described herein include "consisting" and/or "consisting essentially of" aspects and variations.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

The term "about" as used herein refers to the usual error range for the respective value readily known in this technical field. Reference to "about" a value or parameter herein includes (and describes) instances that are directed to that value or parameter per se. For example, description referring to "about X" includes description of "X".

As used herein, a composition refers to any mixture of two or more products, substances, or compounds, including cells. It may be a solution, a suspension, liquid, powder, a paste, aqueous, non-aqueous or any combination thereof.

### VII. EXAMPLES

The following examples are included for illustrative purposes only and are not intended to limit the scope of the invention.

### Example 1 Anti-BCMA CAR-T cell cytolytic activity and cytokine production following incubation with BCMA expressing target cell lines in the presence or absence of lenalidomide

T cells were isolated by immunoaffinity-based enrichment from leukapheresis samples from healthy donors. Isolated cells were transduced with a viral vector encoding one of various exemplary anti-BCMA CARs. Each anti-BCMA CAR contained a human anti-BCMA scFv, a spacer region, a CD28 transmembrane domain, a 4-1BB-derived intracellular co-signaling sequence, and a CD3-zeta derived intracellular signaling domain. The viral vector construct further encoded a truncated EGFR (EGFRt), which served as a surrogate marker for CAR expression; the EGFRt-coding region was separated from the CAR sequence by a T2A skip sequence. After transduction, cells were expanded and the resulting compositions were frozen by cryopreservation.

Cryofrozen anti-BCMA CAR T cells were thawed and were assessed for various responses following co-culture with BCMA-expressing target cells in the presence or absence of lenalidomide. In vitro assays to evaluate target cell killing and cytokine production were conducted using two different BCMA-expressing target multiple myeloma cell lines RPMI-8226 or OPM-2. FIG. 1A shows the surface BCMA expression, as assessed by flow cytometry after staining with an anti-BCMA antibody, of exemplary multiple myeloma cells lines, including RPMI-8226 and OPM-2. The dotted line indicates background of a BCMA-negative cell line stained with anti-BCMA antibody. MFI, median fluorescence intensity. Expression of BCMA was relatively low for both cell lines (see Lee et al. (2016) Br J Haematol. 174:911-922). RPMI-8226 has been shown to be more sensitive to lenalidomide compared with OPM-2 (6.43 and 37.4 µM, respectively) (Wellcome Sanger Institute. Genomics of drug sensitivity in cancer. www.cancerrxgene.org/translation/Drug/1020. Accessed February 7, 2018).

### A. RPMI-8226

### 1. Cytolytic Activity

Cells of the BCMA-expressing target cell line (RPMI-8226) were incubated with exemplary anti-BCMA CAR T cells expressing a CAR with a human anti-BCMA scFv at an effector to target cell (E:T) ratio of 0.3:1 in the presence of 1 µM or 10 µM lenalidomide or in the absence of lenalidomide (vehicle). Co-cultures with T cells not expressing the CAR (mock) or cultures with target cells only (no CAR T) were used as controls, each in the presence or absence (vehicle) of 10 µM or 1 µM lenalidomide. Cells from each condition were plated in triplicate.

The target RPMI-8226 cells were labeled with NucLight Red (NLR) to permit their tracking by microscopy. Cytolytic activity was assessed by measuring the loss of viable target cells over a period of six days, as determined by red fluorescent signal (using the IncuCyte^{®} Live Cell Analysis System, Essen Bioscience). Normalized target cell numbers were generated by dividing target cell counts to cell counts at the start of each culture. The percentage of target killing was assessed by measuring the area under the curve (AUC) for normalized target cell count over time and normalizing the inverse AUC (1/AUC) values by defining a 0% value (target cells alone) and a 100% value (CAR+ T cells co-cultured with target cells in vehicle control).

As shown, co-culture in the presence of 1 µM (FIG. 1C) or 10 µM (FIG. 1B, 1C) lenalidomide resulted in a greater degree of target cell killing by anti-BCMA CAR+ T cells by day 6 of the co-culture, compared to incubation of target cells with anti-BCMA CAR+ T cells in the absence of lenalidomide (set at 100% in FIG. 1B). As shown in FIG. 1C, the observed effect of lenalidomide on cytolytic activity was dose-responsive and delayed, not emerging until after approximately 50 hours in culture. The results were consistent with a role of lenalidomide in promoting continued function and/or survival (such as by preventing exhaustion or cell death) of CAR-T cells after initial activation. Similar results were observed for cells engineered to expressing a number of other anti-BCMA CARs, each having different scFv binding domains.

### 2. Cytokine Production/Accumulation

Levels of various cytokines were assessed in culture supernatants after incubating anti-BCMA CAR T cells with cells of the BCMA-expressing target cell line RPMI-8226 at a 0.3:1 effector to target cell (E:T) ratio in the presence or absence of 10 µM lenalidomide. Culture of T cells not expressing the anti-BCMA CAR (mock) was used as a control. Amounts of IL-2 (FIG. 2A), IFNγ (FIG. 2B), and TNF-α (FIG. 2C) in culture supernatants was assessed at 48 hours after culture initiation. As shown in FIGs. 2A-2C, the presence of lenalidomide was associated with an increase in CAR-dependent cytokine production and/or accumulation following co-culture of anti-BCMA CAR T cells target cells with antigen-specific target cells. These results were consistent with a role for lenalidomide in promoting CAR-mediated effector functions. Similar results were observed with cells expressing various other anti-BCMA CARs, each having different scFv binding domains.

### B. OPM-2

### 3. Cytolytic Activity

Target OPM-2 multiple myeloma cells were incubated with human T cells (isolated from four different independent donors) expressing an exemplary anti-BCMA CAR) at an effector to target cell (E:T) ratio of 1:1 in the presence of 0.01 µM, 0.1 µM, 1.0 µM or 10 µM lenalidomide or in the absence of lenalidomide for a period of 7 days. The OPM-2 cells were labeled with NucLight Red (NLR) to permit tracking of target cells by microscopy substantially as described above. Cytolytic activity was assessed by measuring the loss of viable target cells at the end of the incubation. Degree of cytolytic activity observed for cultures incubated in the absence of lenalidomide was set as baseline, 100 %. The results are shown in FIG. 3A.The addition of lenalidomide was observed to enhance cytolytic activity of the anti-BCMA CAR+ T cells against OPM-2 target cells, in a dose-dependent manner. Similar results were observed in other anti-BCMA CAR-expressing T cells, including those expressing different anti-BCMA CARs each having different scFv binding domains and/or engineered using cells from different donors.

### 4. Cytokine

Anti-BCMA CAR T cells, produced from four independent donors, were incubated with BCMA-expressing target cell line OPM-2 at an effector to target cell (E:T) ratio of 1:1 in the presence of 0.01 µM, 0.1 µM, 1.0 µM or 10 µM lenalidomide or in the absence of lenalidomide (baseline, set at 100%). After 24 hours of culture, the presence of IFNγ (FIG. 3B), IL-2 (FIG. 3C), and TNF-α (FIG. 3D) in culture supernatants was assessed. As shown in FIGS. 3B-3D, lenalidomide was observed to enhance cytokine production and/or accumulation by antigen-stimulated anti-BCMA CAR+ T cells in a dose-dependent manner.

### C. COMPARISON OF ACTIVITY FROM MULTIPLE DONOR-DERIVED ANTI-BCMA CAR+ TCELLS

In another study, anti-BCMA CAR T cells from a representative healthy donor and a multiple myeloma patient (patient was refractory to pomalidomide) were incubated with fluorescently labeled OPM-2 target cells at an effector to target cell (E:T) ratio of 0.3:1 in the presence of varying concentrations of lenalidomide (0.01 µM, 0.1 µM, 1.0 µM or 10 µM lenalidomide) or in the absence of lenalidomide for 6 to 7 days. Cytolytic activity was measured by loss of red fluorescent cells. To assess cytokine production, healthy donor and multiple myeloma patient-derived anti-BCMA CAR-T cells were cocultured with fluorescently labeled OPM-2 target cells at an effector to target cell (E:T) ratio of 1:1 in the presence of varying concentrations of lenalidomide (0.01 µM, 0.1 µM, 1.0 µM or 10 µM lenalidomide) or in the absence of lenalidomide. After 24 hours, the media was sampled to assess the presence of IFNγ and IL-2. The results as shown in FIG. 3E, which are an average of two experiments; antigen-specific anti-BCMA CAR-T cytolytic activity and cytokine production were observed to be increased by lenalidomide in a concentration-dependent manner.

The studies above were extended on anti-BCMA CAR+ T cells generated from cells from two additional healthy donors. Activity of anti-BCMA CAR+ T cells from three healthy donors and one IMiD-refractory patient (patient donor was refractory to pomalidomide) was compared against both OPM-2 and RPMI-8226 BCMA-expressing multiple myeloma cell lines. Cytolytic activity and cytokine production (IFNγ, IL-2 and TNF-α) were assayed substantially as described above. Absolute changes in cytokine levels relative to the vehicle control were calculated. Experiments were performed 2 to 3 times in each donor.

Increased anti-BCMA CAR T cytolytic activity against OPM-2 target cells titrated with increased concentrations of lenalidomide was observed across all donors (*P* = 6.2 × 10⁻⁵) (FIG. 3F). As shown in FIG. 3F, the treatment effect of lenalidomide on CAR T cytolytic activity appeared to be donor-dependent in co-culture with RPMI-8226, with the patient donor showing a significant increase in cytolytic activity (*P* = 1.9 × 10⁻⁸). In addition, all CAR T donors had significantly increased IFN-γ, IL-2, and TNF-α production in a lenalidomide concentration-dependent manner on co-culture with OPM-2 cells (*P* < 0.002, FIG. 3G). Cytokine production by CAR-expressing T cells in RPMI-8226 co-culture was also significantly increased across all donors and cytokines upon treatment with lenalidomide (*P* < 0.003, FIG. 3H).

### Example 2 Effect of Lenalidomide on CAR-T Cell Expansion and Antigen-Specific Function With Serial Restimulation

### A. CAR-T Cell Expansion

The ability of CAR T cells to expand and exhibit antigen-specific function ex vivo following repeated rounds of antigen stimulation can correlate with in vivo function and/or capacity of the cells to persist in vivo (e.g. following administration and initial activation in response to encounter with antigen) (Zhao et al. (2015) Cancer Cell, 28:415-28). Anti-BCMA CAR+ T cells generated as described above were plated in triplicate at 1 × 10⁵ cells/well on 96-well plates. Irradiated BCMA-expressing target cells (MM1.S cells) were added at an effector-to-target (E:T) ratio of 1:2 in the presence or absence of various concentrations (0.01 µM, 0.1 µM, 1.0 µM or 10 µM) of lenalidomide.

Every 3-4 days (start of each new round), CAR T cells were counted. Cells then were harvested and re-plated at the initial seeding density with fresh media, newly-added lenalidomide at the same concentration, where applicable, and newly-thawed, newly-irradiated target cells. 8 rounds of stimulation were carried out during a 31 day culture period. For some rounds, at re-plating, cells were assessed for phenotypic markers via flow cytometry.

Exemplary results are shown in FIG. 4A. As shown, increased expansion of anti-BCMA CAR T cells was observed by day 14 for all concentrations of lenalidomide, compared to wells with no lenalidomide. The assay was performed on various compositions of anti-BCMA CAR+ T cells, each generated by introducing the CAR into T cells derived from one of six different donors. The assay was performed across cells from six different independent donors engineered to express the CAR. For each donor, there was observed an increase or no change in CAR-T expansion at the 0.1 µM concentration of lenalidomide. FIG. 4B shows results of a similar assay, in which cells engineered to express two different human anti-BCMA CARs were subjected to multiple rounds of target cell stimulation in the presence or absence of lenalidomide. As shown, the presence of lenalidomide in the cultures was observed was observed to increase expansion in both cell populations, beginning between day 21 and day 28 The results were consistent with a conclusion that lenalidomide can promote continued CAR+ T cell expansion and/or survival following repeated encounter with cognate antigen.

### B. CAR-T cell count, cytokine production, and activation

Anti-BCMA CAR+ T cells from 3 donors generated as described above were plated in triplicate on 96-well plates with irradiated BCMA-expressing target cells (MM1S cells) at an effector-to-target (E:T) ratio of 1:2, in the presence of 0.1 µM lenalidomide or vehicle control. Culture conditions were reset every 3-4 days. Replating was maintained for 28 days or until the cell count was < 50,000 cells. Experiments were performed in triplicate in 3 donors. Cytokine levels (IFNγ, IL-2, and TNF-α) were assessed 24 hours after re-plating at days 5, 8, and 15 Activation of CAR-T cells was measured on cells collected at days 4, 7, and 14 by flow cytometry for CD25.

FIG. 5A shows the cell counts (projected population doublings) of the anti-BCMA CAR+ T cells for each restimulation time point. The "x" indicates insufficient cells for replating in the assay. The results showed that after repeated stimulation with target cells, all 3 CAR T donors treated with lenalidomide had increased projected cell counts over 28 days relative to controls (P < 0.003). FIG. 5B shows CD25 median fluorescent intensity (MFI) (gated on live CD3⁺ CAR⁺) and FIG. 5C shows cytokine production normalized for cell number plated. Increased cell counts were associated with a significant increase in CAR T CD25 expression (*P* < 3.4 × 10⁻⁴; FIG. 5B) and IL-2, IFN-γ, and TNF-α production in the media (P < 0.5; FIG. 5C). The results showed that anti-BCMA CAR-T cell count, cytokine production, and activation were increased by lenalidomide after repeated stimulations *in vitro.*

### Example 3 Effects of Lenalidomide on BCMA CAR-T proliferation and activation in 3D myeloma model

To assess cell function in the context of a three-dimensional (3-D) human BCMA-expressing tissue microenvironment, a reconstructed bone marrow (rBone^{™}) (zPREDICTA, San Jose, CA) was embedded with BCMA-expressing RPMI-8226. 20,000 T cells expressing another exemplary human anti-BCMA CAR (or mock T cells not expressing the CAR) were incubated in the 3-D model in the presence or absence of 1.0 µM lenalidomide.

After 2 or 7 days, cells were isolated and assessed by flow cytometry for surface expression of CD3, CD25, CD4, and CD8. As shown in FIG. 6A, the presence of lenalidomide was observed to result in an increase in total number of CD3+ cells in cultures with anti-BCMA CAR+ T cells at day 7. An increase in CD25+ expression in CD4+ (FIG. 6B) and CD8+ (FIG. 6C) T cell populations also was observed in the presence of lenalidomide. The results were consistent with a conclusion that lenalidomide can promote increased expansion, survival and/or function of anti-BCMA CAR+ T cells in an antigen-expressing tumor microenvironment.

### Example 4 Effect of lenalidomide on CAR T-cell function in vivo

Anti-tumor effects of anti-BCMA CAR T cells, alone and in combination with lenalidomide, were assessed in two different BCMA-expressing mouse tumor models-an RPMI 8226 human multiple myeloma xenograft mouse model (subcutaneous implant model) and an OPM-2 human multiple myeloma xenograft mouse model (orthotopic bone marrow model).

### A. RPMI-8226 MODEL

Mice were injected subcutaneously (s.c.) with 5 × 10⁶ RPMI-8226 cells, and tumor volume was allowed to grow to approximately 150 mm³. At day 0, a composition containing a sub-optimal (low) dose of anti-BCMA CAR+ T cells (generated by transducing cells derived from samples of human donor subjects essentially as described above) was administered intravenously to mice (with a similar composition of T cells not expressing a CAR (mock) used as a control). Specifically, the composition contained approximately 5 × 10⁵ CAR+ (or mock) CD4+ T cells and 5 × 10⁵ CAR+ (or mock) CD8+ T cells. T cells were adoptively transferred to mice, alone or in combination with lenalidomide, administered daily, at 25 mg/kg, intraperitoneally (i.p.), beginning at d=0 (with T cell administration), continuing through day 21. In another control group, mice were administered lenalidomide alone, without administration of T cells. Tumor volume and survival of animals were monitored throughout the study. A retroorbital (RO) bleed was taken weekly for plasma BCMA and IFN-gamma levels and for pharmacokinetic (PK) assessment of CAR+ T cells.

Tumor volume measurements are shown for individual animals in FIG. 7A, administration of lenalidomide and the low dose of anti-BCMA CAR+ T cells in combination was observed to result in slower tumor growth compared to mice treated with lenalidomide or anti-BCMA CAR+ T cells alone. The observed effect was most evident at later time-points, including those subsequent to the last daily lenalidomide administration (i.e., after day 21). The results are consistent with the ability of lenalidomide to increase the ability of T cells to persist and/or function long-term.

As shown in FIG. 7B, mice that had lenalidomide and anti-BCMA CAR+ T cells exhibited increased survival compared to the other treatment groups. The mean survival (ms) of mice administered anti-BCMA CAR+ T cells and lenalidomide was 85 days (double, compared to the other treatment groups, which exhibited mean survival of 38-43.5 days).

Additionally, the number of CD4+ and CD8+ CAR+ T cells and non-CAR T cells in peripheral blood of each animal was determined at days 7, 14, 21 and 34. The numbers of CD4+ CAR T cells and non-CAR T cells are shown in FIGs. 8A and 8E (days 7 and 14), respectively, and FIG. 8B and 8F (days 21 and 34), respectively. The numbers of CD8+ CAR T cells non-CAR T cells are shown in FIGs. 8C and 8G (days 7 and 14), respectively, and Fig. 8D and 8H (days 21 and 34), respectively. As shown, an increase in numbers of CD4+ and CD8+ CAR+ T cells (but not non-CAR+ T cells) in blood was observed at day 36 in mice having received the combination of anti-BCMA CAR+ T cells and lenalidomide, compared to the other treatment groups.

### B. OPM-2 MODEL

### i. Study 1

The effect of lenalidomide in combination with anti-BCMA CAR T was also assessed in a murine orthotopic tumor model using OPM-2 cells. Mice (NOD.Cg-Prkdc^{scid}IL-2rg^{tm1Wj1}/SzJ mice (NSG; Jackson Labs)) were injected intravenously (i.v.) with 2 × 10⁶ OPM2 (multiple myeloma) cells transfected with firefly luciferase (OPM2-ffluc). Tumor engraftment was allowed to occur for 13 days prior to staging (14 days before CAR-T cell administration) and verified using bioluminescence imaging. Mice were administered one or more compositions in various treatment groups, as follows and summarized in Table E1.

Some groups received 10 mg/kg lenalidomide in phosphate-buffered saline via intraperitoneal injection, either (A) beginning at day -1 (one day prior to administration of CAR+ T cells) (lenalidomide (A)); or (B) at day 14 (day 14 post-initiation of CAR+ T cell administration) (lenalidomide (B)), in each case, daily, for the duration of the study. In groups receiving CAR+ T cells, anti-BCMA CAR (generated by transducing cells derived from samples of human donor subjects essentially as described above) were administered at day 0 (day 14 after tumor cell injection), at a dose of either 5 × 10⁵ (low) or 1 × 10⁶ (high) CAR-expressing T cells. Table E1 summarizes the dosing regimens.

| **Table E1: Study Design** | | |
|---|---|---|
| **Group No.** | **Group Description** | **CAR-T cells administered (or mock-transduced T cells)** |
| 1 | Tumor only | 0 |
| 2 | Mock (high) | (1 x 10⁶) |
| 3 | lenalidomide (A) | n/a |
| 4 | lenalidomide (B) | n/a |
| 5 | Mock + lenalidomide (A) | (1 x 10⁶) |
| 6 | Mock + lenalidomide (B) | (1 x 10⁶) |
| 7 | Anti-BCMA CAR+ T cells (high) | 1 x 10⁶ |
| 8 | Anti-BCMA CAR+ T cells (low) | 5 x 10⁵ |
| 9 | Anti-BCMA CAR+ T cells (high) + lenalidomide (A) | 1 x 10⁶ |
| 10 | Anti-BCMA CAR+ T cells (low) + lenalidomide (A) | 5 x 10⁵ |
| 11 | Anti-BCMA CAR+ T cells (high) + lenalidomide (B) | 1 x 10⁶ |
| 12 | Anti-BCMA CAR+ T cells (low) + lenalidomide (B) | 5 x 10⁵ |

Tumor burden in animals among the various groups was monitored by bioluminescence imaging up to day 39 post-CAR+ T cell dosing. For bioluminescence imaging, mice received intraperitoneal (i.p.) injections of luciferin substrate (CaliperLife Sciences, Hopkinton, MA) resuspended in PBS (15 µg/g body weight). The total flux (photon/s) was determined at each time point.

FIG. 9A and FIG. 9B depict results of tumor burden through up to day 46, in mice treated with lenalidomide daily beginning at day -1 (lenalidomide A) in the presence or absence of the high (1 × 10^6; FIG. 9A) or low (5 × 10^5; FIG. 9B) CAR+ T cell dose. FIG. 9C shows plots for tumor burden of individual animals through up to day 53. FIG. 9D shows plots and tumor imaging results (day 46 post-CAR+ cell administration) for individual animals having received the higher CAR+ dose, with lenalidomide at day -1 (lenalidomide A). FIG. 9E shows plots and tumor imaging results (day 46 post-CAR+ cell administration) for individual animals having received the higher CAR+ dose, without lenalidomide at day -1 (lenalidomide A). Asterisks indicate death or sacrifice of an individual animal at the time-point indicated in plot. As shown, the addition of lenalidomide was observed to result in slower tumor growth and reduced tumor burden in mice administered CAR+ T cells at both CAR+ T cell doses.

FIG. 9F and FIG. 9G depict tumor burden results at a time-point in a study for mice administered lenalidomide beginning at day 14 post-CAR+T administration (lenalidomide B) (vertical lines in FIGs. 9F and 9G) in the presence or absence of the high (1 × 10^6, FIG. 9F) or low (5 × 10^5, FIG. 9G) CAR+ T cell dose. FIG. 9H shows plots for tumor burden of individual animals through up to day 53. FIG. 9I shows plots and tumor imaging results (day 46 post-CAR+ cell administration) for individual animals having received the higher CAR+ dose, with lenalidomide at day -1 (lenalidomide A). FIG. 9J shows plots and tumor imaging results (day 46 post-CAR+ cell administration) for individual animals having received the higher CAR+ dose, without lenalidomide at day -1 (lenalidomide A). As shown, whereas lenalidomide alone was not observed to reduce tumor growth or tumor burden, the addition of lenalidomide was observed to result in a trend towards slower tumor growth and reduced tumor burden in mice administered both doses of CAR-T cells, with clear differences observed beginning at day 30-40 post-CAR-T cell injection for the higher (1 × 10^6) dose of CAR+ T cells. With this dose, combination with lenalidomide was observed to slow tumor growth whether given at day -1 or via delayed dosing.

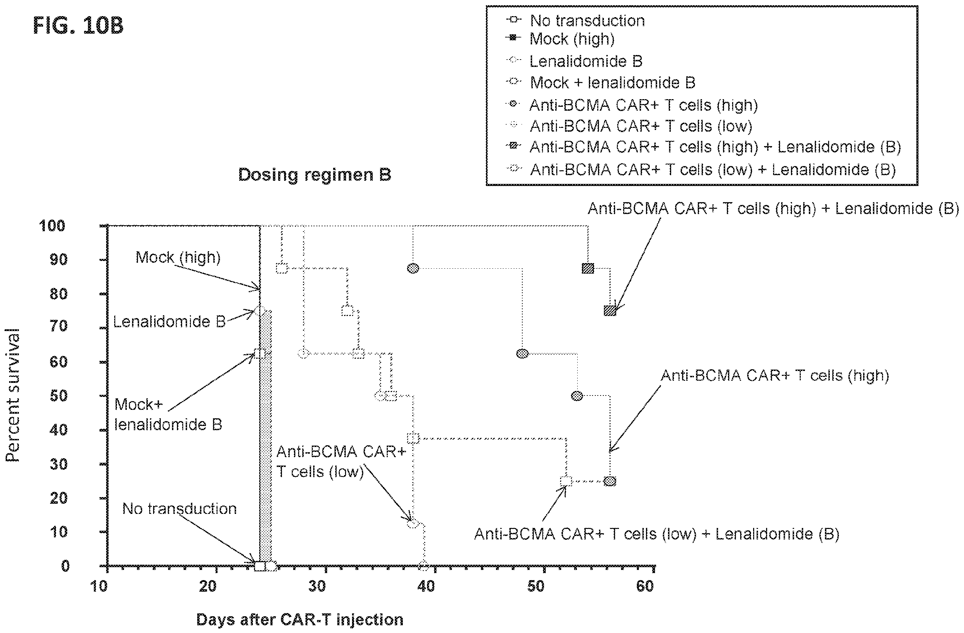

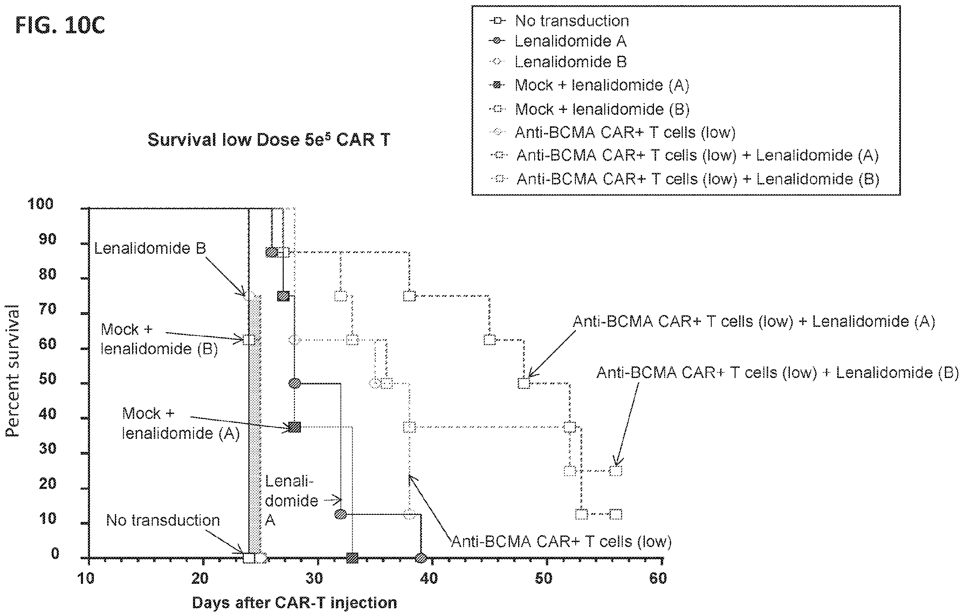

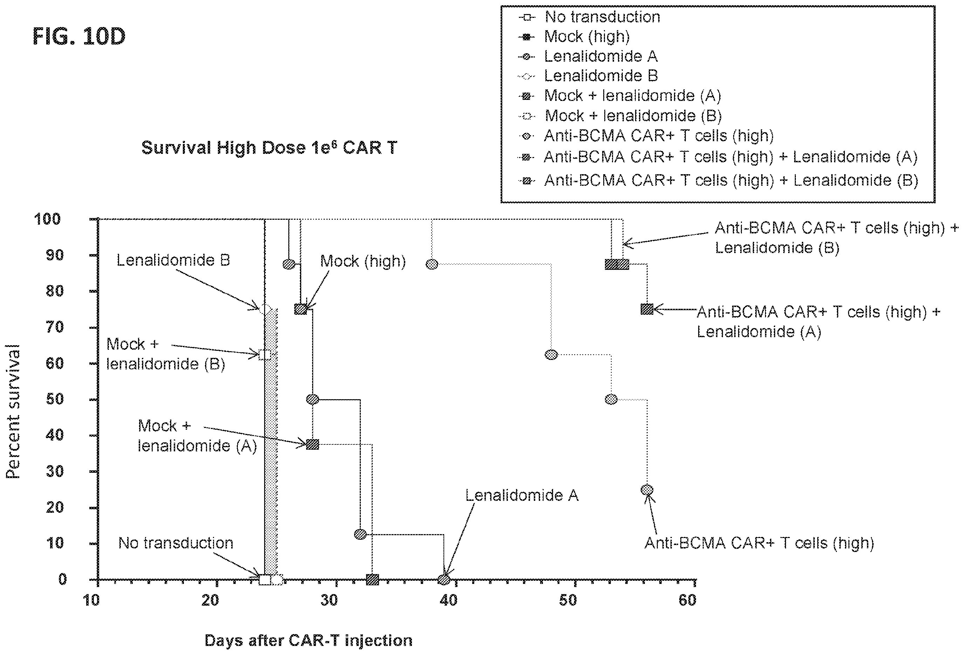

Survival results at a time-point in the study are shown in FIGs. 10A and 10B (for groups receiving lenalidomide via regimens (d-1) and B (d.14 post-CAR (delayed), respectively), and FIGs. 10C and 10D (for groups receiving high and low CAR doses, respectively). As shown, the addition of lenalidomide improved survival effects observed in mice treated with the anti-BCMA CAR+ T cells (at both the high and low doses assessed), when administered either at day -1 (A) or via delayed (d. 14) dosing (B).

Table E2 lists median survival (ms) (as assessed at day 56 post-CAR+ T cell administration) and number of mice in group surviving until day 56-post-CAR+ T cell administration, for each animal group assessed in this study.

| **Table E2: Survival** | | | |
|---|---|---|---|
| **Group** | **Cell Dose** | **Median Survival (assessed at day 6)** | **# animals surviving day 56 post-CART** |
| Mock hi | 1.00E+06 | 24 | 0/8 |
| lenalidomide (A) | N/A | 28 | 0/8 |
| lenalidomide (B) | N/A | 25 | 0/8 |
| Mock+lenalidomide (A) | 1.00E+06 | 28 | 0/8 |
| Mock+lenalidomide (B) | 1.00E+06 | 25 | 0/8 |
| CAR+ T cells (hi) | 1.00E+06 | 56 | 2/8 |
| CAR+ T cells (low) | 5.00E+05 | 35 | 0/8 |
| CAR+ T cells (hi) + lenalidomide (A) | 1.00E+06 | N/A | 6/8 |
| CAR+ T cells (low) + lenalidomide (A) | 5.00E+05 | 52 | 1/8 |
| CAR+ T cells (hi) + lenalidomide (B) | 1.00E+06 | N/A | 6/8 |
| CAR+ T cells (low) + lenalidomide (B) | 5.00E+05 | 36 | 2/8 |

### (ii.) Study 2

In a further study, NOD/Scid/gc^{-/-} (NSG) mice were injected (i.v.) with OPM-2-luciferase cells as described in Study 1 above and allowed to engraft for 14 days prior to CAR-T (or mock) cell; infusion (i.v.). In some groups, daily intraperitoneal administration of 10 mg/kg lenalidomide or vehicle control was initiated either at day -1 (one day prior to CAR-T administration) (concurrent lenalidomide (lenalidomide (C) or vehicle (vehicle (C)) or at day 14 post-CAR-T (or mock) cell administration (delayed lenalidomide (D)).

At 14 days after tumor cell injection (day 0), a subtherapeutic dose of CAR+ T cells (1 × 10^6 CAR-T cells (generated from two different donors)) or mock control cells was injected intravenously. Results are shown in FIG. 10E, 10F, 10G, and 10H. Data are presented as mean ± SEM. For in vivo survival, a Gehan-Breslow-Wilcoxon test was used to compare groups.

FIG. 10E depicts the results of tumor burden assessment through day 60, as analyzed by the bioluminescence measured by flow cytometry. The addition of lenalidomide was observed to result in slower tumor growth and reduced tumor burden in mice administered CAR+ T cells generated from both donor cells. As shown in FIG. 10F, the addition of lenalidomide was observed to improve survival effects in mice treated with the anti-BCMA CAR+ T cells, particularly following concurrent administration of lenalidomide. Linear fixed-effect or mixed-effect models were used to assess the significance of lenalidomide treatments on cytolytic activity, with treatment, donor, and time treated as fixed effects and animal treated as a random effect, nested with time when repeated measurements were derived from the same animal. *P* values were obtained by likelihood ratio tests comparing the full model with the effect of interest against the model without the effect of interest. The addition of concurrent lenalidomide led to a significant decrease in tumor burden for donor 1 (P = 0.02) and increased survival for donor 1 (P = 0.057) and donor 2 (P = 0.04) compared with vehicle-treated animals injected with anti-BCMA CAR T alone. Animals on the concurrent lenalidomide dosing regimen also showed increased CAR T counts in the peripheral blood after 7 days (*P* = 7.3 × 10⁻⁶), but not at later time points. Lenalidomide had a small, but significant mock CAR T effect on tumor burden for donor 1 (*P* = .003) alone. In this study, the addition of delayed dosing of lenalidomide did not improve tumor clearance and survival for both CAR T donors. The results showed that survival and tumor clearance by a subtherapeutic dose of anti-BCMA CAR-T were enhanced by lenalidomide in the in vivo OPM-2 tumor model.

Blood from the treated mice was collected for CAR-T pharmacokinetic analysis, and cells were stained with antibodies to exclude mouse-specific cells (H2-kd, TER119, and muCD45) and analyzed by flow cytometry. Cells were gated on CD45+CD3+CAR+ and the cells per microliter of blood was determined. For pharmacokinetic measurements, each time point was analyzed by one-way ANOVA and Tukey post-hoc test. FIGs. 10G and 10H show the flow cytometric analysis of mock control cells and CAR-T cells in the blood of the mice at days 8, 14, 22, and 28 following injection of the CAR-T cells from two donors. The results showed that increased CAR-T cell counts were observed in peripheral blood at early time points, particularly following concurrent administration of lenalidomide (** P < .01).

### Example 5 Effects of Lenalidomide on anti-CD19 CAR proliferation in sub-optimal stimulation

Anti-CD19 CAR-expressing T cells were generated by engineering CD4+ and CD8+ T cells (which had been isolated by immunoaffinity-based enrichment from healthy human donor subjects) with viral vector encoding the anti-CD19 CAR. The CAR contained an anti-CD19 scFv, an Ig-derived spacer, a human CD28-derived transmembrane domain, a human 4-1BB-derived intracellular signaling domain and a human CD3 zeta-derived signaling domain. The nucleic acid construct encoding the CAR also included a truncated EGFR (tEGFR) sequence for use as a transduction marker, separated from the CAR sequence by a self-cleaving T2A sequence.

Anti-CD19 CAR T cells were subjected to sub-optimal stimulation via incubation with anti-CD3 (without a second reagent such as anti-CD28, designed to provide a costimulatory signal), in the presence of 5 µM lenalidomide or vehicle control. The anti-CD19 CAR-expressing T cells were labeled with CELLTRACE VIOLET dye (CTV; ThermoFisher Scientific, Waltham MA) prior to the incubation; proliferation was assessed by assessing dilution of the dye via flow cytometry. As shown in FIG. 11, in the context of the sub-optimal stimulatory conditions over a period of 72 hours, lenalidomide was observed to have enhanced proliferation of the CAR+ T cells.

### Example 6 Observed relationship between treatment outcomes and levels of peripheral blood CAR+ T cells in cohort of human subjects administered anti-CD19 CAR-expressing cells

Treatment outcomes and numbers of CAR+ T cells in the blood, were evaluated in twenty eight adult subjects with relapsed or refractory (R/R) non-Hodgkin lymphoma (NHL) who had been administered autologous T cells expressing a CD19-targeting chimeric antigen receptor (CAR) including an anti-CD19 scFv antibody and a 4-1BB intracellular signaling domain (administered at approximately 1:1 ratio of CD4+ to CD8+ CAR+ T cells).

Prior to administration of the CAR-expressing T cells, subjects had been treated with 30 mg/m2 fludarabine daily for 3 days and 300 mg/m2 cyclophosphamide daily for 3 days. At d=0, subjects had been treated with 5 × 107 (DL-1) or 1 × 108 (DL-2) CAR-expressing T cells by intravenous infusion.

Response rates observed at a particular time-point in an ongoing study, are shown in Table E3 for a cohort of 20 Diffuse Large B-Cell Lymphoma (DLBCL) subjects treated with a single-dose of DL-1. As shown, an overall response rate (ORR) of 80% (16/20) was observed and 60% (12/20) of subjects were observed to have achieved complete remission (CR). 20% (4/20) of subjects exhibited partial response (PR) and 20% (4/20) exhibited progressive disease (PD). Of the subjects having been chemorefractory (having exhibited stable or progressive disease following last chemo-containing regimen or relapse less than 12 months after autologous SCT) prior to CAR+ T cell administration, the overall response rate was 83% (10 ORR, 7 CR, 3 PR, 2 PD, n=12). Among the subjects having been refractory (having exhibited less than complete remission following last treatment but not deemed chemorefractory), the overall response rate was 77% (13 ORR, 9 CR, 4 PR, 4PD, n=17).

| **Table E3. Overall Response** | | | |
|---|---|---|---|
| | **DLBCL Cohort, DL1 single-dose schedule** | | |
| | **All (n=20)** | **Refractory * (n=17)** | **Chemorefractory ^{†} (n=12)** |
| ORR, n (%) [95% CI] | 16 (80) [56, 94] | 13 (77) [50, 93] | 10 (83) [52, 98] |
| CR, n (%) [95% CI] | 12 (60) [36, 81] | 9 (53) [28, 77] | 7 (58) [28, 85] |
| PR | 4 (20) | 4 (24) | 3 (25) |
| PD | 4 (20) | 4 (24) | 2 (17) |

| | | | |
|---|---|---|---|
| *<CR to last therapy ^{†}SD or PD to last chemo-containing regimen or relapse <12 months after autologous SCT | | | |

Of three DLBCL subjects that at the time of assessment had been treated with two doses of DL-1, two exhibited partial response (PR) and 1 exhibited progressive disease (PD). Among 2 subjects having at the time of assessment been treated with a single-dose of DL-2, both subjects were observed to achieve CR. Among a MCL cohort with a total of two subjected treated at the time of assessment with single-dose of DL-1 1 PR and 1 PD were observed. Two subjects with double-hit, three subjects with triple-hit, and four subjects with double-expressor DLBCL were observed to achieve responses (7 CR, 2 PR).

The number of CAR⁺ T cells in peripheral blood was determined at certain time points post-treatment using a transgene-specific reagent. The number of CD3⁺/CAR⁺ T cells in peripheral blood measured at certain time points post-infusion is shown for subjects grouped by best overall response in FIG. **12A****.** Higher peak CD3⁺/CAR⁺ T cells were observed in responders (CR/PR) than in subjects with progressive disease (PD). FIGs. 12B-D show levels of CD3⁺/CAR⁺ T cells, CD4⁺/CAR⁺ T cells, and CD8⁺/CAR⁺ T cells (cells/µL blood; mean ± SEM) in subjects who achieved a response to treatment, grouped by durability of response (continued response (CR/PR) or PD at 3 months). The Cₘₐₓ (CAR⁺ cells/µL blood) and area under the curve (AUC) for responders (CR/PR) and PD are shown in Table E4. The results were consistent with a conclusion that durable responses correlated with higher CD3⁺/CAR⁺ T cell levels in the blood, over time and at peak expansion.

| **Table E4. Cₘₐₓ and AUC₀₋₂₈ Higher in Patients with CR/PR vs PD** | | | | | | |
|---|---|---|---|---|---|---|
| | **CD3** | | **CD4** | | **CD8** | |
| | **CR/PR (n=16)** | **PD (n=4)** | **CR/PR (n=16)** | **PD (n=4)** | **CR/PR (n=16)** | **PD (n=4)** |
| **Cₘₐₓ(CAR⁺ cells/µL blood)** | | | | | | |
| Mean (SD) | 612 (1919) | 2(1) | 220 (754) | 1 (0.6) | 426 (1314) | 0.5 (0.5) |
| Median (Min, Max) | 33 (1, 7726) | 1 (1, 3) | 8 (1, 3040) | 1 (0, 2) | 4 (0, 5238) | 0.3 (0, 1) |
| Q1, Q3 | 7, 123 | 0.7, 2 | 2, 46 | 0.6, 2 | 0.8, 104 | 0.1, 0.9 |

| **AUC₀₋₂₈** | | | | | | |
|---|---|---|---|---|---|---|
| Mean (SD) | 5883 (18821) | 16 (13) | 2369 (8388) | 10 (7) | 3873 (11963) | 6 (6) |
| Median (Min, Max) | 196 (11, 75773) | 14 (4, 31) | 47 (7, 33740) | 9 (3, 17) | 23 (1, 47834) | 4 (1, 14) |
| Q1, Q3 | 52, 781 | 5, 26 | 16, 261 | 4, 16 | 4, 761 | 1, 10 |

For one subject with chemorefractory transformed DLBCL (germinal center subtype with a BCL2 rearrangement and multiple copies of *MYC* and *BCL6*) who had been administered the CAR+ T cells at DL-1, numbers of CD3+/CAR+, CD4+/CAR+ , CD8+/CAR+ T cells in peripheral blood, measured at certain time points, are shown in FIG. **13A****.** The subject had previously been treated with, and was refractory to, five prior lines of therapy including doseadjusted etoposide, doxorubicin, and cyclophosphamide with vincristine and prednisone plus rituximab (DA-EPOCH-R) and intermediate-intensity allogenic stem-cell transplantation from an 8/8 HLA-matched unrelated donor. Following allogeneic stem cell transplantation and prior to receiving CAR+ T cells, the subject showed 100% donor chimerism in all blood lineages, had ceased taking immunosuppressive therapy, and did not have graft versus host disease (GVHD). Prior to administration of CAR+ T cells, the subject had a periauricular mass and temporal lobe lesion observed by positron-emission tomography and computed tomography (PET-CT) (FIG. 13B) and confirmed by magnetic resonance imaging (MRI) (FIG. 13D).

After receiving anti-CD 19 CAR-T cell treatment, the subject achieved CR 28 days post-infusion, as shown by PET-CT (FIG. 13C) and brain MRI (FIG. 13E), with no observed signs of neurotoxicity or CRS. Three months post-infusion of the CAR-T cells, relapse of the periauricular mass was noted in this subject (FIG. 13F), and an incisional biopsy was performed. As shown in FIG. 13A, following biopsy, the visible tumor receded with no further therapy. Pharmacokinetic analysis showed a marked re-expansion of the CAR+ T cells in peripheral blood (to a level higher than initial expansion observed, with peak levels observed at about 113 days post-infusion) i, which coincided with tumor regression. The subject then went on to achieve a second CR, as confirmed by restaging PET-CT one month following the biopsy (FIG. 13G), and remained in CR at 6 months post CAR-T cell infusion. Further assessment of the subject showed that the CNS response was durable and the subject remained in CR at 12 months.

The results are consistent with a conclusion that re-expansion and activation of CAR+T cells can be initiated *in vivo* following reduction or loss of functional or active CAR+ T cells and/or relapse following anti-tumor response to CAR-T cell therapy. Further, following re-expansion *in vivo* late after initial CAR+ T cell infusion, the CAR+ T cells are able to re-exert anti-tumor activity. This result supports that CAR+ T cell re-expansion and activation can be triggered *in vivo* and that methods of reactivating CAR+ T cells, may further augment their efficacy.

### Example 7 Effects of Lenalidomide on anti-CD19 CAR T cell Activity Following Serial Restimulation

Anti-CD19 CAR+ T cells, generated substantially as described in Example 5, were thawed and were incubated with CD19-expressing cells (K562 cells transduced to express CD 19) at an effector to target cell (E:T) ratio of 2.5:1 in the presence or absence of 1 nM, 5 nM, 60 nM, 550 nM or 5000 nM lenalidomide or in the absence of lenalidomide (control). The target K562-CD 19 cells were labeled with NucLight Red (NLR) as described in Example 1 to permit tracking of target cells by microscopy. Cytolytic activity was assessed by measuring the loss of viable target cells over a period of about 120 hours, as determined by red fluorescent signal (using the IncuCyte^{®} Live Cell Analysis System, Essen Bioscience). Cells from each condition were plated in triplicate. As shown in FIG. 14, the results were consistent with a conclusion that the presence of lenalidomide reduced CAR-mediated cytolytic activity in this assay. In similar assays, results varied depending on E:T ratios and with different anti-CD19 CAR+ T cell compositions (e.g., generated at different times and/or from cells from different donors).

In another study, anti-CD19 CAR+ T cells were incubated with K562-CD19 effector cells at a 2.5:1 E:T ratio in the presence of 100 nM or 1600 nM lenalidomide, 2 nM or 166 nM of an alternative compound targeting a kinase, vehicle control or in the absence of added compound (CAR-T control). After 120 hours of culture, cells were isolated and assessed by flow cytometry for surface expression of CD25 or PD-1 in CD4+ or CD8+ T cell subsets. As shown in FIG. 15A, incubation of anti-CD19 CAR+ T cells with K562-CD19 effector cells in the presence of the highest concentration of lenalidomide (e.g., 1600 nM) resulted in higher levels of CD25 expression in both CD4+ and CD8+T cells as compared to other conditions. No difference in surface expression of PD-1 was observed in CD4+ or CD8+ T cells in the presence of lenalidomide, even at the highest concentration of 1600 nM (FIG. 15B).

In a further study, the amount of IL-10 was assessed in culture supernatants after incubating, for 24 hours, anti-CD19 CAR+ T cells with K562-CD19 effector cells at an effector to target cell (E:T) ratio of 3:1 or 9:1, in the presence or absence of various concentrations of lenalidomide. As shown in FIG. 16, lenalidomide dose-dependently increased secretion and/or accumulation of IL-10 in supernatants of T cell cultures.

### Example 8 Effects of Lenalidomide on anti-CD19 CAR T cell Expansion Following Serial Restimulation

The ability of anti-CD19 CAR+ T cells to expand ex vivo following repeated stimulations was assessed using methods substantially as described in Example 2. Anti-CD19 CAR+ T cells, generated from two donors (pt1 and pt2) substantially as described in Example 5, were cultured with irradiated K562 cells transduced to express CD19 (K562-CD19 cells) at an effector target ratio of 2.5:1 in the presence or absence of 1 µM lenalidomide or 50 nM or 500 nM of an alternative compound targeting a kinase. For each donor, cells were harvested every 3-5 days from each experimental condition in the wells and counted, and restimulated with new target cells using the same culture conditions after resetting cell number to initial seeding density for each round. A total of 4 rounds of stimulation during a 12 day culture period were carried out. For each round of stimulation, the total number of cells was determined, and the results were depicted as the fold-change of cell number after stimulation (FIG. 17A) or number of doublings compared to initial number (FIG. 17B). As shown in FIG. 17A and 17B, no change or only a minor effect in cell expansion of anti-CD19 CAR+ T cells was observed in this restimulation assay when cells were cultured in the presence of lenalidomide versus in the absence of lenalidomide.

At each reset after the pretreatment, cytolytic activity was assessed by incubation of the retimulated cells with the K562-CD19 cells (labeled with NucLight Red (NLR)) at an effector to target cell (E:T) ratio of 1 µM lenalidomide or 50 nM or 500 nM of the alternative compound. Cytolytic activity was assessed by measuring the loss of viable target cells over a period of up to 40-60 hours, as determined by red fluorescent signal (using the IncuCyte^{®} Live Cell Analysis System, Essen Bioscience). Cells from each condition were plated in triplicate. Representative cell killing observed at the 2nd and 4th restimulation for both donors is shown in FIGS. 18A (as normalized to K562-CD19-Nuc-labeled cells at t=0) or in FIG. 18B (% cell killing compared to vehicle only control (set at 100%)). As shown in FIG. 18A and 18B, incubation with lenalidomide was observed to result in a decrease in anti-CD19 CAR+ T cell cytolytic activity in this assay, under the tested stimulation conditions.

### Example 9 Generation of BCMA conjugated beads

B cell maturation antigen (BCMA) was conjugated to beads by covalently coupling a BCMA-Fc fusion polypeptide, containing soluble human BCMA fused at its C-terminus to an Fc region of IgG, to the surface of commercially available tosyl-activated magnetic beads (ThermoFisher, Waltham MA). The beads are superparamagnetic, non-porous, monodisperse, tosylactivated beads that covalently bind primary amino and sulfhydryl groups.. Conjugation was performed using beads having a diameter of approximately 2.8 µm (designated M-280) or 4.5 µm (designated M-450).

The BCMA-Fc (SEQ ID NO: 22) contained the extracellular domain of human BCMA (GenBank No. NP_001183.2) and a human IgG1 Fc connected with a linker as follows:
MLQMAGQCSQNEYFDSLLHACIPCQLRCSSNTPPLTCQRYCNASVTNSVKGTNA (extracellular domain of BCMA; SEQ ID NO: 18)
GGGGS (linker; SEQ ID NO: 19)

PKSSDKTHTCPPCPAPEAEGAPSVFLFPPKPKDTLMISRTPEVTCVVVDVSHEDPEVK
FNWYVDGVEVHNAKTKPREEQYNSTYRVVSVLTVLHQDWLNGKEYKCKVSNKAL
PSSIEKTISKAKGQPREPQVYTLPPSRDELTKNQVSLTCLVKGFYPSDIAVEWESNGQP
ENNYKTTPPVLDSDGSFFLYSKLTVDKSRWQQGNVFSCSVMHEALHNHYTQKSLSL
SPGK(Hum IgG1 Fc; SEQ ID NO: 20.

The clone encoding the human BCMA-Fc fusion construct with the N-terminal CD33 leader sequence (SEQ ID NO: 21) was inserted into an expression vector and expressed in HEK 293 cells. The resulting BCMA-Fc fusion protein was determined to have a purity of greater than 95% as assessed by gel permeation chromatography. To test binding, the BCMA-Fc fusion protein was incubated with T cells expressing anti-BCMA CARs and T cells expressing CARs that do not bind to BCMA. Results from flow cytometry indicated that the BCMA-Fc fusion protein specifically bound to anti-BCMA CAR expressing T cells.

Various concentrations of the BCMA-Fc fusion protein ranging from 5 µg to 200 µg were added to approximately 1 mL of tocylactivated beads (e.g. containing about 4×10⁹ tocylactivated beads having a diameter of 2.8 µm or about 4×10⁸ tocylactivated beads having a diameter of 4.5 µm). Covalent coupling was performed by overnight incubation at 37°C in phosphate buffered solution (PBS) containing 0.1% human serum albumin (HSA). Beads were washed and resuspended in 1 mL PBS with 0.1% HSA. After conjugation, the bead concentration was determined using a Cellometer. In the examples below, the BCMA-conjugated beads used in various studies are referred to with reference either to the amount of BCMA-Fc antigen added per mL or the antigen concentration (µg/mL) during the conjugation, e.g. 5 µg or 5 µg/mL; 50 µg or 50 µg/mL; 200 µg or 200 µg/mL and so on.

### Example 10 Assessment of T cell Markers on anti-BCMA CAR+ T cell Stimulated with BCMA-Conjugated Beads in the Presence or Absence of Lenalidomide

BCMA-conjugated beads (diameter of 4.5 µm) conjugated with various amounts of BCMA antigen as described in Example 9 were incubated with anti-BCMA CAR+ T cells in the presence or absence of lenalidomide, and the expression of T cell markers were assessed.

Approximately 1.5 × 10⁶ CAR+ T cells were added to wells of a 12-well plate and were incubated with beads from a 200 µg/ml BCMA-conjugated bead composition at a ratio of CAR+ T cell to BCMA-conjugated bead of 1:0.3, 1:1 or 1:3 (approximately 0.5×10⁶, 1.5. 10⁶, and 4.5×10⁶ beads per well, respectively). As controls, 5 µg/mL anti-CD3 antibody was coated to wells (sub-optimal concentration for stimulation) or cells were seeded in the absence of any agent (no stimulation control). Each condition was incubated in the presence or absence of 5 µM lenalidomide. The cells were incubated for four days and then analyzed by flow cytometry for surface expression of CD4, CD8, Tim3, PD-1, CD25 and CD69.

As shown in FIG. 19A, _{the presence of 5 µM} lenalidomide increased the proliferative capacity of T cells (observed by decrease in intensity of CTV dye) following incubation for three days with beads conjugated with 200 µg BCMA antigen at a ratio of 1:1 T cells to beads compared to incubation with the beads in the absence lenalidomide (vehicle control). As shown in FIGS. 19B and19C, the presence of lenalidomide during the incubation further increased the extent of surface expression of CD25 in CD4+ and CD8+ T cells induced after incubation of anti-BCMA CAR+ T cells with BCMA-conjugated beads (FIG. 19B) or anti-CD3 stimulation (FIG. 19C).

In a further experiment, anti-BCMA CAR-T cell compositions produced substantially as described in Example 1 were plated in 96-well plates at a density of 5 × 10⁵ cells per well. The tested CAR-T cell compositions contained, on average, approximately 45% anti-BCMA CAR+ cells at plating. Cells from each composition were incubated for 18 hours in the presence of beads from a 5 µg/ml, 50 µg/ml, or 200 µg/ml BCMA-conjugated bead composition at a ratio of 1:1 T cells to beads. As a control, cells were incubated with anti-CD3/anti-CD28 antibody-conjugated beads (positive control) or no added agent (negative control). The incubations were carried out in the absence of lenalidomide or in the presence of 0.5 µM or 5 µM lenalidomide. Following the incubation, the cells were treated with reagents that allowed for extracellular and intracellular antibody staining by flow cytometry for the transcription factors Blimp1, EOMES, GATA-3, ikaros, helios, and Tbet and markers CD25, CD31, and PD-1

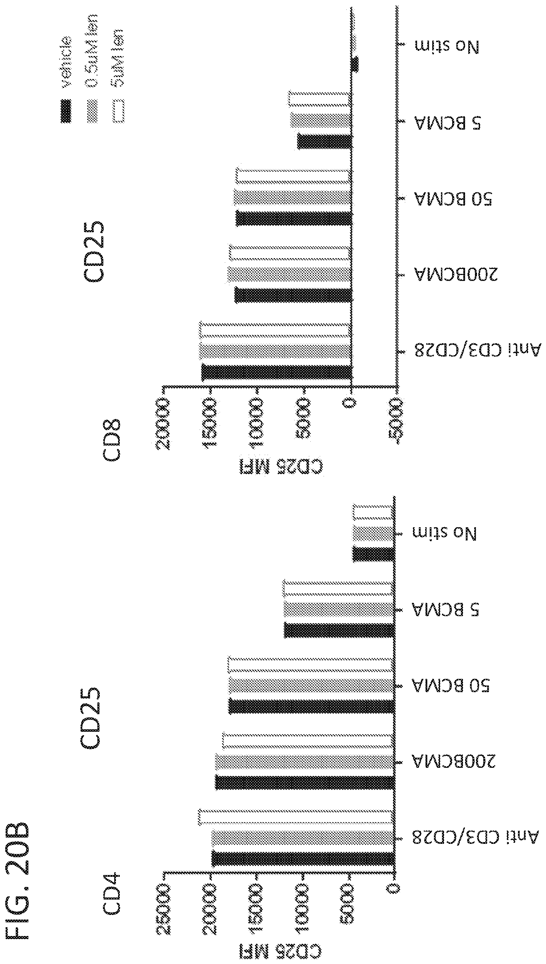

Levels of markers after the incubation of a CAR+ T cell composition from one exemplary donor are shown for BLIMP-1 (FIG. 20A), CD25 (FIG. 20B), CD31 (FIG. 20C), PD-1 (FIG. 20D), Tbet (FIG. 20E), and EOMES (FIG. 20F), GATA-3 (FIG. 20G) Helios (FIG. 20H), and Ikaros (FIG. 20I). As shown, expression of a number of the assessed T effector cellassociated transcription factors and activation markers were increased following stimulation with the BCMA-conjugated beads. For many of the assessed markers, the extent of increased expression was similar to the expression induced by stimulation with anti-CD3/anti-CD28 beads. In some cases, the degree of stimulation with BCMA-conjugated beads was greatest in the presence of 5 µg beads. As shown in FIG. 20I, the expression level of Ikaros was decreased in the presence of lenalidomide in all conditions. Similar results were observed from a CAR+ T cell composition generated from a second donor, except that no change in Helios expression was observed from cells from this donor when stimulated under the tested conditions.

### Example 11 Assessment of Activity of anti-BCMA CAR T cells stimulated with BCMA-conjugated beads in the Presence or Absence of Lenalidomide

### A. Effector Responses

Cryofrozen anti-BCMA CAR T cells, produced substantially as described in Example 2 and formulated at a 1:1 ratio of CD4+ and CD8+ T cells, were thawed. Unless otherwise indicated, beads (diameter about 4.5 µm from a 5 µg/ml or 50 µg/ml BCMA-conjugated bead composition, generated as described in Example 9, were added to the wells at a ratio of T cells to beads of 1:1 in the presence or absence of 5 µM lenalidomide. Cells were incubated up to 14 days and analyzed at various time points for cytokine secretion, cell expansion by flow cytometry for the EGFRt surrogate marker, and for cytolytic activity.

### a. Cytokine Expression

### (i) Presence of cytokines in supernatant

Twenty four hours after addition of BCMA-conjugated beads, the presence of TNF-α, IFNγ, and IL-2 in culture supernatants was assessed. As shown in FIGS. 21A-21C, incubation with BCMA-conjugated beads induced the secretion of IFNγ (FIG. 21A), IL-2 (FIG. 21B), and TNF-α (FIG. 21C) into culture supernatants. The degree of cytokine production was greater when the cells were incubated with beads from the 50 µg/mL BCMA-conjugated bead composition compared to the 5 µg/mL BCMA-conjugated bead composition, demonstrating that CAR stimulation via BCMA beads was dose-dependent. As shown, lenalidomide increased BCMA-induced CAR+ T cell cytokine production following stimulation with the BCMA-conjugated beads.

In a further exemplary study, two different anti-BCMA CAR T cell compositions were generated from different donors, each containing T cells expressing the same anti-BCMA CAR. The cells were thawed and incubated with beads (diameter about 4.5 µm) from a 5 µg/mL or 200 µg/mL BCMA-conjugated bead composition generated as described in Example 1. The incubation was carried out at a ratio of T cells to beads of 1:1 in the presence or absence of 1 µM or 5 µM lenalidomide. Twenty four hours after addition of BCMA-conjugated beads, IL-2 production by the anti-BCMA CAR+ T cells was assessed in culture supernatants. As shown in FIG. 21D, higher production of IL-2 was observed in the presence of high antigen stimulation (200 µg/mL BCMA-conjugated beads) compared to lower antigen stimulation (5 µg/mL BCMA-conjugated beads). Lenalidomide, at either 1 µM or 5 µM, increased cytokine production in the presence of both the high and low antigen stimulation.

### (ii) Intracellular cytokine levels

Anti-BCMA CAR+ T cells were incubated in the presence of 1 µM lenalidomide or a vehicle and 50 µg/mL BCMA-Fc conjugated beads for 2 hours, and cells were assessed by flow cytometry for phosphorylated STAT 5. To assess IFNγ and TNFα cytokine levels, anti-BCMA CAR+ T cells were incubated in the presence of 0.1 µM or 1 µM lenalidomide or a vehicle and 5 µg/mL, 50 µg/mL or 200 µg/mL BCMA-Fc conjugated beads for 24 hours. The cells were gated on transduced, live CD3+ cells, and assessed by flow cytometry for intracellular cytokine accumulation of IFNγ and TNFα in CD4+ and CD8+ cells.

As shown in FIG. 22A, a 2-hour stimulation with antigen increased the percent of cells positive for phosphorylated-STATS compared to the no stimulation control (shown with the dotted line). Results for intracellular cytokine levels of IFNγ and TNFα from anti-BCMA CAR T cells generated from a representative normal CAR-T cell donor are shown in FIG 22B. In this study, anti-BCMA CAR-T cell cytokine production was increased by lenalidomide across a wide range of antigen levels and concentrations.

### b. Cell Proliferation

Total cell count, monitored at day 4 (FIG. 21E) and day 7 (FIG. 21F), was increased following stimulation of anti-BCMA CAR+ T cells with beads from a 50 µg/mL BCMA-conjugated bead composition, but not 5 µg/mL BCMA-conjugated bead composition, compared to the cells present at the time of initiation of the incubation (dashed line). A small increase in proliferation was observed in cells incubated with 50ug beads in the presence of lenalidomide at day 7.

To further assess proliferation, the cells containing anti-BCMA CAR-expressing T cells were labeled with the proliferation marker dye CELL TRACE VIOLET (CTV; ThermoFisher Scientific, Waltham MA) in accordance with the manufacturer's protocol prior to incubation with the BCMA-conjugated beads. Proliferation was assessed by dye dilution using flow cytometry on cells that were stimulated with beads from the 50 µg/mL BCMA-conjugated bead composition. Compared to proliferation in the absence of lenalidomide, there was a slight delay in proliferation as assessed by CTV dilution in the presence of lenalidomide at day 4 but not day 7 (FIG. 21G).

### c. Expansion

Four days and seven days after addition of BCMA-conjugated beads, the incubated cells were stained with CD4 or CD8 and with an anti-EGFR antibody to determine the percentage of cells positive for EGFRt as a surrogate for CAR+ T cells. At the time of plating, 26% of the CD4+ cells expressed anti-BCMA CAR and 39% of the CD8+ cells expressed anti-BCMA CAR as determined by staining with BCMA-Fc. The percent of EGFRt+CD4+ T cells increased from about 26% at the initiation of the incubation to greater than 40% by day 4 (FIG. 21H) and greater than 60% by day 7 (FIG. 21I) when the cells were incubated in the presence of beads from a 50 µg/mL BCMA-conjugated bead composition. As shown in FIG. 21I, the percent of EGFRt+CD8+ T cells increased by day 7 from about 38% at the initiation of the incubation to greater than 60% when the cells were incubated in the presence of beads from the 50 µg/mL BCMA-conjugated bead composition. The extent of cell expansion was greatest when cells were incubated in the presence of beads from a 50 µg/mL BCMA-conjugated bead composition compared to beads from the 5 µg/mL BCMA-conjugated bead composition. The presence of lenalidomide did not substantially impact the extent of CAR+ T cell expansion in this study.

### d. Cytolytic Activity

Cytolytic activity of CAR+ T cells after incubation with BCMA-conjugated beads was assessed by incubation with the BCMA-expressing target cell line RPMI-8226, which is a BCMA+ multiple myeloma cell line. After seven days of incubation of anti-BCMA CAR+ T cells with BCMA-conjugated beads (5 µg/ml or 50 µg/ml) in the presence or absence of lenalidomide, the beads were removed from the cultures and the cells were plated with the RPMI-8226 target cells at a ratio of effector cells to target cells of 3:1 or 1:1 in the further presence or absence of 5 µM lenalidomide. To perform the cytolytic assay, the target RPMI-8226 cells were labeled with NucLight Red (NLR) to permit tracking of target cells by microscopy. Cytolytic activity was assessed by measuring the loss of viable target cells over a period of four days, as determined by red fluorescent signal (using the INCUCYTE^{®} Live Cell Analysis System, Essen Bioscience). The number of viable cells was normalized to cells at day 0 prior to incubation with the RPMI-8226 target cells.

Exemplary results at the 1:1 effector to target cell ratio are shown in FIG. 21J. As shown, the anti-BCMA CAR+ T cells demonstrated effective killing in the assay. Anti-BCMA CAR+ T cells that were stimulated with beads from the 5 µg/ml BCMA-conjugated bead composition were slightly less efficient at cell killing than anti-BCMA CAR+ T cells that were stimulated with beads from the 5 µg/ml BCMA-conjugated bead composition. For all conditions, preincubation with lenalidomide during the seven day incubation prior to the killing assay increased cytolytic activity of the CAR+ T cells. The presence of lenalidomide during the cell killing assay did not substantially affect killing activity. No cell killing was observed when RPMI 8226 cells were cultured alone or in the presence of lenalidomide, demonstrating that lenalidomide did not directly influence target cell viability in this assay.

### B. Serial Restimulation

Anti-BCMA CAR T cell compositions were generated from three different donors, each containing T cells expressing the same anti-BCMA CAR, thawed, and were incubated for seven days with beads (diameter about 4.5 µm) at a 1:1 ratio of beads to cells from a 50 µg/mL BCMA-conjugated bead composition generated as described in Example 9. The incubation was carried out in the presence of 5 µM lenalidomide or in the absence of lenalidomide (vehicle control). Cells were harvested after 7 days and replated for three further rounds up to 28 days, each round involving resetting to initial seeding density and incubating for an additional 7 days in the presence of the same concentration of lenalidomide.

At each reset after the pretreatment, cytolytic activity was assessed by incubation with the BCMA-expressing target cell line RPMI-8226 (labeled with NucLight Red (NLR)) at an effector to target cell (E:T) ratio of 1:1 in the further presence of or absence of lenalidomide. Cytolytic activity was assessed by measuring the loss of viable target cells over a period of up to 80-150 hours, as determined by red fluorescent signal (using the IncuCyte^{®} Live Cell Analysis System, Essen Bioscience). Cells from each condition were plated in triplicate. The % cell killing compared to vehicle only control (set at 100%) was determined.

FIG. 23A shows results for cytolytic activity of anti-BCMA CAR+ T cells from an exemplary donor after pretreatment for 7 days, 14 days or 21 days. As shown, anti-BCMA CAR+ T cells that were preincubated with lenalidomide for 7 days or 14 days exhibited greater cytolytic activity compared to cells that were not preincubated in the presence of lenalidomide. In this donor, an overall decrease in killing efficacy was observed by anti-BCMA CAR+ T cells that were preincubated with lenalidomide for 14 or 21 days compared to day 7. Similar effects on cytolytic activity of anti-BCMA CAR+ T cells after pretreatment with lenalidomide for 7 or 14 days were observed in the donor; cytolytic activity after 21 days lenalidomide pretreatment was not assessed in this donor. As shown in FIG. 23B, increased killing efficacy of anti-BCMA CAR+ T cells was observed in this donor in cells that were pre-incubated with lenalidomide at all time points.

### Example 12 Effects of Lenalidomide on PD-1 expression and PD-L1 signaling

Anti-BCMA CAR-T cells, generated from samples from representative healthy donors or multiple myeloma patient derived material, and cultured with 50 µg/mL BCMA-Fc conjugated beads (generated as described in Example 9) at a ratio of 1:1 bead:CAR+ T cell for 7 days, in the presence of 1 of 1 µM lenalidomide or a vehicle control. Expression of CD25, PD-1, Tim3 and Lag3 on CAR T cells (using an antibody for surrogate CAR marker) cultured under the different conditions then was assessed by flow cytometry.

Such anti-BCMA CAR- T cells prestimulated with beads in the presence or absence of lenalidomide, or freshly thawed anti-BCMA CAR-T cells generated from comparable donor samples, were then debeaded, washed, and cultured with RPMI-8226 target cells (labeled with NucLight Red (NLR) to permit their tracking by microscopy), in the presence of 1 µM lenalidomide or a vehicle control. Specifically, for pretreated cells in which pretreatment had been conducted in the presence of lenalidomide, the cells were cultured with the target cells in the presence of lenalidomide; likewise, for pretreated cells in which pretreatment had been conducted in the presence of vehicle, cells were cultured with the target cells in the presence of vehicle. Following the co-culture, cytolytic activity was assessed by measuring the loss of viable target cells over a period of seven days, as determined by red fluorescent signal. Percentage killing was normalized to anti-BCMA CAR T cells prestimulated on beads in the presence of vehicle. Cytokine production was assessed by ELISA from supernatant following culture with target cells for 24 hours. Experiments were performed twice in 3 donors. Linear fixed-effect or mixed-effect models were used to assess the significance of lenalidomide treatments on cytolytic activity and cytokine production, with treatment, donor, and time treated as fixed effects and animal treated as a random effect, nested with time when repeated measurements were derived from the same animal. *P* values were obtained by likelihood ratio tests comparing the full model with the effect of interest against the model without the effect of interest.

FIG. 24A shows results for CAR antigen-specific cytolytic activity and FIG. 24B shows results for cytokine production for anti-BCMA CAR-T cells that had been prestimulated with BCMA beads (compared to freshly-thawed (non-prestimulated) anti-BCMA CAR-T cells) in the co-cultures, comparing cells cultured in the presence versus absence of lenalidomide. Prestimulated CAR T cells showed decreased cytolytic activity (*P* = 2.1 × 10⁻⁴) and cytokine production (*P* = .03 for IFN-γ) compared with freshly thawed anti-BCMA CAR T cells. In the absence of lenalidomide in pretreatment and subsequent co-culture, the prestimulated CAR-T cells exhibited reduced cell killing and cytokine production compared to fresh CAR-T cells, indicating that chronic prestimulation leads to functional impairment. These results are consistent with an exhaustion-like phenotype having been induced by prestimulation on the BCMA-conjugated beads. The presence of lenalidomide during the prestimulation period preserved cytolytic function (*P* = .04), and there was a trend toward increased cytokine production compared with cells exposed to vehicle during the prestimulation period (FIG. 24B). The presence of lenalidomide in this assay was consistent with an observation that lenalidomide may reduce the effects indicative of functional exhaustion-like phenotype in the prestimulated CAR-T cells.

As shown in FIG. 24C, the phenotype of anti-BCMA CAR T cells stimulated for 7 days on BCMA beads was assessed, and the addition of lenalidomide significantly increased CAR+ viability of anti-BCMA CAR T material across 3 healthy donors (P = 0.04). The addition of lenalidomide did not alter the total cell count across all donors in this 7-day period, and no significant differences were observed in percentage CAR+ between vehicle- and lenalidomidetreated CAR T cells. FIG. 24D shows representative results of flow cytometric analysis of surface CD25 and PD-1 expression (mean fluorescent intensity (MFI), for CD4+ or CD8+ anti-BCMA CAR T-cells after stimulation (pretreatment) with BCMA beads for 7 days, in the presence or absence of 1 µM lenalidomide. As shown, the results indicated that lenalidomide reduced PD-1 expression of BCMA-CAR-T cells, while increasing CD25 expression after prolonged stimulation. As shown in FIG. 24E, flow cytometric analysis across the three CAR T donors indicated that the addition of lenalidomide increased the surface expression ofTim3 in the CD8+ population (P = 4.0 × 10-4), with mixed effects on the CD4+ CAR+ population. Across all donors and in both the CD4+ and CD8+ CAR+ populations, lenalidomide increased CD25 (CD4+ and CD8+; P = 2.2 × 10-16) and the percentage positive for Lag3 expression (CD8+ P < 0.03; CD4+ P = 0.002). Notably, a decrease in the percentage of PD-1+ cells was also observed in the CD4+ population (P = 0.04), with 2 of 3 donors showing a decrease in the CD8+ population as well.

In another study, recombinant human BCMA-conjugated beads were used to stimulate CAR T cells at various concentratons to titrate the magnitude of stimulation, either low (5 µg/ mL), medium (50 µg/ mL), and high (200 µg/ mL) stimulation. At a medium stimulation condition, the secreted cytokine production 24 hours after stimulation was measured, and a 200% increase in IL-2 and TNF-α concentrations were observed compared with vehicle control, with donor-dependent increases in IFN-γ (FIG. 25A). Cells were stimulated with BCMA conjugated beads for 24 hours in the presence of 0.1 µM or 1.0 µM lenalidomide, or vehicle control. A protein transport inhibitor was added in the final hours of incubation, and cells were stained for intracellular IL-2, IFN-γ, and TNF-α.

Anti-BCMA CAR T cells activated on BCMA beads showed stimulation leveldependent effects on cytokine production, with 5-µg BCMA beads causing limited CAR T effector cytokine production compared with 50-µg and 200-µg BCMA beads (FIG. 25B). Lenalidomide increased the percentage of IFN-γ⁺ and TNF-α⁺ intracellular staining at all stimulation levels for both CD4⁺ and CD8⁺ CAR T cells.The magnitude of stimulation either increased or decreased IL-2 in response to lenalidomide, with the lenalidomide decreasing the percentage of IL-2⁺ CAR⁺ T cells at 50-µg and 200-µg stimulation but increasing the percentage of IL-2⁺ CAR⁺ T cells at the 5-µg stimulation condition. In the absence of stimulation, lenalidomide had no effect on CAR T cytokine production, indicating that cytokine enhancement provided by lenalidomide requires stimulation.

In another study, to explore whether the lenalidomide-induced potentiation of CAR T activation and cytokine production could override PD-L1-mediated inhibition, cells were cultured in the presence of BCMA beads generated as described in Example 9, with or without additional conjugation of human recombinant PD-L1-Fc. Healthy donor-or patient-derived CAR T cells were stimulated in the presence of BCMA-conjugated beads or BCMA/PD-L1 conjugated beads for 24 hours in the presence of 1 µM lenalidomide. Cytokine production was measured in the supernatant. Results are shown in FIG. 25C. As shown in FIG. 25C, evaluation of both healthy and patient donor CAR T cells demonstrated that addition of recombinant PD-L1 to recombinant BCMA beads reduced IFN-γ, IL-2, and TNF-α. It was shown that lenalidomide treatment potentiated secreted cytokine levels beyond those from CAR T cells treated with vehicle in the presence of PD-L1. The results were consistent with a conclusion that anti-BCMA CAR-T cytokine production following incubation with BCMA-conjugated beads was increased by lenalidomide in the presence of PD-L1-mediated inhibition.

### Example 13 Gene Expression and Chromatin Accessibility Analysis in CAR T Cells in the Presence or Absence of Lenalidomide

Gene expression and chromatin accessibility was assessed in CAR T cells upon stimulation, in the presence or absence of lenalidomide. Anti-BCMA CAR-expressing T cells, generated from four (4) different independent donors, were stimulated with 50 µg/mL BCMA-conjugated beads for 24 hours (24 hr + stim) or 7 days (d7 + stim), or cultured without stimulation for 24 hours (24 hr), in the presence or absence of lenalidomide (1 µM). Experiments were performed twice in 3 to 4 donors. The CAR-expressing cells were assessed by RNA sequencing (RNA-seq) for gene expression and assayed for transposase-accessible chromatin using sequencing (ATAC-seq) for chromatin accessibility analysis. Assays were performed on 50,000 cells at each time point.

RNA-seq was performed on the complementary DNA (cDNA) samples prepared from the RNA isolated from the cultured anti-BCMA CAR-expressing cells. ATAC-seq was performed generally as described in Buenrostro et al., Nat Methods. (2013) 10(12): 1213-1218. Paired-end ATAC reads were trimmed, aligned with Bowtie2, and filtered for quality, fragment length, duplication, and mitochondrial contribution. ATAC-seq accessibility peaks were called using MACS2 (q<0.01) and a consensus set was generated from overlapping peaks present in 2 or more samples, using DiffBind. Principal component analysis (PCA) was performed for the RNA-seq and ATAC-seq data sets, generated from DESeq2-normalized counts. Differential expression (DE, for RNA-seq) or consensus peak accessibility (DA, for ATAC-seq) were calculated, modeling donor effects (Donors 1-4) and treatment effects (lenalidomide vs. vehicle) at 24 hours and day 7. Differential locus selection cut off was q≤0.05 and log2 fold change ≥ 0.5 for RNA-seq or q≤0.1 for ATAC-seq. Gene ontology (GO) enrichment analysis was performed and activation z-score was determined on the subset of genes differentially expressed at q<0.1 using Ingenuity Pathway Analysis software (Qiagen, Inc.), accounting for donor effects within each treatment condition. A motif enrichment analysis was performed for peaks that were shown to be more accessible in the presence of lenalidomide, with HOMER software, using the consensus peakset as background, for the day 7 stimulation (d7 + stim) ATAC-seq data.

RNA-seq heatmaps were generated on normalized (transcripts per million) expression data, averaged across donors per condition, and row-normalized using z-scores. Motif enrichment in DA peaks at day 7 was performed with HOMER, using the consensus peak set as background.

Results of PCA, representing the overall diversity across gene expression or chromatin accessibility on the genome, are shown in **FIG. 26A** (gene expression; based on RNA-seq results) and **FIG. 26B** (chromatin accessibility; based on ATAC-seq results). Ellipses were drawn to indicate the groups as it was observed that the major factors that contributed to the variation in gene expression or chromatin accessibility were culture time and presence of stimulation. Cells cultured in the presence of lenalidomide (circles) exhibited different overall gene expression and chromatin accessibility compared to cells cultured in the absence of lenalidomide (triangles, vehicle), showing a lenalidomide treatment effect in each donor and culture condition. For lenalidomide treatment, the general direction of change (shown by dotted line between triangle and circle) was similar in each donor, and the degree of change was generally greater in cells cultured for 7 days with stimulation, compared to the change in cells cultured for 24 hours, with or without stimulation. Thus, the PCA demonstrated clustering based on stimulation (stim or no stim) and time (24 hour or 7 days) for both the RNA-seq (Figure 26A) and ATAC-seq (Figure 26B) data sets.

The role of lenalidomide after 24 hours or 7 days of stimulation after accounting for donor-to-donor variability was then examined. **FIGS. 27A-27D** show changes in gene expression (**FIGS. 27A** and **27B****,** following 24 hour and 7 day cultures with stimulation, respectively) or chromatin accessibility (**FIGS. 27C** and **27D****,** following 24 hour and 7 day cultures with stimulation, respectively) in the presence of lenalidomide. RNA-seq analysis showed upregulation of a small set of genes (214) at 24 hours, and a larger number of genes (583) changed after 7 days of stimulation in the presence of lenalidomide (Figures 27A and 27B). ATAC-seq analysis revealed a limited set of chromatin accessibility changes associated with lenalidomide treatment after 24 hours of stimulation, with a dramatic change in profile and an increase in the number of sites with changes in chromatin accessibility (change in chromatin accessibility at 2804 peaks) after 7 days of stimulation in the presence of lenalidomide (Figures 27C and 27D). These results indicated that lenalidomide treatment altered both the transcriptional and epigenetic profile of CAR-T cells.

To further identify specific transcriptional changes associated with lenalidomide treatment, gene ontology analysis was applied to the RNA-seq data set, and biological signaling pathways that were enriched in differentially expressed genes (**FIGS. 28A** and **28B****)** were identified. Directionality and significance of the effects on biological pathways are shown at 24 hours **(****FIG. 28A****)** or 7 days **(****FIG. 28B****).** The results showed that the presence of lenalidomide resulted in increased expression of genes involved in T cell activation and signaling. Results showed that pathways differentially regulated in the presence and absence of lenalidomide showed an enrichment of immune synapse-associated genes, genes involved in cytokine signaling and genes involved in T cell activation pathways. Specifically, pathways associated with T-cell chemotaxis (leukocyte extravasation, integrin, ILK, and CXCR4-associated gene sets), intracellular signaling, and cytoskeleton (Rac/Rho/Cdc42) were upregulated in the presence of lenalidomide within 24 hours of stimulation compared with vehicle controls. In addition, these data support an increase in ICOS-related signaling pathways-a finding that is in line with previous publications demonstrating an increase in ICOS and ICOSL in the CD3⁺ population of peripheral blood mononuclear cells treated with lenalidomide ex vivo (Gorgun et al. (2010) Blood, 116:3227-3237). After 7 days of stimulation, lenalidomide upregulated pathways associated with Th1 T-cell response and co-stimulation, while decreasing Th2-associated gene signatures.

For a selected subset of genes, including genes involved in T cell activation and signaling, the gene expression and chromatin accessibility changes in the presence of lenalidomide were compared for the cells cultured for 7 days with stimulation to determine whether chromatin accessibility correlated with transcription. **FIG. 29** shows individual chromatin accessibility peaks (diamond) and the mean chromatin accessibility change for each gene (circle) plotted against the corresponding gene expression changes measured by RNA-seq showing concordance of signal between the two methods. Across donors, a significant increase in chromatin accessibility was observed across multiple loci associated with IFN-γ and IL-2RA (CD25), and these changes were correlated with a significant increase in transcription. Importantly, the upregulation of IFN-γ and CD25 supported previous findings from chronic stimulation experiments. Additionally, a decrease in CD69 and CCR7 chromatin accessibility and gene transcription on lenalidomide treatment was also observed.

The ATAC-seq data set for motif enrichment was analyzed, and results of the motif enrichment analysis for peaks with increased accessibility in the presence of lenalidomide in day 7 cultures are shown in **FIG. 30****.** Motifs predicted to bind various transcription factors, understood to be involved in T cell activation and signaling, including AP-1/Jun and nuclear factor κB, were enriched in peaks with increased accessibility in the presence of lenalidomide. The results were consistent with an increase in functional activity in the CAR-expressing T cells in the presence of lenalidomide.

Without wishing to be bound by theory, the RNA- and ATAC-seq studies resulted in a number of insights into possible mechanisms for lenalidomide-induced increases in CAR T function. First, the number of transcriptional and chromatin accessibility changes associated with stimulation and time were predominant compared with the effects of lenalidomide, indicating a relatively subtle effect of lenalidomide on transcriptional networks. Second, the changes associated with lenalidomide were broad, including early changes in transcripts associated with cytoskeletal remodeling and chemotaxis. After chronic stimulation, a distinct transcriptional signature emerged that included a decrease in transcripts associated with the Th2 response, G2/M checkpoint, and ATM along with an increase in Th1, peroxisome proliferatoractivated receptor γ, and actin cytoskeleton-associated genes. These effects may support a role for lenalidomide treatment and cell-cycle control and T-cell activation. Previous studies have also demonstrated the effects of IMiDs on Th1- and Th2-associated signatures as well as changes in elements associated with cytoskeletal remodeling and T-cell migration. The demonstrated early alterations in cytokine production by lenalidomide may contribute to an altered T-cell state that is able to enhance aspects of both memory and effector function simultaneously. Overall, these results suggest that additional factors beyond those previously reported are involved in the lenalidomide-induced prolongation of CAR T function, including possible changes in cell-cycle control.

The application of ATAC-seq provided further insights into potential mechanisms of action of lenalidomide. Although both stimulation and time were the predominant drivers of chromatin accessibility changes, lenalidomide treatment was associated with increases in chromatin accessibility in loci enriched in motifs associated with T-cell activation and function after chronic stimulation. These epigenetic changes were coincident with the marked functional changes in CAR T cells incubated with lenalidomide. Alterations in chromatin accessibility signatures have been associated with T-cell exhaustion and may be a more robust indicator of exhaustion compared with T-cell surface ligand expression. These data demonstrated that chronic stimulation with lenalidomide resulted in increased chromatin accessibility and gene expression of IL-2 and CD25 and decreased gene expression and chromatin accessibility of CCR7 and CD69. Previous studies suggested that CCR7-expressing cells produced higher levels of IL-2; however, the current studies indicated that the IL-2 pathway could be altered independently by lenalidomide, resulting in an alternative T-cell state. CD69, a marker of T-cell activation, has a nuclear factor κB-responsive element that is required for the CD69 response to TNF-α. The closing of CD69-associated chromatin and decrease in transcripts may be a reaction to sustained increases in TNF-α production by CAR T cells cultured with lenalidomide, or it may be a T-cell response to increased activation in the presence of lenalidomide. Lenalidomide treated cells demonstrated increased transcription factor motif enrichment of T cell activation associated factors, supporting the idea that these cells are exposed to sustained activation signaling. Overall, the lenalidomide-induced CAR T-cell state has elements of both effector T-cell function, including increased IFN-γ and TNF-α production, and memory T-cell function, including increased IL-2 and long-term proliferation.

### Example 14 Assessment of pharmacodynamic response of Ikaros transcription factor in CAR-expressing T cells in the presence of Compound 1 or lenalidomide

T cell compositions containing anti-CD19 CAR-expressing T cells were generated from leukapheresis samples from three healthy human adult donors by a process including immunoaffinity-based selection of T cells (including CD4+ and CD8+ cells) from the samples, resulting in two compositions, enriched for CD8+ and CD4+ cells, respectively. The cells were incubated in the presence of 3-(5-amino-2-methyl-4-oxo-4H-quinazolin-3-yl)-piperidine-2,6-dione (Compound 1) or lenalidomide, and expression of the transcription factor Ikaros was assessed.

Cells of the enriched CD4+ and CD8+ compositions were separately activated with anti-CD3/anti-CD28 beads and subjected to lentiviral transduction with a vector encoding an anti-CD 19 CAR. The anti-CD 19 CAR contained an anti-CD 19 scFv derived from a murine antibody (variable region derived from FMC63), an immunoglobulin-derived spacer, a transmembrane domain derived from CD28, a costimulatory region derived from 4-1BB, and a CD3-zeta intracellular signaling domain. The expression construct in the viral vector further contained sequences encoding a truncated receptor, which served as a surrogate marker for CAR expression, which was separated from the CAR sequence by a T2A ribosome skip sequence. Transduced populations then were separately incubated in the presence of stimulating reagents for cell expansion. Expanded CD8+ and CD4+ cells were formulated and cryopreserved separately and stored. The cyropreserved CD4+ and CD8+ anti-CD 19 CAR-expressing cells from each donor were thawed, and combined at approximately a 1:1 CAR+ CD4+:CD8+ ratio prior to use.

Approximately 2.5× 10⁵ cells (CD4+ and CD8+ T cells combined at a 1:1 ratio) of the generated CAR+ T cell composition were stimulated overnight with a reagent specific to the CAR, and then the cells were incubated with lenalidomide (100 nM - 10,000 nM), Compound 1 (10 nM - 3000 nM) or a vehicle control overnight at 37°C, 5% CO₂. The evaluated concentrations of Compound 1 and lenalidomide encompassed the reported clinical Cₘₐₓ and C_{min.} After incubation, anti-CD19 CAR-expressing T cells were stained with antibodies and analyzed by flow cytometry to assess surface expression of CD4, CD8 and the surrogate marker for CAR expression, and intracellular levels of Ikaros in CD4+CAR+ or CD8+CAR+ cells. Median fluorescence intensity (MFI) values for Ikaros were normalized and calculated as a percentage relative to vehicle control.

As shown in **FIG. 31****,** a concentration-dependent decrease in intracellular Ikaros expression was observed in both CD4+ anti-CD19 CAR-expressing T cells and CD8+ anti-CD19 CAR-expressing T cells after incubation with Compound 1 or lenalidomide. A greater reduction in Ikaros expression was observed in cells in the presence of Compound 1 compared to lenalidomide. The EC50 for reducing Ikaros expression was calculated as determined from the concentration of the inhibitor that reduced Ikaros MFI to 50% of its maximal MFI in the absence of the inhibitor. EC50 values for Compound 1 and lenalidomide are shown in Table E5.

**Table E5. Ikaros EC50 (nM) in CD4+CAR+ T cells and CD8+CAR+ T cells.**

| | **CD4+** | | **CD8+** | |
|---|---|---|---|---|
| | **Lenalidomide** | **Compound 1** | **Lenalidomide** | **Compound 1** |
| Donor 1 | 61.2 | 67 | 80.9 | 100.9 |
| Donor 2 | ND | 41.5 | ND | 60.8 |
| Donor 3 | 169.8 | 99.8 | 235.5 | 161.1 |

| | | | | |
|---|---|---|---|---|
| ND = not determined | | | | |

### Example 15 Evaluation of Functional Effects on CAR-expressing T cells following incubation with target cells in the presence of Compound 1 or lenalidomide

Anti-CD19 CAR-expressing T cell compositions (containing CD4+ and CD8+ cells combined at a 1:1 ratio) were generated substantially as described in Example 14, and were incubated with target K562 cells transduced with human CD19 (K562.CD19) in the presence of 3-(5-amino-2-methyl-4-oxo-4H-quinazolin-3-yl)-piperidine-2,6-dione (Compound 1) (at concentrations of 10 nM, 100 nM, 500 nM, and 1000 nM), lenalidomide (at concentrations of 100 nM, 1000 nM, and 10,000 nM), or a vehicle control at 37°C, 5% CO₂. Cytokine expression, target cell cytolysis and expression of surface markers of anti-CD 19 CAR-expressing T cells were assessed.

### A. Cytokine production

To assess cytokine production, 1x10⁵ anti-CD19 CAR+ cells (CD4+ and CD8+ T cells combined at a 1:1 ratio) were incubated with K562.CD19 target cells at an E:T ratio of 5:1 or 2.5:1 in the presence or absence of lenalidomide or Compound 1 as described above. After 24 hours, supernatants were harvested and analyzed for IFN-γ, IL-2 and TNF-α cytokine production.

As shown in **FIGS. 32A** (Compound 1) and **32B** (lenalidomide), cytokine production was increased in the presence of Compound 1 or lenalidomide, respectively, in a concentration-dependent manner as compared to vehicle control, at both E:T ratios of 5:1 and 2.5:1. There were differences in cytokine levels among the different donors. Compound 1 treatment resulted in greater cytokine production across multiple conditions compared to lenalidomide treatment at equivalent concentrations. The increase was statistically significant as determined from the increase in cytokine production in the presence of 100 nM and 1000 nM Compound 1 compared to the equivalent concentration of lenalidomide at the 2.5:1 and 5:1 E:T ratio as determined using an unpaired parametric t-test with Welch's correction run, see **Table E6** (P-Values at 2.5:1 E:T/P-Values at 5:1 E:T).

**Table E6. Cytokine production of anti-CD19 CAR-expressing T cells treated with Compound 1 or lenalidomide.**

| | Donor 1 | | Donor 2 | | Donor 3 | |
|---|---|---|---|---|---|---|
| **Concentration (nM):** | **100** | **1000** | **100** | **1000** | **100** | **1000** |
| IFN-γ | ^{∗∗∗}/^{∗} | ^{∗}/ns | ns/ns | ns/^{∗∗∗} | ^{∗∗∗}/^{∗∗∗} | ^{∗∗∗}/^{∗∗∗} |
| IL-2 | ^{∗∗}/^{∗} | ^{∗}/^{∗} | ^{∗∗}/ | ns/ns | ns/^{∗} | ns/ns |
| TNF-α | ^{∗∗∗}/^{∗} | ^{∗∗∗}/^{∗∗∗} | ^{∗∗}/ | ^{∗∗∗}/^{∗∗∗} | ^{∗∗}/^{∗} | ^{∗∗}/^{∗∗} |

| | | | | | | |
|---|---|---|---|---|---|---|
| p ≤0.05 : ^{∗}; p ≤0.01: ^{∗∗}; p ≤0.001: ^{∗∗∗}; ns: not significant | | | | | | |

### B. Cytolytic function

To assess cytolytic function, 1×10⁵ or 5×10⁴ anti-CD19 CAR+ cells (CD4+ and CD8+ T cells combined at a 1:1 ratio) were incubated with 2×10⁴ K562.CD19 target cells at an E:T ratio of 5:1 or 2.5:1 in the presence or absence of lenalidomide or Compound 1 or vehicle control as described above. K562.CD19 target cells were transduced with NucLight Red to permit their tracking by microscopy. Cytolytic activity was assessed by measuring the loss of viable target cells over a period of five days, as determined by red fluorescent signal (using the IncuCyte^{®} Live Cell Analysis System, Essen Bioscience). A killing index was determined using the formula: 1/AUC, and the killing index was normalized to CAR+ cells co-cultured with target cells that had been incubated with a vehicle control (set at 100% killing).

As shown in **FIG. 33****,** Compound 1 and lenalidomide generally had a limited effect on cytolytic function of anti-CD19 CAR-expressing T cells. When the CAR was stimulated in the presence of higher antigen, as present in co-cultures containing a 2.5:1 E:T ratio, Compound 1 and lenalidomide slightly reduced cytolytic activity of anti-CD19 CAR-expressing cells for some donors. When the CAR was stimulated in the presence of lesser antigen, as present in co-cultures containing a 5:1 E:T ratio, a slight but consistent increase in cytolytic activity of anti-CD19 CAR-expressing T cells against target cells was observed from cells that had been incubated in the presence of high concentrations of Compound 1 or lenalidomide for Donor 2 while no effects were observed with Donors 1 and 3.

### C. Expression of T cell surface markers

To assess surface expression of various T cell markers, 1x10⁵ K562.CD19 target cells were incubated with anti-CD19 CAR+ cells (CD4+ and CD8+ T cells combined at a 1:1 ratio) at an E:T ratio of 5:1 or 2.5:1 in the presence or absence of lenalidomide or Compound 1 or vehicle control as described above. After 24 hours, CAR-expressing T cells were stained for CD3, CD4, CD8 and the surrogate marker for CAR expression, and also for the following surface markers: CD69, CD107a, PD-1, CD25, CD62L, CCR7, CD45RO, CD27, and LAG3.

Expression levels of select markers on CD4+ CAR-expressing T cells and CD8+ CAR-expressing T cells were altered, generally less than two-fold, relative to vehicle control co-cultures. Changes in marker expression in the presence of lenalidomide or Compound 1 were donor-dependent, although for assessed memory markers expression of CD45RO was increased and CD27 was decreased across all donors and E:T ratios. Expression of CD27 was downregulated in a concentration-dependent manner in response to Compound 1 or lenalidomide. The expression of CD69 and LAG3 were increased in a concentration-dependent manner for cells derived from donor 3 after incubation with Compound 1 but not following incubation of the same donor-derived CAR+ cells with lenalidomide. Expression of the other assessed activation markers remained unchanged in donors treated with lenalidomide or Compound 1. The results are consistent with an observation that Compound 1 and lenalidomide have the potential to intrinsically modulate early activation phenotypes of CAR-expressing T cells.

### Example 16 Evaluation of cytokine production and surface marker expression of CAR-expressing T cells following anti-idiotypic antibody stimulation in the presence of Compound 1 or lenalidomide

Similar studies as described in Example 15 were carried out to assess cytokine production and surface marker expression following CAR-dependent stimulation of CAR-expressing T cells in the presence or 3-(5-amino-2-methyl-4-oxo-4H-quinazolin-3-yl)-piperidine-2,6-dione (Compound 1) or lenalidomide, except that CAR-expressing cells were stimulated with an anti-idiotypic antibody. The anti-idiotypic antibody was used to simulate variable stimulation levels in co-cultures, which is not generally possible with K562.CD19 target cells because the expression of antigen is uniformly high on K562.CD19 cells.

Anti-CD19 CAR-expressing T cells compositions (containing CD4+ and CD8+ T cells combined at a 1:1 ratio) were generated substantially as described in Example 14. Approximately 1x10⁵ CAR-expressing cells were added to wells of a 96-well plate that had been pre-coated with an anti-idiotypic antibody specific to the scFv of the anti-CD19 CAR-expressing T cells at concentrations of 0, 0.3, 3 and 30 µg/ml. The cells were cultured in the presence of Compound 1 (at concentrations of 100 nM and 1000 nM), lenalidomide (at concentrations of 500 nM and 5000 nM), or a vehicle control at 37°C, 5% CO₂. Cytokine expression and expression of surface markers of anti-CD19 CAR-expressing T cells were assessed.

### A. Cytokine production

Supernatants from the stimulated cultures were harvested after 24 hours and analyzed for cytokine production. In **FIGS. 34A** and **34B****,** the level of cytokine production in the absence of Compound 1 or lenalidomide (vehicle control) is indicated by a dashed line. As shown in **FIGS. 34A** and **34B****,** IFN-γ, IL-2 and TNF-α production were increased relative to the vehicle control following treatment with Compound 1 or lenalidomide, respectively. The increase was particularly evident at an intermediate level of stimulation with 3µg/mL anti-idiotypic antibody.

### B. Expression of T cell surface markers

Surface marker expression on anti-CD19 CAR-expressing T cells was assessed after 4 days in culture with various concentration of the anti-idiotypic antibody in the presence of Compound 1 or lenalidomide. CAR-expressing T cells were stained for CD3, CD4, CD8 and the surrogate marker for CAR expression, and also for the following markers: CD25, PD-1, and CD69.

**FIGs. 35A** and **35B** show cell surface marker expression on CD4+ and CD8+ CAR-expressing cells, respectively, on cells stimulated in the presence of Compound 1, and **FIGs. 36A** and **36B** show cell surface marker expression on CD4+ and CD8+ CAR-expressing cells, respectively, on cells stimulated in the presence of lenalidomide. In the figures, the level of surface marker expression in the absence of Compound 1 or lenalidomide (vehicle control) is indicated by the dashed line. As shown, an increase in surface markers CD25 and CD69 was observed in CD4+ and CD8+ CAR-expressing T cells in the presence of Compound 1 or lenalidomide in some donor-derived CAR-expressing cells and depending on the amount of stimulation through the CAR. Expression of PD-1 also was increased in the presence of Compound 1 or lenalidomide in cells generated from at least one donor, although, PD-1 levels were unchanged or decreased in cells generated from the other donors. Increased expression of the surrogate marker for CAR expression was observed following addition of Compound 1 or lenalidomide at a suboptimal dose of the anti-idiotypic antibody of 0.3µg/ml, but at higher concentrations of the anti-idiotypic antibody expression of the surrogate marker was unchanged or decreased.

### Example 17 Evaluation of surface marker expression and expansion potential of CAR-expressing T cells following serial stimulation in the presence of Compound 1 or lenalidomide

Serial stimulation of anti-CD19 CAR-expressing T cells was carried out to assess short-term and long-term effects of 3-(5-amino-2-methyl-4-oxo-4H-quinazolin-3-yl)-piperidine-2,6-dione (Compound 1) and lenalidomide. Anti-CD19 CAR-expressing T cell compositions (containing CD4+ and CD8+ T cells combined at a 1:1 ratio) were generated substantially as described in Example 14. The generated CAR+ T cell combinations were added at 1x10⁵ cells per well to a 96-well plate and incubated with irradiated red fluorescent positive target K562.CD19 cells in the presence of Compound 1, lenalidomide, or a vehicle control at 37°C, 5% CO₂ at two different effector to target (E:T) ratio of 10: 1 of 2.5:1. The incubation was carried out in the presence of Compound 1 (10, 100 or 500 nM), lenalidomide (100 or 1000 nM) or vehicle control. Every 3-4 days (start of each new round), the cells were counted. Cells then were harvested and re-plated at 1x10⁵ anti-CD19 CAR-expressing cells with fresh media, newly added Compound 1 or lenalidomide at the same concentration, and newly-irradiated K562.CD19 target cells. This was repeated for 7 rounds of serial stimulation, and cells were assessed at various times for surface marker expression, cytolytic activity and expansion potential.

### A. Expression of surface markers

Expression of select surface markers was assessed on cells at day 4 (i.e., 4 days after the first stimulation) and day 28 (i.e., 4 days after the seventh stimulation). Specifically, harvested cells were analyzed by flow cytometry for CD3, CD4, CD8 and the surrogate marker for CAR expression, and also for the following surface markers: CD69, CD107a, PD-1, CD25, CD62L, CCR7, CD45RO, CD27, and LAG3.

Changes in assessed surface markers on CD4+ and CD8+ CAR-expressing cells following incubation with Compound 1 and lenalidomide were variously observed across all donors and E:T ratios, although the expression changes were more pronounced at day 28 compared to day 4. At day 4, CD25 and LAG3 were upregulated across all three donors in response to Compound 1 or lenalidomide treatment, with a greater decrease observed on cells from day 28 compared to day 4. CCR7 was generally decreased at day 28 across treated groups, which is consistent with the possibility that incubation with target cells in the presence of Compound 1 or lenalidomide may have driven the T cell product towards a phenotype associated with a terminally differentiated effector stat. PD-1 was downregulated to some degree in all donors and at both E:T ratios on cells at day 4 and day 28 after treatment with Compound 1 or lenalidomide, with a greater downregulation occurring on cells at day 28. As shown in **FIGs. 37A** and **37B****,** expression of CD28 was decreased in a dose-dependent manner in the presence of increasing concentrations of Compound 1 and lenalidomide, respectively, on both CD4+ and CD8+ CAR-expressing T cells from all three donors. Together, the changes in surface markers at day 28 compared to day 4 are consistent with the ability of Compound 1 and lenalidomide to impact CAR+ T cells following long-term treatment.

### B. Cytolytic function

At day 24 after serial stimulation, cytolytic activity of anti-CD19 CAR-expressing T cells was assessed generally as described in Example 15B. As shown in **FIG. 38****,** long-term treatment with Compound 1 and lenalidomide were both able to increase the cytolytic activity of anti-CD19 CAR-expressing T cells.

### C. Expansion

To evaluate the effect of Compound 1 and lenalidomide on expansion potential of CAR+ T cells, the cell numbers of the anti-CD19 CAR-expressing T cells were counted after each round of stimulation and cell doublings were calculated.

As shown in **FIG. 39A****,** at the 2.5:1 E:T ratio, anti-CD19 CAR-expressing T cells treated with Compound 1 at concentrations of 500nM had a comparable cell count as the treated control group until 3-4 rounds of stimulation for all donors. Similar results were observed at the 10:1 E:T ratio for two donors. At the 500 nM higher concentration of Compound 1, the number of doublings of CAR+ cells in subsequent rounds was lower than the untreated control groups. In contrast, after 24 days of treatment with Compound 1 at lower concentrations of 10nM and 100nM, the cell counts of anti-CD19 CAR-expressing T cells were higher than the untreated controls for two out of three donors **(****FIG. 39B****).**

As shown in **FIG. 40A****,** at the 2.5:1 E:T ratio in cells treated with 1000 nM lenalidomide lower cell doublings were only observed in two of the donors and not until later rounds of stimulation. This result indicates some differences in the activity of Compound 1 and lenalidomide, since 500 nM Compound 1 decreased cell counts across all donors at this E:T ratio. At the 10:1 E:T ratio, decreased cell doublings were observed after 3-4 rounds of stimulation in the presence of1000 nM lenalidomide by cells generated from all donors **(FIG.**

**40A).** As shown in **FIG. 40B****,** treatment with lenalidomide at a lower concentration of 100 nM increased CAR+ T cell counts for two out of three donors.

The result are consistent with an observation that prolonged treatment of CAR-expressing T cells with Compound 1 or lenalidomide at physiologically-relevant concentrations can increase long-term proliferation potential of CAR-expressing T cells, while higher concentrations may be detrimental to long term performance.

### Example 18 Evaluation of anti-tumor efficacy of CAR-expressing T cells in combination with Compound 1 in vivo

The anti-tumor efficacy of CAR-expressing T cells in combination with Compound 1 was assessed by monitoring tumors in a tumor xenograft model. Anti-CD19 CAR-expressing T cell compositions, containing CD4+ and CD8+ T cells combined at a 1:1 ratio, were generated substantially as described in Example 1. T cell compositions were generated from three different donors.

NOD.Cg.Prkdc^{scid}IL2rg^{t*m1Wjl*}SzJ (NSG) mice were injected intravenously (i.v.) with 0.5 × 10⁶ Raji lymphoma tumor cells (an immortalized human B lymphocyte tumor cell line that expresses CD19) that were transfected with firefly luciferase (Raji-ffluc). Tumor engraftment was allowed to occur for 6 days and verified using bioluminescence imaging. On Day 7, mice either received no treatment, or a single intravenous (i.v.) injection of anti-CD19 CAR-expressing cells at a low dose (0.5×10⁶ cells) or a high dose (1.0×10⁶ cells). In one study group (designated "Concurrent"), mice were administered 3-(5-amino-2-methyl-4-oxo-4H-quinazolin-3-yl)-piperidine-2,6-dione (Compound 1)at a dose of 0.3 mg/kg or vehicle control via intraperitoneal injection one day prior to administration of the CAR-expressing cells (day 6), which was continued once a day for the study duration. In a second group (designated "Delayed"), mice were administered either a vehicle control or 3-(5-amino-2-methyl-4-oxo-4H-quinazolin-3-yl)-piperidine-2,6-dione (Compound 1) at a dose of 0.3 mg/kg via intraperitoneal injection starting from day 14, which was after the peak of CAR-expressing T cell expansion, and administration was continued once a day for the study duration. Tumor burden was assessed by bioluminescence every 10 days. For bioluminescence imaging, mice received intraperitoneal (i.p.) injections of luciferin substrate (CaliperLife Sciences, Hopkinton, MA) resuspended in PBS (15 µg/g body weight). The average radiance (p/s/cm²/sr) was determined.

In this study, the combination with Compound 1 was observed to reduce tumor burden and improve survival data in both the "Concurrent" group and the "Delayed" group, as compared to administration of the CAR-expressing cells alone.

The present invention is not intended to be limited in scope to the particular disclosed embodiments, which are provided, for example, to illustrate various aspects of the invention. Various modifications to the compositions and methods described will become apparent from the description and teachings herein.

### SEQUENCES

| **SEQ ID NO.** | **SEQUENCE** | **DESCRIPTION** |
|---|---|---|
| **1** | ESKYGPPCPPCP | spacer (IgG4hinge) (aa) |
| | | Homo sapiens |
| **2** | GAATCTAAGTACGGACCGCCCTGCCCCCCTTGCCCT | spacer (IgG4hinge) (nt) |
| | | Homo sapiens |
| **3** | ESKYGPPCPPCPGQPREPQVYTLPPSQEEMTKNQVSLTCLVKGFYPSDIAVEWES NGQPENNYKTTPPVLDSDGSFFLYSRLTVDKSRWQEGNVFSCSVMHEALHNHYTQ KSLSLSLGK | Hinge-CH3 spacer Homo sapiens |
| **4** | ESKYGPPCPPCPAPEFLGGPSVFLFPPKPKDTLMISRTPEVTCVVVDVSQEDPEV QFNWYVDGVEVHNAKTKPREEQFNSTYRVVSVLTVLHQDWLNGKEYKCKVSNKGL PSSIEKTISKAKGQPREPQVYTLPPSQEEMTKNQVSLTCLVKGFYPSDIAVEWES NGQPENNYKTTPPVLDSDGSFFLYSRLTVDKSRWQEGNVFSCSVMHEALHNHYTQ KSLSLSLGK | Hinge-CH2-CH3 spacer |
| | | Homo sapiens |
| **5** | RWPESPKAQASSVPTAQPQAEGSLAKATTAPATTRNTGRGGEEKKKEKEKEEQEE RETKTPECPSHTQPLGVYLLTPAVQDLWLRDKATFTCFVVGSDLKDAHLTWEVAG KVPTGGVEEGLLERHSNGSQSQHSRLTLPRSL WNAGTSVTCTLNHPSLPPQRLMALREPAAQAPVKLSLNLLASSDPPEAASWLLCE VSGFSPPNILLMWLEDQREVNTSGFAPARPPPQPGSTTFWAWSVLRVPAPPSPQP ATYTCVVSHEDSRTLLNASRSLEVSYVTDH | IgD-hinge-Fc |
| | | Homo sapiens |
| **6** | LEGGGEGRGSLLTCGDVEENPGPR | T2A artificial |
| **7** | RKVCNGIGIGEFKDSLSINATNIKHFKNCTSISGDLHILPVAFRGDSFTHTPPLD PQELDILKTVKEITGFLLIQAWPENRTDLHAFENLEIIRGRTKQHGQFSLAVVSL NITSLGLRSLKEISDGDVIISGNKNLCYANTINWKKLFGTSGQKTKIISNRGENS CKATGQVCHALCSPEGCWGPEPRDCVSCRNVSRGRECVDKCNLLEGEPREFVENS ECIQCHPECLPQAMNITCTGRGPDNCIQCAHYIDGPHCVKTCPAGVMGENNTLVW KYADAGHVCHLCHPNCTYGCTGPGLEGCPTNGPKIPSIATGMVGALLLLLVVALG IGLFM | tEGFR artificial |
| **8** | FWVLWVGGVLACYSLLVTVAFI I FWV | CD28 (amino acids 153-179 of Accession No. P 10747) |
| | | Homo sapiens |
| **9** | IEVMYPPPYLDNEKSNGTIIHVKGKHLCPSPLFPGPSKP FWVLWVGGVLACYSLLVTVAFI I FWV | CD28 (amino acids 114-179 of Accession No. P 10747) |
| | | Homo sapiens |
| **10** | RSKRSRLLHSDYMNMTPRRPGPTRKHYQPYAPPRDFAAYRS | CD28 (amino acids 180-220 of P 10747) |
| | | Homo sapiens |
| **11** | RSKRSRGGHSDYMNMTPRRPGPTRKHYQPYAPPRDFAAYRS | CD28 (LL to GG) |
| | | Homo sapiens |
| **12** | KRGRKKLLYIFKQPFMRPVQTTQEEDGCSCRFPEEEEGGCEL | 4-1BB (amino acids 214-255 of Q07011.1) |
| | | Homo sapiens |
| **13** | RVKFSRSADAPAYQQGQNQLYNELNLGRREEYDVLDKRRGRDPEMGGKPRRKNPQ EGLYN ELQKDKMAEA YSEIGMKGER RRGKGHDGLY QGLSTATKDTYDALHMQALP PR | CD3 zeta |
| | | Homo sapiens |
| **14** | RVKFSRSAEPPAYQQGQNQLYNELNLGRREEYDVLDKRRGRDPEMGGKPRRKNPQ EGLYN ELQKDKMAEA YSEIGMKGER RRGKGHDGLY QGLSTATKDTYDALHMQALP PR | CD3 zeta |
| | | Homo sapiens |
| **15** | RVKFSRSADAPAYKQGQNQLYNELNLGRREEYDVLDKRRGRDPEMGGKPRRKNPQ EGLYN ELQKDKMAEA YSEIGMKGER RRGKGHDGLY QGLSTATKDTYDALHMQALP PR | CD3 zeta |
| | | Homo sapiens |
| **16** | PGGG- (SGGGG) 5-P- wherein P is proline, G is glycine and S is serine | linker |
| **17** | GSADDAKKDAAKKDGKS | Linker |
| **18** | MLQMAGQCSQNEYFDSLLHACIPCQLRCSSNTPPLTCQRYCNASVTNSVKGTNA | Extracellular domain of human BCMA (GenBank No. NP_001183.2) |
| **19** | GGGGS | Linker sequence |
| **20** | PKSSDKTHTCPPCPAPEAEGAPSVFLFPPKPKDTLMISRTPEVTCVVVDVSHEDP EVKFNWYVDGVEVHNAKTKPREEQYNSTYRVVSVLTVLHQDWLNGKEYKCKVSNK ALPSSIEKTISKAKGQPREPQVYTLPPSRDELTKNQVSLTCLVKGFYPSDIAVEW ESNGQPENNYKTTPPVLDSDGSFFLYSKLTVDKSRWQQGNVFSCSVMHEALHNHY TQKSLSLSPGK | Modified Human IgG1 Fc |
| **21** | MPLLLLLPLLWAGALA | CD33 Signal peptide |
| **22** | MPLLLLLPLLWAGALAMLQMAGQCSQNEYFDSLLHACIPCQLRCSSNTPPLTCQR YCNASVTNSVKGTNAGGGGSPKSSDKTHTCPPCPAPEAEGAPSVFLFPPKPKDTL MISRTPEVTCVVVDVSHEDPEVKFNWYVDGVEVHNAKTKPREEQYNSTYRVVSVL TVLHQDWLNGKEYKCKVSNKALPSSIEKTISKAKGQPREPQVYTLPPSRDELTKN QVSLTCLVKGFYPSDIAVEWESNGQPENNYKTTPPVLDSDGSFFLYSKLTVDKSR WQQGNVFSCSVMHEALHNHYTQKSLSLSPGK | BCMA-Fc construct |
| **23** | EGRGSLLTCGDVEENPGP | T2A |
| **24** | GSGATNFSLLKQAGDVEENPGP | P2A |
| **25** | ATNFSLLKQAGDVEENPGP | P2A |
| **26** | QCTNYALLKLAGDVESNPGP | E2A |
| **27** | VKQTLNFDLLKLAGDVESNPGP | F2A |
| **28** | RKVCNGIGIGEFKDSLSINATNIKHFKNCTSISGDLHILPVAFRGDSFTHTPPLD PQELDILKTVKEITGFLLIQAWPENRTDLHAFENLEIIRGRTKQHGQFSLAVVSL NITSLGLRSLKEISDGDVIISGNKNLCYANTINWKKLFGTSGQKTKIISNRGENS CKATGQVCHALCSPEGCWGPEPRDCVSCRNVSRGRECVDKCNLLEGEPREFVENS ECIQCHPECLPQAMNITCTGRGPDNCIQCAHYIDGPHCVKTCPAGVMGENNTLVW KYADAGHVCHLCHPNCTYGCTGPGLEGCPTNGPKIPSIATGMVGALLLLLVVALG IGLFM | tEGFR artificial |
| **29** | ESKYGPPCPPCPAPPVAGPSVFLFPPKPKDTLMISRTPEVTCVVVDVSQEDPEVQ FNWYVDGVEVHNAKTKPREEQFQSTYRVVSVLTVLHQDWLNGKEYKCKVSNKGLP SSIEKTISKAKGQPREPQVYTLPPSQEEMTKNQVSLTCLVKGFYPSDIAVEWESN GQPENNYKTTPPVLDSDGSFFLYSRLTVDKSRWQEGNVFSCSVMHEALHNHYTQK SLSLSLGK | Hinge-CH2-CH3 spacer |
| | | Homo sapiens |
| **30** | QIQLVQSGPELKKPGETVKISCKASGYTFTDYSINWVKRAPGKGLKWMGWINTET REPAYAYDFRGRFAFSLETSASTAYLQINNLKYEDTATYFCALDYSYAMDYWGQG TSVTVSS | Variable heavy (VH) Anti-BCMA |
| **31** | DIVLTQSPPSLAMSLGKRATISCRASESVTILGSHLIHWYQQKPGQPPTLLIQLA SNVQTGVPARFSGSGSRTDFTLTIDPVEEDDVAVYYCLQSRTIPRTFGGGTKLEI K | Variable light (VL) Anti-BCMA |
| **32** | QIQLVQSGPDLKKPGETVKLSCKASGYTFTNFGMNWVKQAPGKGFKWMAWINTYT GESYFADDFKGRFAFSVETSATTAYLQINNLKTEDTATYFCARGEIYYGYDGGFA | Variable heavy (VH) Anti-BCMA |
| | YWGQGTLVTVSA | |
| **33** | DVVMTQSHRFMSTSVGDRVSITCRASQDVNTAVSWYQQKPGQSPKLLIFSASYRY TGVPDRFTGSGSGADFTLTISSVQAEDLAVYYCQQHYSTPWTFGGGTKLDIK | Variable light (VL) Anti-BCMA |
| **34** | EVQLVQSGAEVKKPGESLKISCKGSGYSFTSYWIGWVRQMPGKGLEWMGIIYPGD SDTRYSPSFQGHVTISADKSISTAYLQWSSLKASDTAMYYCARYSGSFDNWGQGT LVTVSS | Variable heavy (VH) Anti-BCMA |
| **35** | SYELTQPPSASGTPGQRVTMSCSGTSSNIGSHSVNWYQQLPGTAPKLLIYTNNQR PSGVPDRFSGSKSGTSASLAISGLQSEDEADYYCAAWDGSLNGLVFGGGTKLTVL G | Variable light (VL) Anti-BCMA |
| **36** | EVQLVQSGAEMKKPGASLKLSCKASGYTFIDYYVYWMRQAPGQGLESMGWINPNS GGTNYAQKFQGRVTMTRDTSISTAYMELSRLRSDDTAMYYCARSQRDGYMDYWGQ GTLVTVSS | Variable heavy (VH) Anti-BCMA |
| **37** | QSALTQPASVSASPGQSIAISCTGTSSDVGWYQQHPGKAPKLMIYEDSKRPSGVS NRFSGSKSGNTASLTISGLQAEDEADYYCSSNTRSSTLVFGGGTKLTVLG | Variable light (VL) Anti-BCMA |
| **38** | EVQLVQSGAEVKKPGSSVKVSCKASGGTFSSYAISWVRQAPGQGLEWMGRIIPIL GIANYAQKFQGRVTMTEDTSTDTAYMELSSLRSEDTAVYYCARSGYSKSIVSYMD YWGQGTLVTVSS | Variable heavy (VH) Anti-BCMA |
| **39** | LPVLTQPPSTSGTPGQRVTVSCSGSSSNIGSNVVFWYQQLPGTAPKLVIYRNNQR PSGVPDRFSVSKSGTSASLAISGLRSEDEADYYCAAWDDSLSGYVFGTGTKVTVL G | Variable light (VL) Anti-BCMA |
| **40** | QVQLVQSGAEVKKPGSSVKVSCKASGGTFSSYAISWVRQAPGQGLEWMGRIIPIL GTANYAQKFQGRVTITADESTSTAYMELSSLRSEDTAVYYCARSGYGSYRWEDSW GQGTLVTVSS | Variable heavy (VH) Anti-BCMA |
| **41** | QAVLTQPPSASGTPGQRVTISCSGSSSNIGSNYVFWYQQLPGTAPKLLIYSNNQR PSGVPDRFSGSKSGTSASLAISGLRSEDEADYYCAAWDDSLSASYVFGTGTKVTV LG | Variable light (VL) Anti-BCMA |
| **42** | QVQLVQSGAEVKKPGASVKVSCKASGYTFTDYYMHWVRQAPGQRLEWMGWINPNS GGTNYAQKFQDRITVTRDTSSNTGYMELTRLRSDDTAVYYCARSPYSGVLDKWGQ GTLVTVSS | Variable heavy (VH) Anti-BCMA |
| **43** | QSVLTQPPSVSGAPGQRVTISCTGSSSNIGAGFDVHWYQQLPGTAPKLLIYGNSN RPSGVPDRFSGSKSGTSASLAITGLQAEDEADYYCQSYDSSLSGYVFGTGTKVTV LG | Variable light (VL) Anti-BCMA |
| **44** | DYGVS | FMC63 CDR H1 |
| **45** | VIWGSETTYYNSALKS | FMC63 CDR H2 |
| **46** | YAMDYWG | FMC63 CDR H3 |
| **47** | HYYYGGSYAMDY | FMC63 HC-CDR3 |
| **48** | RASQDISKYLN | FMC63 CDRL1 |
| **49** | SRLHSGV | FMC63 CDR L2 |
| **50** | HTSRLHS | FMC63 LC-CDR2 |
| **51** | GNTLPYTFG | FMC63 CDR L3 |
| **52** | QQGNTLPYT | FMC63 LC-CDR3 |
| **53** | EVKLQESGPGLVAPSQSLSVTCTVSGVSLPDYGVSWIRQPPRKGLEWLGVIWGSE TTYYNSALKSRLTIIKDNSKSQVFLKMNSLQTDDTAIYYCAKHYYYGGSYAMDYW GQGTSVTVSS | FMC63 VH |
| **54** | DIQMTQTTSSLSASLGDRVTISCRASQDISKYLNWYQQKPDGTVKLLIYHTSRLH SGVPSRFSGSGSGTDYSLTISNLEQEDIATYFCQQGNTLPYTFGGGTKLEIT | FMC63 VL |
| **55** | DIQMTQTTSSLSASLGDRVTISCRASQDISKYLNWYQQKPDGTVKLLIYHTSRLH SGVPSRFSGSGSGTDYSLTISNLEQEDIATYFCQQGNTLPYTFGGGTKLEITGST SGSGKPGSGEGSTKGEVKLQESGPGLVAPSQSLSVTCTVSGVSLPDYGVSWIRQP PRKGLEWLGVIWGSETTYYNSALKSRLTIIKDNSKSQVFLKMNSLQTDDTAIYYC AKHYYYGGSYAMDYWGQGTSVTVSS | FMC63 scFv |
| **56** | KASQNVGTNVA | SJ25C1 CDR L1 |
| **57** | SATYRNS | SJ25C1 CDR L2 |
| **58** | QQYNRYPYT | SJ25C1 CDR L3 |
| **59** | SYWMN | SJ25C1 CDR H1 |
| **60** | QIYPGDGDTNYNGKFKG | SJ25C1 CDR H2 |
| **61** | KTISSVVDFYFDY | SJ25C1 CDRH3 |
| **62** | EVKLQQSGAELVRPGSSVKISCKASGYAFSSYWMNWVKQRPGQGLEWIGQIYPGD GDTNYNGKFKGQATLTADKSSSTAYMQLSGLTSEDSAVYFCARKTISSVVDFYFD YWGQGTTVTVSS | SJ25C1 VH |
| **63** | DIELTQSPKFMSTSVGDRVSVTCKASQNVGTNVAWYQQKPGQSPKPLIYSATYRN SGVPDRFTGSGSGTDFTLTITNVQSKDLADYFCQQYNRYPYTSGGGTKLEIKR | SJ25C1 VL |
| **64** | GGGGSGGGGSGGGGS | Linker |
| **65** | EVKLQQSGAELVRPGSSVKISCKASGYAFSSYWMNWVKQRPGQGLEWIGQIYPGD GDTNYNGKFKGQATLTADKSSSTAYMQLSGLTSEDSAVYFCARKTISSVVDFYFD YWGQGTTVTVSSGGGGSGGGGSGGGGSDIELTQSPKFMSTSVGDRVSVTCKASQN VGTNVAWYQQKPGQSPKPLIYSATYRNSGVPDRFTGSGSGTDFTLTITNVQSKDL ADYFCQQYNRYPYTSGGGTKLEIKR | SJ25C1 scFv |
| **66** | HYYYGGSYAMDY | FMC63 HC-CDR3 |
| **67** | HTSRLHS | FMC63 LC-CDR2 |
| **68** | QQGNTLPYT | FMC63 LC-CDR3 |
| **69** | gacatccagatgacccagaccacctccagcctgagcgccagcctgggcgaccggg tgaccatcagctgccgggccagccaggacatcagcaagtacctgaactggtatca gcagaagcccgacggcaccgtcaagctgctgatctaccacaccagccggctgcac agcggcgtgcccagccggtttagcggcagcggctccggcaccgactacagcctga ccatctccaacctggaacaggaagatatcgccacctacttttgccagcagggcaa cacactgccctacacctttggcggcggaacaaagctggaaatcaccggcagcacc tccggcagcggcaagcctggcagcggcgagggcagcaccaagggcgaggtgaagc tgcaggaaagcggccctggcctggtggcccccagccagagcctgagcgtgacctg caccgtgagcggcgtgagcctgcccgactacggcgtgagctggatccggcagccc cccaggaagggcctggaatggctgggcgtgatctggggcagcgagaccacctact acaacagcgccctgaagagccggctgaccatcatcaaggacaacagcaagagcca ggtgttcctgaagatgaacagcctgcagaccgacgacaccgccatctactactgc gccaagcactactactacggcggcagctacgccatggactactggggccagggca ccagcgtgaccgtgagcagc | Sequence encoding scFv |
| **70** | GSTSGSGKPGSGEGSTKG | Linker |
| **71** | GGGS | Linker |
| **72** | GGGGSGGGGSGGGGS | Linker |
| **73** | GSTSGSGKPGSGEGSTKG | Linker |
| **74** | SRGGGGSGGGGSGGGGSLEMA | Linker |
| **75** | MALPVTALLLPLALLLHAARP | CD8a signal peptide |
| **76** | METDTLLLWVLLLWVPGSTG | signal peptide |
| **77** | EVQLLESGGGLVQPGGSLRLSCAASGFTFSSYAMSWVRQAPGKGLEWVSAISGSG GSTYYADSVKGRFTISRDNSKNTLYLQMNSLRAEDTAVYYCARAEMGAVFDIWGQ GTMVTVSS | Variable heavy (VH) Anti-BCMA |
| **78** | EIVLTQSPATLSLSPGERATLSCRASQSVSRYLAWYQQKPGQAPRLLIYDASNRA TGIPARFSGSGSGTDFTLTISSLEPEDFAVYYCQQRISWPFTFGGGTKVEIK | Variable light (VL) Anti-BCMA |
| **79** | QVQLVESGGGVVQPGRSLRLSCAASGFTFSSYGMHWVRQAPGKGLEWVAVISYDG SNKYYADSVKGRFTISRDNSKNTLYLQMNSLRAEDTAVYYCARDGTYLGGLWYFD LWGRGTLVTVSS | Variable heavy (VH) Anti-BCMA |
| **80** | DIVMTQSPLSLPVTPGEPASISCRSSQSLLHSNGYNYLDWYLQKPGQSPQLLIYL GSNRASGVPDRFSGSGSGTDFTLKISRVEAEDVGVYYCMQGLGLPLTFGGGTKVE IK | Variable light (VL) Anti-BCMA |
| **81** | QVQLVQSGAEVKKPGASVKVSCKASGYTFTSYYMHWVRQAPGQGLEWMGIINPGG GSTSYAQKFQGRVTMTRDTSTSTVYMELSSLRSEDTAVYYCARESWPMDVWGQGT TVTVSS | Variable heavy (VH) Anti-BCMA |
| **82** | EIVMTQSPATLSVSPGERATLSCRASQSVSSNLAWYQQKPGQAPRLLIYGASTRA TGIPARFSGSGSGTEFTLTISSLQSEDFAVYYCQQYAAYPTFGGGTKVEIK | Variable light (VL) Anti-BCMA |
| **83** | QLQLQESGPGLVKPSETLSLTCTVSGGSISSSSYYWGWIRQPPGKGLEWIGSISY SGSTYYNPSLKSRVTISVDTSKNQFSLKLSSVTAADTAVYYCARGRGYATSLAFD IWGQGTMVTVSS | Variable heavy (VH) Anti-BCMA |
| **84** | EIVLTQSPATLSLSPGERATLSCRASQSVSSYLAWYQQKPGQAPRLLIYDASNRA TGIPARFSGSGSGTDFTLTISSLEPEDFAVYYCQQRHVWPPTFGGGTKVEIK | Variable light (VL) Anti-BCMA |
| **85** | EVQLVESGGGLVQPGGSLRLSCAASGFTFSSYSMNWVRQAPGKGLEWVSTISSSSS STIYYADSVKGRFTISRDNAKNSLYLQMNSLRAEDTAVYYCARGSQEHLIFDYWG QGTLVTVSS | Variable heavy (VH) Anti-BCMA |
| **86** | EIVLTQSPATLSLSPGERATLSCRASQSVSRYLAWYQQKPGQAPRLLIYDASNRA TGIPARFSGSGSGTDFTLTISSLEPEDFAVYYCQQRFYYPWTFGGGTKVEIK | Variable light (VL) Anti-BCMA |
| **87** | QVQLVESGGGVVQPGRSLRLSCAASGFTFSSYGMHWVRQAPGKGLEWVAVISYDG SNKYYADSVKGRFTISRDNSKNTLYLQMNSLRAEDTAVYYCARTDFWSGSPPGLD YWGQGTLVTVSS | Variable heavy (VH) Anti-BCMA |
| **88** | DIQLTQSPSSVSASVGDRVTITCRASQGISSWLAWYQQKPGKAPKLLIYGASSLQ SGVPSRFSGSGSGTDFTLTISSLQPEDFATYYCQQIYTFPFTFGGGTKVEIK | Variable light (VL) Anti-BCMA |
| **89** | QVQLVQSGAEVKKPGSSVKVSCKASGGTFSSYAISWVRQAPGQGLEWMGGIIPIF GTANYAQKFQGRVTITADESTSTAYMELSSLRSEDTAVYYCARTPEYSSSIWHYY YGMDVWGQGTTVTVSS | Variable heavy (VH) Anti-BCMA |
| **90** | DIVMTQSPDSLAVSLGERATINCKSSQSVLYSSNNKNYLAWYQQKPGQPPKLLIY WASTRESGVPDRFSGSGSGTDFTLTISSLQAEDVAVYYCQQFAHTPFTFGGGTKV EIK | Variable light (VL) Anti-BCMA |
| **91** | QVQLVESGGGVVQPGRSLRLSCAASGFTFSSYGMHWVRQAPGKGLEWVAVISYDG SNKYYADSVKGRFTISRDNSKNTLYLQMNSLRAEDTAVYYCVKGPLQEPPYDYGM DVWGQGTTVTVSS | Variable heavy (VH) Anti-BCMA |
| **92** | EIVMTQSPATLSVSPGERATLSCRASQSVSSNLAWYQQKPGQAPRLLIYSASTRA TGIPARFSGSGSGTEFTLTISSLQSEDFAVYYCQQHHVWPLTFGGGTKVEIK | Variable light (VL) Anti-BCMA |
| **93** | QVQLVQSGAEVKKPGSSVKVSCKASGGTFSSYAISWVRQAPGQGLEWMGRIIPIL GIANYAQKFQGRVTITADKSTSTAYMELSSLRSEDTAVYYCARGGYYSHDMWSED WGQGTLVTVSS | Variable heavy (VH) Anti-BCMA |
| **94** | LPVLTQPPSASGTPGQRVTISCSGRSSNIGSNSVNWYRQLPGAAPKLLIYSNNQR PPGVPVRFSGSKSGTSASLAISGLQSEDEATYYCATWDDNLNVHYVFGTGTKVTV LG | Variable light (VL) Anti-BCMA |
| **95** | QVQLVQSGSELKKPGASVKVSCKASGYTFTDYSINWVRQAPGQGLEWMGWINTET REPAYAYDFRGRFVFSLDTSVSTAYLQISSLKAEDTAVYYCARDYSYAMDYWGQG TLVTVSS | Variable heavy (VH) Anti-BCMA |
| **96** | DIVLTQSPASLAVSLGERATINCRASESVSVIGAHLIHWYQQKPGQPPKLLIYLA SNLETGVPARFSGSGSGTDFTLTISSLQAEDAAIYYCLQSRIFPRTFGQGTKLEI K | Variable light (VL) Anti-BCMA |
| **97** | EVQLVESGGGLVQPGGSLRLSCAVSGFALSNHGMSWVRRAPGKGLEWVSGIVYSG STYYAASVKGRFTISRDNSRNTLYLQMNSLRPEDTAIYYCSAHGGESDVWGQGTT VTVSS | Variable heavy (VH) Anti-BCMA |
| **98** | DIQLTQSPSSLSASVGDRVTITCRASQSISSYLNWYQQKPGKAPKLLIYAASSLQ SGVPSRFSGSGSGTDFTLTISSLQPEDFATYYCQQSYSTPYTFGQGTKVEIK | Variable light (VL) Anti-BCMA |
| **99** | QVQLVESGGGLVQPGRSLRLSCAASGFTFSNYAMSWVRQAPGKGLGWVSGISRSG ENTYYADSVKGRFTISRDNSKNTLYLQMNSLRDEDTAVYYCARSPAHYYGGMDVW GQGTTVTVSS | Variable heavy (VH) Anti-BCMA |
| **100** | DIVLTQSPGTLSLSPGERATLSCRASQSISSSFLAWYQQKPGQAPRLLIYGASRR ATGIPDRFSGSGSGTDFTLTISRLEPEDSAVYYCQQYHSSPSWTFGQGTKLEIK | Variable light (VL) Anti-BCMA |
| **101** | QVQLVESGGGLVQPGGSLRLSCAVSGFALSNHGMSWVRRAPGKGLEWVSGIVYSG STYYAASVKGRFTISRDNSRNTLYLQMNSLRPEDTAIYYCSAHGGESDVWGQGTT VTVSS | Variable heavy (VH) Anti-BCMA |
| **102** | DIRLTQSPSPLSASVGDRVTITCQASEDINKFLNWYHQTPGKAPKLLIYDASTLQ TGVPSRFSGSGSGTDFTLTINSLQPEDIGTYYCQQYESLPLTFGGGTKVEIK | Variable light (VL) Anti-BCMA |
| **103** | EVQLVESGGGLVQPGGSLRLSCAVSGFALSNHGMSWVRRAPGKGLEWVSGIVYSG | Variable heavy (VH) Anti-BCMA |
| | STYYAASVKGRFTISRDNSRNTLYLQMNSLRPEDTAIYYCSAHGGESDVWGQGTT VTVSS | |
| **104** | EIVLTQSPGTLSLSPGERATLSCRASQSIGSSSLAWYQQKPGQAPRLLMYGASSR ASGIPDRFSGSGSGTDFTLTISRLEPEDFAVYYCQQYAGSPPFTFGQGTKVEIK | Variable light (VL) Anti-BCMA |
| **105** | QIQLVQSGPELKKPGETVKISCKASGYTFRHYSMNWVKQAPGKGLKWMGRINTES GVPIYADDFKGRFAFSVETSASTAYLVINNLKDEDTASYFCSNDYLYSLDFWGQG TALTVSS | Variable heavy (VH) Anti-BCMA |
| **106** | DIVLTQSPPSLAMSLGKRATISCRASESVTILGSHLIYWYQQKPGQPPTLLIQLA SNVQTGVPARFSGSGSRTDFTLTIDPVEEDDVAVYYCLQSRTIPRTFGGGTKLEI K | Variable light (VL) Anti-BCMA |
| **107** | QIQLVQSGPELKKPGETVKISCKASGYTFTHYSMNWVKQAPGKGLKWMGRINTET GEPLYADDFKGRFAFSLETSASTAYLVINNLKNEDTATFFCSNDYLYSCDYWGQG TTLTVSS | Variable heavy (VH) Anti-BCMA |
| **108** | DIVLTQSPASLAMSLGKRATISCRASESVSVIGAHLIHWYQQKPGQPPKLLIYLA SNLETGVPARFSGSGSGTDFTLTIDPVEEDDVAIYSCLQSRIFPRTFGGGTKLEI K | Variable light (VL) Anti-BCMA |
| **109** | QVQLVQSGAEVKKPGASVKVSCKASGYSFPDYYINWVRQAPGQGLEWMGWIYFAS GNSEYNQKFTGRVTMTRDTSINTAYMELSSLTSEDTAVYFCASLYDYDWYFDVWG QGTMVTVSS | Variable heavy (VH) Anti-BCMA |
| **110** | DIVMTQTPLSLSVTPGQPASISCKSSQSLVHSNGNTYLHWYLQKPGQSPQLLIYK VSNRFSGVPDRFSGSGSGTDFTLKISRVEAEDVGIYYCSQSSIYPWTFGQGTKLE IK | Variable light (VL) Anti-BCMA |
| **111** | QVQLVQSGAEVKKPGASVKVSCKASGYSFPDYYINWVRQAPGQGLEWMGWIYFAS GNSEYNQKFTGRVTMTRDTSSSTAYMELSSLRSEDTAVYFCASLYDYDWYFDVWG QGTMVTVSS | Variable heavy (VH) Anti-BCMA |
| **112** | DIVMTQTPLSLSVTPGEPASISCKSSQSLVHSNGNTYLHWYLQKPGQSPQLLIYK VSNRFSGVPDRFSGSGSGADFTLKISRVEAEDVGVYYCAETSHVPWTFGQGTKLE IK | Variable light (VL) Anti-BCMA |
| **113** | QVQLVESGGGLVQPGGSLRLSCEASGFTLDYYAIGWFRQAPGKEREGVICISRSD GSTYYADSVKGRFTISRDNAKKTVYLQMISLKPEDTAAYYCAAGADCSGYLRDYE FRGQGTQVTVSS | Anti-BCMA sdAb |
| **114** | IEVMYPPPYLDNEKSNGTIIHVKGKHLCPSPLFPGPSKP | CD28 spacer |
| **115** | IYIWAPLAGTCGVLLLSLVITLYCN | CD8a TM |
| **116** | LDNEKSNGTIIHVKGKHLCPSPLFPGPSKP | CD28 spacer (truncated) |
| **117** | PTTTPAPRPPTPAPTIASQPLSLRPEACRPAAGGAVHTRGLDFACD | CD8a hinge |
| **118** | TTTPAPRPPTPAPTIASQPLSLRPEACRPAAGGAVHTRGLDFACD | CD8a hinge |
| **119** | FVPVFLPAKPTTTPAPRPPTPAPTIASQPLSLRPEACRPAAGGAVHTRGLDFACD | CD8a hinge |
| **120** | DTGLYICKVELMYPPPYYLGIGNGTQIYVIDPEPCPDSD | CTLA4 hinge |
| **121** | FLLWILAAVSSGLFFYSFLLTAVS | CTLA4 TM |
| **122** | QIKESLRAELRVTERRAEVPTAHPSPSPRPAGQFQTLV | PD-1 hinge |
| **123** | VGVVGGLLGSLVLLVWVLAVI | PD-1 TM |
| **124** | GLAVSTISSFFPPGYQ | FcγRIIIa hinge |
| **125** | EPKSPDKTHTCPPCPAPPVAGPSVFLFPPKPKDTLMIARTPEVTCVVVDVSHEDP EVKFNWYVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPSDIAVEW ESNGQPENNYKTTPPVLDSDGSFFLYSKLTVDKSRWQQGNVFSCSVMHEALHNHY TQKSLSLSPGK | IgG1 hinge |
| **126** | EVQLLESGGGLVQPGGSLRLSCAASGFTFSSYAMSWVRQAPGKGLEWVSAISGSG GSTYYADSVKGRFTISRDNSKNTLYLQMNSLRAEDTAVYYCARAEMGAVFDIWGQ GTMVTVSSGSTSGSGKPGSGEGSTKGEIVLTQSPATLSLSPGERATLSCRASQSV SRYLAWYQQKPGQAPRLLIYDASNRATGIPARFSGSGSGTDFTLTISSLEPEDFA VYYCQQRISWPFTFGGGTKVEIKRAAALDNEKSNGTIIHVKGKHLCPSPLFPGPS KPFWVLVVVGGVLACYSLLVTVAFIIFWVRSKRSRLLHSDYMNMTPRRPGPTRKH YQPYAPPRDFAAYRSRVKFSRSADAPAYQQGQNQLYNELNLGRREEYDVLDKRRG | anti-BCMA CAR |
| | RDPEMGGKPRRKNPQEGLYNELQKDKMAEAYSEIGMKGERRRGKGHDGLYQGLST ATKDTYDALHMQALPPR | |
| **127** | EIVLTQSPATLSLSPGERATLSCRASQSVSRYLAWYQQKPGQAPRLLIYDASNRA TGIPARFSGSGSGTDFTLTISSLEPEDFAVYYCQQRISWPFTFGGGTKVEIKRGS TSGSGKPGSGEGSTKGEVQLLESGGGLVQPGGSLRLSCAASGFTFSSYAMSWVRQ APGKGLEWVSAISGSGGSTYYADSVKGRFTISRDNSKNTLYLQMNSLRAEDTAVY YCARAEMGAVFDIWGQGTMVTVSSAAALDNEKSNGTIIHVKGKHLCPSPLFPGPS KPFWVLVVVGGVLACYSLLVTVAFIIFWVRSKRSRLLHSDYMNMTPRRPGPTRKH YQPYAPPRDFAAYRSRVKFSRSADAPAYQQGQNQLYNELNLGRREEYDVLDKRRG RDPEMGGKPRRKNPQEGLYNELQKDKMAEAYSEIGMKGERRRGKGHDGLYQGLST ATKDTYDALHMQALPPR | anti-BCMA CAR |
| **128** | QVQLVESGGGVVQPGRSLRLSCAASGFTFSSYGMHWVRQAPGKGLEWVAVISYDG SNKYYADSVKGRFTISRDNSKNTLYLQMNSLRAEDTAVYYCARDGTYLGGLWYFD LWGRGTLVTVSSGSTSGSGKPGSGEGSTKGDIVMTQSPLSLPVTPGEPASISCRS SQSLLHSNGYNYLDWYLQKPGQSPQLLIYLGSNRASGVPDRFSGSGSGTDFTLKI SRVEAEDVGVYYCMQGLGLPLTFGGGTKVEIKRAAALDNEKSNGTIIHVKGKHLC PSPLFPGPSKPFWVLVVVGGVLACYSLLVTVAFIIFWVRSKRSRLLHSDYMNMTP RRPGPTRKHYQPYAPPRDFAAYRSRVKFSRSADAPAYQQGQNQLYNELNLGRREE YDVLDKRRGRDPEMGGKPRRKNPQEGLYNELQKDKMAEAYSEIGMKGERRRGKGH DGLYQGLSTATKDTYDALHMQALPPR | anti-BCMA CAR |
| **129** | DIVMTQSPLSLPVTPGEPASISCRSSQSLLHSNGYNYLDWYLQKPGQSPQLLIYL GSNRASGVPDRFSGSGSGTDFTLKISRVEAEDVGVYYCMQGLGLPLTFGGGTKVE IKRGSTSGSGKPGSGEGSTKGQVQLVESGGGVVQPGRSLRLSCAASGFTFSSYGM HWVRQAPGKGLEWVAVISYDGSNKYYADSVKGRFTISRDNSKNTLYLQMNSLRAE DTAVYYCARDGTYLGGLWYFDLWGRGTLVTVSSAAALDNEKSNGTIIHVKGKHLC PSPLFPGPSKPFWVLVVVGGVLACYSLLVTVAFIIFWVRSKRSRLLHSDYMNMTP RRPGPTRKHYQPYAPPRDFAAYRSRVKFSRSADAPAYQQGQNQLYNELNLGRREE YDVLDKRRGRDPEMGGKPRRKNPQEGLYNELQKDKMAEAYSEIGMKGERRRGKGH DGLYQGLSTATKDTYDALHMQALPPR | anti-BCMA CAR |
| **130** | QVQLVQSGAEVKKPGASVKVSCKASGYTFTSYYMHWVRQAPGQGLEWMGIINPGG GSTSYAQKFQGRVTMTRDTSTSTVYMELSSLRSEDTAVYYCARESWPMDVWGQGT TVTVSSGSTSGSGKPGSGEGSTKGEIVMTQSPATLSVSPGERATLSCRASQSVSS NLAWYQQKPGQAPRLLIYGASTRATGIPARFSGSGSGTEFTLTISSLQSEDFAVY YCQQYAAYPTFGGGTKVEIKRAAALDNEKSNGTIIHVKGKHLCPSPLFPGPSKPF WVLVVVGGVLACYSLLVTVAFIIFWVRSKRSRLLHSDYMNMTPRRPGPTRKHYQP YAPPRDFAAYRSRVKFSRSADAPAYQQGQNQLYNELNLGRREEYDVLDKRRGRDP EMGGKPRRKNPQEGLYNELQKDKMAEAYSEIGMKGERRRGKGHDGLYQGLSTATK DTYDALHMQALPPR | anti-BCMA CAR |
| **131** | EIVMTQSPATLSVSPGERATLSCRASQSVSSNLAWYQQKPGQAPRLLIYGASTRA TGIPARFSGSGSGTEFTLTISSLQSEDFAVYYCQQYAAYPTFGGGTKVEIKRGST SGSGKPGSGEGSTKGQVQLVQSGAEVKKPGASVKVSCKASGYTFTSYYMHWVRQA PGQGLEWMGIINPGGGSTSYAQKFQGRVTMTRDTSTSTVYMELSSLRSEDTAVYY CARESWPMDVWGQGTTVTVSSAAALDNEKSNGTIIHVKGKHLCPSPLFPGPSKPF WVLVVVGGVLACYSLLVTVAFIIFWVRSKRSRLLHSDYMNMTPRRPGPTRKHYQP YAPPRDFAAYRSRVKFSRSADAPAYQQGQNQLYNELNLGRREEYDVLDKRRGRDP EMGGKPRRKNPQEGLYNELQKDKMAEAYSEIGMKGERRRGKGHDGLYQGLSTATK DTYDALHMQALPPR | anti-BCMA CAR |
| **132** | QLQLQESGPGLVKPSETLSLTCTVSGGSISSSSYYWGWIRQPPGKGLEWIGSISY SGSTYYNPSLKSRVTISVDTSKNQFSLKLSSVTAADTAVYYCARGRGYATSLAFD IWGQGTMVTVSSGSTSGSGKPGSGEGSTKGEIVLTQSPATLSLSPGERATLSCRA SQSVSSYLAWYQQKPGQAPRLLIYDASNRATGIPARFSGSGSGTDFTLTISSLEP EDFAVYYCQQRHVWPPTFGGGTKVEIKRAAALDNEKSNGTIIHVKGKHLCPSPLF PGPSKPFWVLVVVGGVLACYSLLVTVAFIIFWVRSKRSRLLHSDYMNMTPRRPGP TRKHYQPYAPPRDFAAYRSRVKFSRSADAPAYQQGQNQLYNELNLGRREEYDVLD KRRGRDPEMGGKPRRKNPQEGLYNELQKDKMAEAYSEIGMKGERRRGKGHDGLYQ GLSTATKDTYDALHMQALPPR | anti-BCMA CAR |
| **133** | EIVLTQSPATLSLSPGERATLSCRASQSVSSYLAWYQQKPGQAPRLLIYDASNRA | anti-BCMA CAR |
| | TGIPARFSGSGSGTDFTLTISSLEPEDFAVYYCQQRHVWPPTFGGGTKVEIKRGS TSGSGKPGSGEGSTKGQLQLQESGPGLVKPSETLSLTCTVSGGSISSSSYYWGWI RQPPGKGLEWIGSISYSGSTYYNPSLKSRVTISVDTSKNQFSLKLSSVTAADTAV YYCARGRGYATSLAFDIWGQGTMVTVSSAAALDNEKSNGTIIHVKGKHLCPSPLF PGPSKPFWVLVVVGGVLACYSLLVTVAFIIFWVRSKRSRLLHSDYMNMTPRRPGP TRKHYQPYAPPRDFAAYRSRVKFSRSADAPAYQQGQNQLYNELNLGRREEYDVLD KRRGRDPEMGGKPRRKNPQEGLYNELQKDKMAEAYSEIGMKGERRRGKGHDGLYQ GLSTATKDTYDALHMQALPPR | |
| **134** | EVQLVESGGGLVQPGGSLRLSCAASGFTFSSYSMNWVRQAPGKGLEWVSTISSSS STIYYADSVKGRFTISRDNAKNSLYLQMNSLRAEDTAVYYCARGSQEHLIFDYWG QGTLVTVSSGSTSGSGKPGSGEGSTKGEIVLTQSPATLSLSPGERATLSCRASQS VSRYLAWYQQKPGQAPRLLIYDASNRATGIPARFSGSGSGTDFTLTISSLEPEDF AVYYCQQRFYYPWTFGGGTKVEIKRAAALDNEKSNGTIIHVKGKHLCPSPLFPGP SKPFWVLVVVGGVLACYSLLVTVAFIIFWVRSKRSRLLHSDYMNMTPRRPGPTRK HYQPYAPPRDFAAYRSRVKFSRSADAPAYQQGQNQLYNELNLGRREEYDVLDKRR GRDPEMGGKPRRKNPQEGLYNELQKDKMAEAYSEIGMKGERRRGKGHDGLYQGLS TATKDTYDALHMQALPPR | anti-BCMA CAR |
| **135** | EIVLTQSPATLSLSPGERATLSCRASQSVSRYLAWYQQKPGQAPRLLIYDASNRA TGIPARFSGSGSGTDFTLTISSLEPEDFAVYYCQQRFYYPWTFGGGTKVEIKRGS TSGSGKPGSGEGSTKGEVQLVESGGGLVQPGGSLRLSCAASGFTFSSYSMNWVRQ APGKGLEWVSTISSSSSTIYYADSVKGRFTISRDNAKNSLYLQMNSLRAEDTAVY YCARGSQEHLIFDYWGQGTLVTVSSAAALDNEKSNGTIIHVKGKHLCPSPLFPGP SKPFWVLVVVGGVLACYSLLVTVAFIIFWVRSKRSRLLHSDYMNMTPRRPGPTRK HYQPYAPPRDFAAYRSRVKFSRSADAPAYQQGQNQLYNELNLGRREEYDVLDKRR GRDPEMGGKPRRKNPQEGLYNELQKDKMAEAYSEIGMKGERRRGKGHDGLYQGLS TATKDTYDALHMQALPPR | anti-BCMA CAR |
| **136** | QVQLVESGGGVVQPGRSLRLSCAASGFTFSSYGMHWVRQAPGKGLEWVAVISYDG SNKYYADSVKGRFTISRDNSKNTLYLQMNSLRAEDTAVYYCARTDFWSGSPPGLD YWGQGTLVTVSSGSTSGSGKPGSGEGSTKGDIQLTQSPSSVSASVGDRVTITCRA SQGISSWLAWYQQKPGKAPKLLIYGASSLQSGVPSRFSGSGSGTDFTLTISSLQP EDFATYYCQQIYTFPFTFGGGTKVEIKRAAALDNEKSNGTIIHVKGKHLCPSPLF PGPSKPFWVLVVVGGVLACYSLLVTVAFIIFWVRSKRSRLLHSDYMNMTPRRPGP TRKHYQPYAPPRDFAAYRSRVKFSRSADAPAYQQGQNQLYNELNLGRREEYDVLD KRRGRDPEMGGKPRRKNPQEGLYNELQKDKMAEAYSEIGMKGERRRGKGHDGLYQ GLSTATKDTYDALHMQALPPR | anti-BCMA CAR |
| **137** | DIQLTQSPSSVSASVGDRVTITCRASQGISSWLAWYQQKPGKAPKLLIYGASSLQ SGVPSRFSGSGSGTDFTLTISSLQPEDFATYYCQQIYTFPFTFGGGTKVEIKRGS TSGSGKPGSGEGSTKGQVQLVESGGGVVQPGRSLRLSCAASGFTFSSYGMHWVRQ APGKGLEWVAVISYDGSNKYYADSVKGRFTISRDNSKNTLYLQMNSLRAEDTAVY YCARTDFWSGSPPGLDYWGQGTLVTVSSAAALDNEKSNGTIIHVKGKHLCPSPLF PGPSKPFWVLVVVGGVLACYSLLVTVAFIIFWVRSKRSRLLHSDYMNMTPRRPGP TRKHYQPYAPPRDFAAYRSRVKFSRSADAPAYQQGQNQLYNELNLGRREEYDVLD KRRGRDPEMGGKPRRKNPQEGLYNELQKDKMAEAYSEIGMKGERRRGKGHDGLYQ GLSTATKDTYDALHMQALPPR | anti-BCMA CAR |
| **138** | QVQLVQSGAEVKKPGSSVKVSCKASGGTFSSYAISWVRQAPGQGLEWMGGIIPIF GTANYAQKFQGRVTITADESTSTAYMELSSLRSEDTAVYYCARTPEYSSSIWHYY YGMDVWGQGTTVTVSSGSTSGSGKPGSGEGSTKGDIVMTQSPDSLAVSLGERATI NCKSSQSVLYSSNNKNYLAWYQQKPGQPPKLLIYWASTRESGVPDRFSGSGSGTD FTLTISSLQAEDVAVYYCQQFAHTPFTFGGGTKVEIKRAAALDNEKSNGTIIHVK GKHLCPSPLFPGPSKPFWVLVVVGGVLACYSLLVTVAFIIFWVRSKRSRLLHSDY MNMTPRRPGPTRKHYQPYAPPRDFAAYRSRVKFSRSADAPAYQQGQNQLYNELNL GRREEYDVLDKRRGRDPEMGGKPRRKNPQEGLYNELQKDKMAEAYSEIGMKGERR RGKGHDGLYQGLSTATKDTYDALHMQALPPR | anti-BCMA CAR |
| **139** | DIVMTQSPDSLAVSLGERATINCKSSQSVLYSSNNKNYLAWYQQKPGQPPKLLIY WASTRESGVPDRFSGSGSGTDFTLTISSLQAEDVAVYYCQQFAHTPFTFGGGTKV EIKRGSTSGSGKPGSGEGSTKGQVQLVQSGAEVKKPGSSVKVSCKASGGTFSSYA ISWVRQAPGQGLEWMGGIIPIFGTANYAQKFQGRVTITADESTSTAYMELSSLRS | anti-BCMA CAR |
| | EDTAVYYCARTPEYSSSIWHYYYGMDVWGQGTTVTVSSAAALDNEKSNGTIIHVK GKHLCPSPLFPGPSKPFWVLVVVGGVLACYSLLVTVAFIIFWVRSKRSRLLHSDY MNMTPRRPGPTRKHYQPYAPPRDFAAYRSRVKFSRSADAPAYQQGQNQLYNELNL GRREEYDVLDKRRGRDPEMGGKPRRKNPQEGLYNELQKDKMAEAYSEIGMKGERR RGKGHDGLYQGLSTATKDTYDALHMQALPPR | |
| **140** | QVQLVESGGGVVQPGRSLRLSCAASGFTFSSYGMHWVRQAPGKGLEWVAVISYDG SNKYYADSVKGRFTISRDNSKNTLYLQMNSLRAEDTAVYYCVKGPLQEPPYDYGM DVWGQGTTVTVSSGSTSGSGKPGSGEGSTKGEIVMTQSPATLSVSPGERATLSCR ASQSVSSNLAWYQQKPGQAPRLLIYSASTRATGIPARFSGSGSGTEFTLTISSLQ SEDFAVYYCQQHHVWPLTFGGGTKVEIKRAAALDNEKSNGTIIHVKGKHLCPSPL FPGPSKPFWVLVVVGGVLACYSLLVTVAFIIFWVRSKRSRLLHSDYMNMTPRRPG PTRKHYQPYAPPRDFAAYRSRVKFSRSADAPAYQQGQNQLYNELNLGRREEYDVL DKRRGRDPEMGGKPRRKNPQEGLYNELQKDKMAEAYSEIGMKGERRRGKGHDGLY QGLSTATKDTYDALHMQALPPR | anti-BCMA CAR |
| **141** | EIVMTQSPATLSVSPGERATLSCRASQSVSSNLAWYQQKPGQAPRLLIYSASTRA TGIPARFSGSGSGTEFTLTISSLQSEDFAVYYCQQHHVWPLTFGGGTKVEIKRGS TSGSGKPGSGEGSTKGQVQLVESGGGVVQPGRSLRLSCAASGFTFSSYGMHWVRQ APGKGLEWVAVISYDGSNKYYADSVKGRFTISRDNSKNTLYLQMNSLRAEDTAVY YCVKGPLQEPPYDYGMDVWGQGTTVTVSSAAALDNEKSNGTIIHVKGKHLCPSPL FPGPSKPFWVLVVVGGVLACYSLLVTVAFIIFWVRSKRSRLLHSDYMNMTPRRPG PTRKHYQPYAPPRDFAAYRSRVKFSRSADAPAYQQGQNQLYNELNLGRREEYDVL DKRRGRDPEMGGKPRRKNPQEGLYNELQKDKMAEAYSEIGMKGERRRGKGHDGLY QGLSTATKDTYDALHMQALPPR | anti-BCMA CAR |
| **142** | QSALTQPASVSASPGQSIAISCTGTSSDVGWYQQHPGKAPKLMIYEDSKRPSGVS NRFSGSKSGNTASLTISGLQAEDEADYYCSSNTRSSTLVFGGGTKLTVLGSRGGG GSGGGGSGGGGSLEMAEVQLVQSGAEMKKPGASLKLSCKASGYTFIDYYVYWMRQ APGQGLESMGWINPNSGGTNYAQKFQGRVTMTRDTSISTAYMELSRLRSDDTAMY YCARSQRDGYMDYWGQGTLVTVSSAAAIEVMYPPPYLDNEKSNGTIIHVKGKHLC PSPLFPGPSKPFWVLVVVGGVLACYSLLVTVAFIIFWVRSKRSRLLHSDYMNMTP RRPGPTRKHYQPYAPPRDFAAYRSRVKFSRSADAPAYQQGQNQLYNELNLGRREE YDVLDKRRGRDPEMGGKPRRKNPQEGLYNELQKDKMAEAYSEIGMKGERRRGKGH DGLYQGLSTATKDTYDALHMQALPPR | anti-BCMA CAR |
| **143** | QSVLTQPPSVSGAPGQRVTISCTGSSSNIGAGFDVHWYQQLPGTAPKLLIYGNSN RPSGVPDRFSGSKSGTSASLAITGLQAEDEADYYCQSYDSSLSGYVFGTGTKVTV LGSRGGGGSGGGGSGGGGSLEMAQVQLVQSGAEVKKPGASVKVSCKASGYTFTDY YMHWVRQAPGQRLEWMGWINPNSGGTNYAQKFQDRITVTRDTSSNTGYMELTRLR SDDTAVYYCARSPYSGVLDKWGQGTLVTVSSAAAIEVMYPPPYLDNEKSNGTIIH VKGKHLCPSPLFPGPSKPFWVLVVVGGVLACYSLLVTVAFIIFWVRSKRSRLLHS DYMNMTPRRPGPTRKHYQPYAPPRDFAAYRSRVKFSRSADAPAYQQGQNQLYNEL NLGRREEYDVLDKRRGRDPEMGGKPRRKNPQEGLYNELQKDKMAEAYSEIGMKGE RRRGKGHDGLYQGLSTATKDTYDALHMQALPPR | anti-BCMA CAR |
| **144** | SYELTQPPSASGTPGQRVTMSCSGTSSNIGSHSVNWYQQLPGTAPKLLIYTNNQR PSGVPDRFSGSKSGTSASLAISGLQSEDEADYYCAAWDGSLNGLVFGGGTKLTVL GSRGGGGSGGGGSGGGGSLEMAEVQLVQSGAEVKKPGESLKISCKGSGYSFTSYW IGWVRQMPGKGLEWMGIIYPGDSDTRYSPSFQGHVTISADKSISTAYLQWSSLKA SDTAMYYCARYSGSFDNWGQGTLVTVSSAAAIEVMYPPPYLDNEKSNGTIIHVKG KHLCPSPLFPGPSKPFWVLVVVGGVLACYSLLVTVAFIIFWVRSKRSRLLHSDYM NMTPRRPGPTRKHYQPYAPPRDFAAYRSRVKFSRSADAPAYQQGQNQLYNELNLG RREEYDVLDKRRGRDPEMGGKPRRKNPQEGLYNELQKDKMAEAYSEIGMKGERRR GKGHDGLYQGLSTATKDTYDALHMQALPPR | anti-BCMA CAR |
| **145** | LPVLTQPPSASGTPGQRVTISCSGRSSNIGSNSVNWYRQLPGAAPKLLIYSNNQR PPGVPVRFSGSKSGTSASLAISGLQSEDEATYYCATWDDNLNVHYVFGTGTKVTV LGSRGGGGSGGGGSGGGGSLEMAQVQLVQSGAEVKKPGSSVKVSCKASGGTFSSY AISWVRQAPGQGLEWMGRIIPILGIANYAQKFQGRVTITADKSTSTAYMELSSLR SEDTAVYYCARGGYYSHDMWSEDWGQGTLVTVSSAAAIEVMYPPPYLDNEKSNGT IIHVKGKHLCPSPLFPGPSKPFWVLVVVGGVLACYSLLVTVAFIIFWVRSKRSRL LHSDYMNMTPRRPGPTRKHYQPYAPPRDFAAYRSRVKFSRSADAPAYQQGQNQLY | anti-BCMA CAR |
| | NELNLGRREEYDVLDKRRGRDPEMGGKPRRKNPQEGLYNELQKDKMAEAYSEIGM KGERRRGKGHDGLYQGLSTATKDTYDALHMQALPPR | |
| **146** | QAVLTQPPSASGTPGQRVTISCSGSSSNIGSNYVFWYQQLPGTAPKLLIYSNNQR PSGVPDRFSGSKSGTSASLAISGLRSEDEADYYCAAWDDSLSASYVFGTGTKVTV LGSRGGGGSGGGGSGGGGSLEMAQVQLVQSGAEVKKPGSSVKVSCKASGGTFSSY AISWVRQAPGQGLEWMGRIIPILGTANYAQKFQGRVTITADESTSTAYMELSSLR SEDTAVYYCARSGYGSYRWEDSWGQGTLVTVSSAAAIEVMYPPPYLDNEKSNGTI IHVKGKHLCPSPLFPGPSKPFWVLVVVGGVLACYSLLVTVAFIIFWVRSKRSRLL HSDYMNMTPRRPGPTRKHYQPYAPPRDFAAYRSRVKFSRSADAPAYQQGQNQLYN ELNLGRREEYDVLDKRRGRDPEMGGKPRRKNPQEGLYNELQKDKMAEAYSEIGMK GERRRGKGHDGLYQGLSTATKDTYDALHMQALPPR | anti-BCMA CAR |
| **147** | LPVLTQPPSASGTPGQRVTISCSGRSSNIGSNSVNWYRQLPGAAPKLLIYSNNQR PPGVPVRFSGSKSGTSASLAISGLQSEDEATYYCATWDDNLNVHYVFGTGTKVTV LGSRGGGGSGGGGSGGGGSLEMAQVQLVQSGAEVKKPGSSVKVSCKASGGTFSSY AISWVRQAPGQGLEWMGRIIPILGIANYAQKFQGRVTITADKSTSTAYMELSSLR SEDTAVYYCARGGYYSHDMWSEDWGQGTLVTVSSAAAPTTTPAPRPPTPAPTIAS QPLSLRPEACRPAAGGAVHTRGLDFACDIYIWAPLAGTCGVLLLSLVITLYCNKR GRKKLLYIFKQPFMRPVQTTQEEDGCSCRFPEEEEGGCELRVKFSRSAEPPAYQQ GQNQLYNELNLGRREEYDVLDKRRGRDPEMGGKPRRKNPQEGLYNELQKDKMAEA YSEIGMKGERRRGKGHDGLYQGLSTATKDTYDALHMQALPPR | anti-BCMA CAR |
| **148** | SYELTQPPSASGTPGQRVTMSCSGTSSNIGSHSVNWYQQLPGTAPKLLIYTNNQR PSGVPDRFSGSKSGTSASLAISGLQSEDEADYYCAAWDGSLNGLVFGGGTKLTVL GSRGGGGSGGGGSGGGGSLEMAEVQLVQSGAEVKKPGESLKISCKGSGYSFTSYW IGWVRQMPGKGLEWMGIIYPGDSDTRYSPSFQGHVTISADKSISTAYLQWSSLKA SDTAMYYCARYSGSFDNWGQGTLVTVSSAAAPTTTPAPRPPTPAPTIASQPLSLR PEACRPAAGGAVHTRGLDFACDIYIWAPLAGTCGVLLLSLVITLYCNKRGRKKLL YIFKQPFMRPVQTTQEEDGCSCRFPEEEEGGCELRVKFSRSAEPPAYQQGQNQLY NELNLGRREEYDVLDKRRGRDPEMGGKPRRKNPQEGLYNELQKDKMAEAYSEIGM KGERRRGKGHDGLYQGLSTATKDTYDALHMQALPPR | anti-BCMA CAR |
| **149** | QAVLTQPPSASGTPGQRVTISCSGSSSNIGSNYVFWYQQLPGTAPKLLIYSNNQR PSGVPDRFSGSKSGTSASLAISGLRSEDEADYYCAAWDDSLSASYVFGTGTKVTV LGSRGGGGSGGGGSGGGGSLEMAQVQLVQSGAEVKKPGSSVKVSCKASGGTFSSY AISWVRQAPGQGLEWMGRIIPILGTANYAQKFQGRVTITADESTSTAYMELSSLR SEDTAVYYCARSGYGSYRWEDSWGQGTLVTVSSAAAPTTTPAPRPPTPAPTIASQ PLSLRPEACRPAAGGAVHTRGLDFACDIYIWAPLAGTCGVLLLSLVITLYCNKRG RKKLLYIFKQPFMRPVQTTQEEDGCSCRFPEEEEGGCELRVKFSRSAEPPAYQQG QNQLYNELNLGRREEYDVLDKRRGRDPEMGGKPRRKNPQEGLYNELQKDKMAEAY SEIGMKGERRRGKGHDGLYQGLSTATKDTYDALHMQALPPR | anti-BCMA CAR |
| **150** | QSVLTQPPSVSGAPGQRVTISCTGSSSNIGAGFDVHWYQQLPGTAPKLLIYGNSN RPSGVPDRFSGSKSGTSASLAITGLQAEDEADYYCQSYDSSLSGYVFGTGTKVTV LGSRGGGGSGGGGSGGGGSLEMAQVQLVQSGAEVKKPGASVKVSCKASGYTFTDY YMHWVRQAPGQRLEWMGWINPNSGGTNYAQKFQDRITVTRDTSSNTGYMELTRLR SDDTAVYYCARSPYSGVLDKWGQGTLVTVSSAAAPTTTPAPRPPTPAPTIASQPL SLRPEACRPAAGGAVHTRGLDFACDIYIWAPLAGTCGVLLLSLVITLYCNKRGRK KLLYIFKQPFMRPVQTTQEEDGCSCRFPEEEEGGCELRVKFSRSAEPPAYQQGQN QLYNELNLGRREEYDVLDKRRGRDPEMGGKPRRKNPQEGLYNELQKDKMAEAYSE IGMKGERRRGKGHDGLYQGLSTATKDTYDALHMQALPPR | anti-BCMA CAR |
| **151** | QSALTQPASVSASPGQSIAISCTGTSSDVGWYQQHPGKAPKLMIYEDSKRPSGVS NRFSGSKSGNTASLTISGLQAEDEADYYCSSNTRSSTLVFGGGTKLTVLGSRGGG GSGGGGSGGGGSLEMAEVQLVQSGAEMKKPGASLKLSCKASGYTFIDYYVYWMRQ APGQGLESMGWINPNSGGTNYAQKFQGRVTMTRDTSISTAYMELSRLRSDDTAMY YCARSQRDGYMDYWGQGTLVTVSSAAAPTTTPAPRPPTPAPTIASQPLSLRPEAC RPAAGGAVHTRGLDFACDIYIWAPLAGTCGVLLLSLVITLYCNKRGRKKLLYIFK QPFMRPVQTTQEEDGCSCRFPEEEEGGCELRVKFSRSAEPPAYQQGQNQLYNELN LGRREEYDVLDKRRGRDPEMGGKPRRKNPQEGLYNELQKDKMAEAYSEIGMKGER RRGKGHDGLYQGLSTATKDTYDALHMQALPPR | anti-BCMA CAR |
| **152** | DIVLTQSPPSLAMSLGKRATISCRASESVTILGSHLIHWYQQKPGQPPTLLIQLA | anti-BCMA CAR |
| | SNVQTGVPARFSGSGSRTDFTLTIDPVEEDDVAVYYCLQSRTIPRTFGGGTKLEI KGSTSGSGKPGSGEGSTKGQIQLVQSGPELKKPGETVKISCKASGYTFTDYSINW VKRAPGKGLKWMGWINTETREPAYAYDFRGRFAFSLETSASTAYLQINNLKYEDT ATYFCALDYSYAMDYWGQGTSVTVSSAAATTTPAPRPPTPAPTIASQPLSLRPEA CRPAAGGAVHTRGLDFACDIYIWAPLAGTCGVLLLSLVITLYCKRGRKKLLYIFK QPFMRPVQTTQEEDGCSCRFPEEEEGGCELRVKFSRSADAPAYQQGQNQLYNELN LGRREEYDVLDKRRGRDPEMGGKPRRKNPQEGLYNELQKDKMAEAYSEIGMKGER RRGKGHDGLYQGLSTATKDTYDALHMQALPPR | |
| **153** | DIVLTQSPASLAVSLGERATINCRASESVSVIGAHLIHWYQQKPGQPPKLLIYLA SNLETGVPARFSGSGSGTDFTLTISSLQAEDAAIYYCLQSRIFPRTFGQGTKLEI KGSTSGSGKPGSGEGSTKGQVQLVQSGSELKKPGASVKVSCKASGYTFTDYSINW VRQAPGQGLEWMGWINTETREPAYAYDFRGRFVFSLDTSVSTAYLQISSLKAEDT AVYYCARDYSYAMDYWGQGTLVTVSSAAATTTPAPRPPTPAPTIASQPLSLRPEA CRPAAGGAVHTRGLDFACDIYIWAPLAGTCGVLLLSLVITLYCKRGRKKLLYIFK QPFMRPVQTTQEEDGCSCRFPEEEEGGCELRVKFSRSADAPAYQQGQNQLYNELN LGRREEYDVLDKRRGRDPEMGGKPRRKNPQEGLYNELQKDKMAEAYSEIGMKGER RRGKGHDGLYQGLSTATKDTYDALHMQALPPR | anti-BCMA CAR |
| **154** | DIVLTQSPASLAVSLGERATINCRASESVSVIGAHLIHWYQQKPGQPPKLLIYLA SNLETGVPARFSGSGSGTDFTLTISSLQAEDAAIYYCLQSRIFPRTFGQGTKLEI KGSTSGSGKPGSGEGSTKGQVQLVQSGSELKKPGASVKVSCKASGYTFTDYSINW VRQAPGQGLEWMGWINTETREPAYAYDFRGRFVFSLDTSVSTAYLQISSLKAEDT AVYYCARDYSYAMDYWGQGTLVTVSSAAADTGLYICKVELMYPPPYYLGIGNGTQ IYVIDPEPCPDSDFLLWILAAVSSGLFFYSFLLTAVSKRGRKKLLYIFKQPFMRP VQTTQEEDGCSCRFPEEEEGGCELRVKFSRSADAPAYQQGQNQLYNELNLGRREE YDVLDKRRGRDPEMGGKPRRKNPQEGLYNELQKDKMAEAYSEIGMKGERRRGKGH DGLYQGLSTATKDTYDALHMQALPPR | anti-BCMA CAR |
| **155** | DIVLTQSPASLAVSLGERATINCRASESVSVIGAHLIHWYQQKPGQPPKLLIYLA SNLETGVPARFSGSGSGTDFTLTISSLQAEDAAIYYCLQSRIFPRTFGQGTKLEI KGSTSGSGKPGSGEGSTKGQVQLVQSGSELKKPGASVKVSCKASGYTFTDYSINW VRQAPGQGLEWMGWINTETREPAYAYDFRGRFVFSLDTSVSTAYLQISSLKAEDT AVYYCARDYSYAMDYWGQGTLVTVSSAAAQIKESLRAELRVTERRAEVPTAHPSP SPRPAGQFQTLVVGVVGGLLGSLVLLVWVLAVICSKRGRKKLLYIFKQPFMRPVQ TTQEEDGCSCRFPEEEEGGCELRVKFSRSADAPAYQQGQNQLYNELNLGRREEYD VLDKRRGRDPEMGGKPRRKNPQEGLYNELQKDKMAEAYSEIGMKGERRRGKGHDG LYQGLSTATKDTYDALHMQALPPR | anti-BCMA CAR |
| **156** | EVQLVESGGGLVKPGGSLRLSCAASGFTFSDYYMSWIRQAPGKGLEWVSYISSSG STIYYADSVKGRFTISRDNAKNSLYLQMNSLRAEDTAVYYCAKVDGDYTEDYWGQ GTLVTVSSGGGGSGGGGSGGGGSQSALTQPASVSGSPGQSITISCTGSSSDVGKY NLVSWYQQPPGKAPKLIIYDVNKRPSGVSNRFSGSKSGNTATLTISGLQGDDEAD YYCSSYGGSRSYVFGTGTKVTVLESKYGPPCPPCPAPPVAGPSVFLFPPKPKDTL MISRTPEVTCVVVDVSQEDPEVQFNWYVDGVEVHNAKTKPREEQFQSTYRVVSVL TVLHQDWLNGKEYKCKVSNKGLPSSIEKTISKAKGQPREPQVYTLPPSQEEMTKN QVSLTCLVKGFYPSDIAVEWESNGQPENNYKTTPPVLDSDGSFFLYSRLTVDKSR WQEGNVFSCSVMHEALHNHYTQKSLSLSLGKMFWVLVVVGGVLACYSLLVTVAFI IFWVKRGRKKLLYIFKQPFMRPVQTTQEEDGCSCRFPEEEEGGCELRVKFSRSAD APAYQQGQNQLYNELNLGRREEYDVLDKRRGRDPEMGGKPRRKNPQEGLYNELQK DKMAEAYSEIGMKGERRRGKGHDGLYQGLSTATKDTYDALHMQALPPR | anti-BCMA CAR |
| **157** | EVQLVQSGGGLVQPGRSLRLSCTASGFTFGDYAMSWFKQAPGKGLEWVGFIRSKA YGGTTEYAASVKGRFTISRDDSKSIAYLQMNSLKTEDTAVYYCAAWSAPTDYWGQ GTLVTVSSGGGGSGGGGSGGGGSDIQMTQSPAFLSASVGDRVTVTCRASQGISNY LAWYQQKPGNAPRLLIYSASTLQSGVPSRFRGTGYGTEFSLTIDSLQPEDFATYY CQQSYTSRQTFGPGTRLDIKESKYGPPCPPCPAPPVAGPSVFLFPPKPKDTLMIS RTPEVTCVVVDVSQEDPEVQFNWYVDGVEVHNAKTKPREEQFQSTYRVVSVLTVL HQDWLNGKEYKCKVSNKGLPSSIEKTISKAKGQPREPQVYTLPPSQEEMTKNQVS LTCLVKGFYPSDIAVEWESNGQPENNYKTTPPVLDSDGSFFLYSRLTVDKSRWQE GNVFSCSVMHEALHNHYTQKSLSLSLGKMFWVLVVVGGVLACYSLLVTVAFIIFW VKRGRKKLLYIFKQPFMRPVQTTQEEDGCSCRFPEEEEGGCELRVKFSRSADAPA | anti-BCMA CAR |
| | YQQGQNQLYNELNLGRREEYDVLDKRRGRDPEMGGKPRRKNPQEGLYNELQKDKM AEAYSEIGMKGERRRGKGHDGLYQGLSTATKDTYDALHMQALPPR | |
| **158** | EVQLVESGGGLVKPGGSLRLSCAASGFTFSDYYMSWIRQAPGKGLEWVSYISSSG STIYYADSVKGRFTISRDNAKNSLYLQMNSLRAEDTAVYYCAKVDGPPSFDIWGQ GTMVTVSSGGGGSGGGGSGGGGSSYVLTQPPSVSVAPGQTARITCGANNIGSKSV HWYQQKPGQAPMLVVYDDDDRPSGIPERFSGSNSGNTATLTISGVEAGDEADYFC HLWDRSRDHYVFGTGTKLTVLESKYGPPCPPCPAPPVAGPSVFLFPPKPKDTLMI SRTPEVTCVVVDVSQEDPEVQFNWYVDGVEVHNAKTKPREEQFQSTYRVVSVLTV LHQDWLNGKEYKCKVSNKGLPSSIEKTISKAKGQPREPQVYTLPPSQEEMTKNQV SLTCLVKGFYPSDIAVEWESNGQPENNYKTTPPVLDSDGSFFLYSRLTVDKSRWQ EGNVFSCSVMHEALHNHYTQKSLSLSLGKMFWVLVVVGGVLACYSLLVTVAFIIF WVKRGRKKLLYIFKQPFMRPVQTTQEEDGCSCRFPEEEEGGCELRVKFSRSADAP AYQQGQNQLYNELNLGRREEYDVLDKRRGRDPEMGGKPRRKNPQEGLYNELQKDK MAEAYSEIGMKGERRRGKGHDGLYQGLSTATKDTYDALHMQALPPR | anti-BCMA CAR |
| **159** | SYELTQPPSASGTPGQRVTMSCSGTSSNIGSHSVNWYQQLPGTAPKLLIYTNNQR PSGVPDRFSGSKSGTSASLAISGLQSEDEADYYCAAWDGSLNGLVFGGGTKLTVL GSRGGGGSGGGGSGGGGSLEMAEVQLVQSGAEVKKPGESLKISCKGSGYSFTSYW IGWVRQMPGKGLEWMGIIYPGDSDTRYSPSFQGHVTISADKSISTAYLQWSSLKA SDTAMYYCARYSGSFDNWGQGTLVTVSSESKYGPPCPPCPAPPVAGPSVFLFPPK PKDTLMISRTPEVTCVVVDVSQEDPEVQFNWYVDGVEVHNAKTKPREEQFQSTYR VVSVLTVLHQDWLNGKEYKCKVSNKGLPSSIEKTISKAKGQPREPQVYTLPPSQE EMTKNQVSLTCLVKGFYPSDIAVEWESNGQPENNYKTTPPVLDSDGSFFLYSRLT VDKSRWQEGNVFSCSVMHEALHNHYTQKSLSLSLGKMFWVLVVVGGVLACYSLLV TVAFIIFWVKRGRKKLLYIFKQPFMRPVQTTQEEDGCSCRFPEEEEGGCELRVKF SRSADAPAYQQGQNQLYNELNLGRREEYDVLDKRRGRDPEMGGKPRRKNPQEGLY NELQKDKMAEAYSEIGMKGERRRGKGHDGLYQGLSTATKDTYDALHMQALPPR | anti-BCMA CAR |
| **160** | QSALTQPASVSASPGQSIAISCTGTSSDVGWYQQHPGKAPKLMIYEDSKRPSGVS NRFSGSKSGNTASLTISGLQAEDEADYYCSSNTRSSTLVFGGGTKLTVLGSRGGG GSGGGGSGGGGSLEMAEVQLVQSGAEMKKPGASLKLSCKASGYTFIDYYVYWMRQ APGQGLESMGWINPNSGGTNYAQKFQGRVTMTRDTSISTAYMELSRLRSDDTAMY YCARSQRDGYMDYWGQGTLVTVSSESKYGPPCPPCPAPPVAGPSVFLFPPKPKDT LMISRTPEVTCVVVDVSQEDPEVQFNWYVDGVEVHNAKTKPREEQFQSTYRVVSV LTVLHQDWLNGKEYKCKVSNKGLPSSIEKTISKAKGQPREPQVYTLPPSQEEMTK NQVSLTCLVKGFYPSDIAVEWESNGQPENNYKTTPPVLDSDGSFFLYSRLTVDKS RWQEGNVFSCSVMHEALHNHYTQKSLSLSLGKMFWVLVVVGGVLACYSLLVTVAF IIFWVKRGRKKLLYIFKQPFMRPVQTTQEEDGCSCRFPEEEEGGCELRVKFSRSA DAPAYQQGQNQLYNELNLGRREEYDVLDKRRGRDPEMGGKPRRKNPQEGLYNELQ KDKMAEAYSEIGMKGERRRGKGHDGLYQGLSTATKDTYDALHMQALPPR | anti-BCMA CAR |
| **161** | QSALTQPASVSASPGQSIAISCTGTSSDVGWYQQHPGKAPKLMIYEDSKRPSGVS NRFSGSKSGNTASLTISGLQAEDEADYYCSSNTRSSTLVFGGGTKLTVLGSRGGG GSGGGGSGGGGSLEMAEVQLVQSGAEMKKPGASLKLSCKASGYTFIDYYVYWMRQ APGQGLESMGWINPNSGGTNYAQKFQGRVTMTRDTSISTAYMELSRLRSDDTAMY YCARSQRDGYMDYWGQGTLVTVSSESKYGPPCPPCPAPPVAGPSVFLFPPKPKDT LMISRTPEVTCVVVDVSQEDPEVQFNWYVDGVEVHNAKTKPREEQFQSTYRVVSV LTVLHQDWLNGKEYKCKVSNKGLPSSIEKTISKAKGQPREPQVYTLPPSQEEMTK NQVSLTCLVKGFYPSDIAVEWESNGQPENNYKTTPPVLDSDGSFFLYSRLTVDKS RWQEGNVFSCSVMHEALHNHYTQKSLSLSLGKMFWVLVVVGGVLACYSLLVTVAF IIFWVRSKRSRLLHSDYMNMTPRRPGPTRKHYQPYAPPRDFAAYRSRVKFSRSAD APAYQQGQNQLYNELNLGRREEYDVLDKRRGRDPEMGGKPRRKNPQEGLYNELQK DKMAEAYSEIGMKGERRRGKGHDGLYQGLSTATKDTYDALHMQALPPR | anti-BCMA CAR |
| **162** | EVQLVESGGGLVQPGGSLRLSCAVSGFALSNHGMSWVRRAPGKGLEWVSGIVYSG STYYAASVKGRFTISRDNSRNTLYLQMNSLRPEDTAIYYCSAHGGESDVWGQGTT VTVSSASGGGGSGGRASGGGGSDIQLTQSPSSLSASVGDRVTITCRASQSISSYL NWYQQKPGKAPKLLIYAASSLQSGVPSRFSGSGSGTDFTLTISSLQPEDFATYYC QQSYSTPYTFGQGTKVEIKTTTPAPRPPTPAPTIASQPLSLRPEACRPAAGGAVH TRGLDFACDIYIWAPLAGTCGVLLLSLVITLYCKRGRKKLLYIFKQPFMRPVQTT QEEDGCSCRFPEEEEGGCELRVKFSRSADAPAYKQGQNQLYNELNLGRREEYDVL | anti-BCMA CAR |
| | DKRRGRDPEMGGKPRRKNPQEGLYNELQKDKMAEAYSEIGMKGERRRGKGHDGLY QGLSTATKDTYDALHMQALPPR | |
| **163** | QVQLVESGGGLVQPGRSLRLSCAASGFTFSNYAMSWVRQAPGKGLGWVSGISRSG ENTYYADSVKGRFTISRDNSKNTLYLQMNSLRDEDTAVYYCARSPAHYYGGMDVW GQGTTVTVSSASGGGGSGGRASGGGGSDIVLTQSPGTLSLSPGERATLSCRASQS ISSSFLAWYQQKPGQAPRLLIYGASRRATGIPDRFSGSGSGTDFTLTISRLEPED SAVYYCQQYHSSPSWTFGQGTKLEIKTTTPAPRPPTPAPTIASQPLSLRPEACRP AAGGAVHTRGLDFACDIYIWAPLAGTCGVLLLSLVITLYCKRGRKKLLYIFKQPF MRPVQTTQEEDGCSCRFPEEEEGGCELRVKFSRSADAPAYKQGQNQLYNELNLGR REEYDVLDKRRGRDPEMGGKPRRKNPQEGLYNELQKDKMAEAYSEIGMKGERRRG KGHDGLYQGLSTATKDTYDALHMQALPPR | anti-BCMA CAR |
| **164** | QVQLVESGGGLVQPGGSLRLSCAVSGFALSNHGMSWVRRAPGKGLEWVSGIVYSG STYYAASVKGRFTISRDNSRNTLYLQMNSLRPEDTAIYYCSAHGGESDVWGQGTT VTVSSASGGGGSGGRASGGGGSDIRLTQSPSPLSASVGDRVTITCQASEDINKFL NWYHQTPGKAPKLLIYDASTLQTGVPSRFSGSGSGTDFTLTINSLQPEDIGTYYC QQYESLPLTFGGGTKVEIKTTTPAPRPPTPAPTIASQPLSLRPEACRPAAGGAVH TRGLDFACDIYIWAPLAGTCGVLLLSLVITLYCKRGRKKLLYIFKQPFMRPVQTT QEEDGCSCRFPEEEEGGCELRVKFSRSADAPAYKQGQNQLYNELNLGRREEYDVL DKRRGRDPEMGGKPRRKNPQEGLYNELQKDKMAEAYSEIGMKGERRRGKGHDGLY QGLSTATKDTYDALHMQALPPR | anti-BCMA CAR |
| **165** | EVQLVESGGGLVQPGGSLRLSCAVSGFALSNHGMSWVRRAPGKGLEWVSGIVYSG STYYAASVKGRFTISRDNSRNTLYLQMNSLRPEDTAIYYCSAHGGESDVWGQGTT VTVSSASGGGGSGGRASGGGGSEIVLTQSPGTLSLSPGERATLSCRASQSIGSSS LAWYQQKPGQAPRLLMYGASSRASGIPDRFSGSGSGTDFTLTISRLEPEDFAVYY CQQYAGSPPFTFGQGTKVEIKTTTPAPRPPTPAPTIASQPLSLRPEACRPAAGGA VHTRGLDFACDIYIWAPLAGTCGVLLLSLVITLYCKRGRKKLLYIFKQPFMRPVQ TTQEEDGCSCRFPEEEEGGCELRVKFSRSADAPAYKQGQNQLYNELNLGRREEYD VLDKRRGRDPEMGGKPRRKNPQEGLYNELQKDKMAEAYSEIGMKGERRRGKGHDG LYQGLSTATKDTYDALHMQALPPR | anti-BCMA CAR |
| **166** | QIQLVQSGPDLKKPGETVKLSCKASGYTFTNFGMNWVKQAPGKGFKWMAWINTYT GESYFADDFKGRFAFSVETSATTAYLQINNLKTEDTATYFCARGEIYYGYDGGFA YWGQGTLVTVSAGGGGSGGGGSGGGGSDVVMTQSHRFMSTSVGDRVSITCRASQD VNTAVSWYQQKPGQSPKLLIFSASYRYTGVPDRFTGSGSGADFTLTISSVQAEDL AVYYCQQHYSTPWTFGGGTKLDIKTTTPAPRPPTPAPTIASQPLSLRPEACRPAA GGAVHTRGLDFACDIYIWAPLAGTCGVLLLSLVITLYCKRGRKKLLYIFKQPFMR PVQTTQEEDGCSCRFPEEEEGGCELRVKFSRSADAPAYKQGQNQLYNELNLGRRE EYDVLDKRRGRDPEMGGKPRRKNPQEGLYNELQKDKMAEAYSEIGMKGERRRGKG HDGLYQGLSTATKDTYDALHMQALPPR | anti-BCMA CAR |
| **167** | QIQLVQSGPELKKPGETVKISCKASGYTFTDYSINWVKRAPGKGLKWMGWINTET REPAYAYDFRGRFAFSLETSASTAYLQINNLKYEDTATYFCALDYSYAMDYWGQG TSVTVSSGGGGSGGGGSGGGGSQIQLVQSGPELKKPGETVKISCKASGYTFTDYS INWVKRAPGKGLKWMGWINTETREPAYAYDFRGRFAFSLETSASTAYLQINNLKY EDTATYFCALDYSYAMDYWGQGTSVTVSSTTTPAPRPPTPAPTIASQPLSLRPEA CRPAAGGAVHTRGLDFACDIYIWAPLAGTCGVLLLSLVITLYCKRGRKKLLYIFK QPFMRPVQTTQEEDGCSCRFPEEEEGGCELRVKFSRSADAPAYKQGQNQLYNELN LGRREEYDVLDKRRGRDPEMGGKPRRKNPQEGLYNELQKDKMAEAYSEIGMKGER RRGKGHDGLYQGLSTATKDTYDALHMQALPPR | anti-BCMA CAR |
| **168** | QIQLVQSGPELKKPGETVKISCKASGYTFTDYSINWVKRAPGKGLKWMGWINTET REPAYAYDFRGRFAFSLETSASTAYLQINNLKYEDTATYFCALDYSYAMDYWGQG TSVTVSSGGGGSGGGGSGGGGSDIVLTQSPASLAMSLGKRATISCRASESVSVIG AHLIHWYQQKPGQPPKLLIYLASNLETGVPARFSGSGSGTDFTLTIDPVEEDDVA IYSCLQSRIFPRTFGGGTKLEIKTTTPAPRPPTPAPTIASQPLSLRPEACRPAAG GAVHTRGLDFACDIYIWAPLAGTCGVLLLSLVITLYCKRGRKKLLYIFKQPFMRP VQTTQEEDGCSCRFPEEEEGGCELRVKFSRSADAPAYKQGQNQLYNELNLGRREE YDVLDKRRGRDPEMGGKPRRKNPQEGLYNELQKDKMAEAYSEIGMKGERRRGKGH DGLYQGLSTATKDTYDALHMQALPPR | anti-BCMA CAR |
| **169** | QIQLVQSGPELKKPGETVKISCKASGYTFRHYSMNWVKQAPGKGLKWMGRINTES | anti-BCMA CAR |
| | GVPIYADDFKGRFAFSVETSASTAYLVINNLKDEDTASYFCSNDYLYSLDFWGQG TALTVSSGGGGSGGGGSGGGGSDIVLTQSPPSLAMSLGKRATISCRASESVTILG SHLIYWYQQKPGQPPTLLIQLASNVQTGVPARFSGSGSRTDFTLTIDPVEEDDVA VYYCLQSRTIPRTFGGGTKLEIKTTTPAPRPPTPAPTIASQPLSLRPEACRPAAG GAVHTRGLDFACDIYIWAPLAGTCGVLLLSLVITLYCKRGRKKLLYIFKQPFMRP VQTTQEEDGCSCRFPEEEEGGCELRVKFSRSADAPAYKQGQNQLYNELNLGRREE YDVLDKRRGRDPEMGGKPRRKNPQEGLYNELQKDKMAEAYSEIGMKGERRRGKGH DGLYQGLSTATKDTYDALHMQALPPR | |
| **170** | QIQLVQSGPELKKPGETVKISCKASGYTFTHYSMNWVKQAPGKGLKWMGRINTET GEPLYADDFKGRFAFSLETSASTAYLVINNLKNEDTATFFCSNDYLYSCDYWGQG TTLTVSSGGGGSGGGGSGGGGSDIVLTQSPPSLAMSLGKRATISCRASESVTILG SHLIYWYQQKPGQPPTLLIQLASNVQTGVPARFSGSGSRTDFTLTIDPVEEDDVA VYYCLQSRTIPRTFGGGTKLEIKTTTPAPRPPTPAPTIASQPLSLRPEACRPAAG GAVHTRGLDFACDIYIWAPLAGTCGVLLLSLVITLYCKRGRKKLLYIFKQPFMRP VQTTQEEDGCSCRFPEEEEGGCELRVKFSRSADAPAYKQGQNQLYNELNLGRREE YDVLDKRRGRDPEMGGKPRRKNPQEGLYNELQKDKMAEAYSEIGMKGERRRGKGH DGLYQGLSTATKDTYDALHMQALPPR | anti-BCMA CAR |
| **171** | DIVLTQSPPSLAMSLGKRATISCRASESVTILGSHLIHWYQQKPGQPPTLLIQLA SNVQTGVPARFSGSGSRTDFTLTIDPVEEDDVAVYYCLQSRTIPRTFGGGTKLEI KGSTSGSGKPGSGEGSTKGQIQLVQSGPELKKPGETVKISCKASGYTFTDYSINW VKRAPGKGLKWMGWINTETREPAYAYDFRGRFAFSLETSASTAYLQINNLKYEDT ATYFCALDYSYAMDYWGQGTSVTVSSFVPVFLPAKPTTTPAPRPPTPAPTIASQP LSLRPEACRPAAGGAVHTRGLDFACDIYIWAPLAGTCGVLLLSLVITLYCNHRNR SKRSRLLHSDYMNMTPRRPGPTRKHYQPYAPPRDFAAYRSRVKFSRSADAPAYQQ GQNQLYNELNLGRREEYDVLDKRRGRDPEMGGKPRRKNPQEGLYNELQKDKMAEA YSEIGMKGERRRGKGHDGLYQGLSTATKDTYDALHMQALPPR | anti-BCMA CAR |
| **172** | QVQLVQSGAEVKKPGASVKVSCKASGYSFPDYYINWVRQAPGQGLEWMGWIYFAS GNSEYNQKFTGRVTMTRDTSINTAYMELSSLTSEDTAVYFCASLYDYDWYFDVWG QGTMVTVSSGGGGSGGGGSGGGGSDIVMTQTPLSLSVTPGQPASISCKSSQSLVH SNGNTYLHWYLQKPGQSPQLLIYKVSNRFSGVPDRFSGSGSGTDFTLKISRVEAE DVGIYYCSQSSIYPWTFGQGTKLEIKGLAVSTISSFFPPGYQIYIWAPLAGTCGV LLLSLVITLYCKRGRKKLLYIFKQPFMRPVQTTQEEDGCSCRFPEEEEGGCELRV KFSRSADAPAYQQGQNQLYNELNLGRREEYDVLDKRRGRDPEMGGKPRRKNPQEG LYNELQKDKMAEAYSEIGMKGERRRGKGHDGLYQGLSTATKDTYDALHMQALPPR | anti-BCMA CAR |
| **173** | QVQLVQSGAEVKKPGASVKVSCKASGYSFPDYYINWVRQAPGQGLEWMGWIYFAS GNSEYNQKFTGRVTMTRDTSINTAYMELSSLTSEDTAVYFCASLYDYDWYFDVWG QGTMVTVSSGGGGSGGGGSGGGGSDIVMTQTPLSLSVTPGQPASISCKSSQSLVH SNGNTYLHWYLQKPGQSPQLLIYKVSNRFSGVPDRFSGSGSGTDFTLKISRVEAE DVGIYYCSQSSIYPWTFGQGTKLEIKTTTPAPRPPTPAPTIASQPLSLRPEACRP AAGGAVHTRGLDFACDIYIWAPLAGTCGVLLLSLVITLYCKRGRKKLLYIFKQPF MRPVQTTQEEDGCSCRFPEEEEGGCELRVKFSRSADAPAYQQGQNQLYNELNLGR REEYDVLDKRRGRDPEMGGKPRRKNPQEGLYNELQKDKMAEAYSEIGMKGERRRG KGHDGLYQGLSTATKDTYDALHMQALPPR | anti-BCMA CAR |
| **174** | QVQLVQSGAEVKKPGASVKVSCKASGYSFPDYYINWVRQAPGQGLEWMGWIYFAS GNSEYNQKFTGRVTMTRDTSINTAYMELSSLTSEDTAVYFCASLYDYDWYFDVWG QGTMVTVSSGGGGSGGGGSGGGGSDIVMTQTPLSLSVTPGQPASISCKSSQSLVH SNGNTYLHWYLQKPGQSPQLLIYKVSNRFSGVPDRFSGSGSGTDFTLKISRVEAE DVGIYYCSQSSIYPWTFGQGTKLEIKEPKSPDKTHTCPPCPAPPVAGPSVFLFPP KPKDTLMIARTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLPPSR DELTKNQVSLTCLVKGFYPSDIAVEWESNGQPENNYKTTPPVLDSDGSFFLYSKL TVDKSRWQQGNVFSCSVMHEALHNHYTQKSLSLSPGKIYIWAPLAGTCGVLLLSL VITLYCKRGRKKLLYIFKQPFMRPVQTTQEEDGCSCRFPEEEEGGCELRVKFSRS ADAPAYQQGQNQLYNELNLGRREEYDVLDKRRGRDPEMGGKPRRKNPQEGLYNEL QKDKMAEAYSEIGMKGERRRGKGHDGLYQGLSTATKDTYDALHMQALPPR | anti-BCMA CAR |
| **175** | QVQLVQSGAEVKKPGASVKVSCKASGYSFPDYYINWVRQAPGQGLEWMGWIYFAS GNSEYNQKFTGRVTMTRDTSSSTAYMELSSLRSEDTAVYFCASLYDYDWYFDVWG | anti-BCMA CAR |
| | QGTMVTVSSGGGGSGGGGSGGGGSDIVMTQTPLSLSVTPGEPASISCKSSQSLVH SNGNTYLHWYLQKPGQSPQLLIYKVSNRFSGVPDRFSGSGSGADFTLKISRVEAE DVGVYYCAETSHVPWTFGQGTKLEIKGLAVSTISSFFPPGYQIYIWAPLAGTCGV LLLSLVITLYCKRGRKKLLYIFKQPFMRPVQTTQEEDGCSCRFPEEEEGGCELRV KFSRSADAPAYQQGQNQLYNELNLGRREEYDVLDKRRGRDPEMGGKPRRKNPQEG LYNELQKDKMAEAYSEIGMKGERRRGKGHDGLYQGLSTATKDTYDALHMQALPPR | |
| **176** | QVQLVQSGAEVKKPGASVKVSCKASGYSFPDYYINWVRQAPGQGLEWMGWIYFAS GNSEYNQKFTGRVTMTRDTSSSTAYMELSSLRSEDTAVYFCASLYDYDWYFDVWG QGTMVTVSSGGGGSGGGGSGGGGSDIVMTQTPLSLSVTPGEPASISCKSSQSLVH SNGNTYLHWYLQKPGQSPQLLIYKVSNRFSGVPDRFSGSGSGADFTLKISRVEAE DVGVYYCAETSHVPWTFGQGTKLEIKTTTPAPRPPTPAPTIASQPLSLRPEACRP AAGGAVHTRGLDFACDIYIWAPLAGTCGVLLLSLVITLYCKRGRKKLLYIFKQPF MRPVQTTQEEDGCSCRFPEEEEGGCELRVKFSRSADAPAYQQGQNQLYNELNLGR REEYDVLDKRRGRDPEMGGKPRRKNPQEGLYNELQKDKMAEAYSEIGMKGERRRG KGHDGLYQGLSTATKDTYDALHMQALPPR | anti-BCMA CAR |
| **177** | QVQLVQSGAEVKKPGASVKVSCKASGYSFPDYYINWVRQAPGQGLEWMGWIYFAS GNSEYNQKFTGRVTMTRDTSSSTAYMELSSLRSEDTAVYFCASLYDYDWYFDVWG QGTMVTVSSGGGGSGGGGSGGGGSDIVMTQTPLSLSVTPGEPASISCKSSQSLVH SNGNTYLHWYLQKPGQSPQLLIYKVSNRFSGVPDRFSGSGSGADFTLKISRVEAE DVGVYYCAETSHVPWTFGQGTKLEIKEPKSPDKTHTCPPCPAPPVAGPSVFLFPP KPKDTLMIARTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLPPSR DELTKNQVSLTCLVKGFYPSDIAVEWESNGQPENNYKTTPPVLDSDGSFFLYSKL TVDKSRWQQGNVFSCSVMHEALHNHYTQKSLSLSPGKIYIWAPLAGTCGVLLLSL VITLYCKRGRKKLLYIFKQPFMRPVQTTQEEDGCSCRFPEEEEGGCELRVKFSRS ADAPAYQQGQNQLYNELNLGRREEYDVLDKRRGRDPEMGGKPRRKNPQEGLYNEL QKDKMAEAYSEIGMKGERRRGKGHDGLYQGLSTATKDTYDALHMQALPPR | anti-BCMA CAR |
| **178** | IYIWAPLAGTCGVLLLSLVITLYCNHRN | CD8a TM |
| **179** | IYIWAPLAGTCGVLLLSLVIT | CD8a TM |
| **180** | RAAA | linking peptide |
| **181** | EVQLVESGGGLVKPGGSLRLSCAASGFTFSDYYMSWIRQAPGKGLEWVSYISSSG STIYYADSVKGRFTISRDNAKNSLYLQMNSLRAEDTAVYYCAKVDGDYTEDYWGQ GTLVTVSS | Variable heavy (VH) Anti-BCMA |
| **182** | QSALTQPASVSGSPGQSITISCTGSSSDVGKYNLVSWYQQPPGKAPKLIIYDVNK RPSGVSNRFSGSKSGNTATLTISGLQGDDEADYYCSSYGGSRSYVFGTGTKVTVL | Variable light (VL) Anti-BCMA |
| **183** | EVQLVQSGGGLVQPGRSLRLSCTASGFTFGDYAMSWFRQAPGKGLEWVGFIRSKA YGGTTEYAASVKGRFTISRDDSKSIAYLQMNSLKTEDTAVYYCAAWSAPTDYWGQ GTLVTVSS | Variable heavy (VH) Anti-BCMA |
| **184** | DIQMTQSPAFLSASVGDRVTVTCRASQGISNYLAWYQQKPGNAPRLLIYSASTLQ SGVPSRFRGTGYGTEFSLTIDSLQPEDFATYYCQQSYTSRQTFGPGTRLDIK | Variable light (VL) Anti-BCMA |
| **185** | EVQLVESGGGLVKPGGSLRLSCAASGFTFSDYYMSWIRQAPGKGLEWVSYISSSG STIYYADSVKGRFTISRDNAKNSLYLQMNSLRAEDTAVYYCAKVDGPPSFDIWGQ GTMVTVSS | Variable heavy (VH) Anti-BCMA |
| **186** | SYVLTQPPSVSVAPGQTARITCGANNIGSKSVHWYQQKPGQAPMLVVYDDDDRPS GIPERFSGSNSGNTATLTISGVEAGDEADYFCHLWDRSRDHYVFGTGTKLTVL | Variable light (VL) Anti-BCMA |
| **187** | EVQLVQSGAEVKKPGSSVKVSCKASGGTFSSYAISWVRQAPGQGLEWMGRIIPIL GIANYAQKFQGRVTMTEDTSTDTAYMELSSLRSEDTAVYYCARSGYSKSIVSYMD YWGQGTLVTVSS | Variable heavy (VH) Anti-BCMA |
| **188** | LPVLTQPPSTSGTPGQRVTVSCSGSSSNIGSNVVFWYQQLPGTAPKLVIYRNNQR PSGVPDRFSVSKSGTSASLAISGLRSEDEADYYCAAWDDSLSGYVFGTGTKVTVL G | Variable light (VL) Anti-BCMA |
| **189** | ASGGGGSGGRASGGGGS | Linker |

## Claims

1. A T cell therapy and an immunomodulatory compound for use in a method of treating cancer, wherein the method comprises:
(a) administering a T cell therapy to a subject having a disease or condition that is cancer, wherein the T cell therapy is or comprises genetically engineered cells expressing a recombinant receptor that specifically binds to an antigen, wherein the recombinant receptor is a chimeric antigen receptor (CAR), and wherein the CAR comprises an extracellular domain comprising an antigen-binding domain that specifically binds to a tumor antigen, a spacer, and an intracellular signaling region; and
(b) administering to the subject an immunomodulatory compound, wherein said immunomodulatory compound is thalidomide (2-(2,6-dioxopiperidin-3-yl)-1H-isoindole-1,3(2H)-dione) or an analog or derivative of thalidomide,
and wherein the immunomodulatory compound:
(i) binds to cereblon (CRBN) and/or to the CRBN E3 ubiquitin-ligase complex, and
(ii) enhances the degradation of both Ikaros (IKZF1) and Aiolos (IKZF3);
wherein initiation of administration of the immunomodulatory compound is carried out 7 to 21 days after initiation of administration of the T cell therapy, and
wherein the immunomodulatory compound is administered orally.

2. An immunomodulatory compound for use in a method of treating cancer, the method comprising administering an immunomodulatory compound to a subject having a disease or condition that is cancer, wherein, at the time of initiation of administration of the immunomodulatory compound, the subject has been previously administered a T cell therapy for treatment of the disease or condition, wherein the T cell therapy is or comprises genetically engineered cells expressing a recombinant receptor that specifically binds to an antigen, wherein the recombinant receptor is a chimeric antigen receptor (CAR), and wherein the CAR comprises an extracellular domain comprising an antigen-binding domain that specifically binds to a tumor antigen, a spacer, and an intracellular signaling region,
wherein said immunomodulatory compound is thalidomide (2-(2,6-dioxopiperidin-3-yl)-1H-isoindole- 1,3(2H)-dione) or an analog or derivative of thalidomide,
and wherein the immunomodulatory compound:
(i) binds to cereblon (CRBN) and/or to the CRBN E3 ubiquitin-ligase complex, and
(ii) enhances the degradation of both Ikaros (IKZF1) and Aiolos (IKZF3); and
wherein initiation of administration of the immunomodulatory compound is carried out 7 to 21 days after initiation of administration of the T cell therapy, and
wherein the immunomodulatory compound is administered orally.

3. The T cell therapy and immunomodulatory compound for use of claim 1 or the immunomodulatory compound for use of claim 2, wherein initiation of administration of the immunomodulatory compound is carried out at a time that is greater than 14 days, 15 days, 16 days, 17 days, 18 days, 19, days, or 20 days after initiation of the administration of the T cell therapy.

4. The T cell therapy and immunomodulatory compound for use of claim 1 or claim 3 or the immunomodulatory compound for use of claim 2 or claim 3, wherein the immunomodulatory compound is administered to a subject within a week, such as within 1, 2 or 3 days after:
(i) peak or maximum level of the cells of the T cell therapy are detectable in the blood of the subject;
(ii) the number of cells of the T cell therapy detectable in the blood, after having been detectable in the blood, is not detectable or is reduced, optionally reduced compared to a preceding time point after administration of the T cell therapy;
(iii) the number of cells of the T cell therapy detectable in the blood is decreased by or more than 1.5-fold, 2.0-fold, 3.0-fold, 4.0-fold, 5.0-fold, 10-fold or more the peak or maximum number cells of the T cell therapy detectable in the blood of the subject after initiation of administration of the T cell therapy;
(iv) at a time after a peak or maximum level of the cells of the T cell therapy are detectable in the blood of the subject, the number of cells of or derived from the T cells detectable in the blood from the subject is less than less than 10%, less than 5%, less than 1% or less than 0.1% of total peripheral blood mononuclear cells (PBMCs) in the blood of the subject;
(v) the subject exhibits disease progression and/or has relapsed following remission after treatment with the T cell therapy; and/or
(vi) the subject exhibits increased tumor burden as compared to tumor burden at a time prior to or after administration of the T cells and prior to initiation of administration of the immunomodulatory compound.

5. The T cell therapy and immunomodulatory compound for use of any of claims 1 and 3-4 or the immunomodulatory compound for use of any of claims 2-4, the method comprising, prior to initiation of administration of the immunomodulatory compound, selecting a subject in which:
(i) peak or maximum level of the cells of the T cell therapy are detectable in the blood of the subject;
(ii) the number of cells of the T cell therapy detectable in the blood, after having been detectable in the blood, is not detectable or is reduced, optionally reduced compared to a preceding time point after administration of the T cell therapy;
(iii) the number of cells of the T cell therapy detectable in the blood is decreased by or more than 1.5-fold, 2.0-fold, 3.0-fold, 4.0-fold, 5.0-fold, 10-fold or more the peak or maximum number cells of the T cell therapy detectable in the blood of the subject after initiation of administration of the T cell therapy;
(iv) at a time after a peak or maximum level of the cells of the T cell therapy are detectable in the blood of the subject, the number of cells of or derived from the T cells detectable in the blood from the subject is less than less than 10%, less than 5%, less than 1% or less than 0.1% of total peripheral blood mononuclear cells (PBMCs) in the blood of the subject;
(v) the subject exhibits disease progression and/or has relapsed following remission after treatment with the T cell therapy; and/or
(vi) the subject exhibits increased tumor burden as compared to tumor burden at a time prior to or after administration of the T cells and prior to initiation of administration of the immunomodulatory compound.

6. The T cell therapy and immunomodulatory compound for use of any of claims 1 and 3-5 or the immunomodulatory compound for use of any of claims 2-5, wherein:
(i) the immunomodulatory compound is administered for greater than or greater than about 7 consecutive days, greater than or greater than about 14 consecutive days, greater than or greater than about 21 consecutive days, greater than or greater than about 21 consecutive days, or greater than or greater than about 28 consecutive days; and/or
(ii) the immunomodulatory compound is administered in a cycle comprising administration daily for a plurality of consecutive days followed by a rest period during which the immunomodulatory compound is not administered, optionally wherein the rest period during which the immunomodulatory compound is not administered is greater than 7 consecutive days, greater than 14 consecutive days, greater than 21 days, or greater than 28 days; and/or
(iii) wherein the immunomodulatory compound is administered for at least 2 cycles, at least 3 cycles, at least 4 cycles, at least 5 cycles, at least 6 cycles, at least 7 cycles, at least 8 cycles, at least 9 cycles, at least 10 cycles, at least 11 cycles, or at least 12 cycles.

7. The T cell therapy and immunomodulatory compound for use of any of claims 1 and 3-6 or the immunomodulatory compound for use of any of claims 2-6, wherein the administration of the immunomodulatory compound is continued, from at least after initiation of administration of the T cells, until:
(i) the number of cells of or derived from the administered T cell therapy detectable in the blood from the subject is increased compared to in the subject at a preceding time point just prior to administration of the immunomodulatory compound or compared to a preceding time point after administration of the T-cell therapy;
(ii) the number of cells of the T cell therapy detectable in the blood from the subject is greater than or greater than about 10%, 15%, 20%, 30%, 40%, 50%, or 60% total peripheral blood mononuclear cells (PBMCs) in the blood of the subject;
(iii) the subject exhibits a reduction in tumor burden as compared to tumor burden at a time immediately prior to the administration of the T cell therapy or at a time immediately prior to the administration of the immunomodulatory compound; and/or
(iv) the subject exhibits complete or clinical remission.

8. The T cell therapy and immunomodulatory compound for use of any of claims 1 and 3-7 or the immunomodulatory compound for use of any of claims 2-7, wherein the immunomodulatory compound is a compound of the following formula: 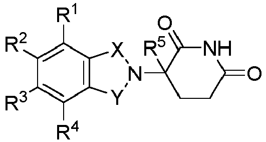 wherein
one of X and Y is -C(O)- and the other of X and Y is -C(O)- or -CH₂-;
(1) each of R¹, R², R³, and R⁴ are independently halo, alkyl of 1 to 4 carbon atoms, or alkoxy or 1 to 4 carbon atoms, or
(2) one of R¹, R³, R⁴, and R⁵ is -NHR^{a} and the remaining of R¹, R², R³, and R⁴ are hydrogen, wherein R^{a} is hydrogen or alkyl of 1 to 8 carbon atoms;
R⁵ is hydrogen or alkyl of 1 to 8 carbon atoms, benzyl, or halo;
provided that R⁵ is other than hydrogen if X and Y are -C(O)- and (i) each of R¹, R², R³, and R⁴ is fluoro; or (ii) one of R¹, R², R³, and R⁴ is amino;
or a pharmaceutically acceptable salt thereof.

9. The T cell therapy and immunomodulatory compound for use of any of claims 1 and 3-8 or the immunomodulatory compound for use of any of claims 2-8, wherein the immunomodulatory compound is a compound of the following formula: 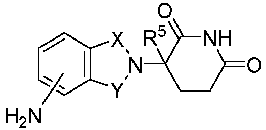 wherein one of X and Y is -C(O)- and the other of X and Y is -C(O)- or -CH₂-, and R⁵ is hydrogen or lower alkyl, or a pharmaceutically acceptable salt thereof.

10. The T cell therapy and immunomodulatory compound for use of any of claims 1 and 3-9 or the immunomodulatory compound for use of any of claims 2-9, wherein the immunomodulatory compound is lenalidomide (3-(4-amino-1-oxo-1,3-dihydro-2H-isoindol-2-yl)piperidine-2,6-dione), pomalidomide (4-amino-2-(2,6-dioxopiperidin-3-yl)isoindole-1,3-dione), or avadomide (3-(5-amino-2-methyl-4-oxo-4H-quinazolin-3-yl)-piperidine-2,6-dione), a stereoisomer of lenalidomide (3-(4-amino-1-oxo-1,3-dihydro-2H-isoindol-2-yl)piperidine-2,6-dione), pomalidomide (4-amino-2-(2,6-dioxopiperidin-3-yl)isoindole-1,3-dione), avadomide (3-(5-amino-2-methyl-4-oxo-4H-quinazolin-3-yl)-piperidine-2,6-dione), or a pharmaceutically acceptable salt, solvate, hydrate, co-crystal, clathrate, or polymorph thereof.

11. The T cell therapy and immunomodulatory compound for use of claim 10 or the immunomodulatory compound for use of claim 10, wherein the immunomodulatory compound is lenalidomide (3-(4-amino-1-oxo-1,3-dihydro-2H-isoindol-2-yl)piperidine-2,6-dione), or a stereoisomer thereof, or a pharmaceutically acceptable salt, solvate, hydrate, co-crystal, clathrate, or polymorph thereof.

12. The T cell therapy and immunomodulatory compound for use of claim 10 or the immunomodulatory compound for use of claim 10, wherein the immunomodulatory compound is lenalidomide (3-(4-amino-1-oxo-1,3-dihydro-2H-isoindol-2-yl)piperidine-2,6-dione).

13. The T cell therapy and immunomodulatory compound for use of claim 10 or the immunomodulatory compound for use of claim 10, wherein the immunomodulatory compound is avadomide (3-(5-amino-2-methyl-4-oxo-4H-quinazolin-3-yl)-piperidine-2,6-dione), or a stereoisomer thereof, or a pharmaceutically acceptable salt, solvate, hydrate, co-crystal, clathrate, or polymorph thereof.

14. The T cell therapy and immunomodulatory compound for use of claim 10 or the immunomodulatory compound for use of claim 10, wherein the immunomodulatory compound is avadomide (3-(5-amino-2-methyl-4-oxo-4H-quinazolin-3-yl)-piperidine-2,6-dione).

15. The T cell therapy and immunomodulatory compound for use of any of claims 1 and 3-7 or the immunomodulatory compound for use of any of claims 2-7, wherein the immunomodulatory compound is 3-[4-(4-morpholin-4-ylmethyl-benzyloxy)-1-oxo-1,3-dihydro-isoindol-2-yl]-piperidine-2,6-dione.

16. The T cell therapy and immunomodulatory compound for use of any of claims 1 and 3-15 or the immunomodulatory compound for use of any of claims 2-15, wherein the immunomodulatory compound is administered in a capsule or a tablet.

17. The T cell therapy and immunomodulatory compound for use of any of claims 1 and 3-16 or the immunomodulatory compound for use of any of claims 2-16, wherein the immunomodulatory compound is administered in an amount from 0.1 mg to 100 mg, from 0.1 mg to 50 mg, from 0.1 mg to 25 mg, from 0.1 mg to 10 mg, from 0.1 mg to 5 mg, from 0.1 mg to 1 mg, from 1 mg to 100 mg, from 1 mg to 50 mg, from 1 mg to 25 mg, from 1 mg to 10 mg, from 1 mg to 5 mg, from 5 mg to 100 mg, from 5 mg to 50 mg, from 5 mg to 25 mg, from 5 mg to 10 mg, from 10 mg to 100 mg, from 10 mg to 50 mg, from 10 mg to 25 mg, from 25 mg to 100 mg, from 25 mg to 50 mg or from 50 mg to 100 mg, each inclusive, wherein the amount is a once daily amount of the immunomodulatory compound.

18. The T cell therapy and immunomodulatory compound for use of any of claims 1 and 3-17 or the immunomodulatory compound for use of any of claims 2-17, wherein:
(i) the immunomodulatory compound is administered once daily, twice daily, three times daily, four times daily, five times daily, or six times daily; and/or
(ii) the immunomodulatory compound is administered at a total daily dosage amount of at least or at least about 0.1 mg per day, 0.5 mg per day, 1.0 mg per day, 2.5 mg per day, 5 mg per day, 10 mg per day, 25 mg per day, 50 mg per day or 100 mg per day; and/or
(iii) the immunomodulatory compound is administered in an amount greater than or greater than about 1 mg per day, 2.5 mg per day, 5 mg per day, 7.5 mg per day, 10 mg per day, 15 mg per day and less than 25 mg per day.

19. The T cell therapy and immunomodulatory compound for use of any of claims 1 and 3-18 or the immunomodulatory compound for use of any of claims 2-18, wherein:
(a) the antigen is selected from among ROR1, B cell maturation antigen (BCMA), carbonic anhydrase 9 (CAIX), Her2/neu (receptor tyrosine kinase erbB2), L1-CAM, CD19, CD20, CD22, mesothelin, CEA, and hepatitis B surface antigen, anti-folate receptor, CD23, CD24, CD30, CD33, CD38, CD44, EGFR, epithelial glycoprotein 2 (EPG-2), epithelial glycoprotein 40 (EPG-40), EPHa2, erb-B2, erb-B3, erb-B4, erbB dimers, EGFR vIII, folate binding protein (FBP), FCRL5, FCRH5, fetal acetylcholine receptor, GD2, GD3, HMW-MAA, IL-22R-alpha, IL-13R-alpha2, kinase insert domain receptor (kdr), kappa light chain, Lewis Y, L1-cell adhesion molecule, (L1-CAM), Melanoma-associated antigen (MAGE)-A1, MAGE-A3, MAGE-A6, Preferentially expressed antigen of melanoma (PRAME), survivin, TAG72, B7-H6, IL-13 receptor alpha 2 (IL-13Ra2), CA9, GD3, HMW-MAA, CD171, G250/CAIX, HLA-AI MAGE Al HLA-A2 NY-ESO-1, PSCA, folate receptor-a, CD44v6, CD44v7/8, avb6 integrin, 8H9, NCAM, VEGF receptors, 5T4, Fetal AchR, NKG2D ligands, CD44v6, dual antigen, a cancer-testes antigen, mesothelin, murine CMV, mucin 1 (MUC1), MUC16, PSCA, NKG2D, NY-ESO-1, MART-1, gp100, G Protein Coupled Receptor 5D (GPCR5D), oncofetal antigen, ROR1, TAG72, VEGF-R2, carcinoembryonic antigen (CEA), Her2/neu, estrogen receptor, progesterone receptor, ephrinB2, CD123, c-Met, GD-2, O-acetylated GD2 (OGD2), CE7, Wilms Tumor 1 (WT-1), a cyclin, cyclin A2, CCL-1, CD138, optionally a human antigen of any of the foregoing; a pathogen-specific antigen; and an antigen associated with a universal tag; or
(b) the antigen is or comprises a multiple myeloma-associated antigen.

20. The T cell therapy and immunomodulatory compound for use of any of claims 1 and 3-18 or the immunomodulatory compound for use of any of claims 2-18, wherein the antigen is or comprises CD19, optionally human CD19.

21. The T cell therapy and immunomodulatory compound for use of any of claims 1 and 3-18 or the immunomodulatory compound for use of any of claims 2-18, wherein the antigen is a BCMA, optionally human BCMA.

22. The T cell therapy and immunomodulatory compound for use of any of claims 1 and 3-21 or the immunomodulatory compound for use of any of claims 2-21, wherein:
the T cell therapy comprises T cells that are CD4+ or CD8+; and/or
the T cell therapy comprises primary cells derived from a subject; and/or
the T cell therapy comprises cells that are autologous to the subject or the T cell therapy comprises T cells that are allogeneic to the subject; and/or
the subject is a human; and/or
the T cell therapy comprises the administration of from or from about 1 × 10⁵ to 1 × 10⁸ total recombinant receptor-expressing cells, total T cells, or total peripheral blood mononuclear cells (PBMCs), from or from about 5 × 10⁵ to 1 × 10⁷ total recombinant receptor-expressing cells, total T cells, or total peripheral blood mononuclear cells (PBMCs) or from or from about 1 × 10⁶ to 1 × 10⁷ total recombinant receptor-expressing cells, total T cells, or total peripheral blood mononuclear cells (PBMCs), each inclusive.

23. The T cell therapy and immunomodulatory compound for use of any of claims 1 and 3-22 or the immunomodulatory compound for use of any of claims 2-22, wherein the method further comprises administering a lymphodepleting chemotherapy prior to administration of the T cell therapy.

24. The T cell therapy and immunomodulatory compound for use of any of claims 1 and 3-23 or the immunomodulatory compound for use of any of claims 2-23, wherein:
(i) the cancer is a B cell malignancy and/or a myeloma, lymphoma or leukemia; and/or
the cancer is mantle cell lymphoma (MCL), multiple myeloma (MM), acute lymphoblastic leukemia (ALL), adult ALL, chronic lymphoblastic leukemia (CLL), non-Hodgkin lymphoma (NHL), Diffuse Large B-Cell Lymphoma (DLBCL) or follicular lymphoma (FL); or
(ii) the cancer is a non-hematological cancer or is a solid tumor.

25. The T cell therapy and immunomodulatory compound for use of any of claims 1 and 3-24 or the immunomodulatory compound for use of any of claims 2-24, wherein;
(i) the T cell therapy exhibits increased or prolonged expansion and/or persistence in the subject as compared to a method in which the T cell therapy is administered to the subject in the absence of the immunomodulatory compound; and/or
(ii) the method reduces tumor burden to a greater degree and/or for a greater period of time as compared to the reduction that would be observed with a comparable method in which the T cell therapy is administered to the subject in the absence of the immunomodulatory compound and/or in which the immunomodulatory compound is administered in the absence of the T cell therapy, optionally at the same dose or dosing schedule.

## Patentansprüche

1. T-Zelltherapie und immunmodulatorische Verbindung zur Verwendung in einem Verfahren zur Behandlung von Krebs, wobei das Verfahren umfasst:
(a) Verabreichen einer T-Zelltherapie an ein Individuum mit einer Krankheit oder einem Leiden, bei der bzw. dem es sich um Krebs handelt, wobei die T-Zelltherapie aus gentechnisch veränderten Zellen besteht oder diese umfasst, die einen rekombinanten Rezeptor exprimieren, der spezifisch an ein Antigen bindet, wobei es sich bei dem rekombinanten Rezeptor um einen chimären Antigenrezeptor (CAR) handelt, und wobei der CAR eine extrazelluläre Domäne, die eine Antigenbindende Domäne umfasst, die spezifisch an ein Tumorantigen bindet, einen Spacer, und eine intrazelluläre Signalregion umfasst; und
(b) Verabreichen einer immunmodulatorischen Verbindung an das Individuum, wobei es sich bei der immunmodulatorischen Verbindung um Thalidomid (2-(2,6-Dioxopiperidin-3-yl)-1H-Isoindol-1,3(2H)-Dion) oder ein Analog oder Derivat von Thalidomid handelt,
und wobei die immunmodulatorische Verbindung:
(i) an Cereblon (CRBN) und/oder den CRBN-E3-Ubiquitin-Ligase-Komplex bindet, und
(ii) den Abbau von sowohl Ikaros (IKZF1) als auch Aiolos (IKZF3) fördert;
wobei der Beginn der Verabreichung der immunmodulatorischen Verbindung 7 bis 21 Tage nach Beginn der Verabreichung der T-Zelltherapie durchgeführt wird, und
wobei die immunmodulatorische Verbindung oral verabreicht wird.

2. Immunmodulatorische Verbindung zur Verwendung in einem Verfahren zur Behandlung von Krebs, wobei das Verfahren Verabreichen einer immunmodulatorischen Verbindung an ein Individuum mit einer Krankheit oder einem Leiden, bei der bzw. dem es sich um Krebs handelt, umfasst, wobei, zum Zeitpunkt des Beginns der Verabreichung der immunmodulatorischen Verbindung, dem Individuum zuvor eine T-Zelltherapie zur Behandlung der Krankheit oder des Leidens verabreicht wurde, wobei die T-Zelltherapie aus gentechnisch veränderten Zellen besteht oder diese umfasst, die einen rekombinanten Rezeptor exprimieren, der spezifisch an ein Antigen bindet, wobei es sich bei dem rekombinanten Rezeptor um einen chimären Antigenrezeptor (CAR) handelt, und wobei der CAR eine extrazelluläre Domäne, die eine Antigenbindende Domäne umfasst, die spezifisch an ein Tumorantigen bindet, einen Spacer, und eine intrazelluläre Signalregion umfasst,
wobei es sich bei der immunmodulatorischen Verbindung um Thalidomid (2- (2, 6-Dioxopiperidin-3-yl)-1H-Isoindol-1,3 (2H)-Dion) oder ein Analog oder Derivat von Thalidomid handelt,
und wobei die immunmodulatorische Verbindung:
(i) an Cereblon (CRBN) und/oder den CRBN-E3-Ubiquitin-Ligase-Komplex bindet, und
(ii) den Abbau von sowohl Ikaros (IKZF1) als auch Aiolos (IKZF3) fördert; und
wobei der Beginn der Verabreichung der immunmodulatorischen Verbindung 7 bis 21 Tage nach Beginn der Verabreichung der T-Zelltherapie durchgeführt wird, und
wobei die immunmodulatorische Verbindung oral verabreicht wird.

3. T-Zelltherapie und immunmodulatorische Verbindung zur Verwendung nach Anspruch 1 oder immunmodulatorische Verbindung zur Verwendung nach Anspruch 2, wobei der Beginn der Verabreichung der immunmodulatorischen Verbindung zu einem Zeitpunkt durchgeführt wird, der später ist als 14 Tage, 15 Tage, 16 Tage, 17 Tage, 18 Tage, 19 Tage, oder 20 Tage nach Beginn der Verabreichung der T-Zelltherapie.

4. T-Zelltherapie und immunmodulatorische Verbindung zur Verwendung nach Anspruch 1 oder Anspruch 3, oder immunmodulatorische Verbindung zur Verwendung nach Anspruch 2 oder Anspruch 3, wobei die immunmodulatorische Verbindung an ein Individuum verabreicht wird, innerhalb einer Woche, wie z. B. innerhalb von 1, 2 oder 3 Tagen, nachdem:
(i) Spitzen- oder maximale Niveaus der Zellen der T-Zelltherapie im Blut des Individuums nachweisbar sind;
(ii) die Anzahl von im Blut nachweisbaren Zellen der T-Zelltherapie, nachdem sie im Blut nachweisbar war, nicht nachweisbar oder verringert ist, wahlweise verringert verglichen mit einem vorausgehenden Zeitpunkt nach Verabreichung der T-Zelltherapie;
(iii) die Anzahl von im Blut nachweisbaren Zellen der T-Zelltherapie um oder um mehr als das 1,5-fache, 2-fache, 3-fache, 4-fache, 5-fache, 10-fache oder mehr des Spitzenwerts oder der maximalen Anzahl von im Blut des Individuums nach Beginn der Verabreichung der T-Zelltherapie nachweisbaren Zellen der T-Zelltherapie verringert ist;
(iv) zu einem Zeitpunkt nachdem ein Spitzen- oder maximales Niveau der Zellen der T-Zelltherapie im Blut des Individuums nachweisbar war, die Anzahl der im Blut von dem Individuum nachweisbaren T-Zellen oder davon abgeleiteten Zellen weniger als 10 %, weniger als 5 %, weniger als 1 % oder weniger als 0,1 % der gesamten peripheren mononukleären Blutzellen (PBMCs) im Blut des Individuums beträgt;
(v) das Individuum Krankheitsfortschritt zeigt und/oder nach Remission nach Behandlung mit der T-Zelltherapie einen Rückfall erlitten hat; und/oder
(vi) das Individuum erhöhte Tumorlast zeigt verglichen mit Tumorlast zu einem Zeitpunkt vor oder nach Verabreichung der T-Zellen und vor Beginn der Verabreichung der immunmodulatorischen Verbindung.

5. T-Zelltherapie und immunmodulatorische Verbindung zur Verwendung nach einem der Ansprüche 1 und 3-4 oder immunmodulatorische Verbindung zur Verwendung nach einem der Ansprüche 2-4, wobei das Verfahren, vor dem Beginn der Verabreichung der immunmodulatorischen Verbindung, Auswählen eines Individuums umfasst, bei dem:
(i) Spitzen- oder maximale Niveaus der Zellen der T-Zelltherapie im Blut des Individuums nachweisbar sind;
(ii) die Anzahl von im Blut nachweisbaren Zellen der T-Zelltherapie, nachdem sie im Blut nachweisbar war, nicht nachweisbar oder verringert ist, wahlweise verringert verglichen mit einem vorausgehenden Zeitpunkt nach Verabreichung der T-Zelltherapie;
(iii) die Anzahl von im Blut nachweisbaren Zellen der T-Zelltherapie um oder um mehr als das 1,5-fache, 2-fache, 3-fache, 4-fache, 5-fache, 10-fache oder mehr des Spitzenwerts oder der maximalen Anzahl von im Blut des Individuums nach Beginn der Verabreichung der T-Zelltherapie nachweisbaren Zellen der T-Zelltherapie verringert ist;
(iv) zu einem Zeitpunkt nachdem ein Spitzen- oder maximales Niveau der Zellen der T-Zelltherapie im Blut des Individuums nachweisbar war, die Anzahl der im Blut von dem Individuum nachweisbaren T-Zellen oder davon abgeleiteten Zellen weniger als 10 %, weniger als 5 %, weniger als 1 % oder weniger als 0,1 % der gesamten peripheren mononukleären Blutzellen (PBMCs) im Blut des Individuums beträgt;
(v) das Individuum Krankheitsfortschritt zeigt und/oder nach Remission nach Behandlung mit der T-Zelltherapie einen Rückfall erlitten hat; und/oder
(vi) das Individuum erhöhte Tumorlast zeigt verglichen mit Tumorlast zu einem Zeitpunkt vor oder nach Verabreichung der T-Zellen und vor Beginn der Verabreichung der immunmodulatorischen Verbindung.

6. T-Zelltherapie und immunmodulatorische Verbindung zur Verwendung nach einem der Ansprüche 1 und 3-5 oder immunmodulatorische Verbindung zur Verwendung nach einem der Ansprüche 2-5, wobei:
(i) die immunmodulatorische Verbindung für mehr als oder mehr als etwa 7 aufeinanderfolgende Tage, mehr als oder mehr als etwa 14 aufeinanderfolgende Tage, mehr als oder mehr als etwa 21 aufeinanderfolgende Tage, mehr als oder mehr als etwa 28 aufeinanderfolgende Tage verabreicht wird; und/oder
(ii) die immunmodulatorische Verbindung in einem Zyklus verabreicht wird, der tägliche Verabreichung für eine Vielzahl von aufeinanderfolgenden Tagen umfasst, gefolgt von einer Ruheperiode, während der die immunmodulatorische Verbindung nicht verabreicht wird, wahlweise wobei die Ruheperiode, während der die immunmodulatorische Verbindung nicht verabreicht wird, länger als 7 aufeinanderfolgende Tage, länger als 14 aufeinanderfolgende Tage, länger als 21 Tage, oder länger als 28 Tage ist; und/oder
(iii) wobei die immunmodulatorische Verbindung für wenigstens 2 Zyklen, wenigstens 3 Zyklen, wenigstens 4 Zyklen, wenigstens 5 Zyklen, wenigstens 6 Zyklen, wenigstens 7 Zyklen, wenigstens 8 Zyklen, wenigstens 9 Zyklen, wenigstens 10 Zyklen, wenigstens 11 Zyklen, oder wenigstens 12 Zyklen verabreicht wird.

7. T-Zelltherapie und immunmodulatorische Verbindung zur Verwendung nach einem der Ansprüche 1 und 3-6 oder immunmodulatorische Verbindung zur Verwendung nach einem der Ansprüche 2-6, wobei die Verabreichung der immunmodulatorischen Verbindung fortgesetzt wird, von wenigstens nach Beginn der Verabreichung der T-Zellen, bis:
(i) die Anzahl von im Blut von dem Individuum nachweisbaren Zellen der verabreichten T-Zelltherapie oder daraus abgeleiteten Zellen erhöht ist, verglichen mit bei dem Individuum zu einem vorausgehenden Zeitpunkt gerade bevor der Verabreichung der immunmodulatorischen Verbindung oder verglichen mit einem vorausgehenden Zeitpunkt nach Verabreichung der T-Zelltherapie;
(ii) die Anzahl von im Blut von dem Individuum nachweisbaren Zellen der T-Zelltherapie größer ist als oder größer ist als etwa 10 %, 15 %, 20 %, 30 %, 40 %, 50 %, oder 60 % der gesamten peripheren mononukleären Blutzellen (PBMCs) im Blut des Individuums;
(iii) das Individuum erhöhte Tumorlast zeigt, verglichen mit Tumorlast zu einem Zeitpunkt unmittelbar vor der Verabreichung der T-Zelltherapie oder zu einem Zeitpunkt unmittelbar vor der Verabreichung der immunmodulatorischen Verbindung; und/oder
(iv) das Individuum vollständige oder klinische Remission zeigt.

8. T-Zelltherapie und immunmodulatorische Verbindung zur Verwendung nach einem der Ansprüche 1 und 3-7 oder immunmodulatorische Verbindung zur Verwendung nach einem der Ansprüche 2-7, wobei die immunmodulatorische Verbindung eine Verbindung der folgenden Formel ist: 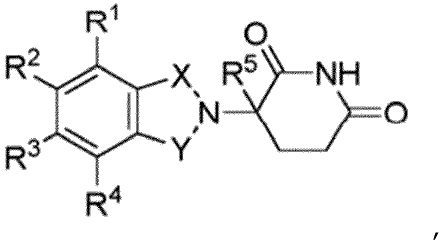 wobei
eines von X und Y -C(O)- und das andere von X und Y -C(O)- oder -CH₂- ist;
(1) jedes von R¹, R², R³, und R⁴ unabhängig voneinander Halo, Alkyl mit 1 bis 4 Kohlenstoffatomen, oder Alkoxy mit 1 bis 4 Kohlenstoffatomen ist, oder
(2) eines von R¹, R³, R⁴, und R⁵ -NHR^{a} ist, und die übrigen von R¹, R², R³, und R⁴ Wasserstoff sind, wobei R^{a} Wasserstoff oder Alkyl mit 1 bis 8 Kohlenstoffatomen ist;
R⁵ Wasserstoff oder Alkyl mit 1 bis 8 Kohlenstoffatomen, Benzyl oder Halo ist;
vorausgesetzt, dass R⁵ etwas anderes als Wasserstoff ist, wenn X und Y -C(O)- sind und (i) jedes von R¹, R², R³, und R⁴ Fluor ist; oder (ii) eines von R¹, R², R³, und R⁴ Amino ist;
oder ein pharmazeutisch akzeptables Salz davon ist.

9. T-Zelltherapie und immunmodulatorische Verbindung zur Verwendung nach einem der Ansprüche 1 und 3-8 oder immunmodulatorische Verbindung zur Verwendung nach einem der Ansprüche 2-8, wobei die immunmodulatorische Verbindung eine Verbindung der folgenden Formel ist: 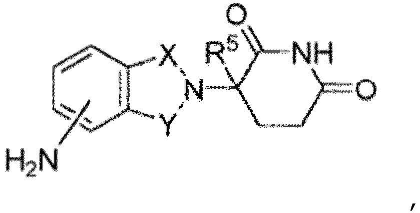 wobei eines von X und Y -C(O)- ist und das andere von X und Y -C(O)- oder -CH₂- ist, und R⁵ Wasserstoff oder ein niederes Alkyl ist, oder ein pharmazeutisch akzeptables Salz davon ist.

10. T-Zelltherapie und immunmodulatorische Verbindung zur Verwendung nach einem der Ansprüche 1 und 3-9 oder immunmodulatorische Verbindung zur Verwendung nach einem der Ansprüche 2-9, wobei die immunmodulatorische Verbindung Lenalidomid (3-(4-Amino-1-oxo-1,3-dihydro-2H-isoindol-2-yl)piperidin-2,6-dion), Pomalidomid (4-Amino-2-(2,6-dioxopiperidin-3-yl)isoindol-1,3-dion), oder Avadomid (3-(5-Amino-2-methyl-4-oxo-4H-quinazolin-3-yl)-piperidin-2,6-dion), ein Stereoisomer von Lenalidomid (3-(4-Amino-1-oxo-1,3-dihydro-2H-isoindol-2-yl)piperidin-2,6-dion), Pomalidomid (4-Amino-2-(2,6-dioxopiperidin-3-yl)isoindol-1,3-dion), oder Avadomid (3-(5-Amino-2-methyl-4-oxo-4H-quinazolin-3-yl)-piperidin-2,6-dion), oder ein pharmazeutisch akzeptables Salz, Solvat, Hydrat, Co-Kristall, Clathrat, oder Polymorph davon ist.

11. T-Zelltherapie und immunmodulatorische Verbindung zur Verwendung nach Anspruch 10 oder immunmodulatorische Verbindung zur Verwendung nach Anspruch 10, wobei die immunmodulatorische Verbindung Lenalidomid (3-(4-Amino-1-oxo-1,3-dihydro-2H-isoindol-2-yl)piperidin-2,6-dion), oder ein Stereoisomer davon, oder ein pharmazeutisch akzeptables Salz, Solvat, Hydrat, Co-Kristall, Clathrat, oder Polymorph davon ist.

12. T-Zelltherapie und immunmodulatorische Verbindung zur Verwendung nach Anspruch 10 oder immunmodulatorische Verbindung zur Verwendung nach Anspruch 10, wobei die immunmodulatorische Verbindung Lenalidomid (3-(4-Amino-1-oxo-1,3-dihydro-2H-isoindol-2-yl)piperidin-2,6-dion) ist.

13. T-Zelltherapie und immunmodulatorische Verbindung zur Verwendung nach Anspruch 10 oder immunmodulatorische Verbindung zur Verwendung nach Anspruch 10, wobei die immunmodulatorische Verbindung Avadomid (3-(5-Amino-2-methyl-4-oxo-4H-quinazolin-3-yl)-piperidin-2,6-dion), oder ein Stereoisomer davon, oder ein pharmazeutisch akzeptables Salz, Solvat, Hydrat, Co-Kristall, Clathrat, oder Polymorph davon ist.

14. T-Zelltherapie und immunmodulatorische Verbindung zur Verwendung nach Anspruch 10 oder immunmodulatorische Verbindung zur Verwendung nach Anspruch 10, wobei die immunmodulatorische Verbindung Avadomid (3-(5-Amino-2-methyl-4-oxo-4H-quinazolin-3-yl)-piperidin-2,6-dion) ist.

15. T-Zelltherapie und immunmodulatorische Verbindung zur Verwendung nach einem der Ansprüche 1 und 3-7 oder immunmodulatorische Verbindung zur Verwendung nach einem der Ansprüche 2-7, wobei die immunmodulatorische Verbindung 3-[4-(4-Morpholin-4-ylmethy]-benzyloxy)-1-oxo-1,3-dihydro-isoindol-2-yl]-piperidin-2,6-dion ist.

16. T-Zelltherapie und immunmodulatorische Verbindung zur Verwendung nach einem der Ansprüche 1 und 3-15 oder immunmodulatorische Verbindung zur Verwendung nach einem der Ansprüche 2-15, wobei die immunmodulatorische Verbindung in einer Kapsel oder einer Tablette verabreicht wird.

17. T-Zelltherapie und immunmodulatorische Verbindung zur Verwendung nach einem der Ansprüche 1 und 3-16 oder immunmodulatorische Verbindung zur Verwendung nach einem der Ansprüche 2-16, wobei die immunmodulatorische Verbindung in einer Menge von 0,1 mg bis 100 mg, von 0,1 mg bis 50 mg, von 0,1 mg bis 25 mg, von 0,1 mg bis 10 mg, von 0,1 mg bis 5 mg, von 0,1 mg bis 1 mg, von 1 mg bis 100 mg, von 1 mg bis 50 mg, von 1 mg bis 25 mg, von 1 mg bis 10 mg, von 1 mg bis 5 mg, von 5 mg bis 100 mg, von 5 mg bis 50 mg, von 5 mg bis 25 mg, von 5 mg bis 10 mg, von 10 mg bis 100 mg, von 10 mg bis 50 mg, von 10 mg bis 25 mg, von 25 mg bis 100 mg, von 25 mg bis 50 mg oder von 50 mg bis 100 mg, jeweils einschließlich, verabreicht wird, wobei die Menge eine einmal tägliche Menge der immunmodulatorischen Verbindung ist.

18. T-Zelltherapie und immunmodulatorische Verbindung zur Verwendung nach einem der Ansprüche 1 und 3-17 oder immunmodulatorische Verbindung zur Verwendung nach einem der Ansprüche 2-17, wobei:
(i) die immunmodulatorische Verbindung einmal täglich, zweimal täglich, dreimal täglich, viermal täglich, fünfmal täglich, oder sechsmal täglich verabreicht wird; und/oder
(ii) die immunmodulatorische Verbindung in einer täglichen Gesamtdosismenge von wenigstens oder wenigstens etwa 0,1 mg pro Tag, 0,5 mg pro Tag, 1,0 mg pro Tag, 2,5 mg pro Tag, 5 mg pro Tag, 10 mg pro Tag, 25 mg pro Tag, 50 mg pro Tag oder 100 mg pro Tag verabreicht wird; und/oder
(iii) die immunmodulatorische Verbindung in einer Menge von mehr als oder mehr als etwa 1 mg pro Tag, 2,5 mg pro Tag, 5 mg pro Tag, 7,5 mg pro Tag, 10 mg pro Tag, 15 mg pro Tag und weniger als 25 mg pro Tag verabreicht wird.

19. T-Zelltherapie und immunmodulatorische Verbindung zur Verwendung nach einem der Ansprüche 1 und 3-18 oder immunmodulatorische Verbindung zur Verwendung nach einem der Ansprüche 2-18, wobei:
(a) das Antigen ausgewählt ist aus ROR1, B-Zell-Reifungsantigen (BCMA), Carboanhydrase 9 (CAIX), Her2/neu (Rezeptortyrosinkinase ErbB2), L1-CAM, CD19, CD20, CD22, Mesothelin, CEA, und Hepatitis-B-Oberflächenantigen, Anti-Folat-Rezeptor, CD23, CD24, CD30, CD33, CD38, CD44, EGFR, epithelialem Glykoprotein 2 (EPG-2), epithelialem Glykoprotein 40 (EPG-40), EPHa2, Erb-B2, Erb-B3, Erb-B4, ErbB-Dimeren, EGFR vIII, Folat-bindendem Protein (FBP), FCRL5, FCRH5, fötalem Acetylcholinrezeptor, GD2, GD3, HMW-MAA, IL-22R-alpha, IL-13R-alpha2, Kinase-Insertdomäne-Rezeptor (KDR), Kappa-leichte-Kette, Lewis Y, L1-Zell-Adhäsionsmolekül, (L1-CAM), Melanoma-assoziiertem Antigen (MAGE)-Al, MAGE-A3, MAGE-A6, Preferentially Expressed Antigen of Melanoma (PRAME), Survivin, TAG72, B7-H6, IL-13-Rezeptor alpha 2 (IL-13Ra2), CA9, GD3, HMW-MAA, CD171, G250/CAIX, HLA-AI MAGE Al, HLA-A2 NY-ESO-1, PSCA, Folatrezeptor-a, CD44v6, CD44v7/8, avb6-Integrin, 8H9, NCAM, VEGF-Rezeptoren, 5T4, fötalem AChR, NKG2D-Liganden, CD44v6, dualem Antigen, einem Cancer-Testis-Antigen, Mesothelin, murinem CMV, Mucin 1 (MUC1), MUC16, PSCA, NKG2D, NY-ESO-1, MART-1, gp100, G-Proteingekoppeltem Rezeptor 5D (GPCR5D), onkofetalem Antigen, ROR1, TAG72, VEGF-R2, Carcinoembryonalem Antigen (CEA), Her2/neu, Östrogenrezeptor, Progesteronrezeptor, EphrinB2, CD123, c-Met, GD-2, O-acetyliertem GD2 (OGD2), CE7, Wilms-Tumor-1 (WT-1), einem Cyclin, Cyclin A2, CCL-1, CD138, wahlweise einem menschlichen Antigen von einem der vorstehenden; einem pathogenspezifischen Antigen, und einem mit einer universellen Markierung verbundenen Antigen, oder
(b) das Antigen ein Multiples-Myelom-assoziiertes Antigen ist oder umfasst.

20. T-Zelltherapie und immunmodulatorische Verbindung zur Verwendung nach einem der Ansprüche 1 und 3-18 oder immunmodulatorische Verbindung zur Verwendung nach einem der Ansprüche 2-18, wobei das Antigen CD19, wahlweise menschliches CD19, ist oder umfasst.

21. T-Zelltherapie und immunmodulatorische Verbindung zur Verwendung nach einem der Ansprüche 1 und 3-18 oder immunmodulatorische Verbindung zur Verwendung nach einem der Ansprüche 2-18, wobei das Antigen BCMA, wahlweise menschliches BCMA, ist oder umfasst.

22. T-Zelltherapie und immunmodulatorische Verbindung zur Verwendung nach einem der Ansprüche 1 und 3-21 oder immunmodulatorische Verbindung zur Verwendung nach einem der Ansprüche 2-21, wobei:
die T-Zelltherapie T-Zellen umfasst, die CD4+ oder CD8+ sind; und/oder
die T-Zelltherapie Primärzellen umfasst, die aus einem Individuum stammen; und/oder
die T-Zelltherapie Zellen umfasst, die autolog gegenüber dem Individuum sind, oder die T-Zelltherapie T-Zellen umfasst, die allogen gegenüber dem Individuum sind; und/oder
das Individuum ein Mensch ist; und/oder
die T-Zelltherapie die Verabreichung von oder von etwa 1 × 10⁵ bis 1 × 10⁸ der gesamten rekombinanten Rezeptor-exprimierenden Zellen, der gesamten T-Zellen, oder der gesamten peripheren mononukleären Blutzellen (PBMCs), von oder von etwa 5 × 10⁵ bis 1 × 10⁷ der gesamten rekombinanten Rezeptor-exprimierenden Zellen, der gesamten T-Zellen, oder der gesamten peripheren mononukleären Blutzellen (PBMCs), oder von 1 × 10⁶ bis 1 × 10⁷ der gesamten rekombinanten Rezeptor-exprimierenden Zellen, der gesamten T-Zellen, oder der gesamten peripheren mononukleären Blutzellen (PBMCs), jeweils einschließlich, umfasst.

23. T-Zelltherapie und immunmodulatorische Verbindung zur Verwendung nach einem der Ansprüche 1 und 3-22 oder immunmodulatorische Verbindung zur Verwendung nach einem der Ansprüche 2-22, wobei das Verfahren ferner Verabreichen einer lymphodepletierenden Chemotherapie vor Verabreichen der T-Zelltherapie umfasst.

24. T-Zelltherapie und immunmodulatorische Verbindung zur Verwendung nach einem der Ansprüche 1 und 3-23 oder immunmodulatorische Verbindung zur Verwendung nach einem der Ansprüche 2-23, wobei:
(i) der Krebs ein B-Zell-Malignom und/oder ein Myelom, Lymphom oder Leukämie ist;
und/oder
der Krebs Mantelzell-Lymphom (MCL), multiples Myelom (MM), akute lymphoblastische Leukämie (ALL), adulte ALL, chronische lymphoblastische Leukämie (CLL), Non-Hodgkin-Lymphom (NHL), diffuses großzelliges B-Zell-Lymphom (DLBCL) oder follikuläres Lymphom (FL) ist; oder
(ii) der Krebs ein nicht-hämatologischer Krebs ist oder ein solider Tumor ist.

25. T-Zelltherapie und immunmodulatorische Verbindung zur Verwendung nach einem der Ansprüche 1 und 3-24 oder immunmodulatorische Verbindung zur Verwendung nach einem der Ansprüche 2-24, wobei:
(i) die T-Zelltherapie erhöhte oder verlängerte Expansion und/oder Persistenz bei dem Individuum zeigt, verglichen mit einem Verfahren, bei dem die T-Zelltherapie dem Individuum in der Abwesenheit der immunmodulatorischen Verbindung verabreicht wird; und/oder
(ii) das Verfahren Tumorlast zu einem größeren Ausmaß und/oder für einen längeren Zeitraum verringert, verglichen mit der Verringerung, die mit einem vergleichbaren Verfahren beobachtet würde, bei dem die T-Zelltherapie dem Individuum in der Abwesenheit der immunmodulatorischen Verbindung verabreicht wird, und/oder bei dem die immunmodulatorische Verbindung in der Abwesenheit der T-Zelltherapie verabreicht wird, wahlweise in derselben Dosis oder demselben Dosierungsschema.

## Revendications

1. Thérapie par cellules T et composé immunomodulateur pour une utilisation dans un procédé de traitement d'un cancer, le procédé comprenant :
(a) une administration d'une thérapie par cellules T à un sujet atteint d'une maladie ou d'une affection qui est un cancer, la thérapie par cellules T étant ou comprenant des cellules génétiquement modifiées exprimant un récepteur recombinant qui se lie spécifiquement à un antigène, le récepteur recombinant étant un récepteur antigénique chimérique (CAR), et le CAR comprenant un domaine extracellulaire comprenant un domaine de liaison à un antigène qui se lie spécifiquement à un antigène tumoral, un espaceur, et une région de signalisation intracellulaire ; et
(b) une administration au sujet d'un composé immunomodulateur, ledit composé immunomodulateur étant le thalidomide (2-(2,6-dioxopipéridin-3-yl)-1H-isoindole-1,3(2H)-dione) ou un analogue ou dérivé de thalidomide,
et le composé immunomodulateur :
(i) se liant à céréblon (CRBN) et/ou au complexe ubiquitine-ligase E3 de CRBN, et
(ii) augmentant la dégradation à la fois d'Ikaros (IKZF1) et d'Aiolos (IKZF3) ;
l'initiation de l'administration du composé immunomodulateur étant réalisée 7 à 21 jours après l'initiation de l'administration de la thérapie par cellules T, et
le composé immunomodulateur étant administré par voie orale.

2. Composé immunomodulateur pour une utilisation dans un procédé de traitement d'un cancer, le procédé comprenant une administration d'un composé immunomodulateur à un sujet atteint d'une maladie ou affection qui est un cancer, au moment de l'initiation de l'administration du composé immunomodulateur, le sujet ayant subi précédemment une administration d'une thérapie par cellules T pour le traitement de la maladie ou de l'affection, la thérapie par cellules T étant ou comprenant des cellules génétiquement modifiées exprimant un récepteur recombinant qui se lie spécifiquement à un antigène, le récepteur recombinant étant un récepteur antigénique chimérique (CAR), et le CAR comprenant un domaine extracellulaire comprenant un domaine de liaison à un antigène qui se lie spécifiquement à un antigène tumoral, un espaceur, et une région de signalisation intracellulaire,
ledit composé immunomodulateur étant le thalidomide (2-(2,6-dioxopipéridin-3-yl)-1H-isoindole-1,3(2H)-dione) ou un analogue ou dérivé de thalidomide,
et le composé immunomodulateur :
(i) se liant à céréblon (CRBN) et/ou au complexe ubiquitine-ligase E3 de CRBN, et
(ii) augmentant la dégradation à la fois d'Ikaros (IKZF1) et d'Aiolos (IKZF3) ; et
l'initiation de l'administration du composé immunomodulateur étant réalisée 7 à 21 jours après l'initiation de l'administration de la thérapie par cellules T, et
le composé immunomodulateur étant administré par voie orale.

3. Thérapie par cellules T et composé immunomodulateur pour une utilisation selon la revendication 1 ou composé immunomodulateur pour une utilisation selon la revendication 2, l'initiation de l'administration du composé immunomodulateur étant réalisée à un moment qui est supérieur à 14 jours, 15 jours, 16 jours, 17 jours, 18 jours, 19 jours, ou 20 jours après l'initiation de l'administration de la thérapie par cellules T.

4. Thérapie par cellules T et composé immunomodulateur pour une utilisation selon la revendication 1 ou la revendication 3 ou composé immunomodulateur pour une utilisation selon la revendication 2 ou la revendication 3, le composé immunomodulateur étant administré à un sujet sous une semaine, tel que dans les 1, 2 ou 3 jours après que :
(i) un taux pic ou maximal des cellules de la thérapie par cellules T est détectable dans le sang du sujet ;
(ii) le nombre de cellules de la thérapie par cellules T détectables dans le sang, après avoir été détectable dans le sang, n'est pas détectable ou est réduit, éventuellement réduit par comparaison avec un moment précédent après une administration de la thérapie par cellules T ;
(iii) le nombre de cellules de la thérapie par cellules T détectables dans le sang est diminué de ou de plus de 1,5 fois, 2,0 fois, 3,0 fois, 4,0 fois, 5,0 fois, 10,0 fois ou plus le nombre pic ou maximal de cellules de la thérapie par cellules T détectables dans le sang du sujet après l'initiation de l'administration de la thérapie par cellules T ;
(iv) à un moment après qu'un taux pic ou maximal des cellules de la thérapie par cellules T est détectable dans le sang du sujet, le nombre de cellules de, ou issues des cellules T détectables dans le sang du sujet est inférieur à inférieur à 10 %, inférieur à 5 %, inférieur à 1 % ou inférieur à 0,1 % de cellules mononucléaires de sang périphérique totales (PBMC) dans le sang du sujet ;
(v) le sujet présente une progression de la maladie et/ou a rechuté à la suite d'une rémission après un traitement avec la thérapie par cellules T ; et/ou
(vi) le sujet présente une charge tumorale augmentée par comparaison avec la charge tumorale à un moment avant ou après une administration des cellules T et avant l'initiation de l'administration du composé immunomodulateur.

5. Thérapie par cellules T et composé immunomodulateur pour une utilisation selon l'une quelconque des revendications 1 et 3-4 ou composé immunomodulateur pour une utilisation selon l'une quelconque des revendications 2-4, le procédé comprenant, avant l'initiation de l'administration du composé immunomodulateur, la sélection d'un sujet dans lequel :
(i) un taux pic ou maximal des cellules de la thérapie par cellules T est détectable dans le sang du sujet ;
(ii) le nombre de cellules de la thérapie par cellules T détectables dans le sang, après avoir été détectable dans le sang, n'est pas détectable ou est réduit, éventuellement réduit par comparaison avec un moment précédent après une administration de la thérapie par cellules T ;
(iii) le nombre de cellules de la thérapie par cellules T détectables dans le sang est diminué de ou de plus de 1,5 fois, 2,0 fois, 3,0 fois, 4,0 fois, 5,0 fois, 10,0 fois ou plus le nombre pic ou maximal de cellules de la thérapie par cellules T détectables dans le sang du sujet après l'initiation de l'administration de la thérapie par cellules T ;
(iv) à un moment après qu'un taux pic ou maximal des cellules de la thérapie par cellules T est détectable dans le sang du sujet, le nombre de cellules de, ou issues des cellules T détectables dans le sang du sujet est inférieur à inférieur à 10 %, inférieur à 5 %, inférieur à 1 % ou inférieur à 0,1 % de cellules mononucléaires de sang périphérique totales (PBMC) dans le sang du sujet ;
(v) le sujet présente une progression de la maladie et/ou a rechuté à la suite d'une rémission après un traitement avec la thérapie par cellules T ; et/ou
(vi) le sujet présente une charge tumorale augmentée par comparaison avec la charge tumorale à un moment avant ou après une administration des cellules T et avant l'initiation de l'administration du composé immunomodulateur.

6. Thérapie par cellules T et composé immunomodulateur pour une utilisation selon l'une quelconque des revendications 1 et 3-5 ou composé immunomodulateur pour une utilisation selon l'une quelconque des revendications 2-5,
(i) le composé immunomodulateur étant administré pendant plus de ou plus d'environ 7 jours consécutifs, plus de ou plus d'environ 14 jours consécutifs, plus de ou plus d'environ 21 jours consécutifs, plus de ou plus d'environ 21 jours consécutifs, ou plus de ou plus d'environ 28 jours consécutifs ; et/ou
(ii) le composé immunomodulateur étant administré dans un cycle comprenant une administration quotidienne pendant une pluralité de jours consécutifs suivie par une période de repos pendant laquelle le composé immunomodulateur n'est pas administré, éventuellement la période de repos pendant laquelle le composé immunomodulateur n'est pas administré étant supérieure à 7 jours consécutifs, supérieure à 14 jours consécutifs, supérieure à 21 jours, ou supérieure à 28 jours; et/ou
(iii) le composé immunomodulateur étant administré pendant au moins 2 cycles, au moins 3 cycles, au moins 4 cycles, au moins 5 cycles, au moins 6 cycles, au moins 7 cycles, au moins 8 cycles, au moins 9 cycles, au moins 10 cycles, au moins 11 cycles, ou au moins 12 cycles.

7. Thérapie par cellules T et composé immunomodulateur pour une utilisation selon l'une quelconque des revendications 1 et 3-6 ou composé immunomodulateur pour une utilisation selon l'une quelconque des revendications 2-6, l'administration du composé immunomodulateur étant poursuivie, à partir d'au moins après l'initiation de l'administration des cellules T, jusqu'à ce que :
(i) le nombre de cellules de, ou issues de la thérapie par cellules T administrée détectables dans le sang du sujet soit augmenté par comparaison avec dans le sujet à un moment précédent juste avant l'administration du composé immunomodulateur ou par comparaison avec un moment précédent après l'administration de la thérapie par cellules T ;
(ii) le nombre de cellules de la thérapie par cellules T détectables dans le sang du sujet soit de plus de ou de plus d'environ 10 %, 15 %, 20 %, 30 %, 40 %, 50 %, ou 60 % de cellules mononucléaires de sang périphérique totales (PBMC) dans le sang du sujet ;
(iii) le sujet présente une réduction de la charge tumorale par comparaison avec la charge tumorale à un moment immédiatement avant l'administration de la thérapie par cellules T ou à un moment immédiatement avant l'administration du composé immunomodulateur ; et/ou
(iv) le sujet présente une rémission complète ou clinique.

8. Thérapie par cellules T et composé immunomodulateur pour une utilisation selon l'une quelconque des revendications 1 et 3-7 ou composé immunomodulateur pour une utilisation selon l'une quelconque des revendications 2-7, le composé immunomodulateur étant un composé de la formule suivante : 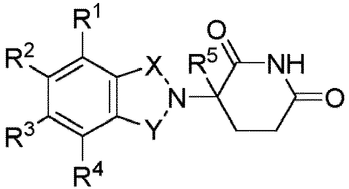
l'un parmi X et Y étant -C(O)- et l'autre parmi X et Y étant -C(O)- ou -CH₂- ;
(1) chacun parmi R¹, R², R³, et R⁴ étant indépendamment halogène, alkyle de 1 à 4 atomes de carbone, ou alcoxy ou 1 à 4 atomes de carbone, ou
(2) l'un parmi R¹, R³, R⁴, et R⁵ étant -NHR^{a} et le reste de R¹, R², R³, et R⁴ étant hydrogène, R^{a} étant hydrogène ou alkyle de 1 à 8 atomes de carbone ;
R⁵ étant hydrogène ou alkyle de 1 à 8 atomes de carbone, benzyle, ou halogène ;
étant entendu que R⁵ est différent d'hydrogène si X et Y sont -C(O)- et (i) chacun parmi R¹, R², R³, et R⁴ est fluoro ; ou (ii) l'un parmi R¹, R², R³, et R⁴ est amino ;
ou un sel pharmaceutiquement acceptable correspondant.

9. Thérapie par cellules T et composé immunomodulateur pour une utilisation selon l'une quelconque des revendications 1 et 3-8 ou composé immunomodulateur pour une utilisation selon l'une quelconque des revendications 2-8, le composé immunomodulateur étant un composé de la formule suivante : 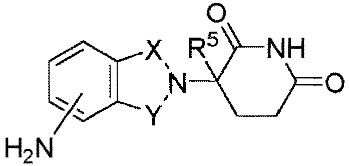 l'un parmi X et Y étant -C(O)- et l'autre parmi X et Y étant -C(O)- ou -CH₂-, et R⁵ étant hydrogène ou alkyle inférieur, ou un sel pharmaceutiquement acceptable correspondant.

10. Thérapie par cellules T et composé immunomodulateur pour une utilisation selon l'une quelconque des revendications 1 et 3-9 ou composé immunomodulateur pour une utilisation selon l'une quelconque des revendications 2-9, le composé immunomodulateur étant lénalidomide (3-(4-amino-1-oxo-1,3-dihydro-2H-isoindol-2-yl)pipéridine-2,6-dione), pomalidomide (4-amino-2-(2,6-dioxopipéridin-3-yl)isoindole-1,3-dione), ou avadomide (3-(5-amino-2-méthyl-4-oxo-4H-quinazolin-3-yl)-pipéridine-2,6-dione), un stéréoisomère de lénalidomide (3-(4-amino-1-oxo-1,3-dihydro-2H-isoindol-2-yl)pipéridine-2,6-dione), pomalidomide (4-amino-2-(2,6-dioxopipéridin-3-yl)isoindole-1,3-dione), avadomide (3-(5-amino-2-méthyl-4-oxo-4H-quinazolin-3-yl)-pipéridine-2,6-dione), ou un sel, un solvate, un hydrate, un co-cristal, un clathrate ou un polymorphe pharmaceutiquement acceptable correspondant.

11. Thérapie par cellules T et composé immunomodulateur pour une utilisation selon la revendication 10 ou composé immunomodulateur pour une utilisation selon la revendication 10, le composé immunomodulateur étant lénalidomide (3-(4-amino-1-oxo-1,3-dihydro-2H-isoindol-2-yl)pipéridine-2,6-dione), ou un stéréoisomère correspondant, ou un sel, un solvate, un hydrate, un co-cristal, un clathrate ou un polymorphe pharmaceutiquement acceptable correspondant.

12. Thérapie par cellules T et composé immunomodulateur pour une utilisation selon la revendication 10 ou composé immunomodulateur pour une utilisation selon la revendication 10, le composé immunomodulateur étant lénalidomide (3-(4-amino-1-oxo-1,3-dihydro-2H-isoindol-2-yl)pipéridine-2,6-dione) .

13. Thérapie par cellules T et composé immunomodulateur pour une utilisation selon la revendication 10 ou composé immunomodulateur pour une utilisation selon la revendication 10, le composé immunomodulateur étant avadomide (3-(5-amino-2-méthyl-4-oxo-4H-quinazolin-3-yl)-pipéridine-2,6-dione), ou un stéréoisomère correspondant, ou un sel, un solvate, un hydrate, un co-cristal, un clathrate ou un polymorphe pharmaceutiquement acceptable correspondant.

14. Thérapie par cellules T et composé immunomodulateur pour une utilisation selon la revendication 10 ou composé immunomodulateur pour une utilisation selon la revendication 10, le composé immunomodulateur étant avadomide (3-(5-amino-2-méthyl-4-oxo-4H-quinazolin-3-yl)-pipéridine-2,6-dione).

15. Thérapie par cellules T et composé immunomodulateur pour une utilisation selon l'une quelconque des revendications 1 et 3-7 ou composé immunomodulateur pour une utilisation selon l'une quelconque des revendications 2-7, le composé immunomodulateur étant 3-[4-(4-morpholin-4-ylméthyl-benzyloxy)-1-oxo-1,3-dihydroisoindol-2-yl]-pipéridine-2,6-dione.

16. Thérapie par cellules T et composé immunomodulateur pour une utilisation selon l'une quelconque des revendications 1 et 3-15 ou composé immunomodulateur pour une utilisation selon l'une quelconque des revendications 2-15, le composé immunomodulateur étant administré dans une capsule ou un comprimé.

17. Thérapie par cellules T et composé immunomodulateur pour une utilisation selon l'une quelconque des revendications 1 et 3-16 ou composé immunomodulateur pour une utilisation selon l'une quelconque des revendications 2-16, le composé immunomodulateur étant administré en une quantité de 0,1 mg à 100 mg, de 0,1 mg à 50 mg, de 0,1 mg à 25 mg, de 0,1 mg à 10 mg, de 0,1 mg à 5 mg, de 0,1 mg à 1 mg, de 1 mg à 100 mg, de 1 mg à 50 mg, de 1 mg à 25 mg, de 1 mg à 10 mg, de 1 mg à 5 mg, de 5 mg à 100 mg, de 5 mg à 50 mg, de 5 mg à 25 mg, de 5 mg à 10 mg, de 10 mg à 100 mg, de 10 mg à 50 mg, de 10 mg à 25 mg, de 25 mg à 100 mg, de 25 mg à 50 mg ou de 50 mg à 100 mg, chacun inclus, la quantité étant une quantité quotidienne unique du composé immunomodulateur.

18. Thérapie par cellules T et composé immunomodulateur pour une utilisation selon l'une quelconque des revendications 1 et 3-17 ou composé immunomodulateur pour une utilisation selon l'une quelconque des revendications 2-17,
(i) le composé immunomodulateur étant administré une fois par jour, deux fois par jour, trois fois par jour, quatre fois par jour, cinq fois par jour ou six fois par jour ; et/ou
(ii) le composé immunomodulateur étant administré à raison d'une quantité de dosage quotidienne totale d'au moins ou d'au moins environ 0,1 mg par jour, 0,5 mg par jour, 1,0 mg par jour, 2,5 mg par jour, 5 mg par jour, 10 mg par jour, 25 mg par jour, 50 mg par jour ou 100 mg par jour ; et/ou
(iii) le composé immunomodulateur étant administré en une quantité de plus de ou de plus d'environ 1 mg par jour, 2,5 mg par jour, 5 mg par jour, 7,5 mg par jour, 10 mg par jour, 15 mg par jour et de moins de 25 mg par jour.

19. Thérapie par cellules T et composé immunomodulateur pour une utilisation selon l'une quelconque des revendications 1 et 3-18 ou composé immunomodulateur pour une utilisation selon l'une quelconque des revendications 2-18,
(a) l'antigène étant choisi parmi ROR1, antigène de maturation des cellules B (BCMA), anhydrase carbonique 9 (CAIX), Her2/neu (récepteur tyrosine kinase erbB2), L1-CAM, CD19, CD20, CD22, mésothéline, CEA, et antigène de surface d'hépatite B, récepteur anti-folate, CD23, CD24, CD30, CD33, CD38, CD44, EGFR, glycoprotéine épithéliale 2 (EPG-2), glycoprotéine épithéliale 40 (EPG-40), EPHa2, erb-B2, erb-B3, erb-B4, dimères erbB, EGFR vIII, protéine de liaison du folate (FBP), FCRL5, FCRH5, récepteur de l'acétylcholine foetale, GD2, GD3, HMW-MAA, IL-22R-alpha, IL-13R-alpha2, récepteur de domaine d'insertion de kinase (kdr), chaîne lumineuse kappa, Lewis Y, molécule d'adhésion des cellules L1, (L1-CAM), antigène associé au mélanome (MAGE)-A1, MAGE-A3, MAGE-A6, antigène exprimé préférentiellement du mélanome (PRAME), survivine, TAG72, B7-H6, récepteur IL-13 alpha 2 (IL-13Ra2), CA9, GD3, HMW-MAA, CD171, G250/CAIX, HLA-AI MAGE A1, HLA-A2 NY-ESO-1, PSCA, récepteur folate a, CD44v6, CD44v7/8, intégrine avb6, 8H9, NCAM, récepteurs VEGF, 5T4, AchR foetal, ligands NKG2D, CD44v6, antigène double, antigène du cancer des testicules, mésothéline, CMV murin, mucine 1 (MUG1), MUC16, PSCA, NKG2D, NY-ESO-1, MART-1, gp100, récepteur 5D couplé à la protéine G (GPCR5D), antigène oncofoetal, ROR1, TAG72, VEGF-R2, antigène carcinoembryonnaire (CEA), Her2/neu, récepteur des oestrogènes, récepteur de la progestérone, ephrinB2, CD123, c-Met, GD-2, GD2 O-acétylée (OGD2), CE7, tumeur de Wilms 1 (WT-1), une cycline, cycline A2, CCL-1, CD138, éventuellement un antigène humain de l'un quelconque des éléments précédents ; un antigène spécifique à un pathogène ; et un antigène associé à une étiquette universelle ; ou
(b) l'antigène étant ou comprenant un antigène associé à un myélome multiple.

20. Thérapie par cellules T et composé immunomodulateur pour une utilisation selon l'une quelconque des revendications 1 et 3-18 ou composé immunomodulateur pour une utilisation selon l'une quelconque des revendications 2-18, l'antigène étant ou comprenant CD19, éventuellement CD19 humain.

21. Thérapie par cellules T et composé immunomodulateur pour une utilisation selon l'une quelconque des revendications 1 et 3-18 ou composé immunomodulateur pour une utilisation selon l'une quelconque des revendications 2-18, l'antigène étant un BCMA, éventuellement un BCMA humain.

22. Thérapie par cellules T et composé immunomodulateur pour une utilisation selon l'une quelconque des revendications 1 et 3-21 ou composé immunomodulateur pour une utilisation selon l'une quelconque des revendications 2-21,
la thérapie par cellules T comprenant des cellules T qui sont CD4+ ou CD8+ ; et/ou
la thérapie par cellules T comprenant des cellules primaires issues d'un sujet ; et/ou
la thérapie par cellules T comprenant des cellules qui sont autologues au sujet ou la thérapie par cellules T comprenant des cellules T qui sont allogéniques au sujet ; et/ou
le sujet étant un humain ; et/ou
la thérapie par cellules T comprenant l'administration de ou d'environ 1 × 10⁵ à 1 × 10⁸ de cellules exprimant un récepteur recombinant totales, de cellules T totales, ou de cellules mononucléaires de sang périphérique totales (PBMC), de ou d'environ 5 × 10⁵ à 1 × 10⁷ de cellules exprimant un récepteur recombinant totales, de cellules T totales, ou de cellules mononucléaires de sang périphérique totales (PBMC) ou de ou d'environ 1 × 10⁶ à 1 × 10⁷ de cellules exprimant un récepteur recombinant totales, de cellules T totales, ou de cellules mononucléaires de sang périphérique totales (PBMC), chacun inclus.

23. Thérapie par cellules T et composé immunomodulateur pour une utilisation selon l'une quelconque des revendications 1 et 3-22 ou composé immunomodulateur pour une utilisation selon l'une quelconque des revendications 2-22, le procédé comprenant en outre une administration d'une une chimiothérapie lymphodéprimante avant une administration de la thérapie par cellules T.

24. Thérapie par cellules T et composé immunomodulateur pour une utilisation selon l'une quelconque des revendications 1 et 3-23 ou composé immunomodulateur pour une utilisation selon l'une quelconque des revendications 2-23,
(i) le cancer étant une malignité de cellules B et/ou un myélome, un lymphome ou une leucémie ; et/ou
le cancer étant un lymphome à cellules du manteau (MCL), un myélome multiple (MM), une leucémie lymphoblastique aiguë (ALL), une ALL d'adulte, une leucémie lymphoblastique chronique (CLL), un lymphome non hodgkinien (NHL), un lymphome diffus à grandes cellules B (DLBCL) ou un lymphome folliculaire (FL) ; ou
(ii) le cancer étant un cancer non hématologique ou étant une tumeur solide.

25. Thérapie par cellules T et composé immunomodulateur pour une utilisation selon l'une quelconque des revendications 1 et 3-24 ou composé immunomodulateur pour une utilisation selon l'une quelconque des revendications 2-24,
(i) la thérapie par cellules T présentant une expansion et/ou une persistance augmentée(s) ou prolongée(s) chez le sujet par comparaison avec un procédé dans lequel la thérapie par cellules T est administrée au sujet en l'absence du composé immunomodulateur ; et/ou
(ii) le procédé réduisant la charge tumorale à un degré plus grand et/ou pour une période de temps plus grande par comparaison avec la réduction qui serait observée avec un procédé comparable dans lequel la thérapie par cellules T est administrée au sujet en l'absence du composé immunomodulateur et/ou dans lequel le composé immunomodulateur est administré en l'absence de la thérapie par cellules T, éventuellement à la même dose ou au même schéma posologique.
